(19) Europäisches Patentamt
European Patent Office
Office européen des brevets

(11) Numéro de publication : 0 238 401 B1

(12) # FASCICULE DE BREVET EUROPEEN

(45) Date de publication du fascicule du brevet : **14.08.91 Bulletin 91/33**

(21) Numéro de dépôt : **87400556.4**

(22) Date de dépôt : **12.03.87**

(51) Int. Cl.$^5$ : **C07C 311/19**, C07C 311/14, C07D 333/14, C07D 333/22, A61K 31/195, A61K 31/20, A61K 31/275, A61K 31/165, A61K 31/22, A61K 31/38

(54) **Nouveaux sulfonamides dérivés de diarylméthanes et leurs procédés de préparation et les compositions pharmaceutiques les contenant.**

(30) Priorité : **12.03.86 FR 8603537**

(43) Date de publication de la demande : **23.09.87 Bulletin 87/39**

(45) Mention de la délivrance du brevet : **14.08.91 Bulletin 91/33**

(84) Etats contractants désignés : **AT BE CH DE ES FR GB GR IT LI LU NL SE**

(56) Documents cités :
**EP-A- 0 064 445**
**EP-A- 0 068 968**
**GB-A- 1 447 054**

(73) Titulaire : **SANOFI**
**40, Avenue George V**
**F-75008 Paris (FR)**
**BE CH DE ES FR GB GR IT LI LU NL SE AT**

(72) Inventeur : **Roger, Pierre**
**6 rue Paul Valéry**
**F-78423 Montigny-Les-Bretonneux (FR)**
Inventeur : **Fournier, Jean-Paul**
**10 rue Fontenay**
**F-78000 Versailles (FR)**
Inventeur : **Martin, Alain**
**6 rue du Colonel Gillon**
**F-92120 Montrouge (FR)**
Inventeur : **Choay, Jean**
**21 rue Saint-Guillaume**
**F-75007 Paris (FR)**

(74) Mandataire : **Moncheny, Michel et al**
**c/o Cabinet Lavoix 2 Place d'Estienne d'Orves**
**F-75441 Paris Cedex 09 (FR)**

**EP 0 238 401 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Description

L'invention est relative à de nouveaux composés, sulfonamides dérivés de diarylméthanes, notamment de benzophénones, et de leurs isostères, ainsi qu'à leurs sels correspondants.

L'invention est également relative à un procédé de préparation de ces composés.

L'invention est également relative à de nouveaux médicaments contenant, à titre de principe actif, ces nouveaux composés, ainsi que leurs sels avec des acides ou des bases organiques ou minéraux physiologiquement acceptables.

On désigne par médicaments, toute composition pharmaceutique contenant, en association avec un véhicule pharmaceutiquement acceptable, l'un au moins des composés chimiques définis ci-après.

On a déjà décrit dans le brevet EP n° 64445 des médicaments tranquillisants ou anxiolytiques, qui comportent, comme substance active, des composés de formule :

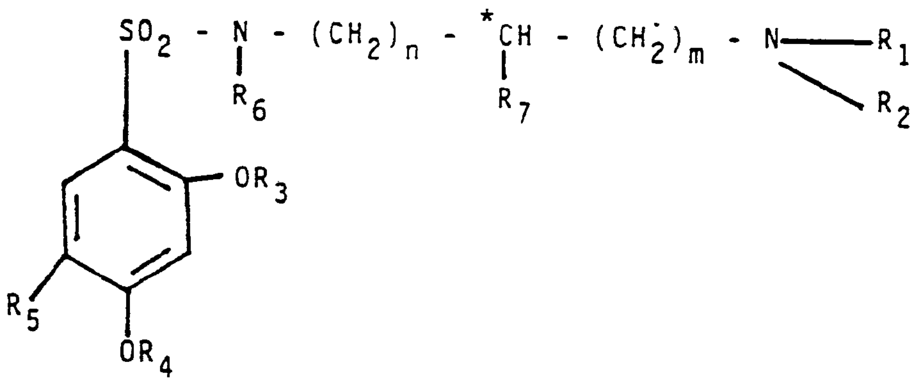

dans laquelle :

n et m prennent les valeurs de O à 4 ;

$R_3$ et $R_4$ représentent notamment un radical alcoyle inférieur ;

$R_1$ et $R_2$ représentent notamment des atomes d'hydrogène, des groupes alcoyle linéaires ou ramifiés ayant de 1 à 4 atomes de carbone ; ou formant conjointement avec l'atome d'azote un groupe hétérocycle ;

$R_5$ représente notamment un atome d'hydrogène, un halogène, le groupe $NO_2$, $NH_2$, $CF_3$, un radical alcoxy ou alcoylsulfonyle ;

$R_6$ et $R_7$ représentent notamment un atome d'hydrogène, un radical alcoyle, ou cycloalcoyle.

On a également décrit dans le brevet EP n° 68968 des médicaments présentant des propriétés normolipémiantes, dont la substance active est constituée par les composés de formule :

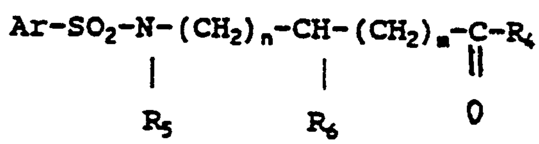

dans laquelle :

Ar représente un cycle aromatique éventuellement substitué ;

n + m + 1 est compris de 3 à 11, étant de préférence égal à 3, 5 ou 1O, ou de 1 à 11 lorsque Ar représente un groupe aryle dont l'un au moins des substituants $R_1$, $R_2$ et $R_3$ est le groupe $CF_3$ ;

$R_5$ et $R_6$ représentent indépendamment l'un de l'autre, notamment un radical alcoyle ;

$R_4$ représente le groupe hydroxy, le radical $OR_7$ dans lequel $R_7$ est notamment un groupe alkyle, le radical $NR_8R_9$ dans lequel $R_8$ et $R_9$ identiques ou différents, représentent notamment un atome d'hydrogène, et forment avec l'azote un hétérocycle azoté à 5 ou 6 chaînons.

On connait également des produits de formule I :

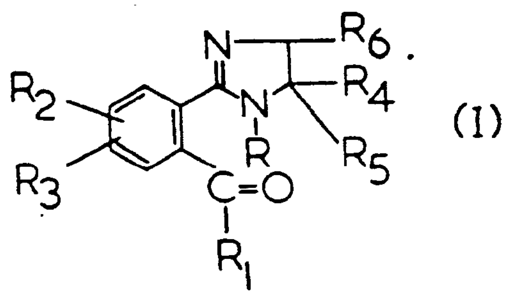

obtenus à partir de composés de formule II :

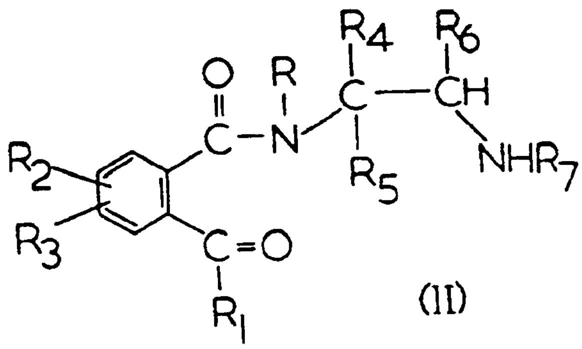

dans lesquelles R est un alcoyle inférieur, $R_1$ est un phényl, monohalophényl, dihalophényl, monoalcoyl(inférieur)-phényl,dialcoyl(inférieur)phényl, trifluorométhylphényl, monoalcoxy(inférieur)phényl, dialcoxy(inférieur)phényl, thiényl,pyridyl, furyl ou 5,6,7,8-tétrahydro-2-naphtyl ; $R_2$ est un hydrogène, halogène, amino, alcoyl(inférieur)amino, alcoyle inférieur ou alcoxy inférieur ; $R_3$ représente l'hydrogène quand $R_2$ et $R_3$ sont différents, et quand $R_2$ et $R_3$ sont identiques ils représentent simultanément un hydrogène, un halogène, un alcoyle inférieur ou alcoxy inférieur ; $R_4$ et $R_5$ sont identiques et représentent l'hydrogène ou un alcoyle inférieur ou $R_4$ est un hydrogène tandis que $R_5$ est un alcoyle inférieur ; et $R_6$ est un hydrogène, méthoxyméthyl ou alcoyle inférieur ; $R_7$ est un alcoylsulfonyl inférieur, phénylsulfonyl, monohalophénylsulfonyl, dihalophénylsulfonyl, monoalcoyl(inférieur)phénylsulfonyl, dialcoyl(inférieur)phénylsulfonyl ou alcoyloxyphénylsulfonyl inférieur.

Les susdits composés de formule I sont doués de propriétés pharmacologiques, et, en particulier on a montré qu'ils présentent l'une ou plusieurs des propriétés suivantes : activité hypoglycémique, activité antireserpine, activité anti-ulcérogène et activité anti-arrhytmique, lorsqu'ils sont testés sur les animaux ; mais aucune activité pharmacologique n'est signalée pour les composés intermédiaires de formule II.

On rappelle que les anti-inflammatoires actuellement connus et utilisés agissent :

– soit en imbibant la biosynthèse de l'acide arachidonique au niveau de la phospholipase $A_2$ ; ce sont les corticoïdes anti-inflammatoires (cortisones et dérivés) dont les effets secondaires limitent l'emploi,

– soit en inhibant la biosynthèse des prostaglandines, au niveau de la cyclooxygénase (voie de la thromboxane synthétase aussi bien que voie de la prostacycline synthétase) ; ce sont les anti-inflammatoires non stéroïdiens.

Parmi les anti-inflammatoires non stéroïdiens, on peut citer les dériver salicylés, tels que l'aspirine, pyrazolés tels que la phénylbutazone, les acides arylalcanoïques, tels que les profènes et les dérivés indoliques, tels que l'indométhacine.

En ce qui concerne les anti-inflammatoires non stéroïdiens ils agissent dans la biosynthèse des prostaglandines en bloquant la cyclooxygénase, bloquant ainsi la formation des thromboxanes et des prostacyclines ; en particulier ils peuvent entraîner des accidents hémorragiques quand ils sont associés à des traitements anticoagulants.

Les anti-inflammatoires non stéroïdiens peuvent entraîner d'autres effets secondaires indésirables, notamment des gastralgies, des nausées ou vomissements et de ce fait ne peuvent pas être utilisés dans le cas d'ulcère gastrique ou duodénal.

Or, dans les phénomènes d'inflammations, intervient une autre classe de médiateurs, les leucotriènes, qui sont des métabolites de l'acide arachidonique.

Les leucotriènes sont des médiateurs puissants qui interviennent dans de nombreuses pathologies inflammatoires, cardio-vasculaires, allergiques, cutanées ou asthmatiques.

L'invention a pour objet de proposer de nouveaux composés susceptibles d'entrer dans la préparation de médicaments présentant notamment des propriétés anti-inflammatoires.

Un aspect avantageux de l'invention est de proposer de nouveaux composés qui n'inhibent pas la cyclooxygénase et n'interviennent qu'à une étape ultérieure dans la biosynthèse des prostaglandines, leur conférant une action plus spécifique.

L'un des aspects de l'invention est de proposer de nouveaux composés entrant dans la préparation de médicaments susceptibles de traiter notamment les phénomènes inflammatoires, sans agir sur la biosynthèse de toutes les prostaglandines.

En effet, ils ont peu ou pas d'action sur la prostacycline synthétase et n'inhibent pas en particulier la biosynthèse de la prostacycline ($PGI_2$) qui est vaso-dilatatrice et anti-agrégante plaquettaire.

En revanche, et de façon avantageuse, ce sont des inhibiteurs de la thromboxane synthétase, enzyme qui intervient dans la biosynthèse des thromboxanes. On sait que le thromboxane $B_2$ en particulier intervient dans les phénomènes de vaso-constriction des vaisseaux et d'agrégation plaquettaire.

Les produits de l'invention ont donc une activité plus spécifique au niveau plaquettaire et sont dénués d'effets secondaires gastriques.

Un aspect avantageux de l'invention est de proposer de nouveaux composés qui sont actifs sur la biosynthèse des leucotriènes, en particulier en inhibant la 5-lipoxygénase.

L'un des aspects de l'invention est de proposer de nouveaux composés qui inhibent la biosynthèse des leucotriènes, plus particulièrement des leucotriènes $B_4$ ($LTB_4$), impliqués dans les processus inflammatoires, et produits par les leucocytes polymorpho-nucléaires, ainsi que des leucotriènes $C_4$($LTC_4$) impliqués dans des réactions allergiques, notamment l'asthme, et certains types de maladies allergiques.

Un autre aspect de l'invention est de proposer de nouveaux composés entrant dans la préparation de médicaments susceptibles de traiter les pathologies où interviennent les leucotriènes, notamment $LTB_4$ et $LTC_4$, telles que les affections allergiques, notamment cutanées et asthmatiques ou des états inflammatoires. Ils pourront même être administrés dans le cas d'insuffisance rénale puisqu'ils n'ont pas d'action inhibitrice sur les prostaglandines rénales vaso-dilatatrices telles que $PGE_2$ et $PGF_2$ alpha.

Ces nouveaux composés peuvent entrer également dans la préparation de médicaments susceptibles de traiter les troubles cardio-vasculaires dans lesquels interviennent les thromboxanes synthétases, prévention d'accidents thrombotiques par exemple.

En particulier, l'invention propose de nouveaux composés entrant dans la constitution de médicaments qui présentent des propriétés anti-inflammatoires efficaces et qui ont un indice thérapeutique amélioré.

Un des aspects de l'invention est de fournir une nouvelle famille de médicaments anti-inflammatoires qui peuvent inhiber la biosynthèse des leucotriènes, et ont une action sélective sur la thromboxane synthétase.

L'invention a pour objet de nouveaux sulfonamides dérivés de diarylméthanes et de leurs isostères répondant à la formule I :

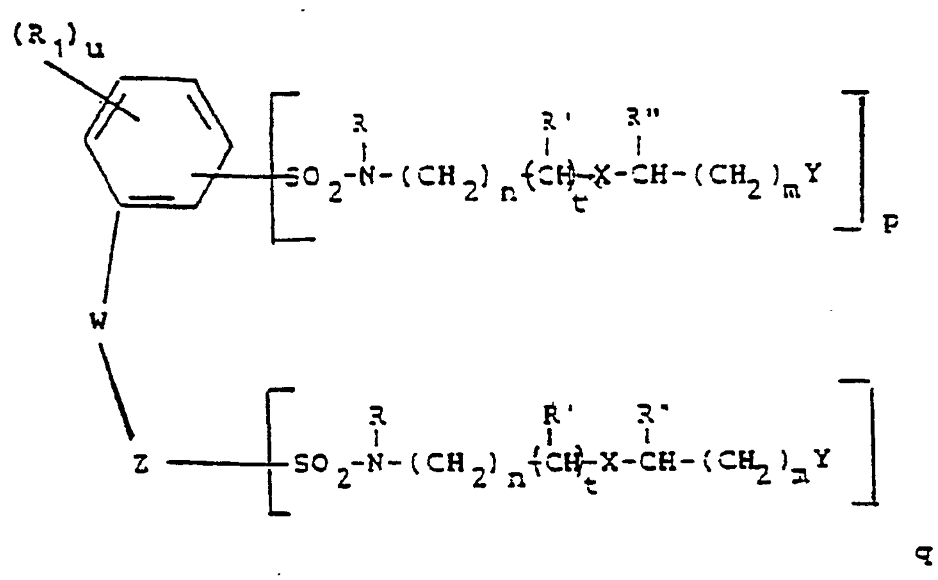

dans laquelle :

– W représente C = O, $CH_2$ ou CHOH,

– Z représente :

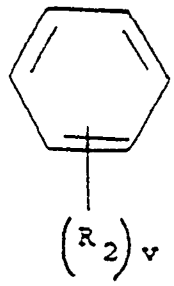 ou 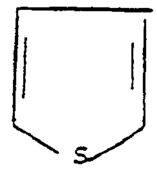

– $R_1$ représente Cl, F, Br, $NO_2$, $NH_2$, $CF_3$, un radical alcoyle de 1 à 4 atomes de carbones, notamment méthyle ou éthyle, un radical alcoxy de 1 à 4 atomes de carbone, acétamido ou benzamido,
– $R_2$ représente Cl, F, Br, $NO_2$, $NH_2$, $CF_3$, un radical alcoyle de 1 à 4 atomes de carbone, notamment méthyle ou éthyle, un radical alcoxy de 1 à 4 atomes de carbone, acétamido ou benzamido,
– R représente H, un radical alcoyle de 1 à 6 atomes de carbone ou un radical benzyle,
– R′ et R″ représentent H ou un radical alcoyle de 1 à 4 atomes de carbone,
– X représente $(CH_2)_r$, r valant O ou 1, -CH = CH,

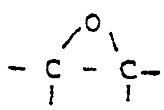 , ou 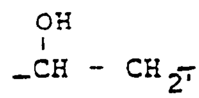

– Y représente $COOR_3$, $R_3$ représentant l'hydrogène ou un radical alcoyle de 1 à 4 atomes de carbone,

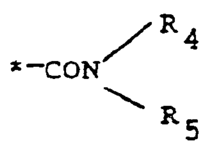 ,

$R_4$ et $R_5$ représentant H ou un radical alcoyle de 1 à 4 atomes de carbone,

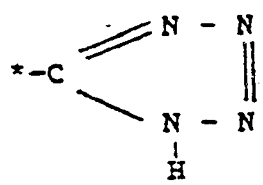 ,

* - C ≡ N,

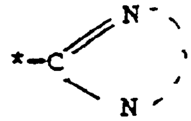 , 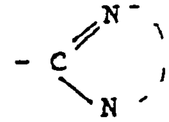

représentant un cycle non aromatique à 5 ou 6 chainons, dans lequel les 2 ou 3 atomes qui entrent dans le cycle sont des atomes de carbone et/ou d'oxygène et/ou d'azote,

* $NH_2$, $NHR_6$, $NR_6R_7$,

$R_6$ et $R_7$ représentant un radical alcoyle de 1 à 4 atomes de carbone, ou susceptible de former avec l'atome d'azote une amine cyclisée non aromatique telle que la morpholine, la pipéridine, la pyrrolidine,

– sous réserve que lorsque Y représente - $NH_2$, X soit différent de

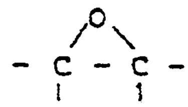

– u est un nombre entier de O à 2
– v est un nombre entier de O à 2
– p vaut O ou 1
– q vaut O ou 1, p + q étant égal ou supérieur à 1
– n est un nombre entier variant de O à 1O
– m est un nombre entier variant de O à 1O
– t vau O ou 1
– le nombre total d'atomes de carbone de la chaîne

$$-(CH_2)_n-(\overset{|}{C}H)_t-X-\overset{|}{C}H-(CH_2)_m\,Y,$$

variant de 2 à 2O,
ainsi que leus sels physiologiquement acceptables obtenus avec des acides ou bases organiques ou minéraux.
L'invention vise également les isomères cis et trans de ces composés, lorque X représente CH = CH, ainsi que les isomères optiques, lorsque

$$\underset{R'}{\underset{|}{CH}} \text{ ou } X$$

comporte un atome de carbone asymétrique, ainsi que les racémiques correspondants et les diastéroisomères lorsque

$$\underset{R'}{\underset{|}{CH}} \text{ et } X$$

comportent chacun un atome de carbone asymétrique.
Parmi les composés de formule I, un groupe préféré de composés est constitué par ceux dans lesquels W représente C = O.
Ces composés répondent à la formule II suivante :

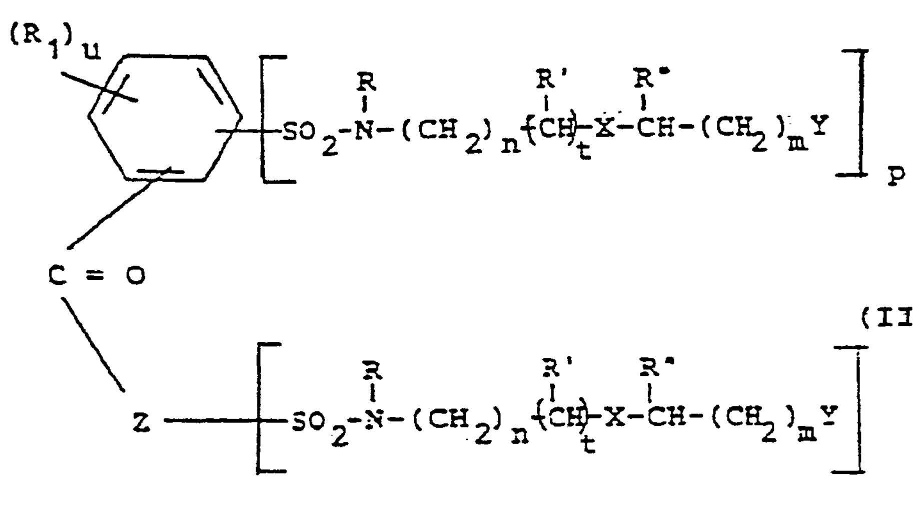

(II)

dans laquelle $R_1$, R, R', R'', X, Y, Z, m, n, p, q, t et u ont les significations indiquées à propos de la formule I.

Un autre groupe avantageux de composés de l'invention est constitué par ceux de formule I dans lesquels W représente $CH_2$.

Ces composés répondent à la formule III suivante :

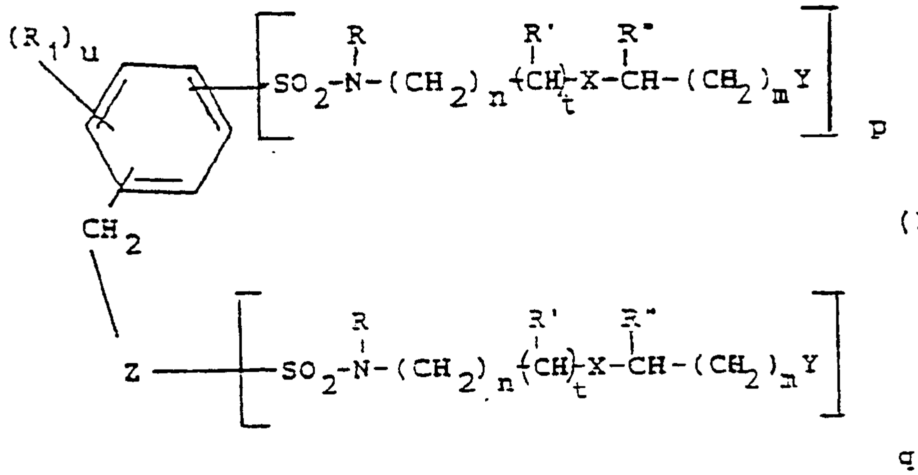

(III)

dans laquelle $R_1$, R, R', R'', X, Y, Z, m, n, p, q, t et u ont les significations indiquées à propos de la formule I.

Un autre groupe avantageux de composés de l'invention est constitué par ceux de formule I dans lesquels W représente CHOH.

Ces composés répondent à la formule IV suivante :

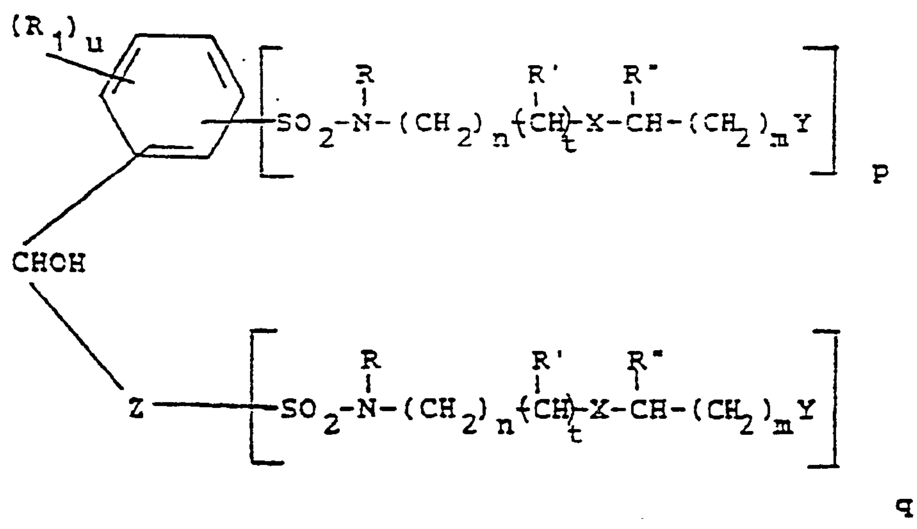

(IV)

dans laquelle $R_1$, R, R', R'', X, Y, Z, m, n, p, g, t et u ont les significations indiquées à propos de la formule I.

Parmi les composés de formule II, un groupe avantageux de composés est constitué par ceux dans lesquels Z représente

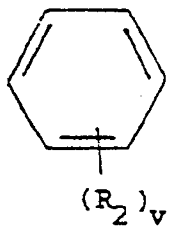

Ces composés répondent répondent à la formule V suivante :

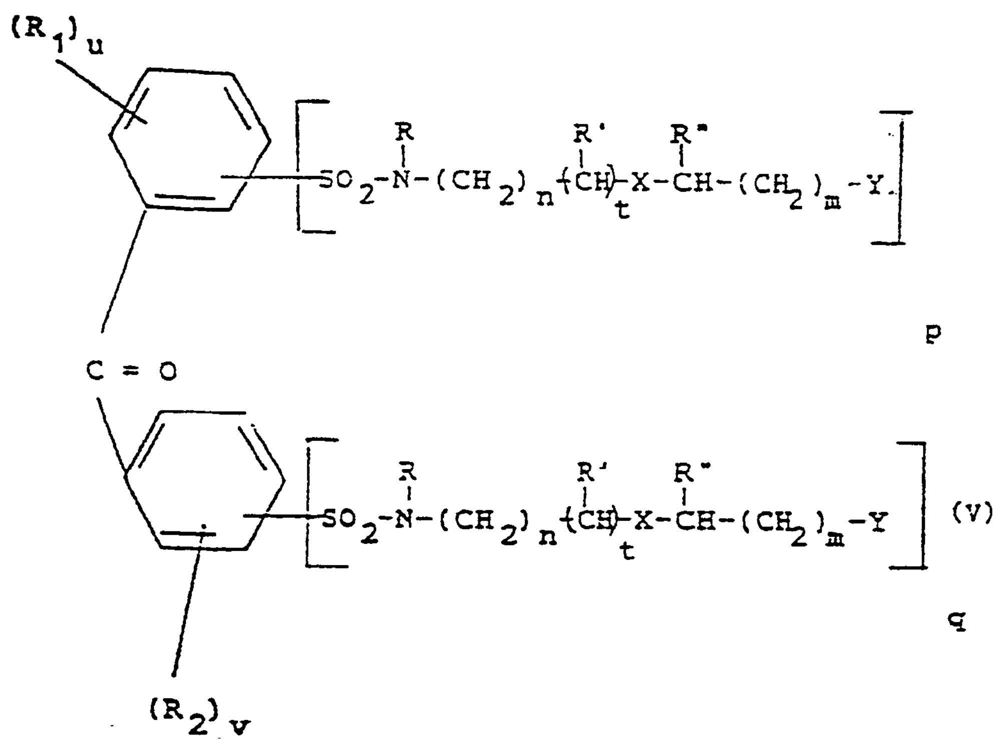

(V)

dans laquelle $R_1$, $R_2$,R, R′, R″, X, Y, m, n, p, q, t, u et v ont les significations indiquées à propos de la formule I.

Parmi les composés de formule V, un groupe avantageux de composés de l'invention est constitué par ceux dans lesquels Y représente $-COOR_3$, $R_3$ représentant H ou un radical alcoyle de 1 à 4 atomes de carbone.

Parmi les composés de formule V, un autre groupe avantageux de composés de l'invention est constitué par ceux dans lesquels Y représente $NH_2$, $NHR_6$, $NR_6R_7$, $R_6$ et $R_7$ représentant un radical alcoyle de 1 à 4 atomes de carbone, ou susceptible de former avec l'atome d'azote une amine cyclisée non aromatique telle que la morpholine, la pipéridine, la pyrrolidine,

- sous réserve que lorsque Y représente $-NH_2$, X soit différent de

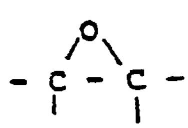

Parmi les composés de formule V, un groupe avantageux de composés de l'invention est constitué par ceux dans lesquels p + q vaut 1, c'est-à-dire des composés ne comportant qu'une chaîne ; dans le cas où p = 1 ; ces composés répondent à la formule Vbis suivante :

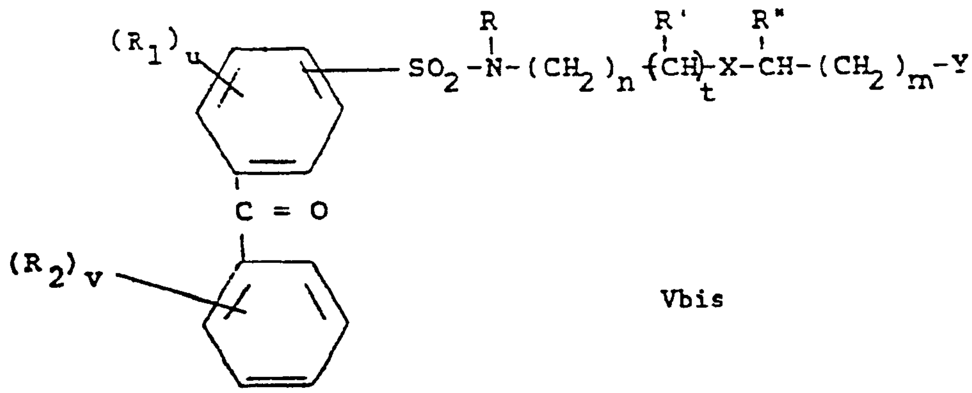

Vbis

dans laquelle $R_1$, $R_2$, R, R', R'', X, Y, n, m, u, t et v ont les significations indiquées à propos de la formule I.

Parmi les composés de formule Vbis, un groupe avantageux de composés de l'invention est constitué par ceux dans lesquels

– u + v est égal ou supérieur à 1

– le nombre total d'atomes de carbone de la chaîne

$$-(CH_2)_n-(CH)_t-X-CH-(CH_2)_m-Y$$

est égal ou supérieur à 5.

Parmi les composés de formule V, un groupe avantageux de composés de l'invention est constitué par ceux dans lesquels

– p = 1

– q = 1

– u + v est égal ou supérieur à 1

– le nombre total d'atomes de carbone des chaînes

$$-(CH_2)_n-(CH)_t-X-CH-(CH_2)_m-Y$$

est égal ou supérieur à 5.

Ces composés répondent à la formule Vter suivante :

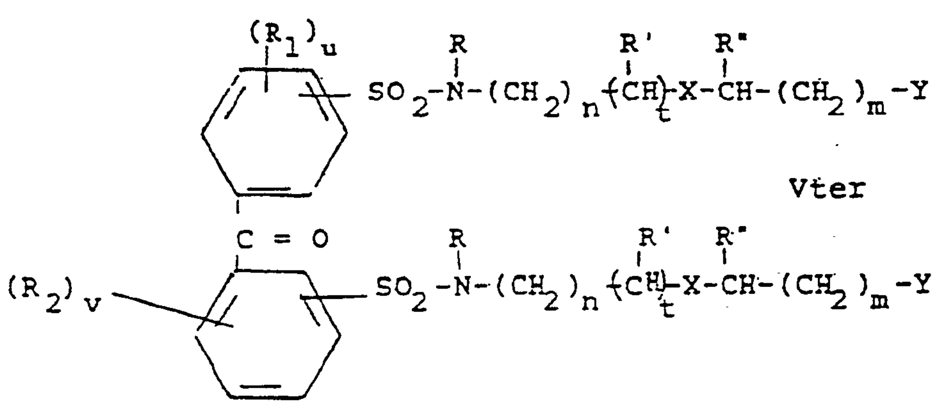

Vter

dans laquelle $R_1$, $R_2$, R, R', R'', X, Y, u, v, n, m et t ont les significations indiquées à propos de la formule I.

Parmi les composés de formule I, un groupe avantageux de composés de l'invention est consitués par ceux dans lesquels p + q vaut 1, c'est-à-dire des composés ne comportant qu'une seule chaîne ; dans le cas où p = 1, ces composés répondent à la formule VI suivante

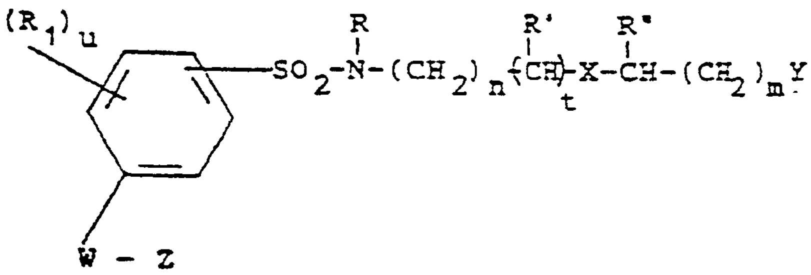

(VI)

dans laquelle $R_1$, R, R', R", X, Y, Z, W, m, n, t et u ont les significations indiquées à propos de la formule I.

Parmi les composés de formule VI, un groupe avantageux de composés de l'invention est constitué par ceux dans lesquels la chaîne latérale est en position méta par rapport à W.

Ces composés répondent à la formule VII suivante

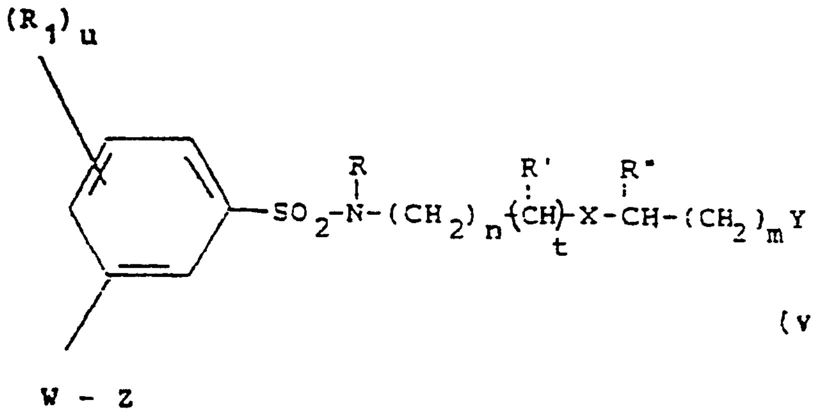

(VII)

dans laquelle $R_1$, R, R', R", X, Y, Z, W, m, n, t et u ont les significations indiquées à propos de la formule I.

Parmi les composés de formule VII, un groupe avantageux de composés de l'invention est constitué par ceux dans lesquels Y représente $COOR_3$, $R_3$ représentant H ou un radical alcoyle de 1 à 4 atomes de carbone.

Parmi les composés de formule VII, dans lesquels Y représente $COOR_3$, $R_3$ représentant H ou un radical alcoyle de 1 à 4 atomes de carbone, un groupe avantageux de composés est constitué par ceux dans lesquels W représente C = O.

Ces composés répondent à la formule VIII suivante :

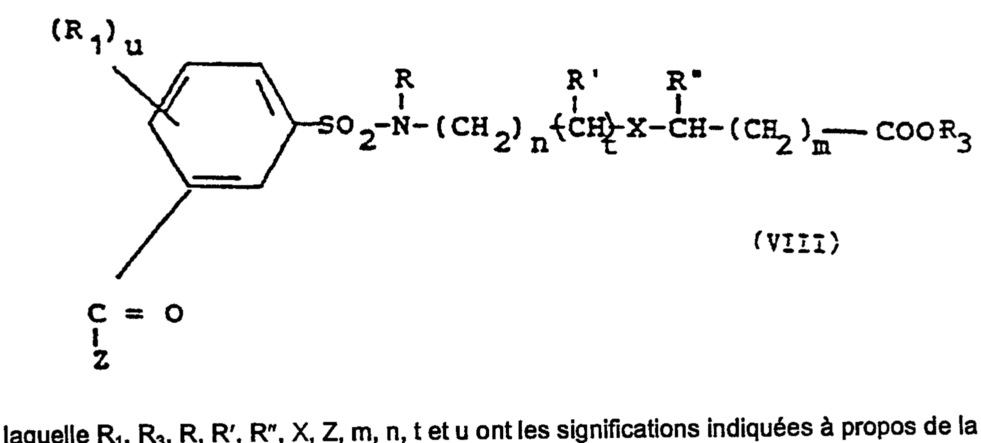

(VIII)

dans laquelle $R_1$, $R_3$, R, R', R", X, Z, m, n, t et u ont les significations indiquées à propos de la formule I.

Parmi les composés de formule VII, dans lesquels Y représente $COOR_3$, $R_3$ représentant H ou un radical alcoyle de 1 à 4 atomes de carbone, un groupe avantageux de composés est constitué par ceux dans lesquels W représente $CH_2$.

Ces composés répondent à la formule IX suivante :

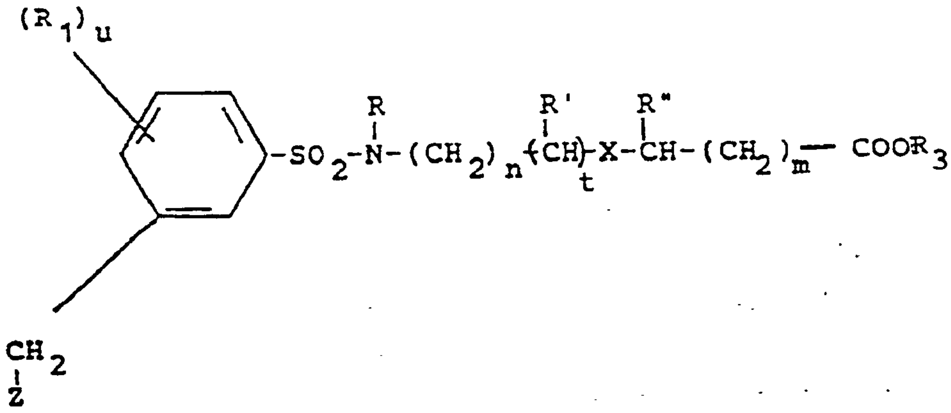

IX

dans laquelle $R_1$, $R_3$, R, R′, R″, X, Z, m, n, t et u ont les significations indiquées à propos de la formule I.

Parmi les composés de formule VII, dans lesquels Y représente $COOR_3$, $R_3$ représentant H ou un radical alcoyle de 1 à 4 atomes de carbone, un groupe avantageux de composés est constitué par ceux dans lesquels W représente CHOH.

Ces composés répondent à la formule X suivante :

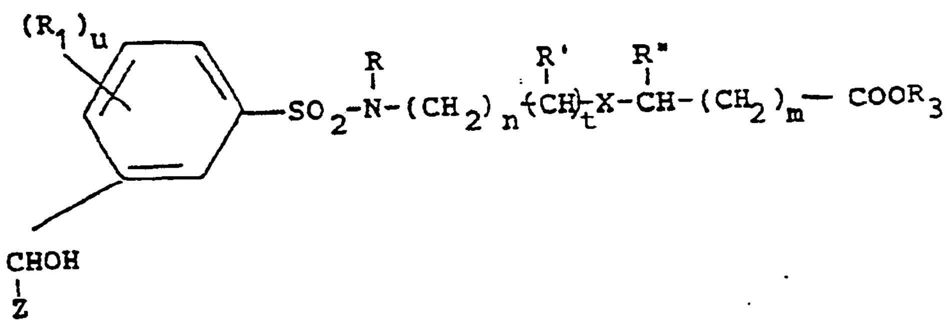

X

dans laquelle $R_1$ $R_3$, R, R′, R″, X, Z, m, n, t et u ont les significations indiquées à propos de la formule I.

Parmi les composés de formule VIII, un groupe avantageux de composés est constitué par ceux dans lesquels le nombre total d'atomes de carbone de la chaîne

$$(CH_2)_n-(CH)_t-X-CH-(CH_2)_m-Y$$

est égal ou supérieur à 5

Parmi les composés de formule IX, un groupe avantageux de composés est constitué par ceux dans lesquels le nombre total d'atomes de carbone de la chaîne

$$(CH_2)_n-(CH)_t-X-CH-(CH_2)_m-Y$$

est égal ou supérieur à 5

Parmi les composés de formule X, un groupe avantageux de composés est constitué par ceux dans lesquels le nombre total d'atomes de carbone de la chaîne

$$(CH_2)_n-(CH)_t-X-CH-(CH_2)_m-Y$$

est égal ou supérieur à 5

Parmi les composés de formule VII, dans lesquels Y représente $COOR_3$, $R_3$ représentant H ou un radical alcoyle

de 1 à 4 atomes de carbone, un autre groupe avantageux de composés est constitués par ceux dans lesquels
– Z représente

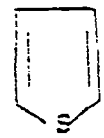

– le nombre total d'atomes de carbone de la chaîne

$$(CH_2)_n-(CH)_t-X-CH-(CH_2)_m-Y$$

est égal ou supérieur à 5
Ces composés répondent à la formule XI suivante :

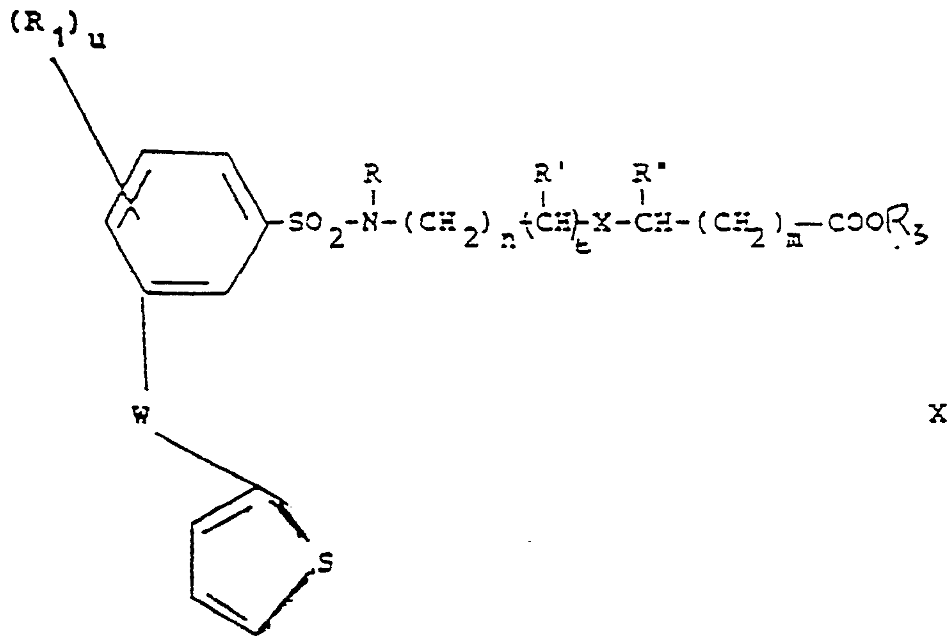

XI

dans laquelle $R_1$, $R_3$, R, R', R'', X, W, m, n, t et u ont les significations indiquées à propos de la formule I.
Parmi les composés de formule VI, un groupe de composés avantageux de l'invention est constitué par ceux de formule VIbis :

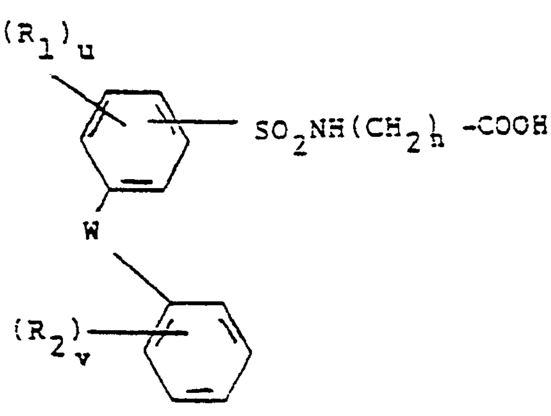

VIbis

dans laquelle $R_1$ et $R_2$ représentent un halogène, u et v varient de O à 2, W représente C=O, CHOH, $CH_2$ et n varie de 3 à 11.

Parmi les composés de formule VI, un groupe de composés avantageux est constitué par ceux de formule VIter:

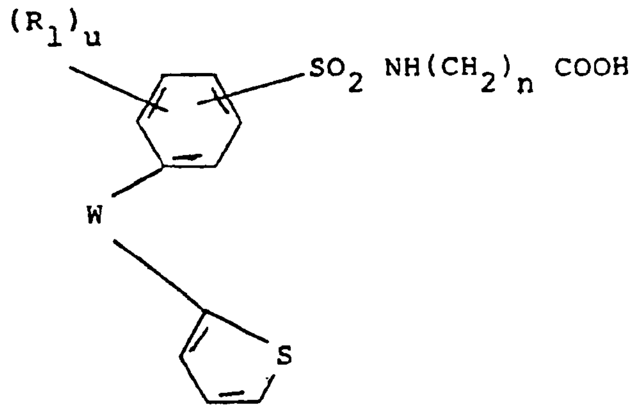

VIter

dans laquelle $R_1$ représente un halogène, u varie de O à 2, W représente C=O, CHOH, $CH_2$, et n varie de 3 à 11.

Des composés particulièrement avantageux de l'invention répondent aux formules suivantes :

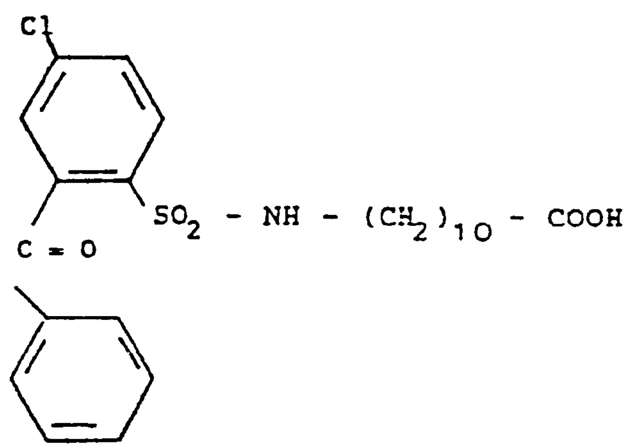

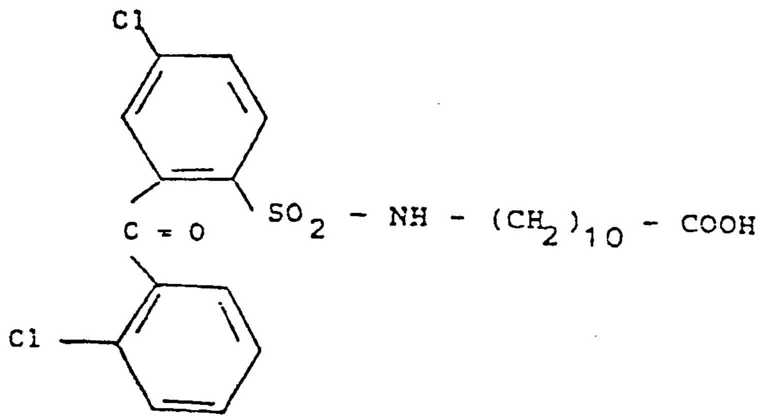

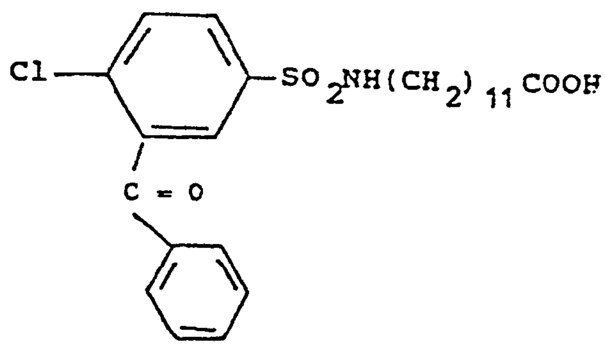

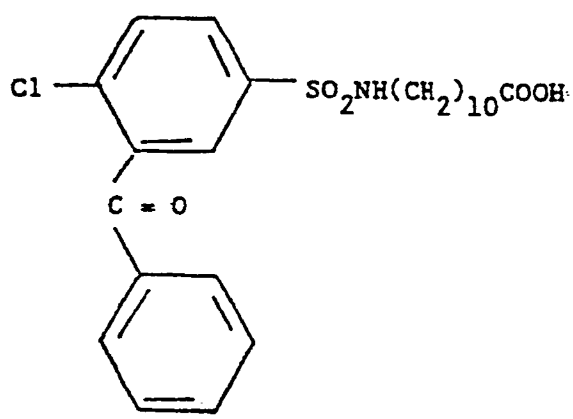

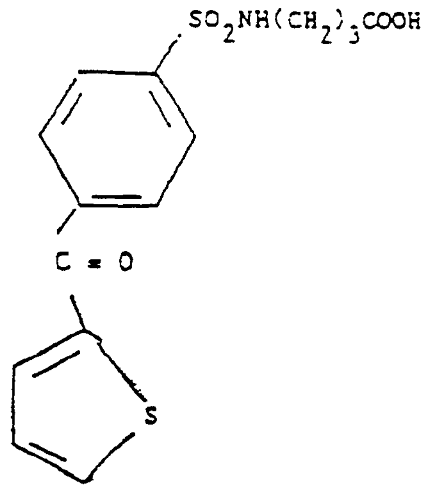

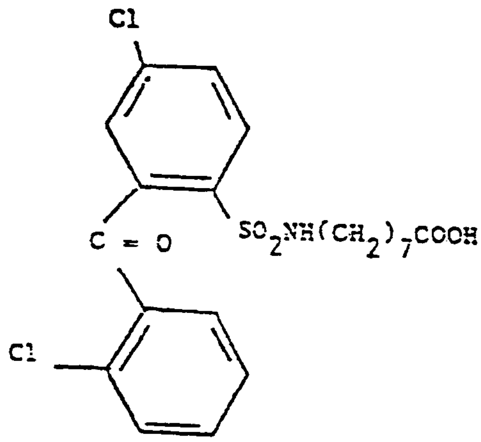

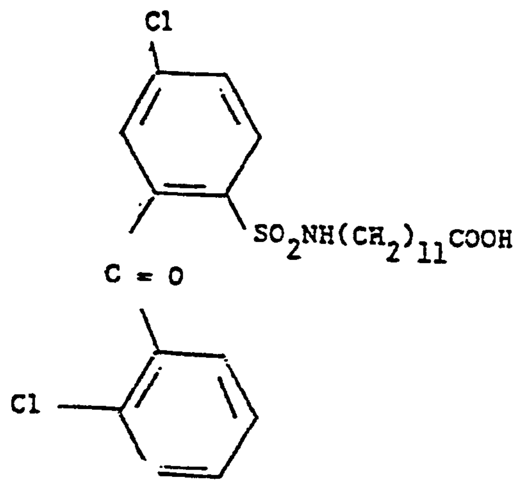

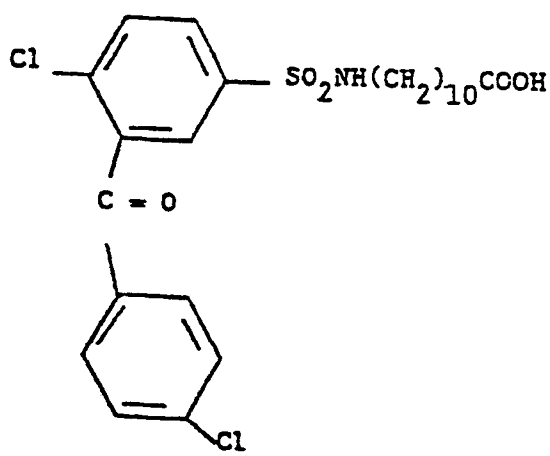

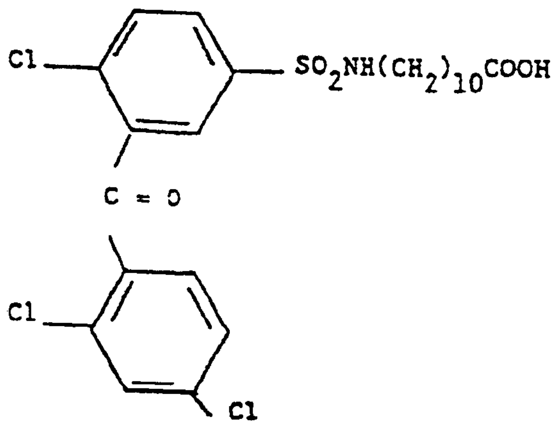

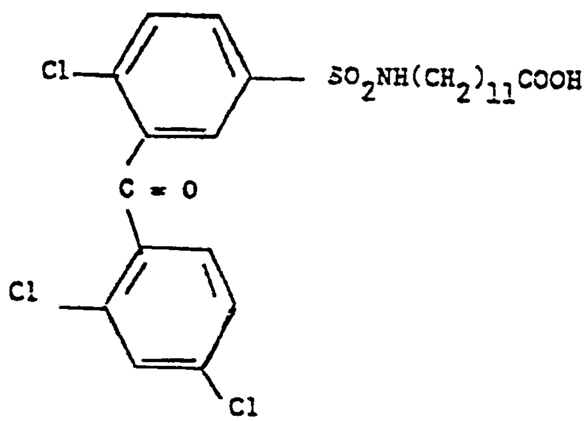

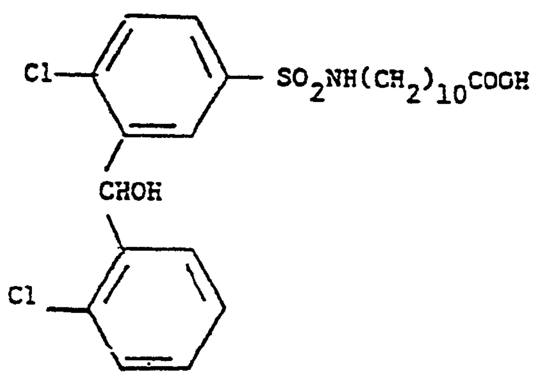

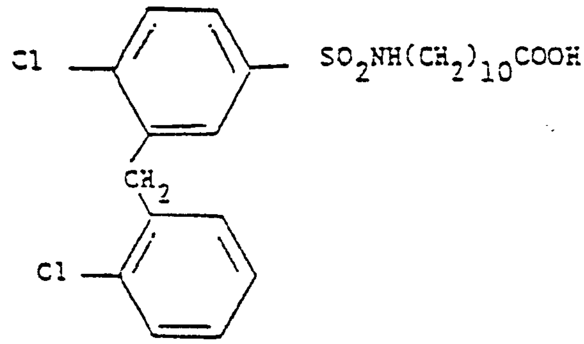

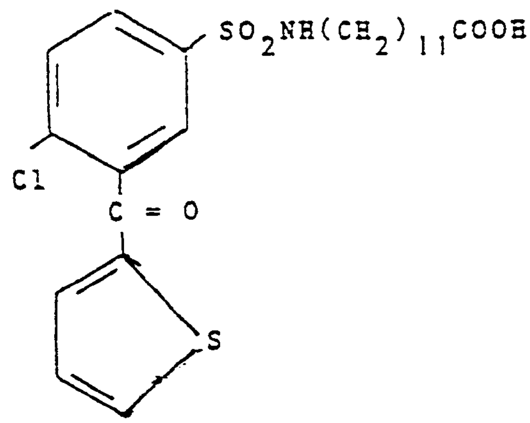

PREPARATION DES COMPOSES DE L'INVENTION

Pour préparer les composés de l'invention répondant à la formule I

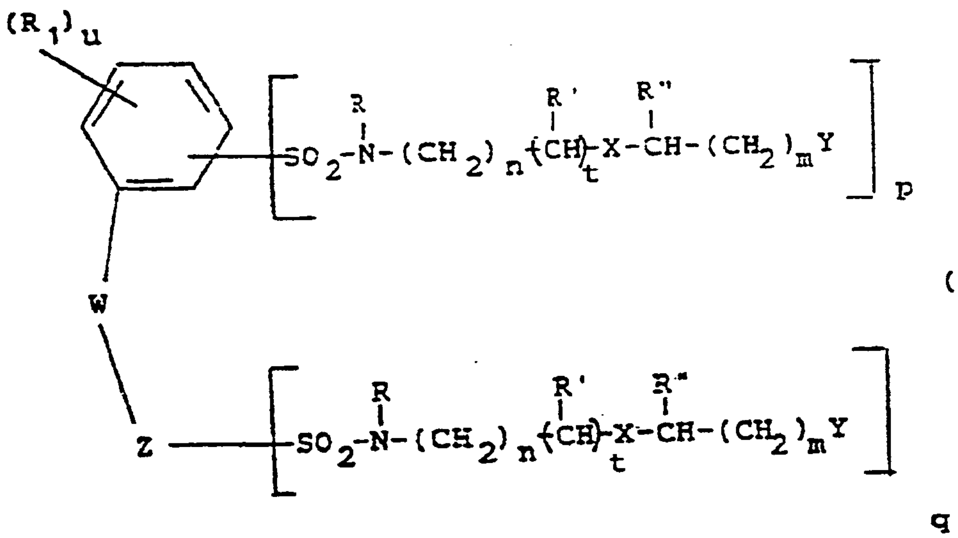

(I)

dans laquelle W, Z, $R_1$, R, R', R'', X, Y, m, n, p, t et q ont les significations indiqués ci-dessus, on peut soumettre un composé de formule

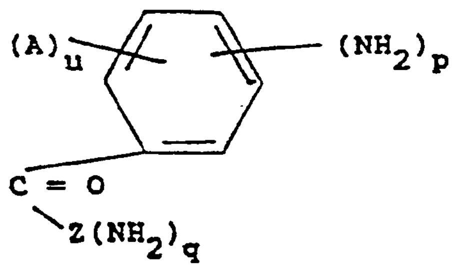

dans laquelle

– A a les significations indiquées pour $R_1$, à l'exception de $NH_2$, c'est-à-dire A représente Cl, F, Br, $NO_2$, $CF_3$, un radical alcoyle de 1 à 4 atomes de carbone, notamment méthyle ou éthyle, un radical alcoxy de 1 à 4 atomes de carbone, acétamido ou benzamido, et de préférence Cl ou $NO_2$,

– Z représente

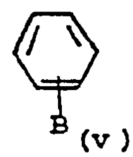

ou

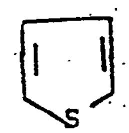

– B a les significations indiquées pour $R_2$, à l'exception de $NH_2$, c'est-à-dire B représente Cl, Br, $NO_2$, $CF_3$, un radical alcoyle de 1 à 4 atomes de carbone, notamment méthyle ou éthyle, un radical alcoxy de 1 à 4 atomes de carbone, acétamido ou benzamido et de préférence Cl ou $NO_2$,

– u, v, p et q ont les significations indiquées ci-dessus,

éventuellement à une réduction, notamment à l'aide de chlorure d'aluminium et d'hydrure d'aluminium et de lithium pour réduire - C = O en $CH_2$, et obtenir le composé de formule

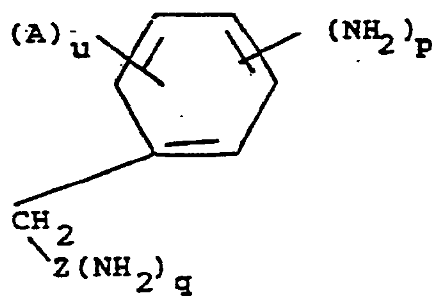

dans laquelle A, Z, u, p et q ont les significations indiquées ci-dessus,

. on peut ensuite soumettre le composé aminé de formule indiquée ci-dessus à la réaction de Sandmeyer (formation du sel de diazonium et action de l'anhydride sulfureux), pour obtenir un sulfohalogénure, notamment sulfochlorure de formule :

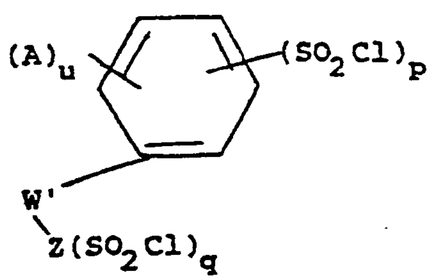

dans laquelle A, Z, u, p et q ont les significations indiquées ci-dessus et W' représente C = O ou $CH_2$,

. on fait ensuite réagir le sulfohalogénure, notamment le sulfochlorure, de formule indiquée ci-dessus, sur un composé de formule :

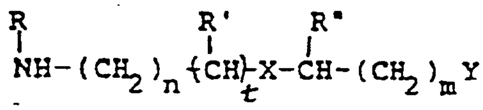

dans laquelle

– R représente H, un radical alcoyle de 1 à 6 atomes de carbone ou un radical benzyle,

– R' et R" représentent H ou un radical aclcoyle de 1 à 4 atomes de carbone,

– X représente $(CH_2)_r$, r valant O ou 1, -Ch = CH-,

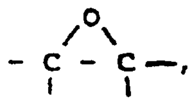 ou 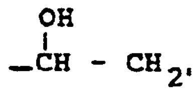

– Y représente $COOR_3$, $R_3$ représentant H ou un radical alcoyle de 1 à 4 atomes de carbone,

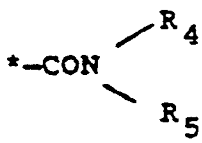 ,

$R_4$ et $R_5$ représentant H ou un radical alcoyle de 1 à 4 atomes de carbone,

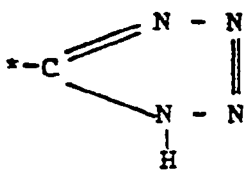 ,

* - C ≡ N,

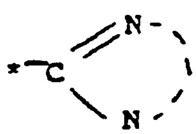 , 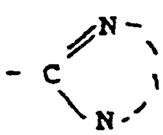

représentant un cycle non aromatique à 5 ou 6 chainons, dans lequel les 2 ou 3 atomes qui entrent dans le cycle sont des atomes de carbone et/ou d'oxygène et/ou d'azote,

* $NH_2$, $NHR_6$, $NR_6R_7$,

$R_6$ et $R_7$ représentant un radical alcoyle de 1 à 4 atomes de carbone, ou susceptible de former avec l'atome d'azote une amine cyclisée non aromatique telle que la morpholine, la pipéridine, la pyrrolidine,

– sous réserve que lorsque Y représente - $NH_2$, X soit différent de

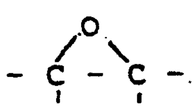

– n est un nombre entier variant de O à 10

– m est un nombre entier variant de O à 10

– t vaut O ou 1

– le nombre total d'atomes de la chaîne

$$-(CH_2)_n-(CH)_t-X-CH-(CH_2)_m\,Y,$$

variant de 2 à 2O,
pour obtenir le composé de formule :

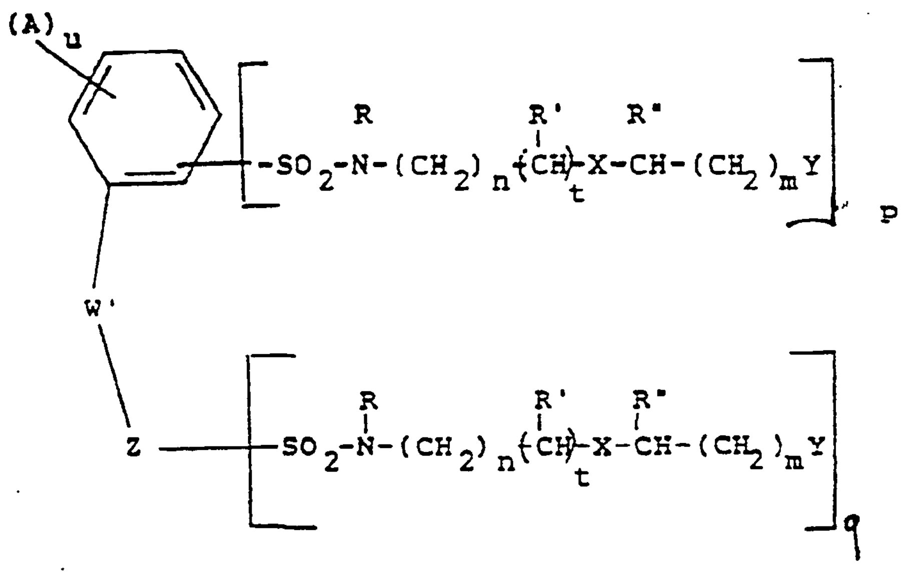

. et dans le cas où W′ représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$.
Pour préparer les composés de l'invention de formule II

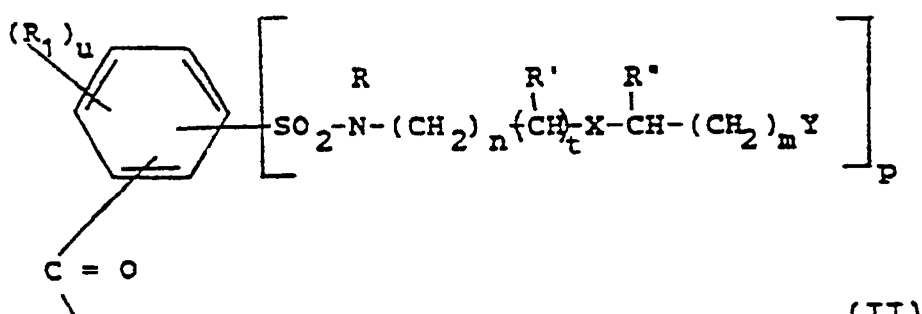

(II)

dans laquelle $R_1$, R, R′, R″, X, Y, Z, m, n, p, q, t et u ont les significations indiquées ci-dessus, on peut faire réagir un sulfohalogénure, notamment un sulfochlorure de formule

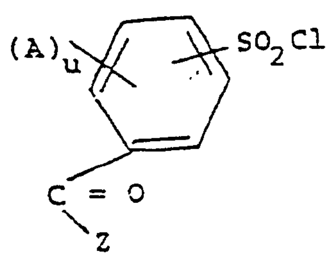

dans laquelle A, Z et u ont les significations indiquées ci-dessus ou un disulfohalogénure, notamment un disul-

fochlorure de formule

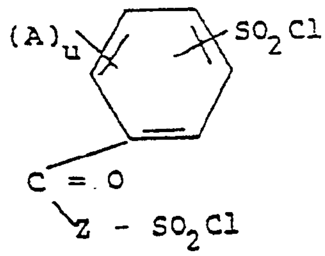

dans laquelle A, u et Z ont les significations précédemment indiquées sur un ou deux composés de formule

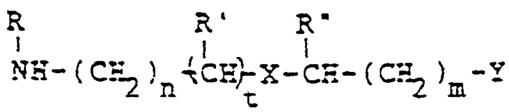

dans laquelle R, R′, R″, n, m, t, X et Y ont les significations indiquées ci-dessus,
- suivi éventuellement d'une réduction lorsque A et/ou B représente $NO_2$.
Pour préparer les composés de l'invention de formule II,
– on peut estérifier un composé tel qu'obtenu ci-dessus, et dans lequel Y représente COOH, en un composé de formule II, dans lequel Y représente $COOR_3$, $R_3$ représentant un radical alcoyle de 1 à 4 atomes de carbone
– ou on peut effectuer une réaction appropriée pour transformer un composé tel qu'obtenu ci-dessus et dans lequel Y représente COOH en un composé de formule II dans lequel Y représente

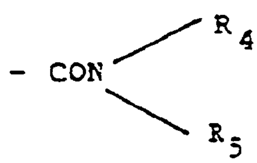

$R_4$, $R_5$ représentant H ou un radical alcoyle de 1 à 4 atomes de carbone,

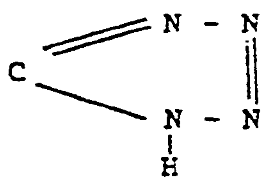

- C≡N

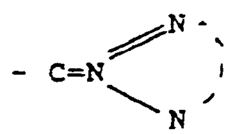

La préparation des composés de l'invention dans lesquels Y représente :

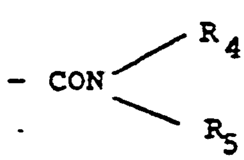

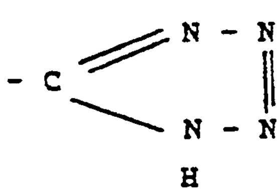

- C ≡ N

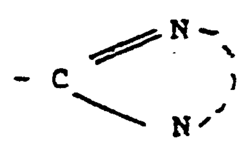

- $R_4$ et $R_5$ représentant un groupe alcoyle de 1 à 4 atomes de carbone,

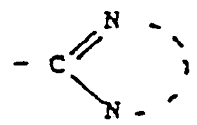

représentant un cycle non aromatique à 5 ou 6 chaînons dans lequel les 2 ou 3 atomes qui entrent dans le cycle sont des atomes de carbone et/ou d'oxygène et/ou d'azote,
est obtenue à partir des composés dans lesquels Y représente COOH, selon des méthodes classiques, par exemple comme indiqué ci-après :
Le passage d'un acide

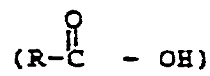

à un amide peut se faire notamment selon deux méthodes :
La première de ces méthodes consiste :
1) à former un chlorure d'acide intermédiaire selon le schéma réactionnel suivant :

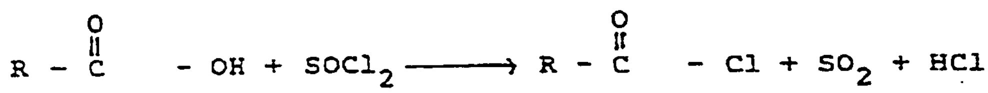

2) puis à faire réagir une amine sur le chlorure d'acide, en présence d'un accepteur d'acide chlorhydrique, par exemple une amine tertiaire selon le schéma réactionnel suivant :

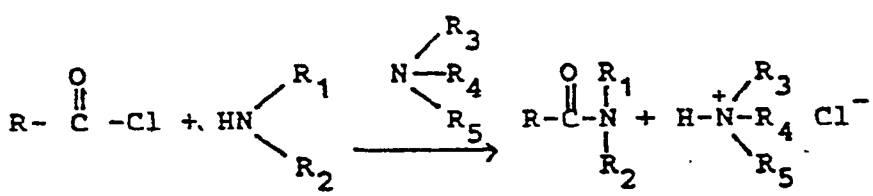

Dans la première étape, on peut également utiliser $PCl_3$ ou $PCl_5$ à la place de $SOCl_2$.
La deuxième méthode consiste :
1) à former un ester intermédiaire selon le schéma réactionnel suivant :

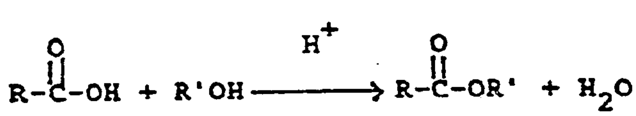

$H^+$ provenant avantageusement de HCl ou HBr,
2) à faire réagir une amine, de préférence sous pression et à température élevée suivant le schéma réactionnel suivant :

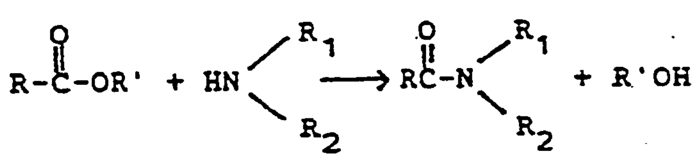

Ces deux méthodes s'appliquent aussi bien à la préparation des amides primaires, secondaires et tertiaires, selon l'amine de départ utilisée.
Le passage d'un groupe amide a un groupe nitrile peut se faire selon deux méthodes.
La première de ces méthodes consiste à utiliser un réactif deshydratant (oxychlorure de phosphore $POCl_3$, pentoxyde de phosphore $P_2O_5$, chlorure de thionyle $SOCl_2$) sur un amide

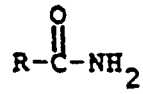

selon le schéma réactionnel suivant :

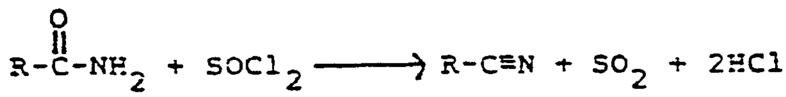

La deuxième méthode consiste à faire une pyrolyse de l'amide à température élevée, par exemple d'environ 200°C

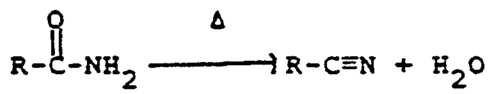


Dans ce cas, R peut représenter soit le sulfonamide dérivé de diarylméthane de formule I, dans lequel Y représente $CONH_2$, soit la chaîne aminée, condensée ensuite sur le sulfochlorure pour éviter des dégradations. Le passage du groupe nitrile au groupe tétrazole peut se faire par cyclocondensation du nitrile avec l'azoture de sodium selon le schéma réactionnel suivant :

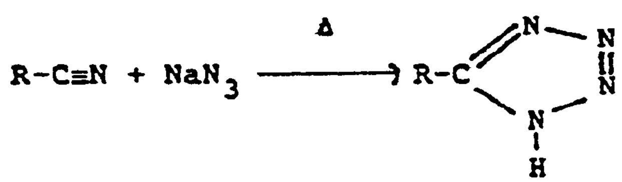


Le passage d'un amide à un composé cyclique tel que le dihydroimidazole ou la tétrahydropyrimidine peut se faire par une deuxième méthode consistant à former un thioamide intermédiaire selon le schéma réactionnel suivant :

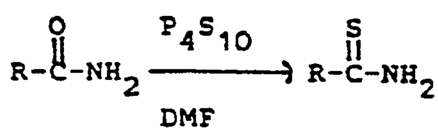


(dans ce cas, R peut représenter soit le sulfonamide dérivé de diarylméthane de formule I, dans lequel Y représente $CONH_2$, soit la chaîne aminée, condensée ensuite sur le sulfochlorure pour éviter des dégradations), puis à former un isothiouronium selon le schéma réactionnel suivant :

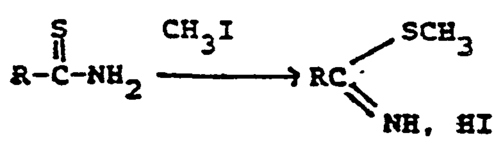


puis à cycliser par une diamine selon le schéma réactionnel suivant :

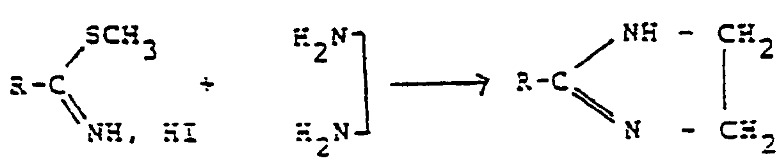

Le passage d'un acide

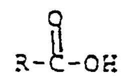

au noyau imidazole peut se faire par condensation directe de l'éthylènediamine sur l'acide selon le schéma réactionnel suivant :

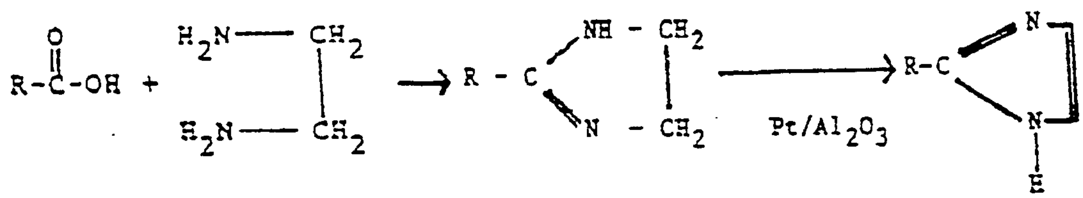


Pour préparer les composés de l'invention de formule II, dans lesquels Y représente $NH_2$, $NHR_6$, ou $NR_6R_7$, $R_6$, $R_7$ représentant un radical alcoyle de 1 à 4 atomes de carbone, ou forme avec l'azote une amine cyclisée non aromatique telle que la morpholine, la pipéridine ou la pyrrolidine, on peut faire réagir un sulfohalogenure, notamment sulfochlorure de formule :

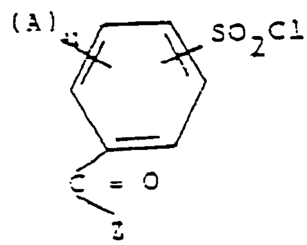

dans laquelle :
- A représente $R_1$, à l'exception de $NH_2$, c'est-à-dire Cl, F, Br, $NO_2$, $CF_3$, un radical alcoyle de 1 à 4 atomes de carbone, un radical alcoxy de 1 à 4 atomes de carbone, acétamido ou benzamido,
- Z représente :

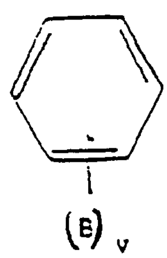

ou

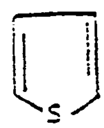

,

- B a les significations indiquées pour $R_2$, à l'exception de $NH_2$, c'est à dire B représente Cl, Br, F, $NO_2$, $CF_3$, un radical alcoxy ou alcoyle de 1 à 4 atomes de carbone, acétamido ou benzamido et de préférence Cl, ou $NO_2$.
- u représente un nombre entier de O à 2
- v représente un nombre entier de O à 2 ou un disulfohalogénure, notamment disulfochlorure de formule

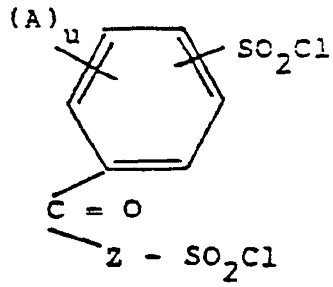

dans laquelle A, u, Z ont les significations indiquées ci-dessus ;
sur une aminoalcoylamine de formule :

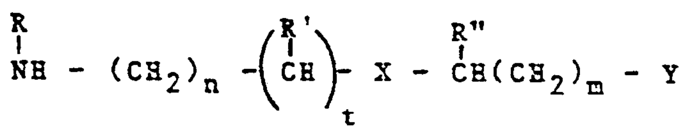

dans laquelle :

- R représente H, un radical alcoyle de 1 à 6 atomes de carbone ou un radical benzyle,
- R' et R" représentent H ou un radical alcoyle de 1 à 4 atomes de carbone,
- n représente un nombre entier variant de O à 1O,
- m représente un nombre entier variant de O à 1O,
- t vaut O ou 1,
- Y ayant la signification $NH_2$, $NHR_6$, ou $NR_6R_7$, tel que défini ci-dessus,
- X ayant la signification indiquée ci-dessus.

L'obtention des composés ci-dessus dans lesquels $R_1$ et/ou R2 représentent $NH_2$, est réalisée en réduisant les composés dans lesquels A et/ou B représentent $NO_2$, par hydrogénation catalytique ou par réduction chimique.

La réaction qui a été décrite ci-dessus à propos des sulflochlorures s'applique également aux autres sulfohalogènures, notamment aux sulfobromures.

Un mode de préparation avantageuse des composés de l'invention dans lesquels Y représente $NH_2$, $NHR_6$ ou $NR_6R_7$, défini ci-dessus, consiste à procéder comme indiqué ci-après, à titre d'exemple général.

A O,1 mole de chlorure d'(aroyl)-phénylsulfonyle en solution dans un solvant organique (par exemple l'éther éthylique, le chlorure de méthylène, le chloroforme ...) et refroidi dans un bain de glace, on ajoute sous agitation O,1 mole d'une amine tertiaire (telle que la triéthylamine, la pyridine ...) et lentement O,1 mole d'aminoalcoylamine en solution dans le même solvant organique.

Le milieu réactionnel est ramené à température ambiante, après environ 12 heures de contact, toujours sous agitation, et est filtré ; le filtrat est évaporé à sec puis repris par de l'eau.

Le produit obtenu est :

- soit sous forme de cristaux qui sont lavés à l'eau, essorés, séchés puis recristallisés dans un solvant approprié,
- soit sous forme d'huile, qui est soit cristallisée directement dans un solvant, ou soit après extraction par un solvant organique (notamment éther, acétate d'éthyle, chlorure de méthylène, chloroforme) séchage et évaporation, est purifiée sur colonne de chromatographie sur silice puis cristallisée dans un solvant approprié.

Pour préparer les composés comportant deux chaînes latérales

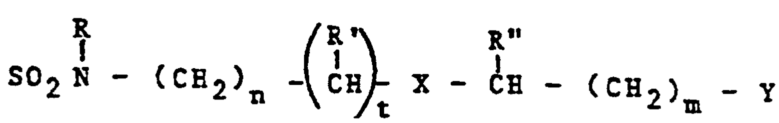

on opère comme indiqué ci-après :

On part d'un équivalent de disulfochlorures et on le condense avec au moins deux équivalents d'aminoacide ou d'aminoalcoylamine appropriés.

Un groupe de sulfochlorures avantageusement utilisés dans la préparation des composés de l'invention répondant à la formule II, est constitué par :

– le chlorure de benzoyl - 2 nitro - 4 benzènesulfonyle
– le chlorure de benzoyl - 2 chloro - 4 benzènesulfonyle
– le chlorure de chloro - 4 (chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

Un groupe d'amino-acides avantageusement utilisés dans la préparation des composés de l'invention de formule II est constitué par :

$$- NH_2-(CH_2)_n-COOH$$

n variant de 1 à 11.

Un groupe de dialcoylaminoalcoylamines avantageusement utilisés dans la préparation des composés de formule II de l'invention est constitué par :

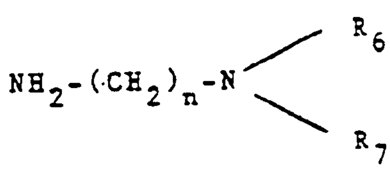

n = 2 ou 3

$R_6$ et $R_7$ représentant un groupe alcoyle de 1 à 4 atomes de carbone ou formant un groupe morpholine avec l'atome d'azote.

Pour préparer les composés de l'invention de formule III

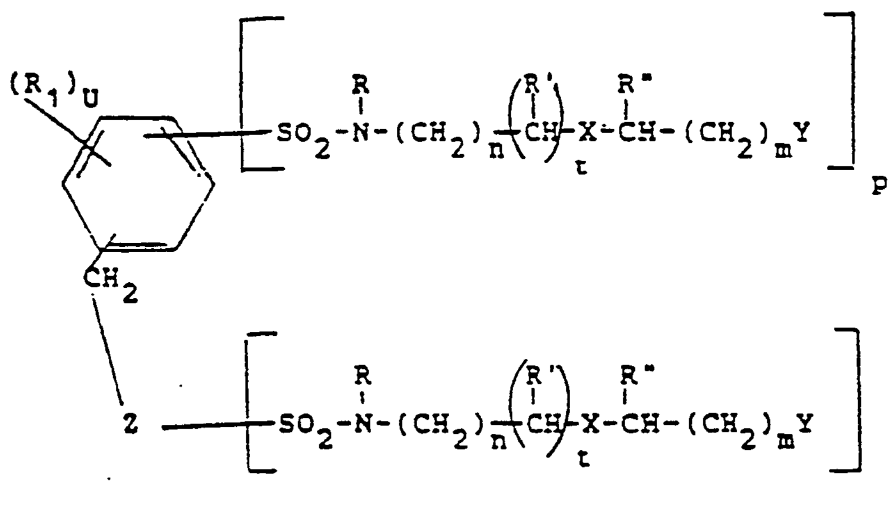

(III)

dans laquelle $R_1$, R, R′, R″, X, Y, Z, m, n, p, q, t et u ont les significations indiquées ci-dessus, on peut soumettre le composé de formule

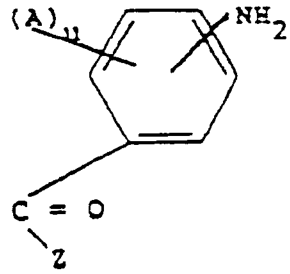

ou de formule

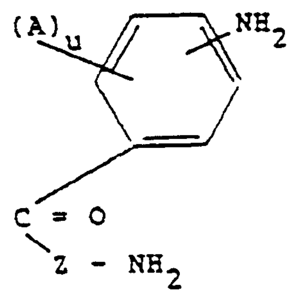

à une réduction, notamment à l'aide de chlorure d'aluminium et d'hydrure de lithium et d'aluminium, pour réduire C = O en $CH_2$ et obtenir le composé de formule

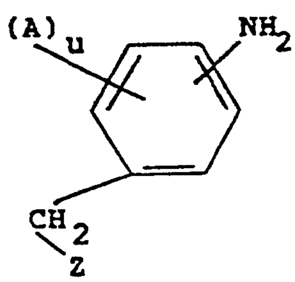

ou

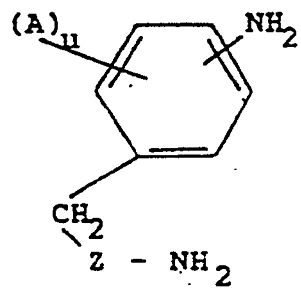

– on peut soumettre l'un de ces composés à la réaction de Sandmeyer (formation du sel de diazonium et action de l'anhydride sulfureux) pour obtenir les composés de formule :

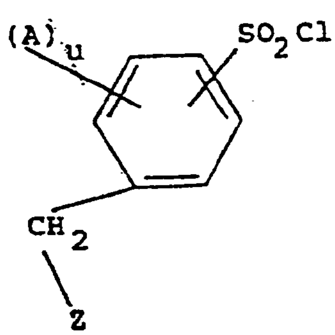

ou

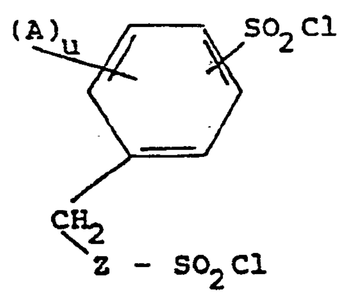

– on peut ensuite condenser le sulfohalogénure ou le disulfohalogénure de formule indiquée ci-dessus, sur un composé de formule :

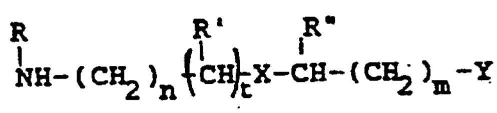

dans laquelle R, R', R", X, Y, n, m et t ont les significations indiquées ci-dessus, suivi éventuellement d'une réduction lorsque A et/ou B représente $NO_2$.

Un groupe amino-acides ou de dialcoylaminoalcoylamines avantageusement utilisés pour la préparation des composés de formule III, est choisi parmi

$$- NH_2 - (CH_2)_n - COOH$$

n variant de 1 à 11,

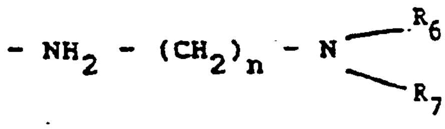

$n = 2$ ou 3

$R_6$ et $R_7$ représentant un groupe alcoyle de 1 à 4 atomes de carbone ou formant un groupe morpholine avec l'atome d'azote.

Les composés de formule

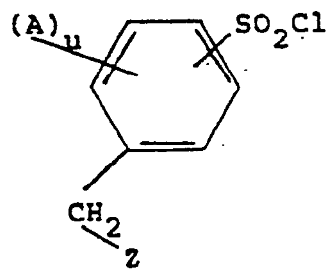

préférés pour la préparation de formule III sont choisis parmi

- chlorure de (dichloro-2,4 benzyl)-3 chloro-4 benzènesulfonyle
- chlorure de (chloro-4 benzyl)-3 chloro-4 benzènesulfonyle
- chlorure de benzyl-2 chloro-4 benzènesulfonyle
- chlorure de (dichloro-2,4 benzyl)-2 chloro-4 benzènesulfonyle
- chlorure de (chloro-4 benzyl)-2 chloro-4 benzènesulfonyle
- chlorure de benzyl-4 chloro-3 benzènesulfonyle
- chlorure de (dichloro-2,4 benzyl)-4 chloro-3 benzènesulfonyle
- chlorure de (chloro-4 benzyl)-4 chloro-3 benzènesulfonyle
- chlorure de benzyl-3 chloro-4 benzènesulfonyle.

Pour préparer les composés de l'invention de formule IV

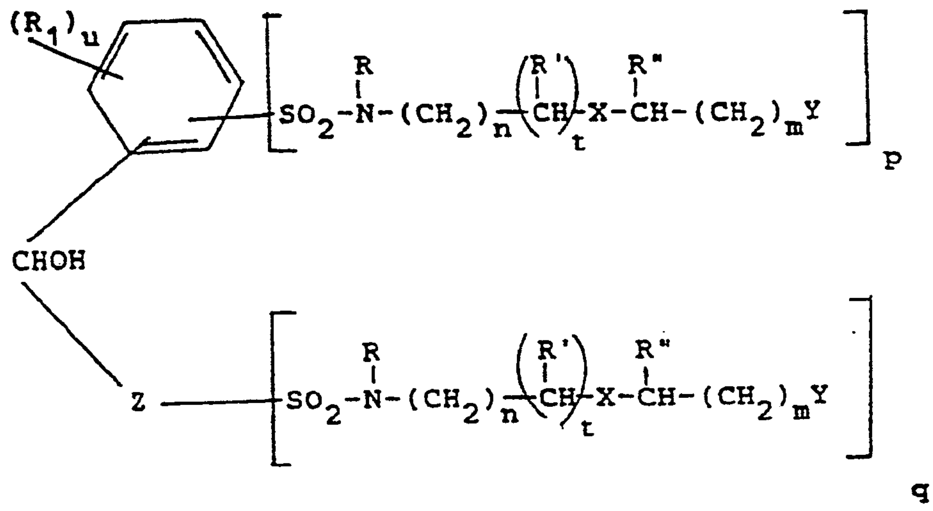

(IV)

dans laquelle $R_1$, R, R', R'', X, Y, Z, m, n, p, q, t et u ont les significations indiquées ci-dessus, on peut soumettre le composé de formule II

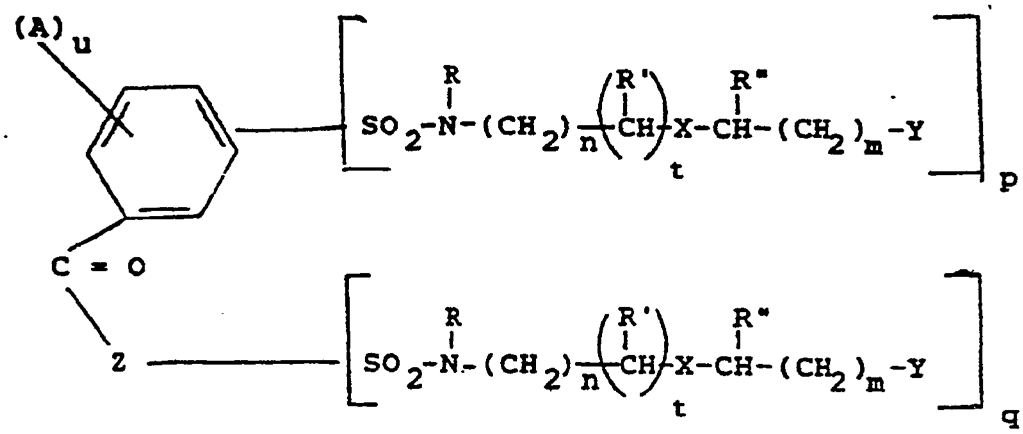

dans laquelle A, X, Y, Z, R, R', R'', m, n, p, q, t et u ont les significations indiquées ci-dessus, et tel qu'obtenu selon ci-dessus, avant la réduction éventuelle de A et/ou B lorsqu'ils représentent $NO_2$,
à une réduction pour transformer C = O en CHOH, à l'aide de borohydrure alcalin, notamment le borohydrure de sodium.
Un groupe d'amino-acides ou de dialcoylaminoalcoylamines avantageusement utilisés pour la préparation des composés de formule IV est choisi parmi

$$- NH_2 - (CH_2)_n - COOH$$

n variant de 1 à 11,

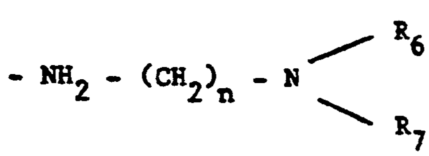

n = 2 ou 3

$R_6$ et $R_7$ représentant un groupe alcoyle de 1 à 4 atomes de carbone ou formant un groupe morpholine avec l'atome d'azote.

Les sulfochlorures avantageusement utilisés pour la préparation des composés de formule IV sont choisis parmi

– le chlorure de benzoyl - 2 nitro - 4 benzènesulfonyle
– le chlorure de benzoyl - 2 chloro - 4 benzènesulfonyle
– le chlorure de chloro - 4(chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

Pour préparer les composés de l'invention répondant à la formule V suivante

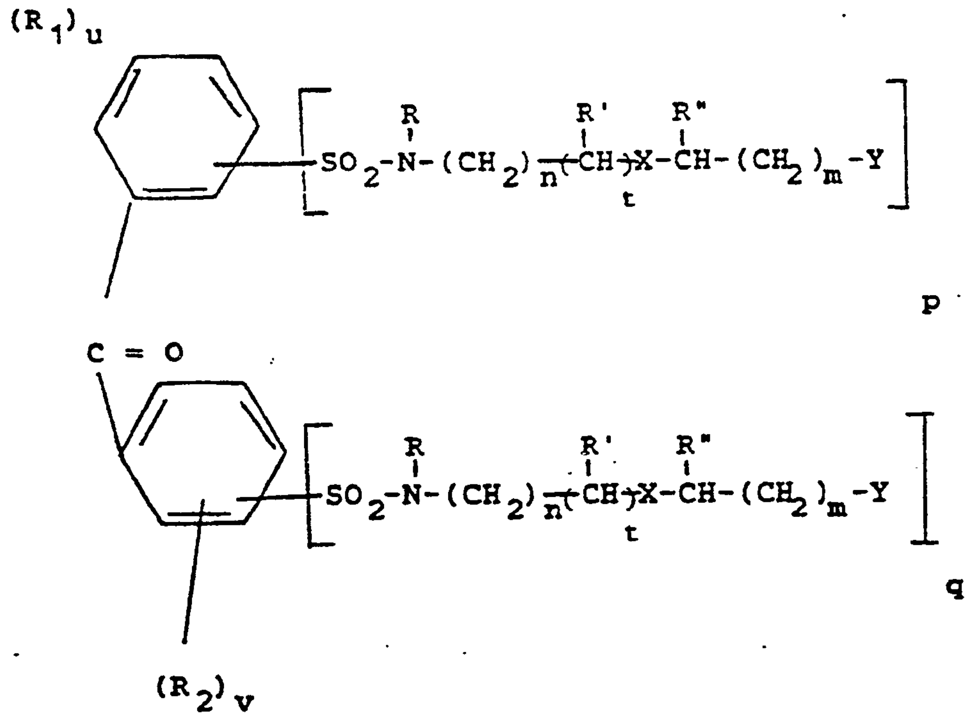

dans laquelle $R_1$, $R_2$, R, R', R'', X, Y, m, n, p, q, t, u et v ont les significations indiquées ci-dessus, on peut faire réagir un sulfohalogénure, notamment un sulfochlorure de formule

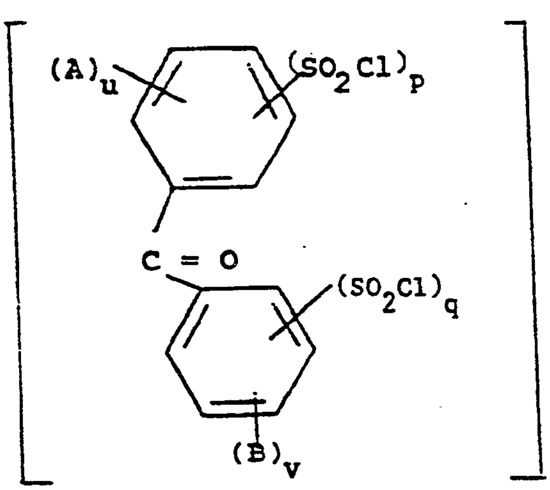

Vquater

dans laquelle A, B, p, q, u et v ont les significations indiquées ci-dessus, ou un disulfohalogénure, notamment un disulfochlorure de formule

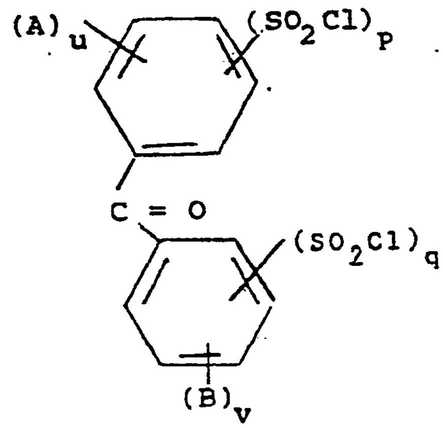

dans laquelle A, B, p, q, u et v ont les significations précédemment indiquées sur un composé de formule :

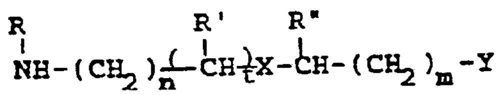

dans laquelle R, R', R", m, n, t, X et Y ont les significations indiquées ci-dessus,
suivi éventuellement d'une réduction lorsque A et/ou B représente $NO_2$.
Pour préparer les composés de formule V dans laquelle
- Y représente COOH,
. on peut faire réagir un sulfochlorure de formule Vquater
sur un amino-acide de formule :

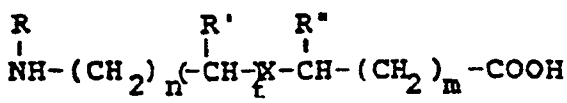

dans laquelle R, R', R", n, m, t et X ont les significations indiquées ci-dessus.
La préparation des composés dans lesquels Y représente $COOR_3$ ($R_3$ étant différent de H), notamment $COOC_2H_5$, peut être obtenue par estérification des composés de l'invention dans lesquels Y représente -CO-OH.
La préparation des composés de l'invention de formule V, dans laquelle Y représente $COOR_3$, $R_3$ représentant H ou un groupe alcoyle de 1 à 4 atomes de carbone, et dans lesquels $R_1$ représente $NH_2$ et/ou $R_2$ représente $NH_2$, est effectuée en réduisant les composés dans lesquels A représente $NO_2$ et/ou B représente $NO_2$, par hydrogénation catalytique, ou par réduction chimique.
La réaction qui a été décrite ci-dessus, à propos des sulfochlorures s'applique également aux autres sulfohalogènures, notamment aux sulfobromures.
Pour préparer les composés de l'invention, notamment ceux de formule V, dans laquelle Y représente $COOR_3$, $R_3$ représentant H ou un radical alcoyle de 1 à 4 atomes de carbone, on procède avantageusement comme suit à titre d'exemple général :
A O,1 mole de chlorure d'(aroyl)-phénylsulfonyle, en solution dans un solvant organique très peu soluble dans l'eau (par exemple ether éthylique, chlorure de méthylène, chloroforme), on ajoute O,2 mole d'aminoacide en solution dans 4 équivalents d'une solution basique aqueuse (par exemple soude, potasse, ammoniaque).
Le milieu réactionnel est agité vigoureusement jusqu'à disparition du sulfochlorure (suivi en chromatographie sur couche mince ou chromatographie en phase gazeuse).
Après élimination de la phase organique, la phase aqueuse est amenée à un pH compris d'environ 1 à 3, par addition d'une solution aqueuse acide 1 N à 1O N, de préférence 5 N (par exemple acide chlorhydrique ou acide sulfurique).
Or

Le produit obtenu se présente :
– soit sous forme de cristaux qui sont essorés puis séchés avant d'être recristallisés dans le solvant approprié,
– soit sous forme d'huile qui est soit cristallisée directement dans un solvant, ou soit après extraction par un solvant organique (notamment éther, acétate d'éthyle, chlorure de méthylène, chloroforme) séchage et évaporation, est purifiée sur colonne de chromatographie sur silice puis cristallisée dans un solvant approprié.
Pour préparer les composés de l'invention de formule VI,

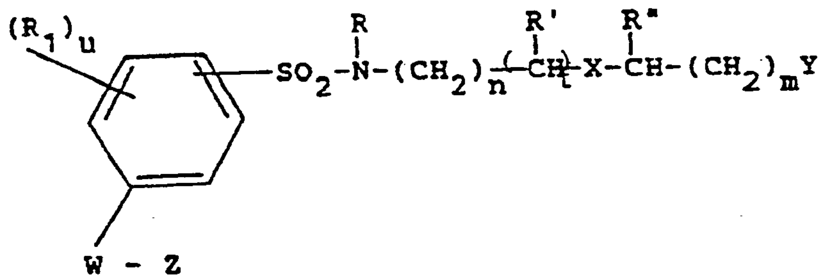

(VI)

dans laquelle $R_1$, R, R', R", X, Y, Z, W, m, n, t et u ont les significations indiquées ci-dessus, on peut soumettre un composé de formule

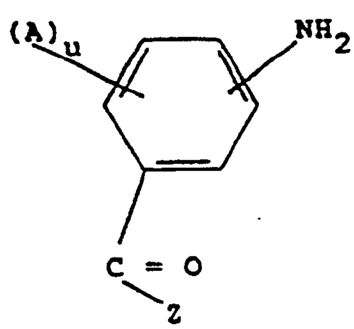

dans laquelle A, Z et u ont les singifications indiquées ci-dessus,
éventuellement à une réduction, notamment à l'aide de chlorure d'aluminium et d'hydrure d'aluminium et de lithium pour réduire - C = O en $CH_2$, et obtenir le composé de formule

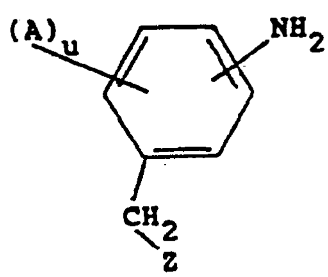

dans laquelle A, Z, u ont les significations indiquées ci-dessus,
. on peut ensuite soumettre l'un des composés aminés de formule indiquée ci-dessus à la réaction de Sandmeyer (formation du sel de diazonium et action de l'anhydride sulfureux), pour obtenir un sulfohalogénure de formule :

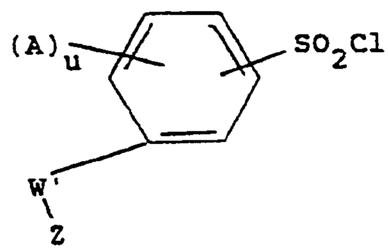

dans laquelle A, Z et u ont les significations indiquées ci-dessus et W′ représente C = O ou $CH_2$,

. on fait réagir le sulfohalogénure, notamment le sulfochlorure, de formule indiquée ci-dessus, sur un composé de formule :

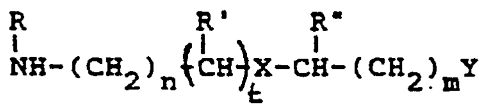

dans laquelle R, R′, R″, m, n, t, X et Y ont les significations indiquées ci-dessus,
pour obtenir le composé de formule

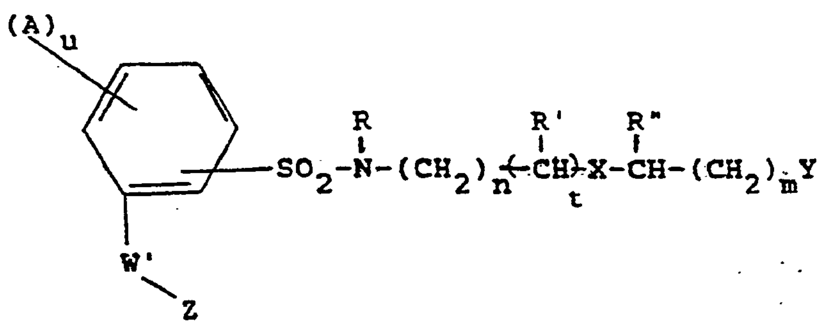

. et dans le cas où W′ représente C = O, on soumet éventuellement le composé ci-dessus à une réduction, notamment à l'aide de borohydrure alcalin, tel que le borohydrure de sodium, pour réduire C = O en CHOH,

. on effectue ensuite éventuellement une réduction lorsque A et/ou B représente $NO_2$.

Pour préparer les composés de l'invention de formule VII,

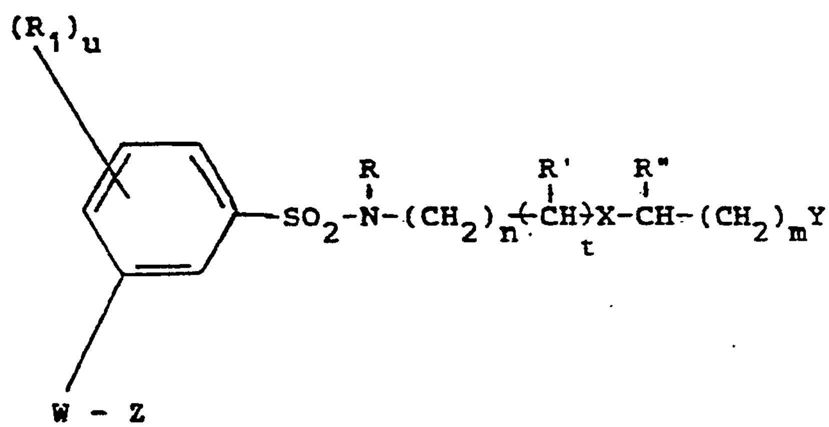

dans laquelle $R_1$, R, R′, R″, X, Y, Z, W, m, n, t et u ont les significations indiquées ci-dessus, on peut soumettre un composé de formule

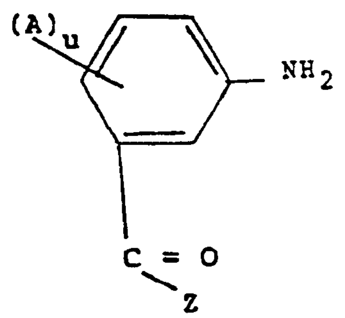

dans laquelle A, Z et u ont les significations indiquées ci-dessus,
éventuellement à une réduction, notamment à l'aide de chlorure d'aluminium et d'hydrure d'aluminium et de lithium pour réduire - C = O en $CH_2$, et obtenir le composé de formule

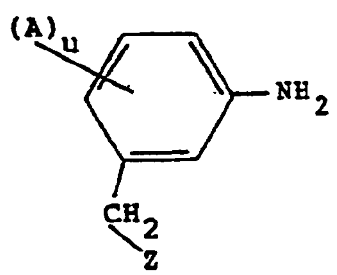

dans laquelle A, Z et u ont les significations indiquées ci-dessus,
. on peur ensuite soumettre l'un des composés aminés de formule indiquée ci-dessus à la réaction de Sandmeyer (formation du sel de diazonium et action de l'anhydride sulfureux), pour obtenir un sulfohalogénure de formule :

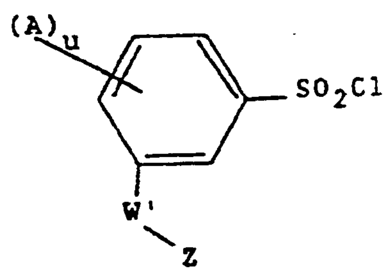

dans laquelle A, Z et u ont les significations indiquées ci-dessus et W' représente C = O ou $CH_2$,
. on fait réagir le sulfohalogénure, notamment le sulfochlorure, de formule indiquée ci-dessus, sur un ou deux composés de formule

$$\overset{R}{N}H-(CH_2)_n-(\overset{R'}{C}H)_t-X-\overset{R''}{C}H-(CH_2)_m-Y$$

dans laqelle R, R', R", X, Y, m, n et t ont les significations indiquées ci-dessus,
pour obtenir le composé de formule

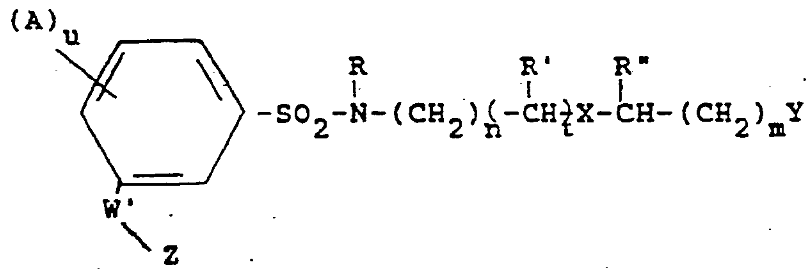

. et dans le cas où W' représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$.
Pour préparer les composés de l'invention de formule VII,
dans laquelle ,
- Y représente $COOR_3$, $R_3$ représentant H ou un radical alcoyle de 1 à 4 atomes de carbone,
. on peut soumettre un composé de formule

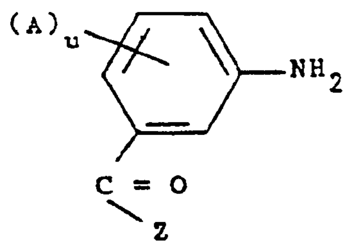

dans laquelle A, Z et u ont les significations indiquées ci-dessus,
éventuellement à une réduction, notamment à l'aide de chlorure d'aluminium et d'hydrure d'aluminium et de lithium pour réduire - C = O en $CH_2$, et obtenir le composé de formule

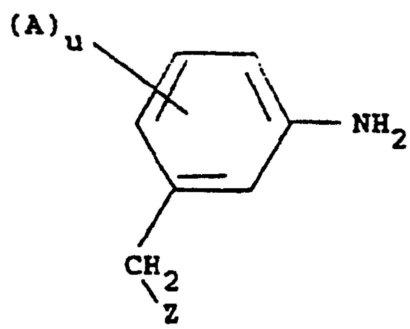

dans laquelle A, Z, et u ont les significations indiquées ci-dessus,
. on peut ensuite soumettre l'un des composés aminés de formule indiquée ci-dessus à la réaction de Sandmeyer (formation du sel de diazonium et action de l'anhydride sulfureux), pour obtenir un sulfohalogénure de formule :

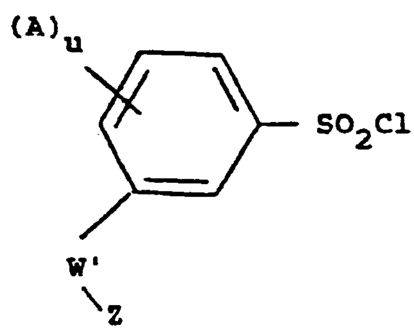

dans laquelle A, Z et u ont les significations indiquées ci-dessus et W′ représente C = O ou $CH_2$,
. on peut faire réagir le sulfohalogénure, notamment le sulfochlorure, de formule indiquée ci-dessus, sur un ou deux composés de formule

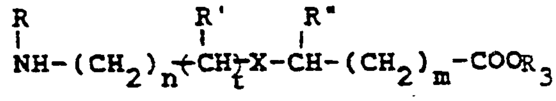

dans laquelle R, R′, R″, X, $R_3$, m, n et t ont les significations indiquées ci-dessus,
pour obtenir le composé de formule

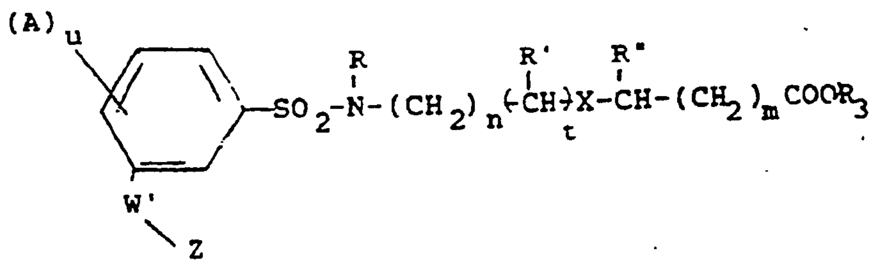

. et dans le cas où W′ représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$.
Pour préparer les composés de formule VIII, IX, X correspondant aux composés de formule VII dans lesquels Y représente $COOR_3$, $R_3$ représentant H ou un groupe alcoyle de 1 à 4 atomes de carbone, et dans lesquels W représente respectivement C = O (composés de formule VIII), $CH_2$ (composés de formule IX), CHOH (composés de formule X), on procède comme indiqué à propos des composés de formule VII, dans lesquels Y représente $COOR_3$, en ayant recours aux composés intermédiaires appropriés.
Pour préparer les composés de l'invention de formule XI

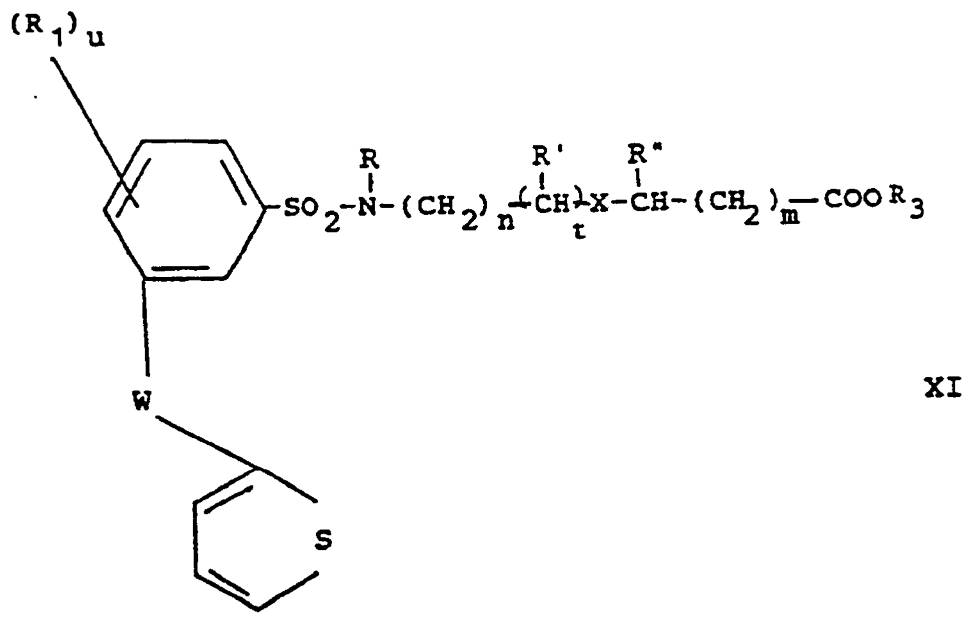

XI

- dans laquelle
le nombre total d'atomes de carbone de la chaîne

$$(CH_2)_n-(CH)_t-X-CH-(CH_2)_m-Y$$

est égal ou supérieur à 5, et dans laquelle $R_1$, $R_3$, R, R', R", X, W, m, n, t et u ont les significations indiquées ci-dessus, on peut soumettre le composé de formule

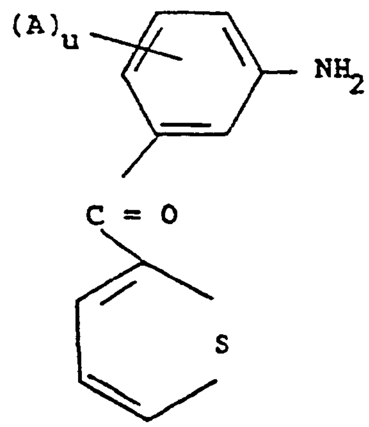

dans laquelle A et u ont les significations indiquées ci-dessus, éventuellement à une réduction, notamment à l'aide de chlorure d'aluminium et d'hydrure d'aluminium et de lithium pour réduire - C = O en $CH_2$, et obtenir le composé de formule

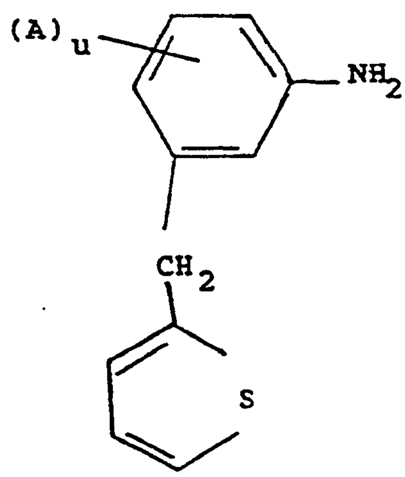

dans laquelle A, et u ont les significations indiquées ci-dessus,

. on peut ensuite soumettre le composé aminé de formule indiquée ci-dessus à la réaction de Sandmeyer (formation du sel de diazonium et action de l'anhydride sulfureux), pour obtenir un sulfohalogénure de formule :

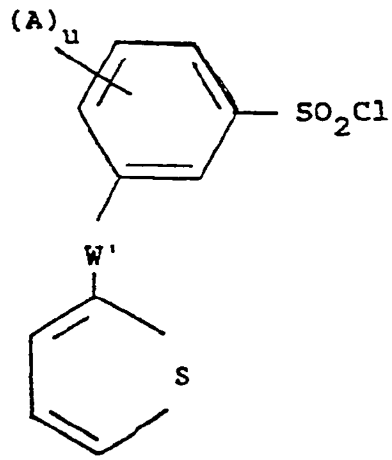

dans laquelle A, et u ont les significations indiquées ci-dessus et W′ représente C = O ou $CH_2$,
. on peut ensuite faire réagir le sulfohalogénure, notamment le sulfochlorure, de formule indiquée ci-dessus, sur un composé de formule

$$R\text{-}NH-(CH_2)_n-(CH(R'))_t-X-CH(R'')-(CH_2)_m-COOR_3$$

dans laquelle R, R′, R″, X, m, n, t et $R_3$ ont les significations indiquées ci-dessus, pour obtenir le composé de formule

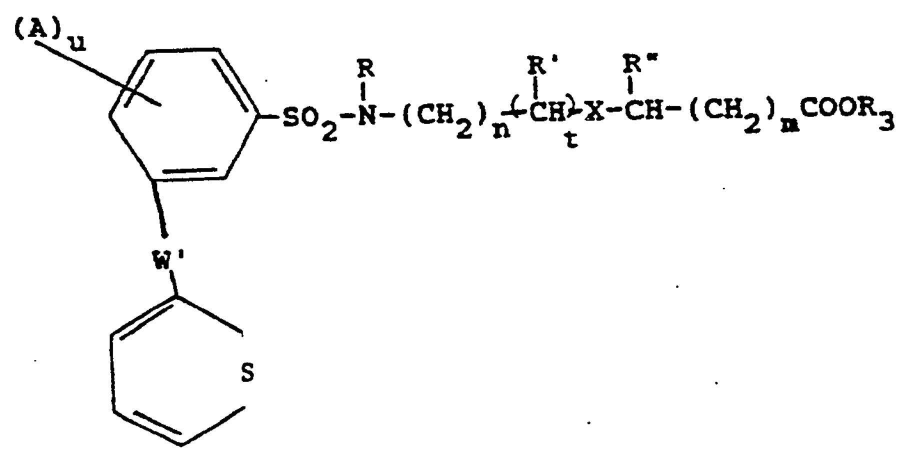

. et dans le cas où W′ représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$

Les sulfochlorures entrant dans la préparation dés composés de l'invention peuvent être préparés selon des procédés classiques.

Pour préparer les sulfochlorures qui conduisent à des composés de l'invention dans lesquels la chaîne latérale est en ortho par rapport au C = O, on peut avoir recours à la nitro - 2 benzophénone, éventuellement substituée, qui est réduit par une méthode connue, en amino - 2 benzophénone, éventuellement substituée, laquelle par une réaction de Sandmeyer est transformée en sulfohalogénure, notamment en chlorure de benzoyl - 2 benzènesulfonyle.

Pour préparer les sulfochlorures qui conduisent à des composés de l'invention dans lesquels la chaîne latérale est en méta ou en para par rapport au C = O, on procède comme indiqué précedemment en utilisant les produits de départ appropriés.

Les disulfochlorures sont préparés à partir des composés diaminés correspondants par une réaction de Sandmeyer qui consiste en :

1) la diazotation par de l'acide nitreux naissant ($NaNO_2$, HCl ou $NaNO_2$, acide formique ou $NaNO_2$, acide acétique),

2) l'addition du sel de diazonium à une solution saturée d'anhydride sulfureux en solution dans l'acide acétique en présence de chlorure cuivrique.

Les diamines indiquées ci-après sont préparées suivant les modes opératoires donnés dans la littérature

(amino-2 phényl) (amino-3-phényl) cétone

décrit dans CA 61 7186e

(amino-2 phényl) (amino-4 phényl) cétone

décrit dans CA 44 2960g à partir du dinitro correspondant réduit par voie chimique

décrit dans CA 61 7186e

(amino-3 phényl) (amino-3 phényl) cétone

décrit dans CA 53 3136e, 8061e, CA 61 7186e

Pour préparer les chlorures de thénoylbenzènesulfonyle, on procède suivant la technique de G. Holt et B. Padgin, J. Chem. Soc.1960, 2508, par réaction de Friedel et Crafts :

– soit en partant directement d'un chlorure de benzoyle chlorosulfoné en faisant agir le thiophène en présence de chlorure d'aluminium,

– soit en partant d'un chlorure de nitro benzoyle que l'on fait agir avec le thiophène en présence de chlorure d'aluminium, la nitrodiarylcétone obtenue étant alors réduite chimiquement ou de façon catalytique en amino diarylcétone laquelle donne après une réaction de Sandmeyer le sulfochlorure attendu.

Les exemples ci-après, relatifs à la préparation d'un certain nombre de sulfochlorures et de nouveaux composés de l'invention servent à illustrer l'invention mais ne sont pas limitatifs.

Exemple 1 : Préparation du chlorure de benzoyl - 2 nitro - 4 benzènesulfonyle.

72,6 g (0,3 mole) d'amino - 2 nitro - 5 benzophénone sont ajoutés à un mélange de 225 ml d'une solution d'acide chlorhydrique (d = 1,18)) et 75 ml d'acide acétique pur par petites portions. Le milieu réactionnel est mis sous agitation puis refroidi vers - 5°C et maintenu à cette température lors de l'addition, goutte à goutte, d'une solution de nitrite de sodium (21,75 g dans 75 ml d'eau.

L'addition terminée, le milieu réactionnel est agité à - 5° C pendant 45 minutes, puis filtré.

Le filtrat est ajouté par portions à une solution saturée d'anhydride sulfureux dans l'acide acétique (750 ml) refroidi à 0° Celsius contenant 16,5 g de chlorure cuivrique.

Le mélange laissé sous agitation vive pendant 3 heures, est versé sur de la glace pilée. Le précipité de sulfochlorure est essoré, lavé à plusieurs reprises avec de l'eau puis mis à sécher sous vide.

Le produit (62,5 g) est cristallisé dans un mélange benzène-cyclohexane. Il a pour formule brute :

$C_{13}H_8ClNO_5S$ (325,71) - Rendement 65 % F : 161° C.

Ce composé est un produit nouveau.

Exemple 2 : Préparation du chlorure de chloro-4 (chloro-2 benzoyl)-2 benzène-sulfonyle.

On procède comme indiqué ci-dessus à l'exemple I en utilisant de l'(amino-2 chloro-5 phényl)(chloro-2 phényl) cétone comme produit de départ.

Les caractéristiques du produit obtenu sont les suivantes :

$C_{13}H_7Cl_3O_3S$ (349,61). Rendement 58 % - F : 100° C.

Ce composé est un produit nouveau.

Exemple 3 : Préparation du chlorure de benzoyl-2 benzènesulfonyle

On procède comme indiqué ci-dessus à l'exemple I en utilisant de l'amino-2 benzophénone comme produit de départ.

Les caractéristiques du produit obtenu sont les suivantes :

$C_{13}H_9ClO_3S$ (280,72). Rendement 30 % - F : 99° C

Exemple 4 : Préparation du chlorure de (chloro-4 benzoyl)-2 benzènesulfonyle

On procède comme indiqué ci-dessus à l'exemple 1 en utilisant, comme produit de départ de l'(amino-2 phényl)(chloro-4 phényl) cétone.

Le produit obtenu a les caractéristiques suivantes :

$C_{13}H_8Cl_2O_3S$ (315,17). Rendement 53 % - F : 70° C

Exemple 5 : Préparation du chlorure de benzoyl-4 benzènesulfonyle

On utilise le procédé décrit ci-dessus à l'exemple 1, en ayant recours à l'amino 4-benzophénone.

Les caractéristiques du produit obtenu sont les suivantes :

$C_{13}H_8Cl_4O_3S$ (280,72). Rendement 70 % - F : 90° C

Exemple 6 : Préparation d'un composé de l'invention dans lequel la chaîne latérale est acide :

acide [(benzoyl-2 chloro-4 phényl)sulfonamido]-11 undécanoïque (composé n° 1572).

A 37,9 g (0,12 mole) de chlorure de benzoyl-2 chloro-4 benzènesulfonyle dans 250 ml de chlorure de methylène sont ajoutés, à 0° C et sous agitation 24,16 g (0,12 mole) d'acide amino-11 undécanoïque dans 250 ml

de soude normale.

Après six heures d'agitation à température ambiante, la phase organique est éliminée. La phase aqueuse est amenée à pH2 par addition d'une solution d'acide chlorhydrique 2N.

Après extraction par de l'acétate d'éthyle, lavage à l'eau de la phase organique, séchage sur sulfate de sodium, et évaporation sous pression réduite, le résidu est cristallisé dans l'éther de pétrole pour donner 44,5 g de cristaux blancs fondant à 70° C.

Le rendement de la réaction est de 77,2 %.

Exemple 7 : Préparation d'un composé de l'invention comportant deux chaînes latérales acides.

On utilise le procédé ci-dessus à l'exemple 6 en mettant en jeu 2 molécules d'amino-acide pour une molécule de sulfochlorure.

Exemple 8 : Préparation d'un composé de l'invention dans laquelle la chaîne latérale est aminée.

(composé n° 1344) benzoyl-2 chloro-4 N(morpholino-3 propyl)benzènesulfonamide.

A 4,72 g (0,015 mole) de chlorure de benzoyl-2 chloro-4 benzènesulfonyle dans 45 ml de chlorure de méthylène anhydre sont ajoutés 7,5 ml de triéthylamine redistillée puis, à 0° C, 2,16 g (0,015 mole) de morpholino-3 propylamine dans 10 ml de chlorure de méthylène anhydre.

Après trois heures d'agitation à température ambiante, le précipité (chlorhydrate de triéthylamine) est essoré. Le filtrat évaporé à sec sous vide est repris par 20 ml d'eau.

Les cristaux obtenus sont essorés, lavés avec de l'eau jusqu'à l'abscence d'ion chlorure puis séchés avant d'être recristallisés dans un mélange cyclohexane - benzène (1.1).

On obtient 4,93 g de cristaux blancs fondant à 116° C. Le rendement de la réaction est de 80 %.

Exemple 9 : Préparation de l'acide [[(thénoyl-2)-4 phényl] sulfonamido]-4 butyrique

a) préparation de la (nitro-4 phényl)(thiényl-2) cétone

A 11,14 g (0,06 mole) du chlorure de nitro-4 benzoyle dans 150 ml de chlorure de méthylène sont ajoutés avec précaution 15 g de chlorure d'aluminium puis 10 ml de thiophène (0,18 mole).

Après 4 heures d'agitation à température ambiante, le milieu réactionnel est versé sur de la glace. Après extraction au dichlorométhane, séchage de la phase organique sur sulfate de sodium et évaporation sous vide, le résidu est purifié par chromatographie sur silice l'éluant est le dichlorométhane.

On obtient 6,25 g du dérivé nitré (produit jaune) fondant à 172°C.

b) préparation de l'(amino-4 phéyl)(thiényl-2) cétone

L'(amino-4 phényl)(thiényl-2) cétone est obtenu par réduction catalytique du composé nitré précédent à pression ordinaire en utilisant comme catalyseur le palladium sur charbon à 10 % et comme solvant l'acétate d'éthyle.

Après absorption de la quantité théorique d'hydrogène, le catalyseur est éliminé par filtration, la phase organique est évaporée à sec, le résidu repris par une solution d'acide chlorhydrique 2N.

La phase aqueuse acide lavée avec de l'éther est ensuite amenée à pH alcalin. Les cristaux obtenus correspondant au produit attendu ont un point de fusion de 120°C.

c) préparation du chlorure de (thénoyl-2)-4 benzènesulfonyle

Le mode opératoire utilisé est celui décrit précédemment pour la réaction de Sandmeyer (Exemple 1).

A partir de 609 mg ($3.10^{-3}$ mole) de l'(amino-4 phényl)(thiényl-2) cétone, on obtient 400 mg de chlorure de (thénoyl-2)-4 benzènesulfonyle sous forme de cristaux fondant à 118°C.

d) préparation de l'acide [[(thénoyl-2)-4 phényl] sulfonamido]-4 butyrique

A 287 mg ($1.10^{-3}$ mole) du chlorure de (thénoyl-2) - 4 benzènesulfonyle dans 5 ml d'éther éthylique, sont ajoutés goutte à goutte 103 mg ($1.10^{-3}$ mole) d'acide amino-4 butyrique en solution dans 4 ml d'une solution de soude 0,5 N.

Après deux heures d'agitation à température ambiante, la phase organique est éliminée par décantation. La phase aqueuse est amenée à pH 2 par addition d'une solution d'acide chlorhydrique 4 N.

Les cristaux sont essorés, puis lavés par le miniumum d'eau jusqu'à absence d'ion $Cl^-$.

Après séchage sous vide, les cristaux obtenus (200 mg) ont un point de fusion de 128° - 130°C.

Les composés du tableau I ci-après ont été préparés conformément au procédé décrit dans l'exemple 6.

Exemple 10 :

Préparation du chlorure de (dichloro-2,4 benzoyl)-3 chloro-4 benzènesulfonyle

a) Préparation de la trichloro-2,2',4' nitro-5 benzophénone

A 5 g. de chlorure de chloro-2 nitro-5 benzoyle dans 60 ml de dichlorométhane sont ajoutés 6 g. de chlorure

d'aluminium, puis goutte à goutte, 30 ml de dichloro-1,3 benzène. Le milieu réactionnel est porté deux heures à 80°C puis refroidi, versé sur de la glace pilée. Après extraction au dichlorométhane, lavages à l'eau et séchage sur sulfate de sodium, la phase organique est évaporée à sec sous vide et le résidu cristallin est recristallisé dans le cyclohexane (3,6 g).

$C_{13}H_6Cl_3NO_3$ (330,55) Rdt. 48 % F : 117°C

b) Préparation de l'amino-5 trichloro-2,2',4' benzophénone

7,5 g de trichloro-2,2',4' nitro-5 benzophénone dans 100 ml d'acétate d'éthyle sont hydrogénés à pression ordinaire et à température ambiante en présence de palladium sur charbon.

Après élimination du catalyseur, le milieu réactionnel est évaporé à sec sous vide et donne un résidu cristallin 6,6 g.

$C_{13}H_8Cl_3NO$ (300,57) Rdt. 88 % F : 103°C

c) Préparation du chlorure de chloro-4 (dichloro-2,4 benzoyl)-3 benzènesulfonyle

On procède de la même façon qu'à l'exemple 1.

$C_{13}H_6Cl_4O_3S$ (384,06) Rdt. 60 % F 118°C

Exemple 11 :

Préparation du chlorure de benzoyl-3 chloro-4 benzènesulfonyle

a) Préparation de la chloro-2 nitro-5 benzophénone

On procède comme dans l'exemple 10 a) par action du benzène sur le chlorure de chloro-2 nitro-5 benzoyle en présence de chlorure d'aluminium.

$C_{13}H_8ClNO_3$ (261,66) Rdt. 55 % F : 85°C

b) Préparation de l'amino-5 chloro-2 benzophénone

On procède comme dans l'exemple 10 b) à partir du chloro-2 nitro-5 benzophénone.

c) Préparation du chlorure de benzoyl-3 chloro-4 benzènesulfonyle

On procède de la même façon qu'à l'exemple 1.

$C_{13}H_8Cl_2O_3S$ (315,14) Rdt. 40 % F : 92°C

Exemple 12 :

Préparation du chlorure de (chloro-4 benzoyl)-3 chloro-4 benzènesulfonyle

a) Préparation de la dichloro-2,4 nitro-5 benzophénone

On procède comme dans l'exemple 10 a) par action du chlorobenzène sur le chlorure de chloro-2 nitro-5 benzoyle en présence de chlorure d'aluminium.

$C_{13}H_7Cl_2O_3$ (296,11) Rdt. 76 % F : 92°C

b) Préparation de l'amino-5 dichloro-2,4' benzophénone

On procède comme dans l'exemple 10 b) à partir de la dichloro-2, 4' nitro-5 benzophénone.

c) Préparation du chlorure de (chloro-4 benzoyl)-3 chloro-4 benzène sulfonyle

On procède comme dans l'exemple 1.

$C_{13}H_7Cl_3O_3S$ (349,62) Rdt. 65 % F : 110°C

Exemple 13 :

Préparation du chlorure de benzoyl-4 chloro-3 benzènesulfonyle

a) Préparation de la chloro-2 nitro-4 benzophénone

On procède comme dans l'exemple 10 a) par action du benzène sur le chlorure de chloro-2 nitro-4 benzoyle en présence de chlorure d'aluminium.

$C_{13}H_8ClNO_3$ (261,66) Rdt. 75 % F : 90°C

b) Préparation de l'amino-4 chloro-2 benzophénone

On procède de la même façon qu'à l'exemple 10 b).

c) Préparation du chlorure de benzoyl-4 chloro-3 benzènesulfonyl

On procède de la même façon qu'à l'exemple 10 c).

Exemple 14

Préparation du chlorure de chloro-4(thénoyl-2)-3 benzènesulfonyle

a) Préparation de la (chloro-2 nitro-5 phényl) (thiényl-2) cétone

On procède comme indiqué à l'exemple 9 a) en partant du chlorure de chloro-2 nitro-5 benzoyle.

$C_{11}H_6ClNO_3S$ (267,69) Rdt. 85 % F : 105°-106°C

b) Préparation du chlorydrate de l'(amino-5 chloro-2 phényl) (thiényl-2) cétone

On procède comme indiqué à l'exemple 9 b).

$C_{11}H_8ClNOS$, HCl (274,17) Rdt. 95 % F : 115°-120°C

c) Préparation du chlorure de chloro-4(thénoyl-2)-3 benzènesulfonyle

On procède comme indiqué à l'exemple 1.

$C_{11}H_{16}Cl_2O_3S_2$ (321,20) Rdt. 72 % F : 84°C

Exemple 15

Préparation de l'acide[[[(chloro-4 phényl) hydroxyméthyl]-3 phényl] sulfonamido]-12 dodécanoïque IC-1810

A 4,8 g d'acide[(benzoyl-3 chloro-4 phényl)sulfonamido]12 dodécanoïque dans 200 ml d'éthanol absolu sont ajoutés 1,1 g de borohydrure de sodium par petites portions en maintenant la température entre 0° et 5°C. Après 2 heures d'agitation à température ambiante, le milieu réactionnel est neutralisé par addition d'éthanol chlorydrique puis évaporé à sec sous vide. Le résidu est repris par de l'acétate d'éthyle, la phase organique est lavée à l'eau, séchée sur sulfate de sodium puis évaporée à sec sous vide. On obtient le produit sous forme de cristaux (4g).

$C_{25}H_{34}ClNOS_5S$ (490,02) Rdt. 63 % F : 122°C

Les composés IC-1796 et IC-1807 sont préparés par cette méthode.

Exemple 16

Acide[(benzyl-3 chloro-4 phényl), sulfonamido]11 undécanoïque IC-1808

a) Préparation de la benzyl-3 chloro-4 aniline

On ajoute, sous azote, dans 1000 ml d'éther anhydre refroidi à 0°C, et par petites portions, 6,8 g d'hydrure de lithium, aluminium puis 44,4 g de chlorure d'aluminium puis 22 g d'amino-5 chloro-2 benzophénone en solution dans de l'éther anhydre. Après 2 heures d'agitation et au reflux, on décompose l'excès des réactifs par une addition très lente de 7 ml de soude à 15 % et enfin de 21 ml d'eau. Après filtration et lavages du précipité avec 2 x 200 ml d'éther, la phase organique est lavée, séchée sur $Na_2SO_4$ puis évaporée à sec sous vide. Après purification sur colonne de silice éluant hexane : 3 $CH_3COOC_2H_5$ : 2, on isole 17 g du produit sous forme d'une laque incolore.

$C_{13}H_{12}ClN$ (217,68) Rdt. 82 %

b) Préparation du chlorure de benzyl-3 chloro-4 benzènesulfonyle

On procède comme indiqué à l'exemple 1.

$C_{13}H_{10}Cl_2O_2S$ (301,19) Rdt. 52 %

c) Acide [(benzyl-3 choro-4 phényl) sulfonamido]-11 dodécanoïque

On procède comme indiqué à l'exemple 6.

$C_{24}H_{32}ClNO_4S$ (467,04) Rdt. 30% F : 88-90°C.

Le composé IC-1797 est préparé de façon similaire.

Exemple 17

Préparation du [(benzoyl-2 chloro-4 phényl) sulfonamido]-6 capronitrile

A 15,7 g de chlorure de chloro-4 benzoyl-2 benzènesulfonyle dans 100 ml de dichlorométhane anhydre sont ajoutés 15 ml de triéthylamine redistillée, puis à 0°C et goutte à goutte 5,6 g d'amino-6 capronitrile dans 20 ml de dichlorométhane.

Après une nuit d'agitation à 20°C, le milieu réactionnel est évaporé à sec sous vide. Le résidu est repris dans 100 ml d'eau et extrait par 2 x 200 ml d'acétate d'éthyle.

Après séchage, évaporation à sec sous vide et purification sur colonne de silice ($CH_2Cl_2$), on obtient 19 g d'un produit sirupeux.

$C_{19}H_{19}ClN_2S$ (390,89) Rdt. 95%

Exemple 18

Préparation du [(benzoyl-2 chloro-4 phényl) sulfonamido]-11 undécanoate d'éthyle IC 1740

A 5,3 g du chlorhydrate de l'amino-11 undécanoate d'éthyle dans 30 ml de chlorure de méthylène anhydre sont ajoutés 7 ml de triéthylamine anhydre puis après une heure d'agitation à température ambiante, goutte à goutte, 6,3 g du chloure de benzoyl-2 chloro-4 benzènzsulfonyle. Après 12 heures d'agitation, le milieu réactionnel est évaporé à sec sous vide repris dans l'eau, extrait à l'acétate d'éthyle. La phase organique lavée à l'eau jusqu'à absence d'ion $Cl^-$ est séchée sur sulfate de sodium et évaporée à sec sous vide.

Par purification sur colonne de silice éluant hexane : 5 acétate d'héthyle : 1, on obtient le produit pur sous forme de cristaux.

$C_{26}H_{34}ClNO_5S$ (508,08) Rdt. 80% F : 33°-34°C

Les composés IC-1741 et 1742 sont préparés de manière similaire.

Exemple 19

Préparation du [[(chloro-2 benzoyl)-2 chloro-4 phényl] sulfonamido]-11 undécanamide

a) Préparation du chlorure de [[(chloro-2 benzoyl)-2 chloro-4 phényl] sulfonamido]-11 undécanoyle

A 10,3 g de l'acide [(chloro-2 benzoyl)-2 chloro-4 phényl] sulfonamido-11 undécanoïque dans 120 ml de benzène anhydre son ajoutés 19 g de chlorure de thionyle redistillé. Après deux heures au reflux, le milieu réactionnel est évaporé à sec sous vide et donne une huile incolore qui est utilisée sans purification ultérieure.

$C_{24}H_{28}Cl_3NO_4S$ (532,91) Rdt. 98 %

b) Préparation du [[(chloro-2 benzoyl)-2 chloro-4 phényl] sulfonamido]-11 undécanamide

Une solution de 2 g du chlorure de l'acide [(chloro-2 benzoyl)-2 chloro-4 phényl] sulfonamido-11 undécanoïque dans 15 ml de benzène anhydre est saturée par de l'ammoniaque à 10°C. Après deux heures d'agitation, le milieu réactionnel est évaporé à sec sous vide, le résidu est repris dans l'eau puis extrait à l'acétate d'éthyle. La phase organique conduit après évaporation à des cristaux qui sont recristallisés dans le benzène (1,22 g).

$C_{24}H_{30}Cl_2N_2O_4S$ (513,48) Rdt. 70% F : 95°-97°C

Les composés du tableau I ci-après ont été préparés conformément au procédé décrit dans l'exemple 6.

TABLEAU I

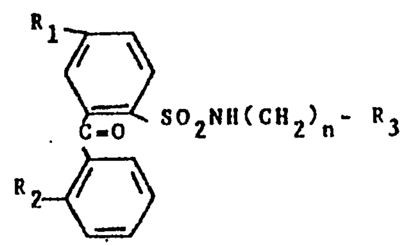

| Numéro de composés | $R_1$ | $R_2$ | $R_3$ | n | Formule brute | Masse moléculaire | Point de fusion | Rendement % |
|---|---|---|---|---|---|---|---|---|
| 1564 | $NO_2$ | H | COOH | 1 | $C_{15}H_{12}N_2O_7S$ | 364,34 | 120° | 67 % |
| 1565 | Cl | H | COOH | 1 | $C_{15}H_{12}ClNO_5S$ | 353,78 | 125° | 59 % |
| 1566 | $NO_2$ | H | COOH | 3 | $C_{17}H_{16}N_2O_7S$ | 393,39 | 162° | 66 % |
| 1567 | Cl | H | COOH | 3 | $C_{17}H_{16}ClNO_5S$ | 381,84 | 148° | 60 % |
| 1568 | $NO_2$ | H | COOH | 5 | $C_{19}H_{20}N_2O_7S$ | 420,45 | 100° | 65 % |
| 1569 | Cl | H | COOH | 5 | $C_{19}H_{20}ClNO_5S$ | 409,90 | 94° | 78 % |
| 1570 | Cl | Cl | COOH | 5 | $C_{19}H_{19}Cl_2NO_5S$ | 444,35 | 128° | 40 % |
| 1571 | $NO_2$ | H | COOH | 10 | $C_{24}H_{30}N_2O_7S$ | 490,58 | 67° | 87 % |
| 1572 | Cl | H | COOH | 10 | $C_{24}H_{30}ClNO_5S$ | 480,03 | 70° | 77 % |
| 1573 | Cl | Cl | COOH | 3 | $C_{17}H_{15}Cl_2NO_5S$ | 416,279 | 112° | 27 % |
| 1574 | Cl | Cl | COOH | 10 | $C_{24}H_{29}Cl_2NO_5S$ | 514,47 | 99° | 77 % |
| 1575 | Cl | Cl | COOH | 1 | $C_{15}H_{11}Cl_2NO_5S$ | 388,22 | 166° | 55 % |
| 1682 | H | H | COOH | 10 | $C_{24}H_{31}NO_5S$ | 445,58 | 70° | 65 % |
| 1683 | H | H | COOH | 11 | $C_{25}H_{33}NO_5S$ | 459,61 | 80° | 65 % |

TABLEAU I (Suite)

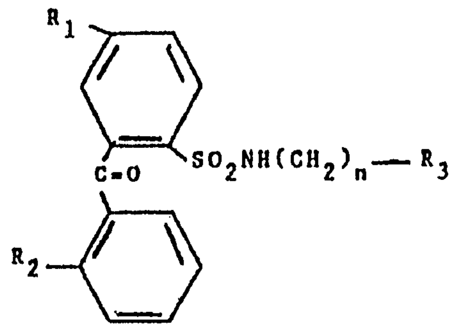

| Numéro de composés | $R_1$ | $R_2$ | $R_3$ | n | Formule brute | Masse moléculaire | Point de fusion en °C | Rendément % |
|---|---|---|---|---|---|---|---|---|
| 1740 | Cl | H | $COOC_2H_5$ | 10 | $C_{26}H_{34}ClNO_5S$ | 508,08 | 33-34 | 80 |
| 1741 | Cl | H | $COOC_2H_5$ | 5 | $C_{21}H_{24}ClNO_5S$ | 437,94 | 55 | 55 |
| 1742 | Cl | H | $COOC_2H_5$ | 7 | $C_{23}H_{28}ClNO_5S$ | 465,99 | 45 | 70 |
| 1743 | Cl | Cl | COOH | 7 | $C_{21}H_{23}Cl_2NO_5S$ | 472,38 | 76 | 60 |
| 1744 | Cl | H | COOH | 7 | $C_{21}H_{24}ClNO_5S$ | 437,94 | 96 | 60 |
| 1745 | Cl | H | CN | 5 | $C_{19}H_{19}ClN_2O_3S$ | 390,89 | sirupeux | 95 |
| 1755 | Cl | Cl | COOH | 11 | $C_{25}H_{31}Cl_2NO_5S$ | 528,50 | 76-78 | 58 |
| 1760 | Cl | Cl | $CONH_2$ | 10 | $C_{24}H_{30}Cl_2N_2O_4S$ | 513,48 | 95-97 | 65 |

En mettant en oeuvre les produits de départ (sulfochlorures et amino-acides décrits ci-dessus dans la préparation de composés de l'invention), on obtient les produits suivants :

acide [[chloro-4 (chloro-4 benzoyl)-2 phényl] sulfonamido] -12 dodécanoïque,

acide [[chloro-4 (chloro-4 benzoyl)-2 phényl] sulfonamido] -11 undécanoïque,

acide [[chloro-4 (chloro-4 benzoyl)-2 phényl] sulfonamido] -8 octanoïque,

acide [[chloro-4 (chloro-4 benzoyl)-2 phényl] sulfonamido] -6 hexanoïque.

acide [[chloro-4 (dichloro-2,4 benzoyl)-2 phényl] sulfonamido]-12 dodécanoïque,

acide [[chloro-4 (dichloro-2,4 benzoyl)-2 phényl] sulfonamido]-11 undécanoïque,

acide [[chloro-4 (dichloro-2,4 benzoyl)-2 phényl] sulfonamido]-8 octanoïque,

acide [[chloro-4 (dichloro-2,4 benzoyl)-2 phényl] sulfonamido]-6 héxanoïque.

acide [[(chloro-4 benzoyl)-2 phényl] sulfonamido]-12 dodécanoïque,

acide [[(chloro-4 benzoyl)-2 phényl] sulfonamido]-11 undécanoïque,

acide [[(chloro-4 benzoyl)-2 phényl] sulfonamido]-8 octanoïque,

acide [[(chloro-4 benzoyl)-2 phényl] sulfonamido]-6 héxanoïque.

acide [[(dichloro-2-4 benzoyl)-2 phényl] sulfonamido]-12 dodécanoïque,

acide [[(dichloro-2-4 benzoyl)-2 phényl] sulfonamido] - undécanoïque,

acide [[(dichloro-2-4 benzoyl)-2 phényl] sulfonamido] - octanoïque,

acide [[(dichloro-2-4 benzoyl)-2 phényl] sulfonamido] -6 héxanoïque.

Les composés du tableau II ci-après ont été préparés conformément au procédé décrit dans l'exemple 6.

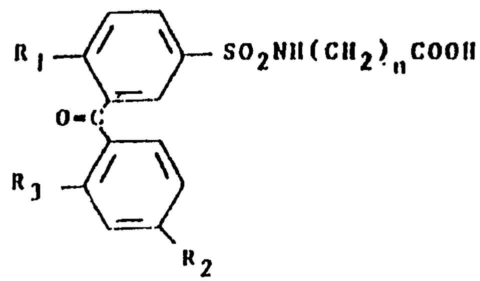

TABLEAU II

| Numéro des composés | $R_1$ | $R_2$ | $R_3$ | n | Formule Brute | Masse moléculaire | Point de fusion | rendement |
|---|---|---|---|---|---|---|---|---|
| 1684 | H | H | H | 10 | $C_{24}H_{31}NO_5S$ | 445,58 | 80° | 65% |
| 1685 | H | H | H | 11 | $C_{25}H_{33}NO_5S$ | 459,61 | 70°-75° | 65% |
| 1736 | Cl | H | H | 11 | $C_{25}H_{32}ClNO_5S$ | 494,05 | 75° | 41% |
| 1737 | Cl | H | H | 10 | $C_{24}H_{30}ClNO_5S$ | 480,03 | 79°-80° | 43% |
| 1761 | Cl | Cl | H | 10 | $C_{24}H_{29}Cl_2NO_5S$ | 514,47 | 80° | 60% |
| 1762 | Cl | Cl | H | 11 | $C_{25}H_{31}Cl_2NO_5S$ | 528,49 | 93° | 67% |
| 1763 | Cl | Cl | Cl | 10 | $C_{24}H_{28}Cl_3NO_5S$ | 548,91 | 94° | 38% |
| 1764 | Cl | Cl | Cl | 11 | $C_{25}H_{30}Cl_3NO_5S$ | 562,94 | 88° | 62% |

En mettant en oeuvre les produits de départ (sulfochlorures et amino-acides décrits ci-dessus dans la préparation de composés de l'invention), on obtient les produits suivants :

acide [(benzoyl-3 chloro-4 phényl) sulfonamido]-12 dodécanoïque,
acide [(benzoyl-3 chloro-4 phényl) sulfonamido]-11 undécanoïque,
acide [(benzoyl-3 chloro-4 phényl) sulfonamido]-8 octanoïque,
acide [(benzoyl-3 chloro-4 phényl) sulfonamido]-6 héxanoïque.
acide [[(dichloro-2,4 benzoyl)-3 phényl] sulfonamido]-12 dodécanoïque,
acide [[(dichloro-2,4 benzoyl)-3 phényl] sulfonamido]-11 undécanoïque,
acide [[(dichloro-2,4 benzoyl)-3 phényl] sulfonamido] -8 octanoïque,
acide [[(dichloro-2,4 benzoyl)-3 phényl] sulfonamido] -6 héxanoïque.
acide [[(chloro-4 benzoyl)-3 phényl] sulfonamido] -12 dodécanoïque,
acide [[(chloro-4 benzoyl)-3 phényl] sulfonamido]-11 undécanoïque,
acide [[(chloro-4 benzoyl)-3 phényl] sulfonamido] - 8 octonoïque,
acide [[(chloro-4 benzoyl)-3 phényl] sulfonamido] - 6 héxanoïque.
acide [[chloro-4 (dichloro-2,4 benzoyl)-3 phényl] sulfonamido]-12 dodécanoïque,
acide [[chloro-4 (dichloro-2,4 benzoyl)-3 phényl] sulfonamido]-11 undécanoïque,
acide [[chloro-4 (dichloro-2,4 benzoyl)-3 phényl] sulfonamido]-8 octanoïque,
acide [[chloro-4 (dichloro-2,4 benzoyl)-3 phényl] sulfonamido]-6 héxanoïque.

Les composés du tableau III ci-après ont été préparés conformément au procédé décrit dans l'exemple 6.

TABLEAU III

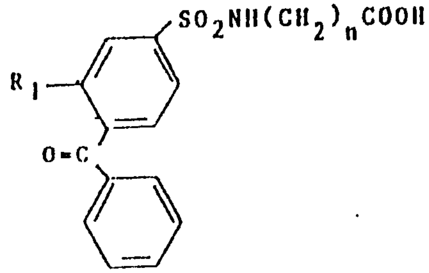

| Numéro des composés | $R_1$ | n | Formule Brute | Masse moléculaire | Point de fusion | Rendement |
|---|---|---|---|---|---|---|
| 1686 | H | 10 | $C_{24}H_{31}NO_5S$ | 445,58 | 128° | 65% |
| 1687 | H | 11 | $C_{25}H_{33}NO_5S$ | 459,61 | 172° | 75% |
| 1739 | Cl | 11 | $C_{25}H_{32}ClNO_5S$ | 494,10 | 107° | 50% |

En mettant en oeuvre les produits de départ (sulfochlorures et amino-acides décrits ci-dessus dans la préparation de composés de l'invention), on obtient les produits suivants :

acide [(benzoyl-4 chloro-3 phényl)sulfonamido] -12 dodécanoïque,

acide [(benzoyl-4 chloro-3 phényl)sulfonamido] -11 undécanoïque,

acide [(benzoyl-4 chloro-3 phényl)sulfonamido] -8 octanoïque,

acide [(benzoyl-4 chloro-3 phényl)sulfonamido] -6 héxanoïque.

acide [[(chloro-4 benzoyl)-4 phényl] sulfonamido]-12 dodécanoïque,

acide [[(chloro-4 benzoyl)-4 phényl]sulfonamido] -11 undécanoïque,

acide [[(chloro-4 benzoyl)-4 phényl] sulfonamido] -8 octanoïque,

acide [[(chloro-4 benzoyl)-4 phényl]sulfonamido] -6 héxanoïque.

acide [[(dichloro-2,4 benzoyl)-4 phényl] sulfonamido]-12 dodécanoïque,

acide [[(dichloro-2,4 benzoyl)-4 phényl] sulfonamido]-11 undécanoïque,

acide [[(dichloro-2,4 benzoyl)-4 phényl]sulfonamido] -8 octanoïque,

acide [[(dichloro-2,4 benzoyl)-4 phényl]sulfonamido] -6 héxanoïque.

acide [[chloro-4 (dichloro-2,4 benzoyl)-4 phényl] sulfonamido]-12 dodécanoïque,

acide [[chloro-4 (dichloro-2,4 benzoyl)-4 phényl] sulfonamido]-11 undécanoïque,

acide [[chloro-4 (dichloro-2,4 benzoyl)-4 phényl] sulfonamido]-8 octanoïque,

acide [[chloro-4 (dichloro-2,4 benzoyl)-4 phényl] Sulfonamido] -6 héxanoïque,

Les composés du tableau IV ci-après ont été préparés conformément au procédé décrit dans l'exemple 7

TABLEAU IV

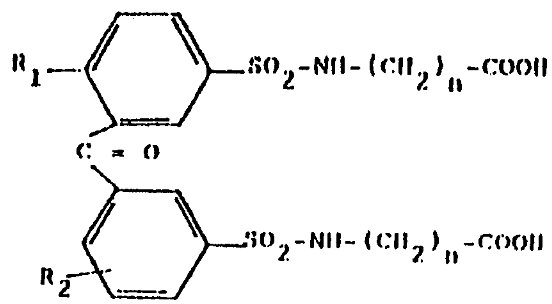

| Numéro de composés | $R_1$ | $R_2$ | n | Formule brute | Masse moléculaire | Point de fusion °C | Rendement % |
|---|---|---|---|---|---|---|---|
| 1688 | Cl | H | 10 | $C_{35}H_{51}ClN_2O_9S$ | 743,38 | 100-110 | 60 |
| 1689 | Cl | H | 11 | $C_{37}H_{55}ClN_2O_9S$ | 769,42 | 123 | 50 |

En mettant en oeuvre les produits de départ (sulfochlorures et amido-acides décrits ci-dessus dans la préparation de composés de l'invention), on obtient les produits suivants :

acide [[[[(carboxy-7 heptyl sulfamoyl] -3 benzoyl] -3 chloro-4 phényl] sulfonamido] -8 octanoïque,

acide [[[[(carboxy-5 pentyl sulfamoyl] -3 benzoyl] - 3 chloro-4 phényl] sulfonamido] -6 héxanoïque,

acide [[[[(carboxy-11 undécyl) sulfamoyl] -3 benzoyl] -2 chloro-3 phényl] sulfonamido] -12 dodécanoïque,

acide [[[[(carboxy-10 décyl) sulfamoyl] -3 benzoyl] 2-chloro-3 phényl] sulfonamido] -11 undécanoïque,

acide [[[[(carboxy-7 heptyl) sulfamoyl] -3 benzoyl] 2-chloro-3 phényl] sulfonamido] -8 octanoïque,

acide [[[[(carboxy-5 pentyl) sulfamoyl] -3 benzoyl] 2-chloro-3 phényl] sulfonamido] -6 héxanoïque,

acide [[[[(carboxy-11 undécyl) sulfamoyl] -3 benzoyl] -4 chloro-2 phényl] sulfonamido] -12 dodécanoïque,

acide [[[[(carboxy-10 décyl) sulfamoyl] -3 benzoyl] -4 chloro-2 phényl] sulfonamido] -11 undécanoïque,

acide [[[[(carboxy-7 heptyl) sulfamoyl] -3 benzoyl] -4 chloro-2 phényl] sulfonamido] -8 octanoïque,

acide [[[[(carboxy-5 pentyl) sulfamoyl] -3 benzoyl] -4 chloro-2 phényl] sulfonamido] -6 héxanoïque.

Les composés du tableau V ci-après ont été préparés conformément au procédé décrit dans l'exemple 8.

**TABLEAU V**

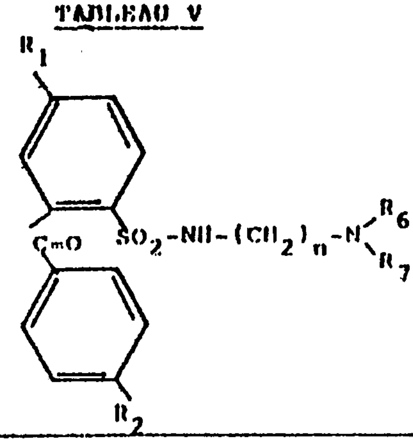

| Numéro de composés | $R_1$ | $R_2$ | 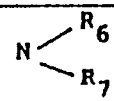 | n | Formule brute | Masse moléculaire | Point de fusion °C | Rendement % |
|---|---|---|---|---|---|---|---|---|
| 1340 | H | H | 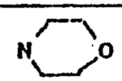 | 3 | $C_{20}H_{24}N_2O_4S$ | 388,49 | 121 | 65 |
| 1341 | H | H | 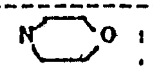 | 2 | $C_{19}H_{22}N_2O_4S$ HCl | 410,93 | 166 | 45 |
| 1342 | H | H | 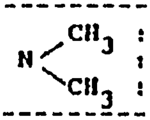 | 3 | $C_{18}H_{22}N_2O_3S$ HCl | 382,92 | 153 | 45 |

TABLEAU V (SUITE)

| Numéro de composés | $R_1$ | $R_2$ | N $R_7$ | n | Formule brute | Masse moléculaire | Point de fusion °C | Rendement % |
|---|---|---|---|---|---|---|---|---|
| 1343 | Cl | H | 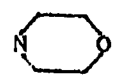 | 2 | $C_{19}H_{21}ClN_2O_4S$ | 408,91 | 157 | 70 |
| 1344 | Cl | H | 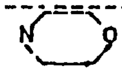 | 3 | $C_{20}H_{23}ClN_2O_4S$ | 422,94 | 116 | 80 |
| 1345 | Cl | H | 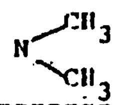 | 3 | $C_{18}H_{21}ClN_2O_3S$ $CH_3SO_3H$ | 477,01 | 164 | 25 |
| 1354 | $NO_2$ | H | 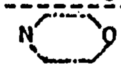 | 2 | $C_{19}H_{21}N_3O_6S$ | 419,46 | 138 | 50 |
| 1360 | Cl | H | 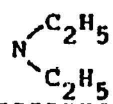 | 2 | $C_{19}H_{23}ClN_2O_3S$ HCl | 431,89 | 125 | 45 |

TABLEAU V (SUITE)

| Numéro de composés | $R_1$ | $R_2$ | N R₇ | n | Formule brute | Masse moléculaire | Point de fusion °C | Rendement % |
|---|---|---|---|---|---|---|---|---|
| 1361 | $NO_2$ | H | 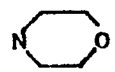 | 3 | $C_{20}H_{23}N_3O_6S$ | 433,49 | 129 | 66 |
| 1362 | $NO_2$ | H | 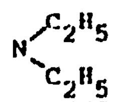 | 2 | $C_{18}H_{21}N_3O_5S$ | 391,45 | 200 | 30 |
| 1409 | H | Cl | 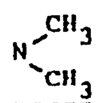 | 3 | $C_{18}H_{21}ClN_2O_3S$ | 380,90 | 98 | 42 |
| 1410 | H | Cl | 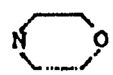 | 3 | $C_{20}H_{23}ClN_2O_4S$ | 422,94 | 130 | 55 |

Les composés des tableaux VI et VIbis ci-après ont été préparés conformément à l'exemple 9.

**TABLEAU VI**

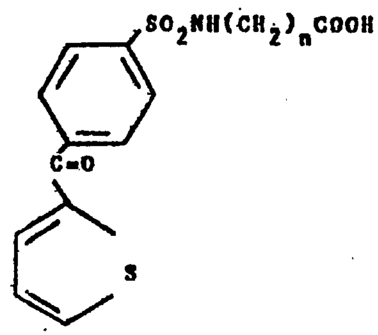

| Numéro de composés | n | Formule brute | Masse moléculaire | Point de fusion en °C | Rendement % |
|---|---|---|---|---|---|
| 1738 | 3 | $C_{15}H_{15}NO_5S_2$ | 353,42 | 128-130 | 60 |

TABLEAU VI BIS

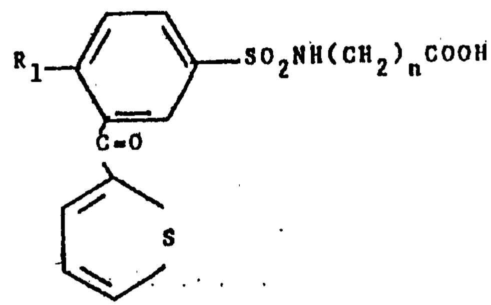

| Numéro de composés | $R_1$ | n | Formule brute | Masse moléculaire | Point de fusion en °C | Rendement % |
|---|---|---|---|---|---|---|
| 1813 | Cl | 11 | $C_{23}H_{30}ClNO_5S_2$ | 500,08 | 74-75 | 73 |
| 1815 | Cl | 10 | $C_{22}H_{28}ClNO_5S_2$ | 486,05 | 62-63 | 66 |

Les composés du tableau VII ci-après ont été préparés conformément à l'exemple 15.

TABLEAU VII

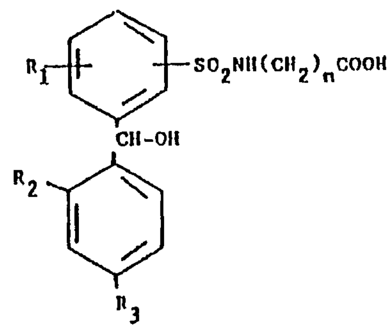

| Numéro de composés | $R_1$ | $R_2$ | $R_3$ | n | Position chaîne | Formule brute | Masse moléculaire | Point de fusion °C | Rendement % |
|---|---|---|---|---|---|---|---|---|---|
| 1796 | 5-Cl | Cl | H | 10 | en 2 | $C_{24}H_{31}Cl_2NO_5S$ | 516,48 | 125-130 | 66 |
| 1807 | 2-Cl | Cl | Cl | 11 | en 5 | $C_{25}H_{32}Cl_3NO_5S$ | 564,96 | 113.4 | 85 |
| 1810 | 2-Cl | H | H | 11 | en 5 | $C_{25}H_{34}ClNO_5S$ | 496,02 | 122 | 83 |

Les composés du tableau VIIbis ci-après ont été préparés conformément à l'exemple 9 et à l'exemple 15.

TABLEAU VII BIS

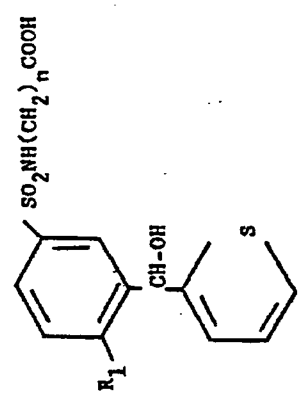

| Numéro de composés | $R_1$ | n | Formule brute | Masse moléculaire | Point de fusion °C | Rendement % |
|---|---|---|---|---|---|---|
| 1816 | Cl | 10 | $C_{22}H_{30}ClNO_5S_2$ | 488,07 | 109 | 50 |
| 1817 | Cl | 11 | $C_{23}H_{32}ClNO_5S_2$ | 502,10 | 117-118 | 59 |

Les composés du tableau VIII ci-après ont été préparés conformément à l'exemple 16.

TABLEAU VIII

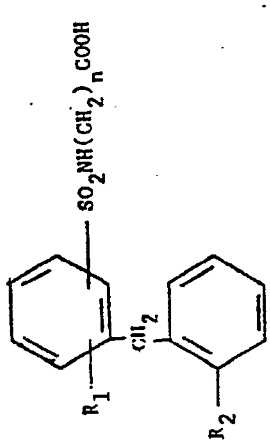

| Numéro de composés | $R_1$ | $R_2$ | n | Position chaîne | Formule brute | Masse moléculaire | Point de fusion °C | Rendement % |
|---|---|---|---|---|---|---|---|---|
| 1797 | 5-Cl | Cl | 10 | en 2 | $C_{24}H_{31}Cl_2NO_4S$ | 500,49 | 77-80 | 49 |
| 1808 | 2-Cl | H | 10 | en 5 | $C_{24}H_{32}ClNO_4S$ | 467,04 | 88-90 | 30 |

Les nouveaux composés de l'invention ainsi que leurs sels organiques ou minéraux physiologiquement acceptables présentent de remarquables propriétés pharmacologiques.
Parmi les sels physiologiquement acceptables des composés selon l'invention, on peut mentionner les suivants en particulier :

concernant les sels formés à partir d'un acide : les sels des métaux alcalins ou alcalinoterreux ou les sels d'une base organique, tel que le méglumate et l'acéglumate ;

en ce qui concerne les sels formés à partir des amines :
chlorhydrates, sulfates, phosphates, méthanesulfonates, tartrates, acetates, fumarates, succinates, pyruvates, phenoxyacetates, lactates, citrates, maléates.
Les sels des médicaments selon l'invention sont les suivants :
1. soit ceux formés à partir d'une salification du groupe carboxylique -COOH dans les composés de formule suivante :

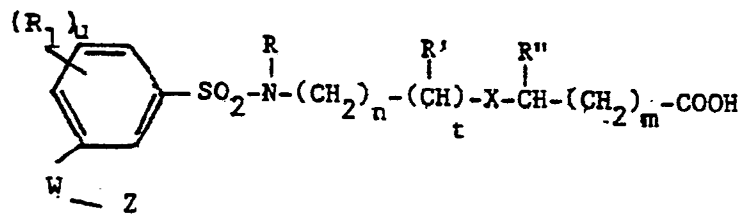

2. soit les sels formés à partir d'une salification par une base forte (NaOH, KOH) des groupes -NH et -COOH dans les composés de formule suivante :

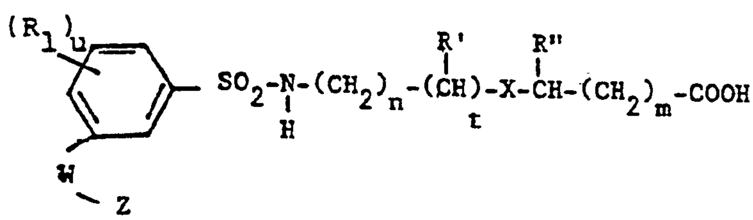

3. ou bien les sels des composés obtenus par salification du groupe d'amino substituants du noyau aromatique dans les composés de formule suivante :

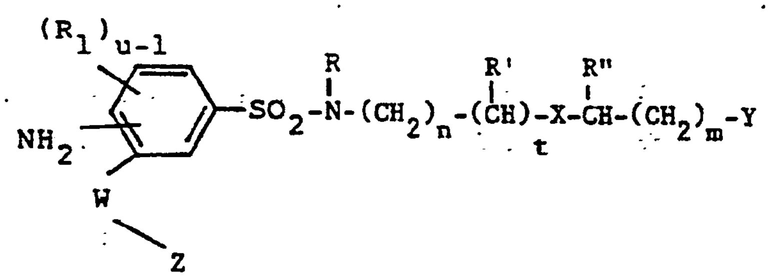

4. ou bien les sels des composés obtenus par salification de Y, lorsque Y représente $NH_2$, $NHR_6$ ou $NR_6R_7$.
Dans le premier cas, le composé non salifié est transformé en sel en faisant réagir, de façon connue en soi, la base et le produit de l'invention, en une quantité équimoléculaire.
Dans le deuxième cas, le composé non salifié est transformé en sel en faisant réagir, de façon connue en soi, deux moles de la base et une mole du produit de l'invention.
Dans le troisième cas et le quatrième cas, le composé non salifié est transformé en sel en faisant réagir de façon connue en soi, l'acide et le produit de l'invention, en une quantité équimoléculaire.
Les composés selon l'invention exercent une action anti-inflammatoire et analgésique. Certains d'entre eux bloquent fortement et sélectivement la biosynthèse des leucotriènes $B_4$ (au niveau les leucocytes polymorphonucléaires) impliqués dans les processus inflammatoires, et des leucotriènes $C_4$ impliqués dans des réactions allergiques telles que l'asthme et certains types de maladies allergiques.
Les composés selon l'invention se distinguent aussi par le fait qu'ils sont dépourvus de toxicité aux doses actives.

Leur indice thérapeutique est compatible avec leur utilisation comme médicament.

A titre d'exemple, différents essais pour la mise en évidence des propriétés pharmacologiques et de la bonne tolérance des composés selon l'invention sont rapportés ci-après.

I - Activité anti-inflammatoire.

1) Test de l'arthrite au kaolin.

1.1 première expérience :

L'injection de O,O5 ml d'une suspension stérile de kaolin à 1O % dans la gaine des fléchisseurs métatarsiens de la patte postérieure du rat provoque dans un premier temps une réaction oedémateuse. Une arthrite inflammatoire se consistue ensuite.

La réaction oedémateuse précoce apparaît dès la première heure et atteint son maximum entre la quatrième heure et la sixième heure. C'est à cette manifestation que l'on s'intéresse. L'appréciation du volume de la patte permet d'étudier l'action des substances sur la réaction oedémateuse. Les mesures sont faites à l'aide du pléthysmomètre de Giono et Chevilard.

Les produits à tester sont administrés par voie orale (sonde gastrique), une heure avant l'injection du kaolin.

Les résultats sont exprimés en % de la diminution de l'inflammation chez les animaux traités par rapport à une série d'animaux contrôle.

Les lots sont de 1O animaux par dose et 1O animaux témoins.

Les résultats sont également comparés à des séries d'animaux traités par un produit de référence : le kétoprofène.

Les résultats relatifs aux composés de l'invention sont rassemblés dans le tableau IX ci-après.

Les chiffres fournis représentent la moyenne de 1O animaux.

TABLEAU IX

| | 25 mg/kg | 50 mg/kg | 100 mg/kg |
|---|---|---|---|
| Ketoprofène | -30 % | -51 % | -73 % |
| 1573 | -15 % | -30 % | -52 % |
| 1570 | -10 % | -25 % | -52 % |
| 1572 | -17 % | -26 % | -50 % |
| 1565 | -15 % | -20 % | -45 % |
| 1568 | -15 % | -18 % | -40 % |
| 1575 | -18 % | -21 % | -37 % |
| 1569 | -28 % | -30 % | -45 % |
| 1567 | -12 % | -15 % | -20 % |
| 1410 | -18 % | -32 % | -57 % |
| 1361 | -25 % | -30 % | -48 % |
| 1340 | -10 % | -25 % | -45 % |
| 1344 | -10 % | -15 % | -18 % |
| 1343 | - | - | - |
| 1571 | -17 % | -26 % | -50 % |
| 1682 | -35 % | -35 % | -40 % |
| 1683 | -25 % | -30 % | -40 % |
| 1684 | -28 % | -42 % | -45 % |
| 1686 | -50 % | -50 % | -50 % |
| 1687 | -23 % | -28 % | -50 % |
| 1688 | -25 % | -28 % | -48 % |

Les résultats montrent qu'à une dose de 100 mg/kg, la majorité des composés testés présentent une activité anti-inflammatoire très nette tout en présentant par rapport au kétoprophène l'avantage de ne pas entraîner d'effets secondaires indésirables par suite de l'absence d'action sur la biosynthèse des prostaglandines.

1.2 Deuxième expérience :

Dans une deuxième expérience, une étude plus poussée a été effectuée afin de connaître l'action des produits, d'une part sur la phase aiguë de la reaction inflammatoire (mesurée par le volume de la patte à la 8ème heure), et d'autre part sur la phase chronique (activité mesurée par le poids de la patte à la 12ème heure).

Comme dans l'expérience précédente, le Ketoprofène a été utilisé comme produit de comparaison :

- Résultats de la phase aiguë

Les résultats sont exprimé en % de diminution significative du volume de la patte traitée par rapport aux témoins. Ils sont rapportés dans le tableau IXbis ci-après :

## TABLEAU IXBIS

| PRODUIT | DOSE TESTEE 25 mg/kg | 50 mg/kg | 100 mg/kg | DOSE ACTIVE 50 |
|---|---|---|---|---|
| 1574 Ketoprofène | -60 ±6<br>-60 ±5 | -65 ±6<br>-68 ±5 | -68 ±5<br>-68 ±5 | 25mg/kg |
| 1736 Ketoprofène | -45 ±5<br>-47 ±6 | -50 ±5<br>-48 ±5 | -55 ±5<br>-55 ±5 | 50mg/kg |
| 1755 Ketoprofène | -48 ±5<br>-45 ±6 | -50 ±5<br>-48 ±2 | -55 ±5<br>-50 ±5 | 25mg/kg |
| 1761 Ketoprofène | -38 ±5<br>-40 ±5 | -45 ±5<br>-48 ±5 | -50 ±5<br>-60 ±5 | 100mg/kg |
| 1763 Ketoprofène | -35 ±5<br>-37 ±5 | -45 ±5<br>-48 ±6 | -50 ±5<br>-52 ±3 | 100mg/kg |
| 1764 Ketoprofène | -45 ±5<br>-47 ±6 | -55 ±5<br>-53 ±3 | -60 ±5<br>-58 ±4 | 50mg/kg |
| 1796 Ketoprofène | -50 ±5<br>-48 ±5 | -55 ±5<br>-55 ±5 | -60 ±5<br>-58 ±5 | 25mg/kg |
| 1797 Ketoprofène | -50 ±5<br>-30 ±5 | -55 ±5<br>-55 ±5 | -60 ±5<br>-58 ±5 | 25mg/kg |

- résultats de la phase chronique :

Les résultats sont exprimés en % de diminution significative du poids de la patte traitée par rapport aux témoins et rapportés dans le tableau IXter ci-après :

TABLEAU IXTER

| PRODUIT | DOSE TESTEE 25 mg/kg | 50 mg/kg | 100 mg/kg | DOSE ACTIVE 50 |
|---|---|---|---|---|
| 1574<br>Ketoprofène | -58 ±5<br>-58 ±5 | -60 ±5<br>-65 ±5 | -60 ±5<br>-65 ±5 | 25mg/kg |
| 1736<br>Ketoprofène | -45 ±5<br>-48 ±5 | -55 ±5<br>-55 ±5 | -58 ±5<br>-60 ±5 | 50mg/kg |
| 1755<br>Ketoprofène | -45<br>-44 ±5 | -55<br>-52 ±6 | -58<br>-55 ±5 | 25mg/kg |
| 1761<br>Ketoprofène | -40 ±5<br>-45 ±5 | -45 ±5<br>-50 ±6 | -50 ±5<br>-55 ±7 | 100mg/kg |
| 1763<br>Ketoprofène | -38 ±5<br>-45 ±5 | -40 ±5<br>-48 ±5 | -48 ±5<br>-55 ±5 | 100 mg/kg |
| 1764<br>Ketoprofène | -45 ±5<br>-47 ±5 | -52 ±5<br>-51 ±5 | -58 ±5<br>-57 ±5 | 50mg/kg |
| 1796<br>Ketoprofène | -50 ±5<br>-50 ±5 | -52 ±5<br>-52 ±5 | -58 ±5<br>-55 ±5 | 25mg/kg |
| 1797<br>Ketoprofène | -50 ±5<br>-50 ±5 | -58 ±5<br>-55 ±5 | -60 ±5<br>-56 ±5 | 25mg/kg |

2) Test de l'oedème à la carragénine.

Dans ce test, on utilise 10 rats par composé étudié de l'invention. Des lots témoins de 10 rats également reçoivent soit le composé de comparaison (kétoprofène), soit uniquement une solution de NaCl.

Une injection de carragénine dans la base de la patte postérieure d'un rat provoque un oedème dont le volume est mesuré au pléthysmomètre de Lence (technique voisine de celle de C.A. Winter, E.A. Risley et G.W. Nuss, Proc. Soc. Exp. Biol. Med. 1962, 111, 544 et P. Lence, Arch. Iny. Pharmacodyn. 1962, 136, 237).

Les composés de l'invention sont administrés par voie orale (sonde gastrique) une heure avant l'injection de carragénine.

La carragénine est mise en suspension à 1 %, dans une solution isotonique de chlorure de sodium à 9 ‰. Le volume injecté dans la patte du rat est de 0,20 ml.

Le volume de la patte est mesuré avant l'injection et 3 heures après. L'effet antagoniste des produits vis-à-vis de la carragénine se traduit par l'inhibition du volume de l'oedème par rapport à des rats témoins. Ces derniers ne reçoivent que du soluté isotonique de NaCl dans des conditions identiques.

Les résultats sont exprimés en % d'inhibition de l'inflammation par rapport aux rats témoins (moyenne de 10 animaux).

2.1 première expérience :

Les résultats sont rassemblés dans le tableau X ci-après :

TABLEAU X

| | 25 mg/kg | 50 mg/kg | 100 mg/kg |
|---|---|---|---|
| Kétoprofène | -25 % | -45 % | -65 % |
| 1573 | -17 % | -28 % | -50 % |
| 1570 | -20 % | -29 % | -40 % |
| 1572 | -18 % | -25 % | -49 % |
| 1565 | -18 % | -22 % | -49 % |
| 1568 | -15 % | -20 % | -35 % |
| 1575 | -15 % | -18 % | -35 % |
| 1569 | -30 % | -35 % | -50 % |
| 1567 | - 8 % | -10 % | -10 % |
| 1410 | -15 % | -25 % | -48 % |
| 1361 | -15 % | -25 % | -45 % |
| 1340 | - 5 % | - 5 % | -10 % |
| 1344 | - | - | - |
| 1343 | - 7 % | - 8 % | - 8 % |
| 1571 | -23 % | -27 % | -48 % |
| 1682 | -33 % | -42 % | -45 % |
| 1683 | -25 % | -30 % | -35 % |
| 1684 | -30 % | -45 % | -55 % |
| 1686 | -50 % | -50 % | -55 % |
| 1687 | -25 % | -30 % | -48 % |
| 1688 | -25 % | -32 % | -50 % |

2.2 Deuxième expérience :
Les résultats sont rassemblés dans le tableau Xbis ci-après :

TABLEAU XBIS

| PRODUIT | DOSE | | |
|---|---|---|---|
| | 25 mg/kg | 50 mg/kg | 100 mg/kg |
| 1574 | -40% ±5 | -45% ±5 | -45% ±5 |
| Ketoprofène | -45% ±5 | -50% ±5 | -50% ±5 |
| 1736 | -45% ±5 | -48% ±5 | -55% ±5 |
| Ketoprofène | -48% ±6 | -50% ±3 | -60% ±5 |
| 1755 | -45% ±5 | -52% ±5 | -55% ±5 |
| Ketoprofène | -44% ±5 | -55% ±6 | - 58% ±6 |
| 1761 | -30% ±5 | -35% ±5 | -45% ±5 |
| Ketoprofène | -35% ±2 | -40% ±5 | -55% ±5 |
| 1763 | -30% ±5 | -35% ±5 | -45% ±5 |
| Ketoprofène | -35% ±5 | -45% ±6 | -55% ±3 |
| 1764 | -45% ±5 | -48% ±5 | -52% ±5 |
| Ketoprofène | -47% ±5 | -49% ±7 | -50% ±5 |
| 1796 | -45% | -48% | -55% |
| Ketoprofène | -50% ±5 | -50% ±5 | -55% ±5 |
| 1797 | -45% ±5 | -48% ±5 | -55% ±5 |
| Ketoprofène | -45% ±5 | -50% ±5 | -55% ±5 |

3) Test du granulome expérimental par corps étranger.

3.1 Première expérience :

Etude des composés suivants :

1573, 157O, 1572, 1341, 1361, 141O.

Ce test consiste à étudier la phase cellulaire subaiguë qui se développe dans les 8 jours qui suivent l'implantation d'un corps étranger, éponge de matière plastique non résorbable, dans le tissu conjonctif sous-cutané dorsal chez le rat.

Les granulomes formés sont prélevés 8 jours après l'implantation ; une étude histologique est réalisée après fixation du tissu et coloration des coupes par l'hématéine-éosine. L'observation porte plus particulièrement sur la population cellulaire de la phase primaire de la formation du tissu de granulation ; les cellules réticulo-histiocytaires, les lymphocytes et plus spécialement les macrophages.

Les animaux utilisés sont des rats Wistar de 12O-14O g de même origine, maintenus dans les mêmes conditions de stabulation pendant toute la durée du traitement (8 jours).

Les composés de l'invention sont administrés par voie orale, dans une suspension de gomme, le matin à jeun, à l'aide d'une sonde oesophagienne.

Après 8 jours de traitement, les rats sont sacrifiés par décapitation, les granulomes prélevés et préparés en vue de l'examen histologique. ,

Les conditions expérimentales sont les suivantes :

– Les animaux sont répartis par lots de 1O.
– Chaque produit à tester est administré à 2 doses respectivement de 5O et 1OOmg à 2 lots de 1O animaux.
– Le kétoprofène est utilisé à titre de produit de référence aux 2 mêmes doses et dans les mêmes conditions.
– Un lot de 1O animaux témoins ne reçoit aucun traitement.

Les résultats figurent dans le tableau XI ci-après.

TABLEAU XI

| N° de composé | |
|---|---|
| 1573 | Aux deux doses, l'activité est très nette, le tissu conjonctif est peu dense et ne présente pas les caractéristiques d'un tissu inflammatoire. |
| 1570 | L'activité anti-inflammatoire se manifeste dès la dose de 50 mg/kg et est nette pour la dose de 100 mg/kg. |
| 1572 | Idem. |
| 1361 | L'activité anti-inflammatoire se manifeste aux deux doses, sans inhiber toutefois la formation d'un tissu conjonctif de type cicatriciel. |
| 1410 | La réaction inflammatoire est atténuée aux deux doses. L'action se manifeste en particulier par l'absence de réaction congestive du tissu de granulation. |
| 1341 | Réaction inflammatoire nettement atténuée aux deux doses. Les résultats sont supérieurs à ceux du composé 1410. |
| KETOPROFENE | Aux deux doses, on constate une diminution très importante de la réaction inflammatoire. |
| TEMOIN | Aspect très caractéristique d'un granulome en voie de formation de type inflammatoire. |

En conclusion, tous les produits testés ont manifesté une activité anti-inflammatoire se traduisant par une modification histologique des tissus comparativement aux animaux témoins non traités.

3.2 Deuxième expérience :

Etudes des composés suivants :

1574 - 1736 - 1755 - 1761 - 1763 - 1764 - 1796 - 1797

Dans cette expérience, on effectue d'une part une étude histologique des granulomes par prélèvement au 8ème jour (résultats rapportés dans le tableau XI bis, d'autre part une étude du poids des granulomes 1O jours après

l'implantation (résultats rapportés dans le tableau XI ter).

TABLEAU XI BIS

| Numéro du composé | |
|---|---|
| 1574 | Aux trois doses très nette raréfaction du tissu conjonctif inflammatoire. Tissu conjonctif lâche, peu de fibroblastes. Entre les fibres, présence de cellules mobiles du tissu conjonctif. Cellules géantes à corps étranger très rares. |
| 1736 | Ralentissement très net de la formation du tissu de granulation. Coque périphérique peu épaisse. Raréfaction des fibroblastes. Fibres de collagène minces et éparpillées. Présence de lymphocytes de polymorphonucléaires ; macrophages peu nombreux. |

TABLEAU XI bis SUITE

| | |
|---|---|
| 1755 | Granulome peu développé. Fibres de collagène mince, peu de fibroblastes. Vascularisation normale. Présence de cellules mobiles du tissu conjonctif. Tissu conjonctif peu dense. |
| 1761 | Production atténuée du tissu de granulation inflammatoire par rapport au témoin. Coque périphérique du granulome formée de fibroblastes et de fibres de collagène peu épaisse. Présence de lymphocytes, de macrophages, d'histiocytes. Sous la coque périphérique, tissu de granulation peu dense, formé de fibroblastes éparpillés entre lesquels on observe la présence de cellules mobiles du tissu conjonctif. Tissu bien vascularisé. |
| 1763 | Coque périphérique peu dense, formée de fibroblastes et de fibres de collagène peu denses et éparpillées entre les fibroblastes et les fibres. Présence de cellules mobiles du tissu conjonctif : polymorphonucléaires, lymphocytes et histiocytes, rares cellules géantes à corps étranger accolés aux fragments d'éponge. |
| 1764 | Coque périphérique peu dense et peu cellulaire. Présence de cellules mobiles du tissu conjonctif entre les fibroblastes et les fibres du collagène. Présence de lymphocytes, macrophages, histiocytes et rares cellules géantes à corps étranger. Tissu de granulation peu inflammatoire. |

TABLEAU XI bis SUITE

| | |
|---|---|
| 1796 | Présence de nombreuses cellules mobiles du tissu conjonctif entre les fibroblastes et les fibres du collagène (peu épaisse). Présence de lymphocytes, macrophages, histiocytes. Coque périphérique peu dense et peu cellulaire. Rares cellules géantes à corps étranger. Tissu ce granulation peu inflammatoire. |
| 1797 | Granulome peu développé autour du corps étranger. Coque périphérique mince. Fibres de collagène minces. Vascularisation normale. Présence de cellules mobiles du tissu conjonctif. Sous la coque périphérique, tissu conjonctif peu inflammatoire. |

TABLEAU XI ter

| PRODUIT | DOSE | | |
|---|---|---|---|
| | 25 mg/kg | 50 mg/kg | 100 mg/kg |
| 1574<br>Ketoprofène | -48%<br>-50% ±5 | -50%<br>-55% ±5 | -55%<br>-55% ±5 |
| 1736<br>Ketoprofène | -48% ±5<br>-46% ±5 | -52% ±5<br>-50% ±5 | -55% ±5<br>-55% ±5 |
| 1755<br>Ketoprofène | -45% ±5<br>-44% ±5 | -50% ±5<br>-50% ±5 | -55% ±5<br>-60% ±5 |
| 1761<br>Ketoprofène | -25% ±5<br>-30% ±5 | -30% ±5<br>-40% ±6 | -45% ±5<br>-55% ±7 |
| 1763<br>Ketoprofène | -15% ±5<br>-25% ±5 | -20% ±5<br>-25% ±5 | -45% ±5<br>-48% ±6 |

TABLEAU XI ter SUITE

| | | | |
|---|---|---|---|
| 1764<br>Ketoprofène | -45% ±5<br>-48% ±5 | -50% ±5<br>-50% ±5 | -55% ±5<br>-57% ±5 |
| 1796<br>Ketoprofène | -50%<br>-50% ±5 | -55%<br>-50% ±5 | -60%<br>-58% ±6 |
| 1797<br>Ketoprofène | -50% ±5<br>-50% ±5 | -55% ±5<br>-50% ±5 | -60% ±5<br>-55% ±5 |

II - Activité analgésique

Test de Randall et Selitto. (Arch. Int. Pharmacodyn. 1957, CXI, 409-419).

L'inflammation accroît la sensibilité à la douleur, notamment à la pression, et les analgésiques élèvent le seuil de cette sensibilité.

L'inflammation est obtenue par injection, sous l'aponévrose plantaire d'une des pattes postérieures, de 0.1 ml d'une suspension aqueuse de levure de bière à 20 %. La douleur est provoquée par une force appliquée sur la face plantaire et augmentée graduellement de 16 g/seconde. Le seuil douloureux est apprécié par la force nécessaire et suffisante pour déclencher une réaction caractéristique de retrait de la patte.

La sensibilité à la douleur de la patte enflammée atteint un plateau 4 heures après l'injection de levure de bière et reste alors stable jusqu'à la sixième heure. Le seuil douloureux de la patte enflammee est, chez des rats témoins de 140 g, atteint pour des pressions de 50 à 60 g, alors qu'il se situe autour de 160 g pour la patte intacte.

On exprime l'action de la substance administrée par voie orale à des séries de 10 rats par le pourcentage d'augmentation du seuil moyen de la douleur par rapport à celui mesuré chez les rats d'une serie témoin de 10 animaux, n'ayant pas reçu de composé de l'invention.

Les produits à tester sont administrés par voie orale (sonde gastrique), une heure avant l'injection de levure de bière.

Le composé de comparaison utilisé est le kétoprofène.

1. PREMIERE EXPERIENCE

Les résultats sont rassemblés dans le tableau XII ci-après :

TABLEAU XII

| | 25 mg/kg | 50 mg/kg | 100 mg/kg |
|---|---|---|---|
| Kétoprofène | + 22% | + 55% | + 78% |
| 1573 | + 20% | + 35% | + 60% |
| 1570 | + 15% | + 25% | + 53% |
| 1572 | + 18% | + 30% | + 55% |
| 1565 | + 18% | + 28 % | + 42% |
| 1568 | + 18% | + 25% | + 48% |
| 1575 | + 15% | + 19% | + 35% |
| 1569 | + 10% | + 15% | + 25% |
| 1567 | + 12% | + 15% | + 20% |
| 1410 | + 19% | + 37% | + 58 % |
| 1361 | + 20% | + 35% | + 45% |
| 1340 | + 10% | + 20% | + 49% |
| 1344 | + 18% | + 18% | + 25% |
| 1343 | + 10% | + 12% | + 12% |
| 1571 | + 25% | + 32% | + 36% |
| 1682 | + 12% | + 15% | + 20% |
| 1683 | + 15% | + 20% | + 30% |
| 1684 | + 35% | + 45% | + 50% |
| 1686 | + 35% | + 50% | + 50% |
| 1687 | + 25% | + 35% | + 50% |
| 1688 | + 25% | + 32% | + 50% |

2. DEUXIEME EXPERIENCE

Les résultats sont rassemblés dans le tableau XIIbis ci-après :

TABLEAU X-IIBIS

| PRODUIT | DOSE | | | dose active 50 |
|---|---|---|---|---|
| | 25 mg/kg | 50 mg/kg | 100 mg/kg | |
| 1574<br>Ketoprofène | +45% ±6<br>+50% ±5 | +50% ±5<br>+55% ±5 | +50% ±5<br>+58% ±5 | 50mg/kg |
| 1736<br>Ketoprofène | +52%<br>+51% ±6 | +58%<br>+55% ±5 | +60%<br>+60% ±5 | 25mg/kg |
| 1755<br>Ketoprofène | +40% ±5<br>+38% ±6 | +50% ±5<br>+52% ±5 | +55% ±5<br>+55% ±6 | |
| 1761<br>Ketoprofène | +40% ±5<br>+45% ±2 | +45% ±5<br>+48% ±5 | +50% ±5<br>+55% ±5 | |
| 1763<br>Ketoprofène | +40% ±5<br>+42% ±6 | +42% ±5<br>+48% ±7 | +45% ±5<br>+55% ±8 | |
| 1764<br>Ketoprofène | +50% ±5<br>+50% ±5 | +55% ±5<br>+57% ±5 | +58% ±5<br>+60% ±5 | 25 mg/kg |
| 1796<br>Ketoprofène | +50% ±5<br>+49% ±5 | +55% ±5<br>+50% ±5 | +58% ±5<br>+55% ±5 | 25 mg/kg |
| 1797<br>Ketoprofène | +50% ±5<br>+55% ±5 | +55% ±5<br>+55% ±5 | +58% ±5<br>+58% ±5 | |

III - Mécanisme d'action biochimique.

Les phospholipides membranaires sont métabolisés par action de la phospholipase $A_2$ en acide arachidonique, celui-ci conduisant par la voie de la cyclooxygénase à la biosynthèse des prostaglandines et des thromboxanes et par la voie de la lipoxygénase à la biosynthèse des leucotriènes.

Les produits de l'invention ont été testés d'une part sur la voie de la cyclooxygénase plaquettaire humaine en utilisant des agents agrégants (Thrombine ou concentration subagrégante d'acide arachidonique). Aucune modification (pas d'effet anticyclooxygénase) n'est décelée avec les produits de l'invention.

Des études complémentaires ont été réalisées pour étudier une éventuelle activité des produits sur la thromboxanes synthétase et sur la prostacycline synthétase, étape postérieure de la biosynthèse des prostaglandines.

D'autre part, les produits de l'invention ont été testés sur la voie de la 5 lipoxygénase en utilisant des leucocytes polymorphonucléaires humaines ce qui a montré que certains d'entre eux étaient des inhibiteurs de la 5-lipoxygénase.

1) Etude de l'action spécifique vis-à-vis de la thromboxane synthétase :

1.1 Système de suspensions plaquettaires humaines

Les plaquettes sont mises à incuber avec de l'acide arachidonique (5 µg/ml) en présence de doses croissantes de produits à tester ($10^{-6}$M à $5.10^{-5}$M). Les plaquettes stimulées par l'acide arachidonique (AA) fabriquent une quantité importante de thromboxane $A_2$ ($TXA_2$) mesurable en radio-immunologie sous forme de métabolite stable $TXB_2$.

Le dosage de la quantité de $TXB_2$ dans le milieu est effectué après une incubation de 10 à 15 minutes à 37°C, les mesures étant faites par rapport à une suspension témoin n'ayant pas été incubée avec les produits à tester.

Résultats :

ils sont exprimés en IC 50, c'est-à-dire en concentration du produit testé permettant d'inhiber 50 % de la production de $TXB_2$.

| Produit testé | IC 50 µM |
|---|---|
| 1572 | 12 |
| 1574 | 8 |
| 1736 | 6.2 |
| 1796 | 7.8 |
| 1797 | 5 |
| 1813 | 11 |

Les IC 50 obtenues montrent que les produits testés sont fortement inhibiteurs de la production de $TBX_2$.

De plus, aux doses étudiées ($10^{-6}$M à $5.10^{-5}$M), on a pu constater une relation dose-effet pour les produits testés vis-àvis de l'inhibition de la production de $TXB_2$.

1.2. Modèle de préparation rénale

Les glomérules humains synthétisent à la fois des thromboxanes par l'intermédiaire de la thromboxane synthétase et des prostaglandines par l'intermédiaire de la prostacycline synthétase. L'essai consiste à stimuler des cellules de glomérules par de l'acide arachidonique (5 µg/ml) et à doser par radio-immunologie la quantité de thromboxane $B_2$ ($TXB_2$) et de la prostacycline ($PGI_2$) produites en présence de concentrations croissantes d produits testés ($10^{-6}$M à $5.10^{-5}$M).

Comme pour le modèle précédent, les résultats sont exprimés en IC 50 par rapport à une suspension témoin.

Résultats

| Produits testés | IC 50 | |
|---|---|---|
| | $TXB_2$ | $PGI_2$ |
| 1572 | 7 | 100 |
| 1574 | 5 | 100 |
| 1736 | 2.6 | >50 |
| 1796 | 3.2 | 50 |
| 1797 | ·4.4 | >50 |
| 1813 | 2 | >50 |

Les IC 50 obtenues (<10 µM) montrent que les produits testés sont fortement inhibiteurs de la production de $TXB_2$, alors qu'ils ne sont que faiblement inhibiteurs de la production de $PGI_2$ (IC 50 $\geqq$ 50 µM), et qu'ils est donc possible d'obtenir une activité spécifique vis-à-vis de $TXB_2$.

De plus, aux doses utilisées ($10^{-6}$M à $5.10^{-5}$M), on observe une relation dose-effet vis-à-vis de l'inhibition de $TXB_2$.

2) Activité vis-à-vis de la biosynthèse des leucotriènes

2.1. Première expérience

Les leucocytes polymorphonucléaires sont isolés à partir du sang humain et sont utilisés dans les 6 heures suivant le prélèvement. La viabilité des cellules est vérifiée par exclusion du bleu trypan.

5 x $10^6$ cellules sont incubées 10 minutes à 37°C en présence du produit à tester mis en solution dans du DMSO, et stimulées par du Ionophore A 23187 (Galbiochem, 10 µM), dans du tampon PBS à pH 7,4 $Ca^{2+}$ et $Mg^{2+}$ = 1 µM.

100 ng de prostaglandine B2 (PGB2) en solution dans du méthanol sont ajoutés à la fin de la réaction pour servir de standard interne.

Par centrifugation, on prélève le surnageant de culture. Celui-ci est dégagé sous Argon et stocké à -80°C jusqu'à l'analyse HPLC.

Cette analyse est faite par injection de 250 µl sur colonne ultrasphère ODS 5 µm (Beckmann), avec un gradient acétonitrile (20-80 %) dans de l'eau, à pH 7,4.

Les quantités des différents produits absorbant à 269 nm ; leucotriènes $B_4$ ($LTB_4$), isomères de $LTB_4$ et métabolites oxydés de $LTB_4$, sont estimées par rapport au standard interne.

Le produit à tester est utilisé à la concentration de 40 µM.

Les résultats rapportés dans le tableau ci-après montrent l'activité des composés sur :

1 $LTB_4$

2 les isomères de $LTB_4$

3 les métabolites oxydés de $LTB_4$

Ils sont exprimés

soit en % d'inhibition,

soit en % d'augmentation de la production des $LTB_4$, isomères de $LTB_4$ et métabolites oxydés de $LTB_4$ (noté par +).

| Produit | $LTB_4$ | Isomères | Métabolites oxydés |
|---|---|---|---|
| 1571 | 60% | 53% | 70% |
| 1572 | 84% | 100% | 90% |
| 1574 | 95% | 100% | 100% |
| 1682 | +30% | +30% | 40% |
| 1683 | +15% | +15% | 30% |
| 1684 | 20% | 35% | 60% |
| 1685 | 50% | 70% | 70% |
| 1686 | 0% | 5% | 25% |
| 1688 | +35% | +35% | 20% |
| 1689 | +30% | +30% | 20% |
| 1687 | +15% | 0 | +30% |

2.2. Deuxième expérience :

Le modèle expérimental est le même que décrit dans le paragraphe 2.1. D'autres produits sont testés par rapport au composition de l'invention n° 1574, utilisé comme témoin.

Résultats

| Produit testé | % d'inhibition de la formation de $LTB_4$ |
|---|---|
| 1574 | 95 |
| 1734 | 70 |
| 1736 | 100 |
| 1737 | 95 |
| 1743 | 20 |
| 1738 | 20 |
| 1755 | 100 |
| 1761 | 100 |
| 1763 | 100 |
| 1764 | 100 |
| 1796 | 100 |
| 1797 | 100 |

Les résultats montrent qu'à la concentration de 4O µM, la plupart des produits testés inhibent très fortement la biosynthèse de $LTB_4$.

2.3. Troisième expérience

Des leucocytes polymorphonucléaires humains sont stimulés par du ionophore de calcium A 23187 (Calbiochem, 5 µM), en présence ou non des produits à tester.

Après 15 minutes d'incubation à 37°C, on effectue le dosage des leucotriènes $C_4$ ($LTC_4$) par radio-immunologie.

Les résultats sont exprimés en IC 5O, concentration inhibant 5O % de la production de $LTC_4$, par rapport à une suspension témoin n'ayant pas été incubée en présence des produits à tester.

| Résultats<br>Produit testé | IC 50<br>µM |
|---|---|
| 1574 | 3 |
| 1736 | 3 |
| 1796 | 1.7 |
| 1797 | 1 |
| 1813 | 1 |

Les IC 5O obtenues montrent que les produits sont fortement inhibiteurs de la production de $LTC_4$.

On obseve également une relation dose-effet entre les doses testées $1O^{-6}$ et $5.1O^{-5}$M, et l'inhibition de la biosynthèse de $LTC_4$.

2.4. Quatrième expérience

Les leucocytes polymorphonucléaires humains, mis en suspension avec de l'acide arachidonique et stimulés par du ionophore de calcium, produisent des leucotriènes, $B_4$ ($LTB_4$).

Le test consiste à incuber des leucocytes humains frais avec de l'acide arachidonique à la dose de 1O µg/ml, additionné d'une dose traceuse d'acide arachidonique marqué au titrium. On stimule les leucocytes par du ionophore de calcium A 23187 (Calbiochem, 5 µM) et on dose ensuite en HPLC la quantité d'acide 5-hydroxy eicosa tétraénoïque(5-HETE) et de $LTB_4$ produits.

Le 5-HETE est le métabolite produit à partir de l'acide arachidonique par la 5-lipoxygénase, et à partir duquel est produit le $LTB_4$ par l'action d'une isomérase.

Les résultats sont exprimés en intégration de la surface du pic obtenu en HPLC par rapport à celui obtenu avec les leucocytes témoins n'ayant pas été incubés avec les produits à tester.

Les résultats entre parenthèses sont exprimés en % d'inhibition de la production de 5-HETE et de $LTB_4$.

<u>Résultats</u>

| | Leucocytes témoins | 1736 | 1796 | 1797. | 1813 |
|---|---|---|---|---|---|
| LTB4 | 3.419 | Indétectable (100) | 152 (95.7) | Indétectable (100) | Indétectable (100) |
| 5-HETE | 3.690 | 384 (89.6) | 127 (96.6) | Indétectable (100) | 181 (95.1) |

Les résultats montrent que les produits testés inhibent très fortement à la fois la production de 5-HETE et celle le $LTB_4$ ce qui indique que l'inhibition a lieu au niveau de la 5-lipoxygénase.

IV - <u>Etude toxicologique</u>

On a étudié la $DL_{50}$ approchée par voie orale, des composés suivants : 1361, 1489, 1572 chez le rat et la souris et 1341, 1573, 1574, 1361, 1495, 1579, 1489, 1572 chez la souris.
Les composés ont été mis en suspension dans une solution de gomme arabique à 3 %, de manière à apporter sous un volume de 2 ml pour les rats, l'équivalent de 50, 100, 200, 400 et 800 mg/kg et sous un volume de 1 ml pour les souris, 100, 200, 400, 800, 1 600 mg/kg et pour 1573, 1579, 3 200 mg/kg.
Le gavage a été pratiqué à l'aide d'une sonde métallique sur des lots de 5 animaux pour chaque série.
Les animaux ont été observés quotidiennement pendant 14 jours.
Les $DL_{50}$ approchées figurent dans le tableau suivant.

TABLEAU XIII

$DL_{50}$ Voie orale

| Rats Wistar mâles 200-400 g | | Souris femelles Swiss CF Poids 20 g | |
|---|---|---|---|
| Composé N° | | Composé N° | |
| 1361 | > 800 mg/kg | 1341 ~ | 250 mg/kg |
| 1572 | > 800 mg/kg | 1573 | > 3 200 mg/kg |
| 1489 | > 800 mg/kg | 1574 | > 1 600 mg/kg |
| | | 1361 | > 1 600 mg/kg |
| | | 1572 | > 1 600 mg/kg |
| | | 1495 | > 1 600 mg/kg |
| | | 1579 | > 3 200 mg/kg |
| | | 1489 | > 1 600 mg/kg |

Les produits testés s'avèrent bien tolérés par voie orale pour le rat et la souris. Leur $DL_{50}$ est très supérieure à celle connue du kétoprofène.
Les composés sont avantageusement introduits comme principe actif dans des médicaments destinés au traitement des états inflammatoires, des états allergiques, notamment cutanés ou asthmatiques ou au traitement de certaines pathologies cardiovasculaires ou de l'insuffisance rénale.
Les composés selon l'invention sont à cet effet conditionnés avec les excipients et adjuvants traditionnels, notamment ceux utilisés pour la préparation de comprimés, poudres, gélules, ampoules buvables, solutions injectables.
Les medicaments de l'invention peuvent être avantageusement administrés par voie orale, notamment sous forme de comprimés, gelules, poudres, solutions, suspensions, sirop.
Les medicaments selon l'invention peuvent également être administrés par voie topique telle que crème, lotion, pommade, gel.
Les medicaments selon l'invention peuvent également être administrés par voie parentérale, par exemple lorsque la substance active est sous forme de sel.
Pour cette raison, ils sont présentés sous forme de solutions stériles ou stérilisables, injectables ou appropriées pour cette utilisation, pour la préparation extemporanée de solutions injectables. Ces solutions peuvent être présentées sous forme de solutions physiologiques aqueuses, en particulier solutions isotoniques de l'un de ces composés, par exemple solutions isotoniques de glucose ou salines, ces exemples n'ayant évidemment aucune nature limitative dans la définition des produits physiologiquement acceptables qui peuvent être utilisés pour former les solutions isotoniques injectables.
Les medicaments selon l'invention peuvent également être administrés sous forme de suppositoires.
Les doses à administrer seront d'environ 10 à 500 mg de substance active par jour.
Dans les présentations pharmaceutiques destinées à l'administration orale, la dose unitaire est composé

d'environ 1O à environ 5OO mg, de préference d'environ 25 à 25O mg de substance active, par dose unitaire. Dans les présentations pharmaceutiques destinées à l'administration parentérale, les préparations injectables comprennent environ 1O à 1OO mg de substance active, par dose unitaire.

## Revendications

**Revendications pour les Etats contractants suivants : AT, BE, CH, DE, FR, GB, IT, LI, LU, NL, SE**

1. Composés répondant à la formule I

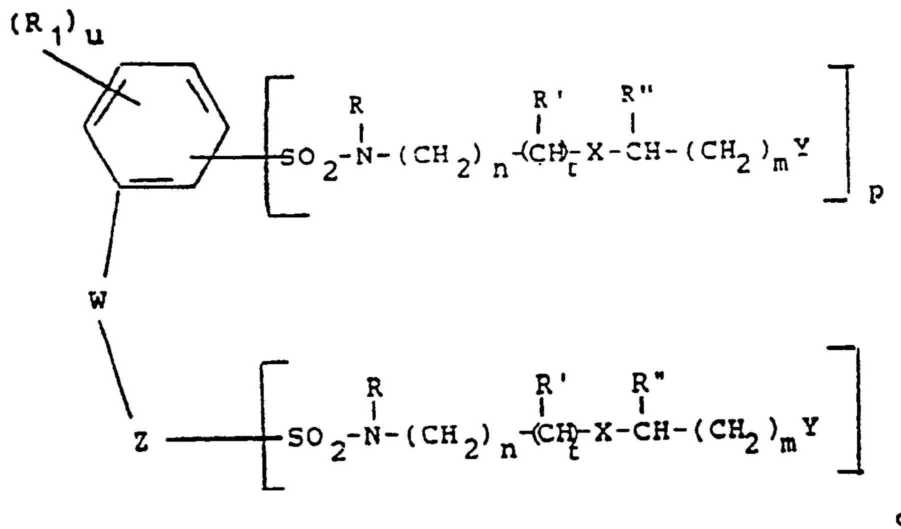

(I)

dans laquelle :
- W représente C = O, $CH_2$ ou CHOH,
- Z représente :

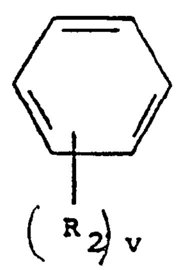

ou

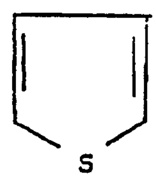

- $R_1$ représente Cl, F, Br, $NO_2$, $NH_2$ , $CF_3$, un radical alcoyle de 1 à 4 atomes de carbone, notamment méthyle ou éthyle, un radical alcoxy de 1 à 4 atomes de carbone, acétamido ou benzamido,
- $R_2$ représente Cl, F, Br, $NO_2$, $NH_2$ , $CF_3$, un radical alcoyle de 1 à 4 atomes de carbone, un radical alcoxy de 1 à 4 atomes de carbone, acétamido ou benzamido,
- R représente H, un radical alcoyle de 1 à 6 atomes de carbone ou un radical benzyle,
- R′ et R″ représente H ou un radical alcoyle de 1 à 4 atomes de carbone,
- X représente $(CH_2)_r$, r valant O à 1, -CH = CH,

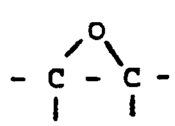, ou 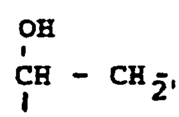,

– Y représente $COOR_3$, $R_3$ représentant un hydrogène ou un radical alcoyle de 1 à 4 atomes de carbone,

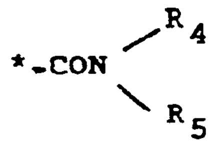 ,

$R_4$ et $R_5$ représentant H ou un radical alcoyle de 1 à 4 atomes de carbone,

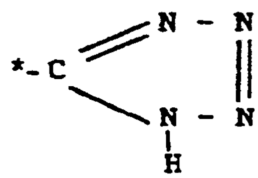 ,

* - C ≡ N,

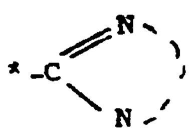 , 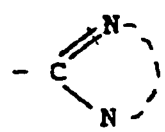

représentant un cycle non aromatique à 5 ou 6 chainons, dans lequel les 2 ou 3 atomes qui entrent dans le cycle sont des atomes de carbone et/ou d'oxygène et/ou d'azote,

* $NH_2$, $NHR_6$, $NR_6R_7$,

$R_6$ et $R_7$ représentant un radical alcoyle de 1 à 4 atomes de carbone, ou susceptible de former avec l'atome d'azote une amine cyclisée non aromatique,

– sous réserve que lorsque Y représente - $NH_2$, X soit différent de

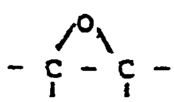

– u est un nombre entier de O à 2
– v est un nombre entier de O à 2
– p vaut O ou 1
– q vaut O ou 1, p + q étant égal ou supérieur à 1
– n est un nombre entier variant de O à 1O
– m est un nombre entier variant de O à 1O
– t vaut O ou 1,
– le nombre total d'atomes de carbone de la chaîne

$$-(CH_2)_n-(CH)_t-X-CH-(CH_2)_m\ Y,$$

variant de 2 à 2O,

ainsi que leurs sels physiologiquement acceptables.

2. Composé selon la revendication 1, répondant à la formule II suivante

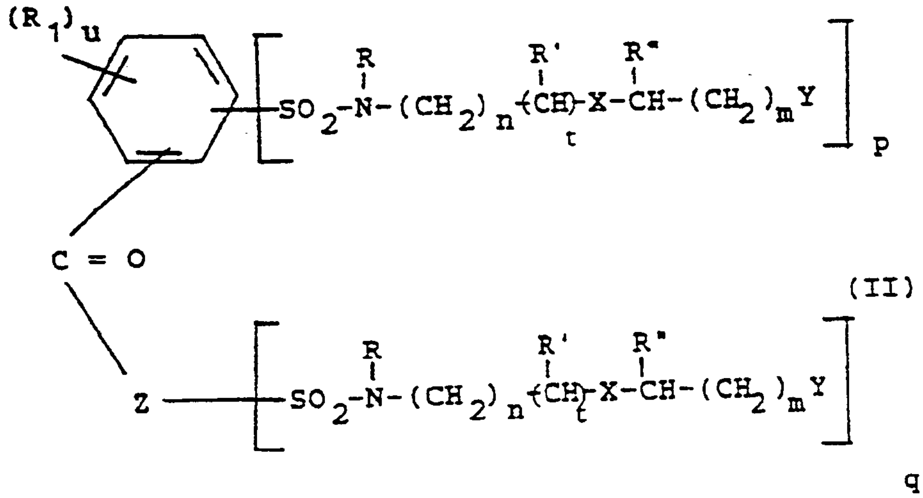

(II)

dans laquelle $R_1$, R, R′, R″, X, Y, Z, m, n, t, p, q et u ont les significations indiquées à la revendication 1.

3. Composé selon la revendication 1, répondant à la formule III suivante

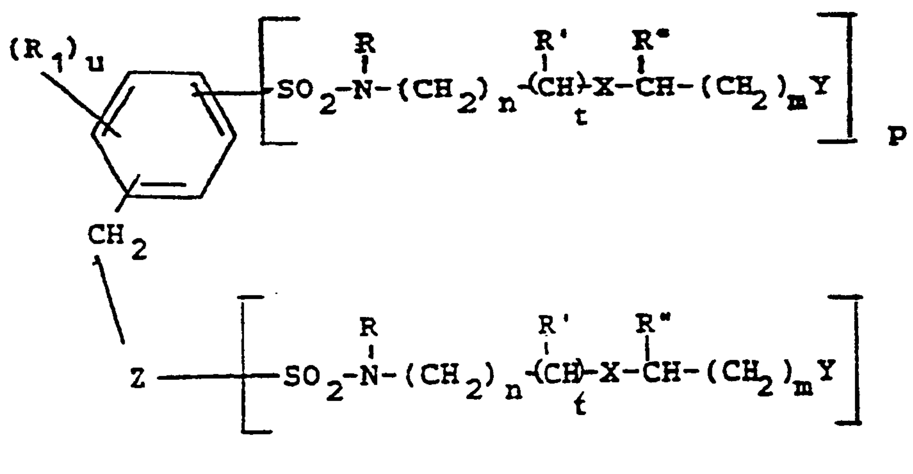

(III)

dans laquelle $R_1$, R, R′, R″, X, Y, Z, m, n, t, p, q et u ont les significations indiquées à la revendication 1.

4. Composé selon la revendication 1, répondant à la formule IV suivante

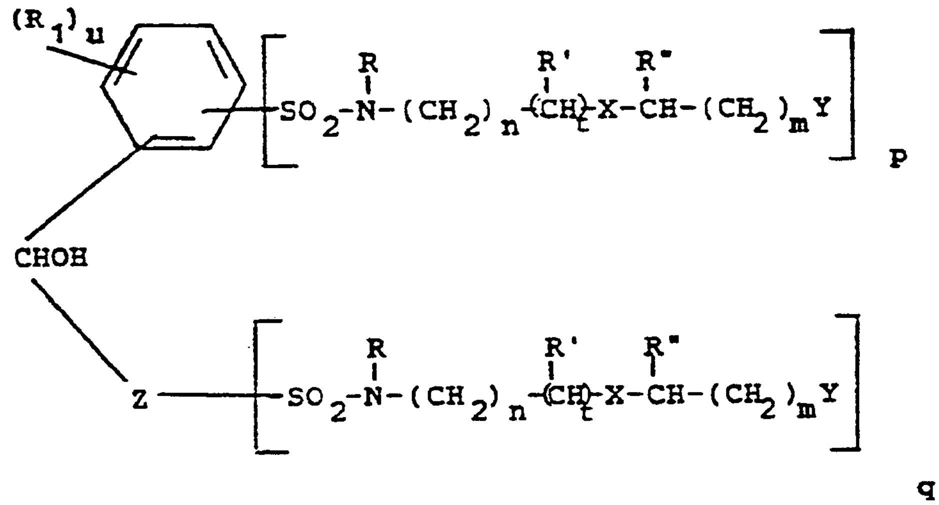

(IV)

dans laquelle $R_1$, R, R', R'', X, Y, Z, m, n, t, p, q et u ont les significations indiquées à la revendication 1.

5. Composé selon la revendication 2, répondant à la formule V suivante

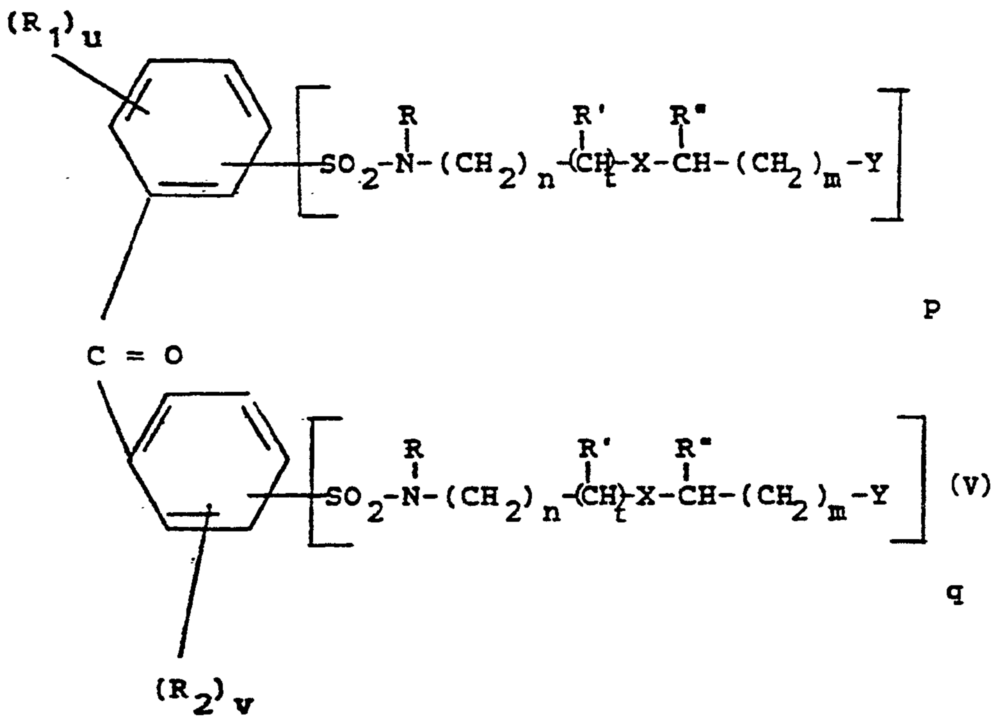

(V)

dans laquelle $R_1$, $R_2$, R, R', R'', X, Y, Z, m, n, p, q, t, u et v ont les significations indiquées à la revendication 1.

6. Composé selon la revendication 5, répondant à la formule V
dans laquelle
- Y représente $COOR_3$,
$R_3$ représentant H ou un radical alcoyle de 1 à 4 atomes de carbone.

7. Composé selon la revendication 5, répondant à la formule V
dans laquelle
- Y représente $NH_2$ , $NHR_6$ ou $NR_6R_7$, $R_6$ et $R_7$ ayant les significations indiquées à la revendication 1.

8. Composé selon la revendication 5, répondant à la formule V

dans laquelle p ou q vaut O.

9. Composé selon la revendication 8, caractérisé en ce que

– u + v supérieur ou égal à 1,

– le nombre total d'atomes de carbone de la chaîne

$$(CH_2)_n - (\overset{|}{C}H)_t - X - \overset{|}{C}H - (CH_2)_m - Y$$

est égal ou supérieur à 5.

1O. Composé selon la revendication 5, répondant à la formule V, dans laquelle

– p = 1,

– q = 1,

– u + v supérieur ou égal à 1,

– le nombre total d'atomes de carbone de la chaîne

$$(CH_2)_n - (\overset{|}{C}H)_t - X - \overset{|}{C}H - (CH_2)_m - Y$$

est égal ou supérieur à 5.

11. Composé selon la revendication 1, répondant à la formule VI suivante

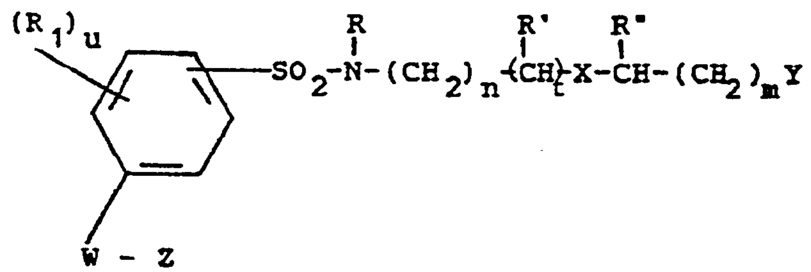

(VI)

dans laquelle $R_1$, R, R', R", X, Y, Z, W, m, n, u et t, ont les significations indiquées à la revendication 1.

12. Composé selon la revendication 11, répondant à la formule VII suivante

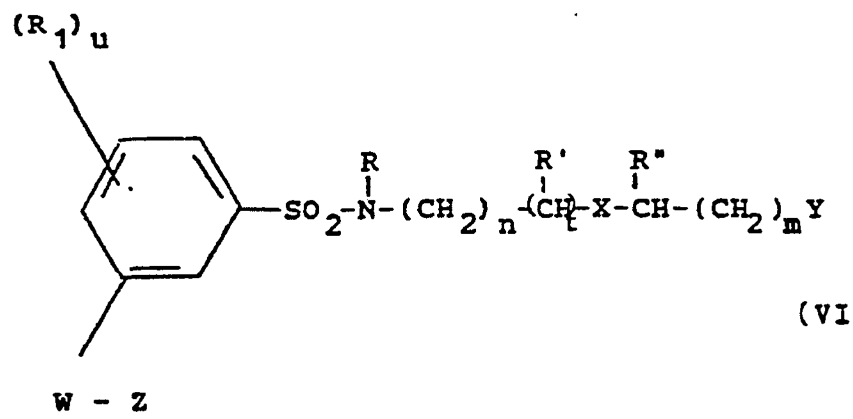

(VII)

dans laquelle $R_1$, R, R', R", X, Y, Z, W, m, n, u et t ont les significations indiquées à la revendication 1.

13. Composé selon la revendication 12, répondant à la formule VII dans laquelle

- Y représente $COOR_3$, $R_3$ représentant H ou un radical alcoyle de 1 à 4 atomes de carbone.

14. Composé selon la revendication 13, répondant à la formule VIII suivante

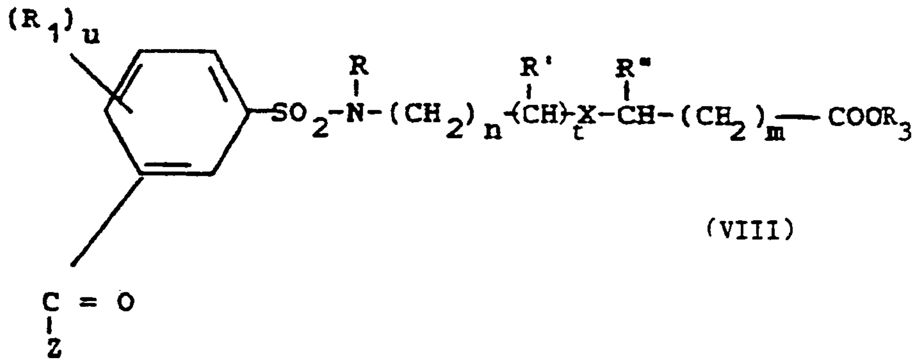

(VIII)

dans laquelle $R_1$, $R_3$, R, R′, R″, X, Z, m, n, u et t ont les significations indiquées à la revendication 1.

15. Composé selon la revendication 13, répondant à la formule IX suivante

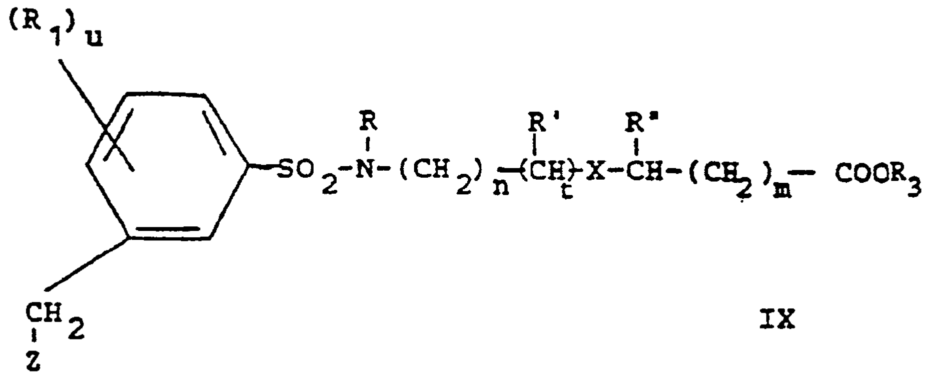

IX

dans laquelle $R_1$, $R_3$, R, R′, R″, X, Z, m, n, u et t ont les significations indiquées à la revendication 1.

16. Composé selon la revendication 13 répondant à la formule X suivante

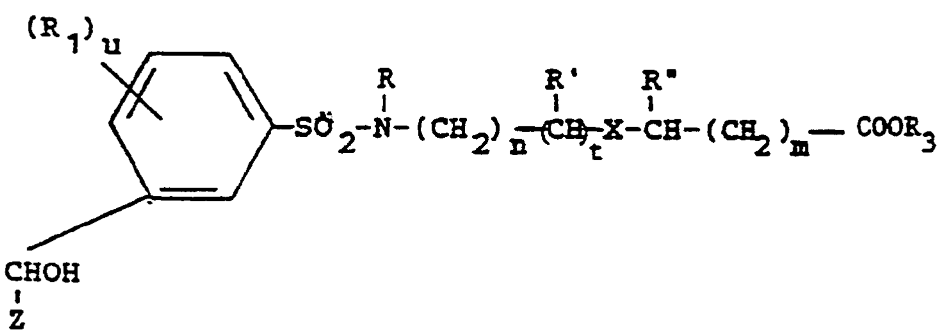

X

dans laquelle $R_1$, $R_3$, R, R′, R″, X, Z, m, n, u et t ont les significations indiquées à la revendication 1.

17. Composé selon la revendication 14, répondant à la formule VIII dans laquelle le nombre total d'atomes de carbone de la chaîne

$$(CH_2)_n - \overset{|}{(CH)}_t - X - \overset{|}{CH} - (CH_2)_m - Y$$

est égal ou supérieur à 5.

18. Composé selon la revendication 15, répondant à la formule IX dans laquelle le nombre total d'atomes de carbone de la chaîne

$$(CH_2)_n-(\overset{|}{C}H)_t-X-\overset{|}{C}H-(CH_2)_m-Y$$

est égal ou supérieur à 5.
19. Composé selon la revendication 16, répondant à la formule X
dans laquelle le nombre total d'atomes de carbone de la chaîne

$$(CH_2)_n-(\overset{|}{C}H)_t-X-\overset{|}{C}H-(CH_2)_m-Y$$

est égal ou supérieur à 5.
2O. Composé selon la revendication 13, répondant à la formule XI suivante

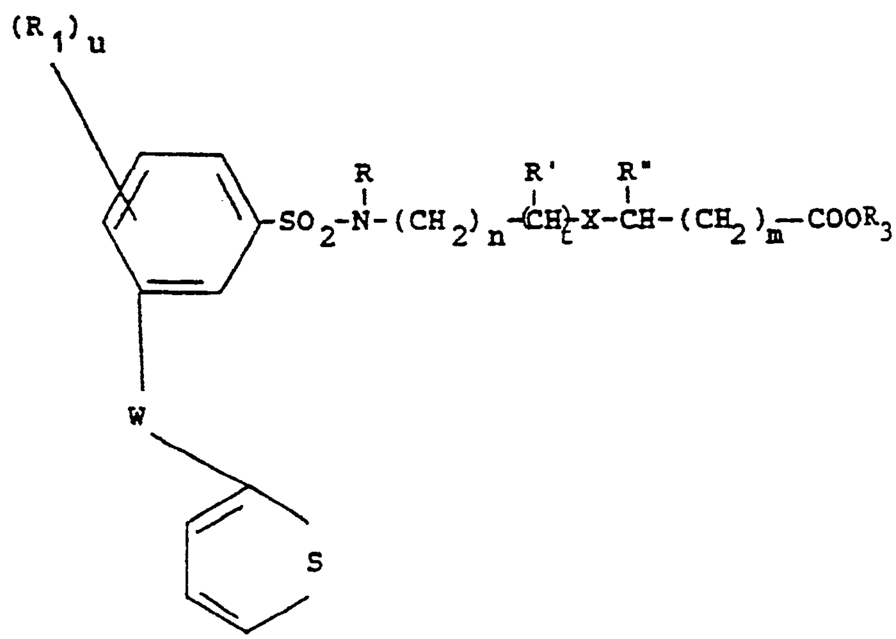

XI

- dans laquelle
le nombre total d'atomes de carbone de la chaîne

$$(CH_2)_n-(\overset{|}{C}H)_t-X-\overset{|}{C}H-(CH_2)_m-Y$$

est égal ou supérieur à 5, et dans laquelle
$R_1$, $R_3$, R, R′, R″, X, W, m, n, t et u ont les significations indiquées à la revendication 1.
21. Composé selon la revendication 11, répondant à la formule VIbis suivante :

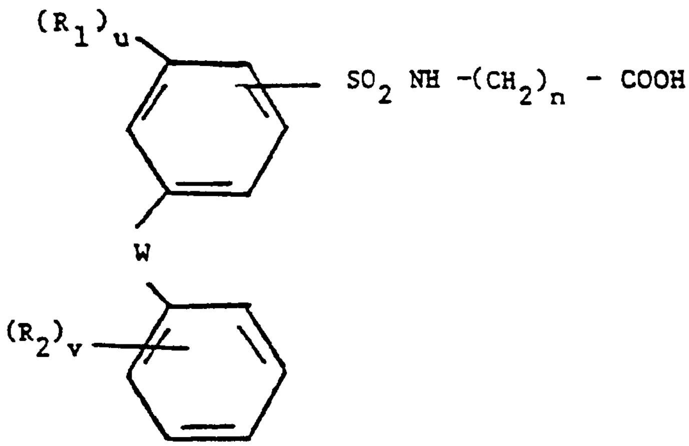

dans laquelle $R_1$et $R_2$ représentent un halogène
u varie de O à 2, v varie de O à 2,
W représente C = O, CHOH, $CH_2$,
n vaut de 3 à 11

22. Composé selon la revendication 11 répondant à la formule VIter suivante :

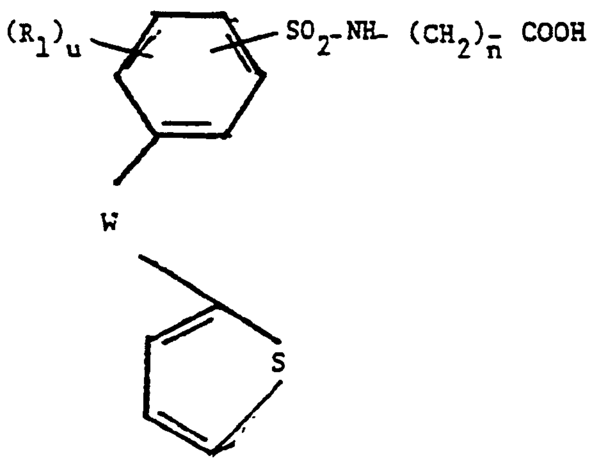

dans laquelle $R_1$ représente un halogène
u vaut O à 2
W représente C=O, CHOH, $CH_2$
n vaut 3 à 11.

23. Composés selon la revendication 1, répondant aux formules suivantes

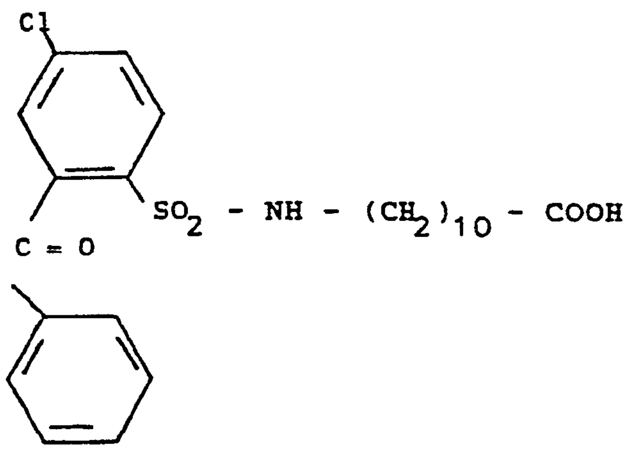

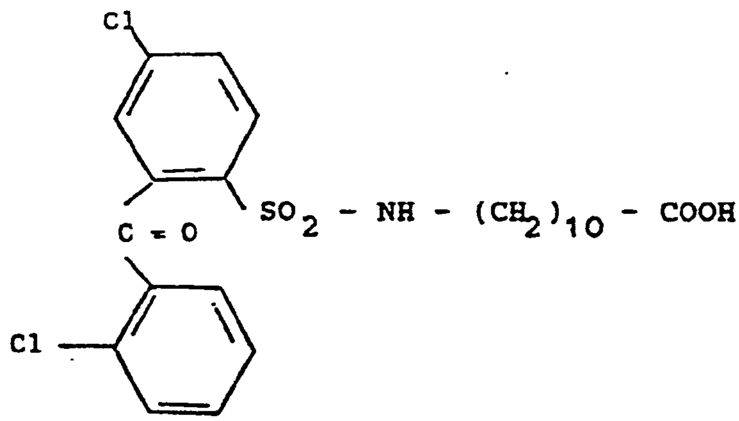

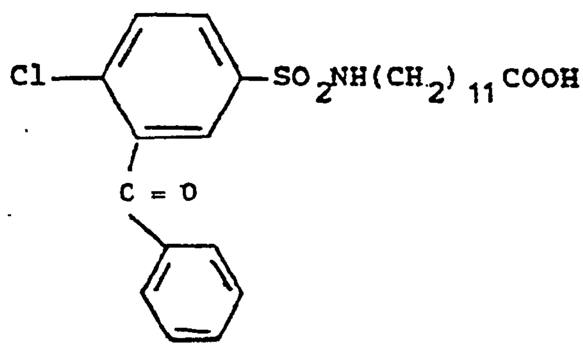

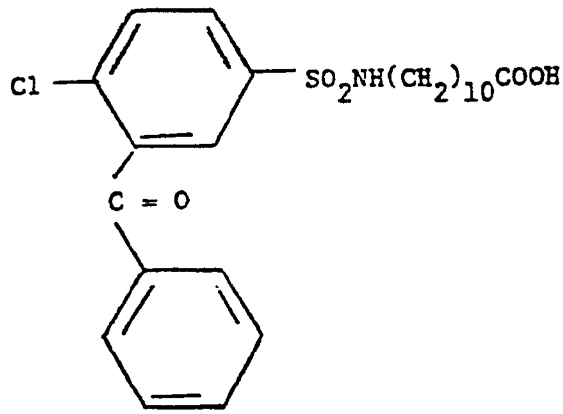

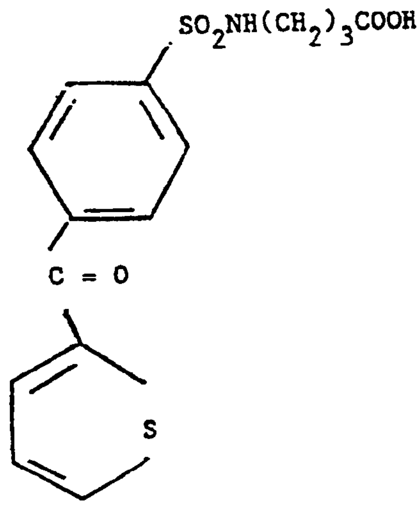

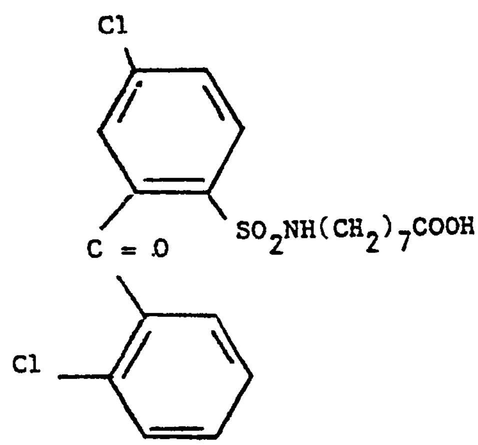

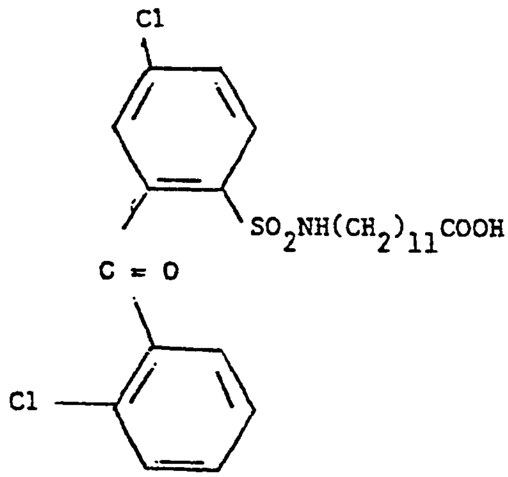

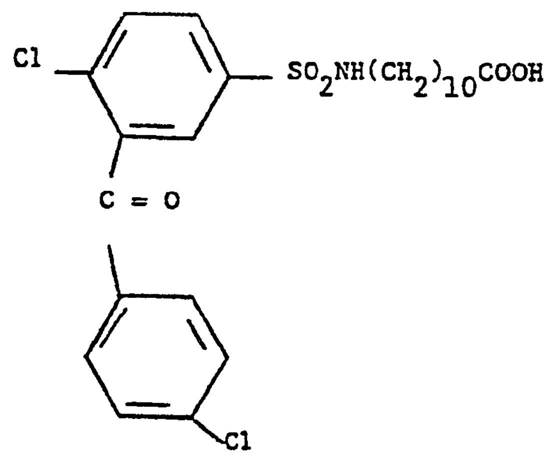

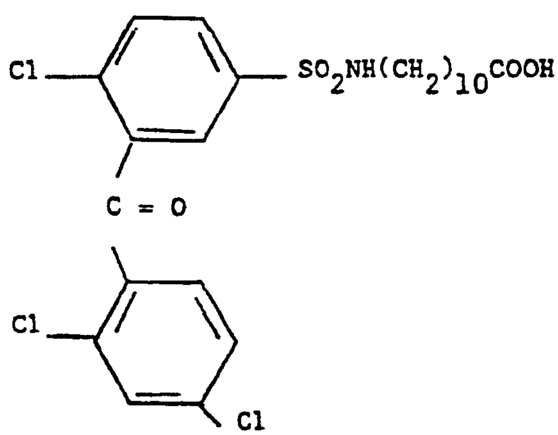

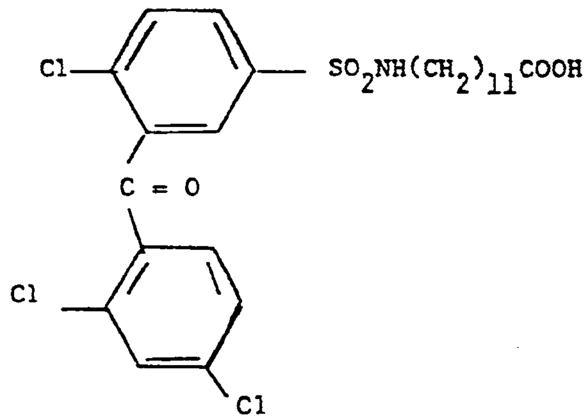

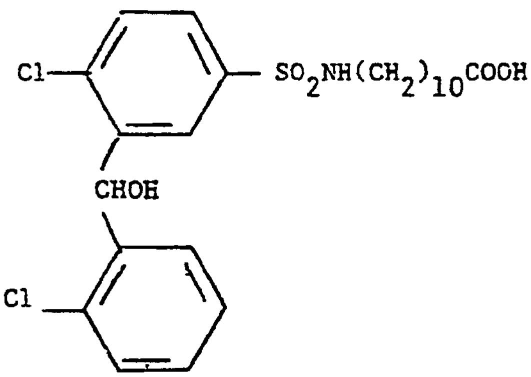

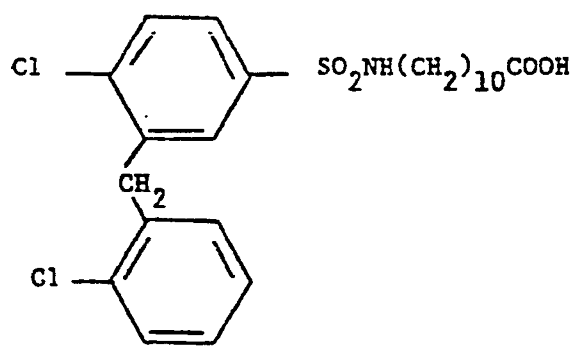

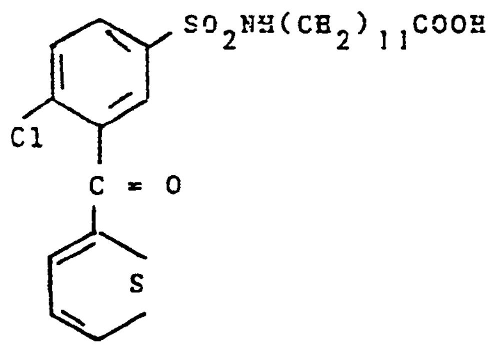

24. Procédé de préparation des composés de formule I, selon la revendication 1, caractérisé en ce que l'on soumet un composé de formule

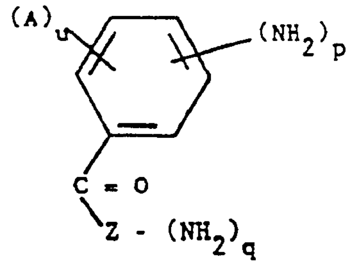

dans laquelle

– A a les significations indiquées pour $R_1$, à l'exception de $NH_2$, c'est-à-dire A représente Cl, F, Br, $NO_2$, $CF_3$, un radical alcoyle de 1 à 4 atomes de carbone, un radical alcoxy de 1 à 4 atomes de carbone, acétamido ou benzamido,

– Z représente

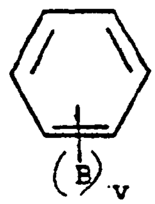

ou

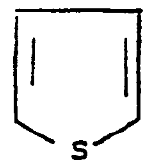

– B a les significations indiquées pour $R_2$, à l'exception de $NH_2$, c'est-à-dire B représente Cl, $NO_2$, $CF_3$, un radical alcoyle de 1 à 4 atomes de carbone, un radical alcoxy de 1 à 4 atomes de carbone, acétamido ou benzamido et

– u représente un nombre entier de O à 2,

– v représente un nombre entier de O à 2,

– p = O ou 1,

– q = O ou 1, p + q étant égal ou supérieur à 1,

éventuellement à une réduction, notamment à l'aide de chlorure d'aluminium et d'hydrure d'aluminium et de lithium pour réduire - C = O en $CH_2$, et obtenir le composé de formule

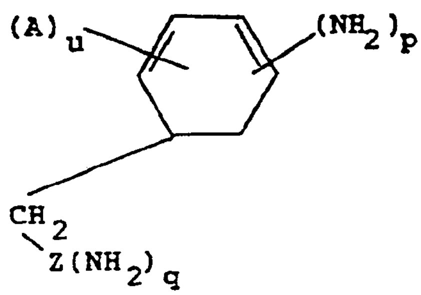

dans laquelle A, Z, u, p et q ont les significations indiquées ci-dessus,

. on soumet l'un des composés aminés de formule indiquée ci-dessus à la réaction de Sandmeyer, pour obtenir un sulfohalogénure de formule :

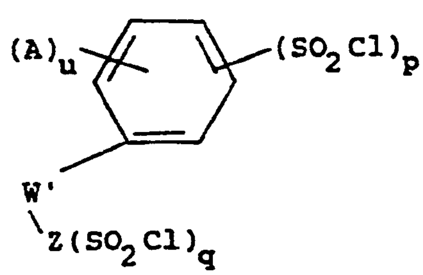

dans laquelle A, Z, u, p et q ont les significations indiquées ci-dessus et W' représente C = O ou $CH_2$,
. on fait réagir le sulfohalogénure, notamment le sulfochlorure, de formule indiquée ci-dessus, sur un ou deux composés de formule

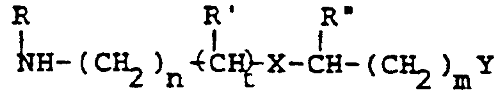

dans laquelle
– R représente H, un radical alcoyle de 1 à 6 atomes de carbone ou un radical benzyle,
– R' et R" représente H ou un radical alcoyle de 1 à 4 atomes de carbone,
– X représente $(CH_2)_{r'}$, r valant O ou 1 ou -CH = CH,

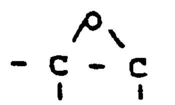

, ou

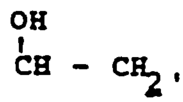

– Y représente $COOR_3$, $R_3$ représentant H ou un radical alcoyle de 1 à 4 atomes de carbone,

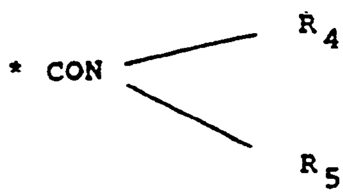

, $R_4$ et $R_5$ représentant H ou un radical alcoyle de 1 à 4 atomes de carbone,

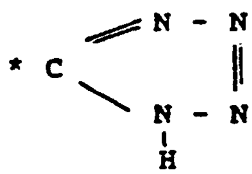

,

- C ≡ N,

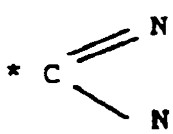

,

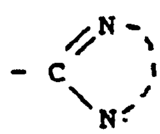

représentant un cycle non aromatique à 5 ou 6 chainons, dans lequel les 2 ou 3 atomes qui entrent dans le cycle sont des atomes de carbone et/ou d'oxygène et/ou d'azote,
* $NH_2$, $NHR_6$, $NR_6N_7$,
$R_6$ et $R_7$ représentant un radical alcoyle de 1 à 4 atomes de carbone, ou susceptible de former avec l'atome d'azote une amine cyclisée non aromatique,
– sous réserve que lorsque Y représente $-NH_2$, X soit différent de

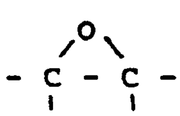

– n est un nombre entier variant de O à 1O

– m est un nombre entier variant de O à 1O
– t vaut O ou 1
– le nombre total d'atomes de carbone de la chaîne

$$-(CH_2)_n-(CH)_t-X-CH-(CH_2)_m\,Y,$$

variant de 2 à 2O,
pour obtenir le composé de formule

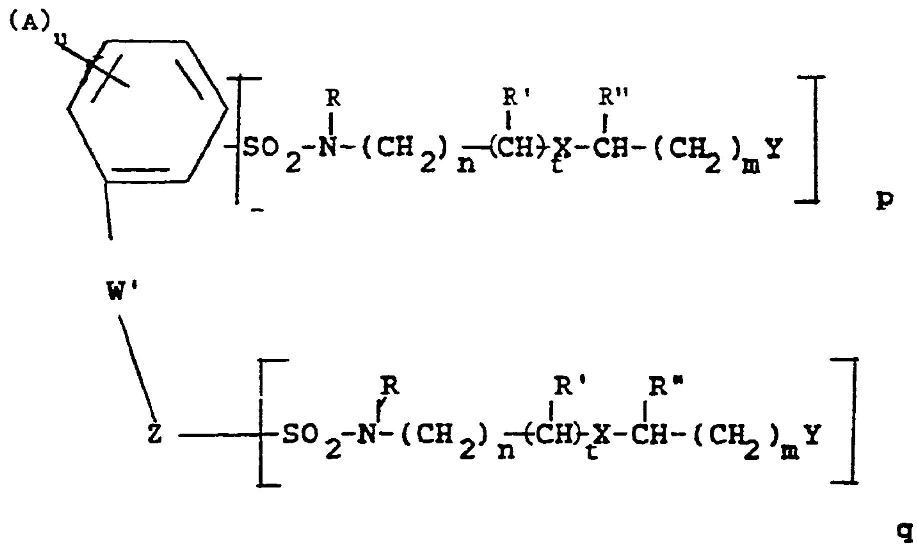

dans laquelle A, Z, W', R, R', R'', X, Y, m, n, t, q et u ont les significations indiquées ci-dessus :
. et dans le cas où W' représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$.
25. Procédé selon la revendication 24 de préparation des composés de formule II

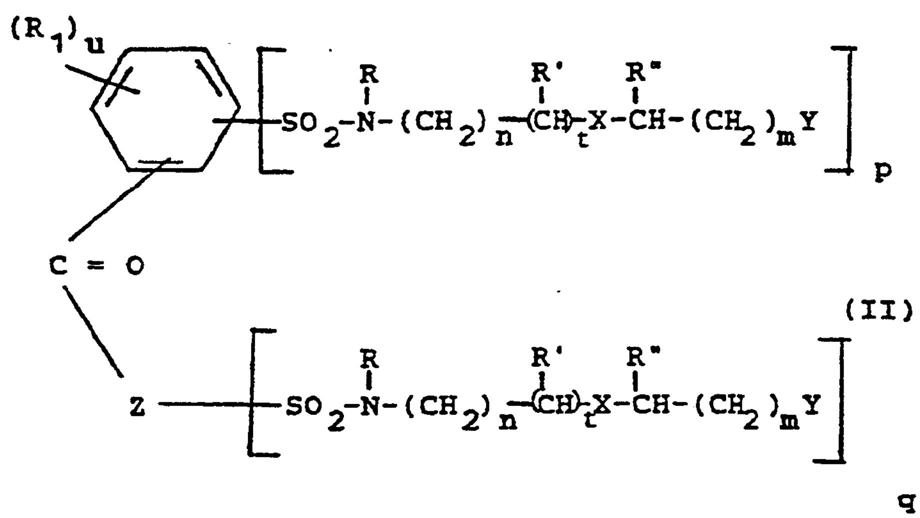

(II)

dans laquelle $R_1$, R, R', R'', X, Y, Z, m, n, p, q, u et t ont les significations indiquées à la revendication 2, caractérisé en ce que l'on fait réagir un sulfohalogénure, notamment un sulfochlorure de formule

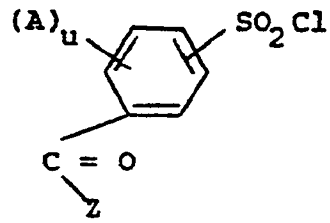

dans laquelle A, Z et u ont les significations indiquées à la revendication 24 ou un disulfohalogénure, notamment un disulfochlorure de formule

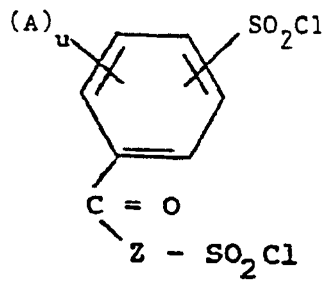

dans laquelle A, u et Z ont les significations précédemment indiquées sur un ou deux composés de formule

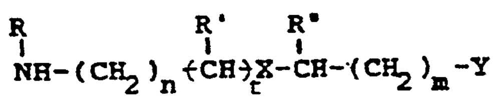

dans laquelle R, R', R", n, m, t, X et Y ont les significations indiquées à la revendication 24,
- suivi éventuellement d'une réduction lorsque A et/ou B représente $NO_2$.

26. Procédé selon la revendica tion 25, caractérisé en ce que l'on fait réagir un sulfochlorure de formule

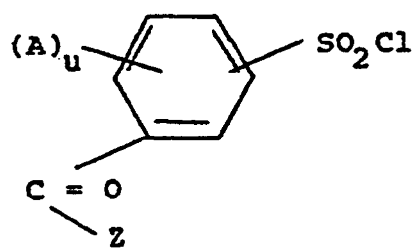

dans laquelle A, u, Z ont les significations indiquées à la revendication 25, ou un disulfochlorure de formule

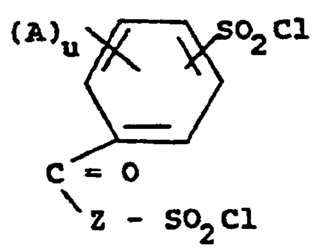

dans lequel A, u, Z ont les significations indiquées à la revendication 25, sur un ou deux amino-acides de formule

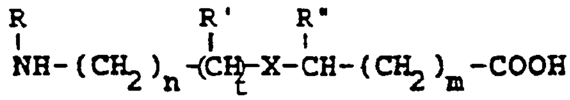

dans laquelle R, R', R'', n, m, t et X ont les significations indiquées ci-dessus à la revendication 25.

27. Procédé selon la revendication 25, caractérisé en ce que

– l'on estérifie un composé tel qu'obtenu selon la revendication 26 , et dans lequel Y représente COOH, en un composé de formule II, dans lequel Y représente $COOR_3$, $R_3$ représentant un radical alcoyle de 1 à 4 atomes de carbone

– ou en ce que l'on transforme un composé tel qu'obtenu selon la revendication 26, en un composé de formule II dans lequel Y représente

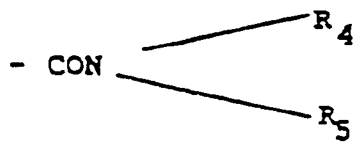

$R_4$, $R_5$ représentant H ou un radical alcoyle de 1 à 4 atomes de carbone,

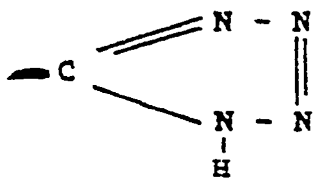 , - C≡N 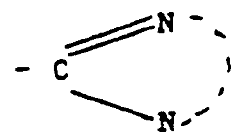

28. Procédé selon la revendication 25, caractérisé en ce que l'on fait réagir un sulfochlorure de formule

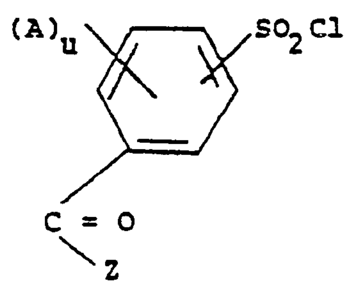

dans laquelle :

– A a les significations indiquées pour $R_1$, à l'exception de $NH_2$, c'est-à-dire Cl, F, Br, $NO_2$, $CF_3$, un radical alcoyle de 1 à 4 atomes de carbone, un radical alcoxy de 1 à 4 atomes de carbone, acétamido ou benzamido,

– Z représente :

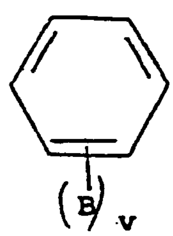

ou

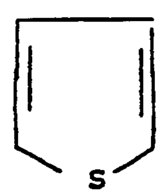

– B a les significations indiquées pour $R_2$, à l'exception de $NH_2$, c'est-à-dire B représente Cl, F, Br, $NO_2$, $CF_3$, un radical alcoyle de 1 à 4 atomes de carbone, un radical alcoxy de 1 à 4 atomes de carbone, acétamido ou benzamido,

– u represente un nombre entier de O à 2

– v représente un nombre entier de O à 2 ou un disulfohalogénure, notamment disulfochlorure de formule

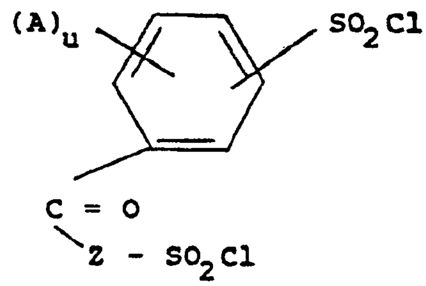

dans laquelle A, u, Z ont les significations indiquées ci-dessus ;
sur une aminoalcoylamine de formule :

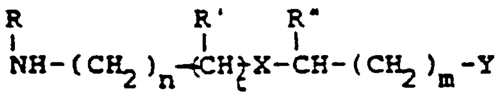

dans laquelle :

– R représente H, un radical alcoyle de 1 à 6 atomes de carbone ou un radical benzyle,
– R′ et R″ représentent H ou un radical alcoyle de 1 à 4 atomes de carbone,
– n représente un nombre entier variant de O à 1O,
– m représente un nombre entier variant de O à 1O,
– t vaut O ou 1
– Y représente $NH_2$, $NHR_6$, $NR_6R_7$, $R_6$ et $R_7$ représentent un radical alcoyle de 1 à 4 atomes de carbone, ou forment avec l'azote une amine cyclisée non aromatique.
– $X_a$ la signification donnée dans la formule I

29. Procédé de préparation selon la revendication 25, caractérisé en ce que l'amino-acide utilisé ou la dialcoylaminoalcoylamine utilisée est choisi respectivement parmi :

$$- NH_2 -(CH_2)_n - COOH ,$$

n variant de 1 à 11

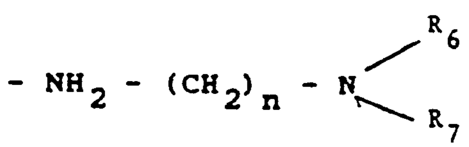

n = 2 ou 3

– $R_6$ et $R_7$ représentant un alcoyle de 1 à 4 atomes de carbone ou un noyau morpholine avec l'atome d'azote et le sulfochlorure utilisé est choisi respectivement parmi :
– le chlorure de benzoyl - 2 nitro - 4 benzènesulfonyle
– le chlorure de benzoyl - 2 chloro - 4 benzènesulfonyle
– le chlorure de chloro - 4(chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

3O. Procédé selon la revendication 24 de préparation des composés de formule III

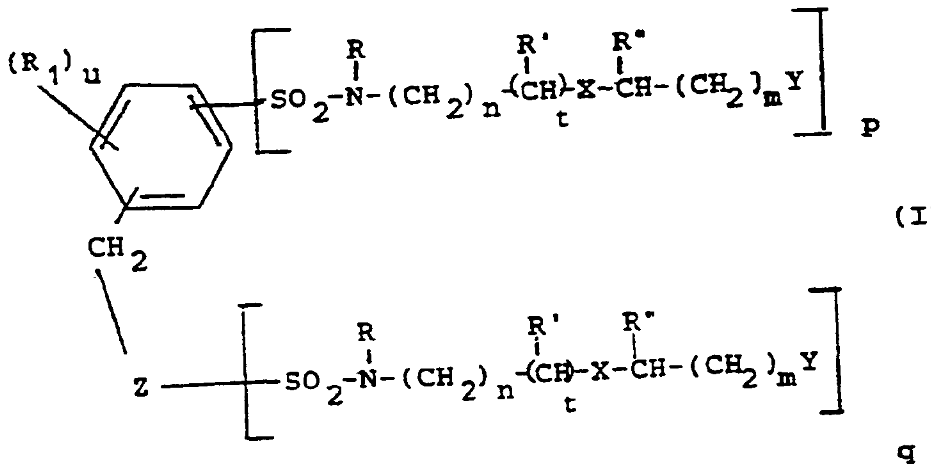

(III)

dans laquelle $R_1$, R, R′, R″, X, Y, Z, m, n, t, p, q et u ont les significations indiquées à la revendication 3 caractérisé en ce que l'on soumet le composé de formule

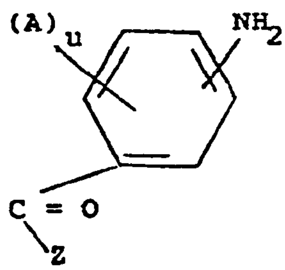

ou de formule

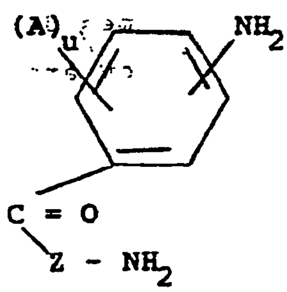

dans laquelle A a la signification indiquée à la revendication 24, à une réduction, pour réduire C = O en $CH_2$ et obtenir le composé de formule

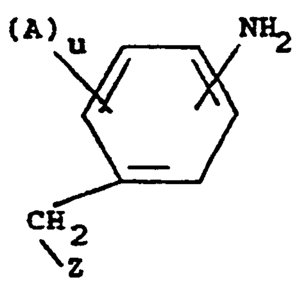

ou

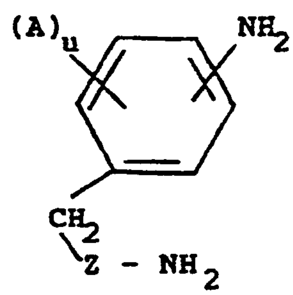

– on soumet l'un de ces composés à la réaction de Sandmeyer, pour obtenir les composés de formule :

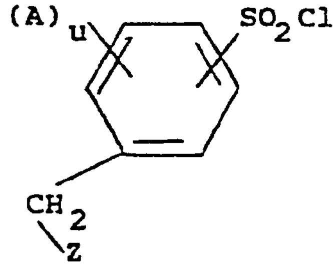

ou

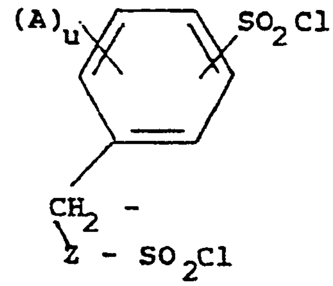

– on condense le sulfohalogénure ou le disulfohalogénure de formule indiquée ci-dessus, sur un composé de formule

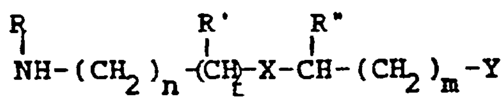

dans laquelle R, R', R", X, Y, n, m et t ont les significations indiquées à la revendication 24 suivi éventuellement d'une réduction lorsque A et/ou B représente $NO_2$.

31. Procédé de préparation selon la revendication 3O, caractérisé en ce que l'amino-acide utilisé ou la dialcoylaminoalcoylamine utilisée est choisie parmi

$$- NH_2 -(CH_2)_n - COOH ,$$

n variant de 1 à 11

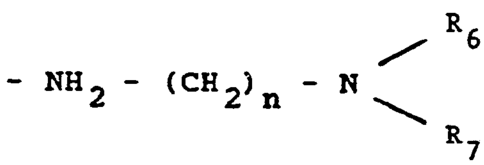

n = 2 ou 3

– $R_6$ et $R_7$ représentant un groupe alcoyle de 1 à 4 atomes de carbone ou un noyau morpholine avec l'atome d'az et le composé de formule

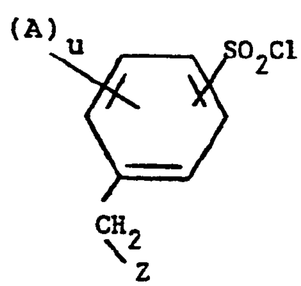

est choisi parmi

– chlorure de (dichloro-2,4 benzyl)-3 chloro-4 benzènesulfonyle
– chlorure de (chloro-4 benzyl)-3 chloro-4 benzènesulfonyle
– chlorure de benzyl-2 chloro-4 benzènesulfonyle
– chlorure de (dichloro-2,4 benzyl)-2 chloro-4 benzènesulfonyle
– chlorure de (chloro-4 benzyl)-2 chloro-4 benzènesulfonyle
– chlorure de benzyl-4 chloro-3 benzènesulfonyle
– chlorure de (dichloro-2,4 benzyl)-4 chloro-3 benzènesulfonyle
– chlorure de (chloro-4 benzyl)-4 chloro-3 benzènesulfonyle
– chlorure de benzyl-3 chloro-4 benzènesulfonyle.

32. Procédé selon la revendication 24, de préparation des composés de formules IV

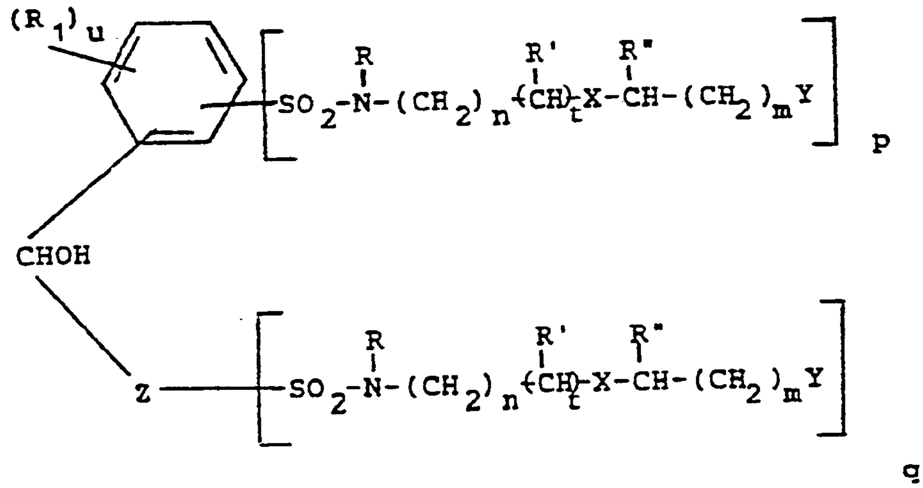

(IV)

dans laquelle $R_1$, R, R', R'', X, Y, Z, m, n, t, p, q et u ont les significations indiquées à la revendication 4, caractérisé en ce que l'on soumet le composé de formule

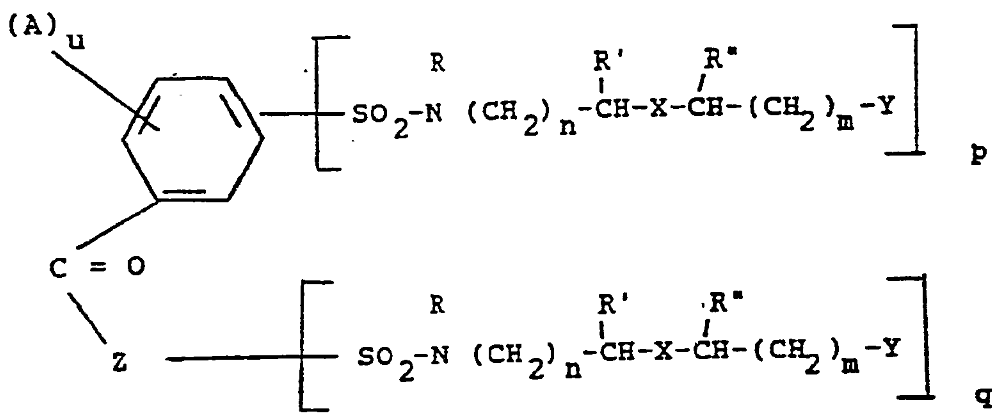

dans laquelle A, X, Y, Z, R, R', R'', m, n, p, q, t et u ont les significations indiquées à la revendication 24, et tel qu'obtenu selon la revendication 25, avant la réduction éventuelle de A et/ou B lorsqu'ils représentent $NO_2$, à une réduction pour transformer C = O en CHOH, à l'aide de borohydrure alcalin.

33. Procédé de préparation selon la revendication 32, caractérisé en ce que l'amino-acide utilisé ou la dialcoylaminoalcoylamine utilisée est choisie parmi

$$- NH_2 - (CH_2)_n - COOH ,$$

n variant de 1 à 11

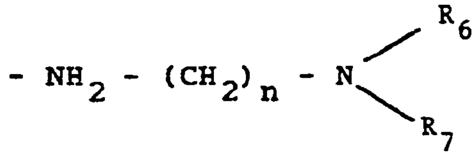

n = 2 ou 3

– $R_6$ et $R_7$ représentant un groupe alcoyle de 1 à 4 atomes de carbone, ou formant un noyau morpholine, avec l'atome d'azote et le sulfochlorure utilisé est choisi parmi

– le chlorure de benzoyl - 2 nitro - 4 benzènesulfonyle
– le chlorure de benzoyl - 2 chloro - 4 benzènesulfonyle
– le chlorure de chloro - 4(chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

34. Les composés selon l'une des revendications 1 à 23 en tant que substance thérapeutique.

35. Composition pharmaceutique caractérisée en ce qu'elle contient à titre de substance active l'un des composés selon l'une quelconque des revendications 1 à 23, en association avec un véhicule pharmaceutiquement acceptable.

36. Composition pharmaceutique destinée au traitement des états inflammatoires, allergiques ou asthmatiques caractérisée en ce qu'elle contient comme substance active l'un des composés selon l'une des revendications 1 à 23.

37. Composition pharmaceutique destinée au traitement de l'insuffisance rénale et de pathologies cardiovasculaires caractérisée en ce qu'elle contient comme substance active l'un des composés selon l'une des revendications 1 à 23.

38. Compositions pharmaceutiques comprenant à titre de substance active l'un au moins des composés selon l'une des revendications 1 à 23 associé à un véhicule pharmaceutiquement acceptable, sous forme de préparations injectables comprenant de 10 à 100 mg de substance active par dose unitaire.

39. Compositions pharmaceutiques comprenant à titre de substance active l'un au moins des composés selon l'une des revendications 1 à 23, associé à un véhicule pharmaceutiquement acceptable, sous forme de présentations destinées à l'administration orale, contenant de 10 à 500 mg, par dose unitaire.

**Revendications pour l'Etat contractant suivant : GR**

1. Composés répondant à la formule I

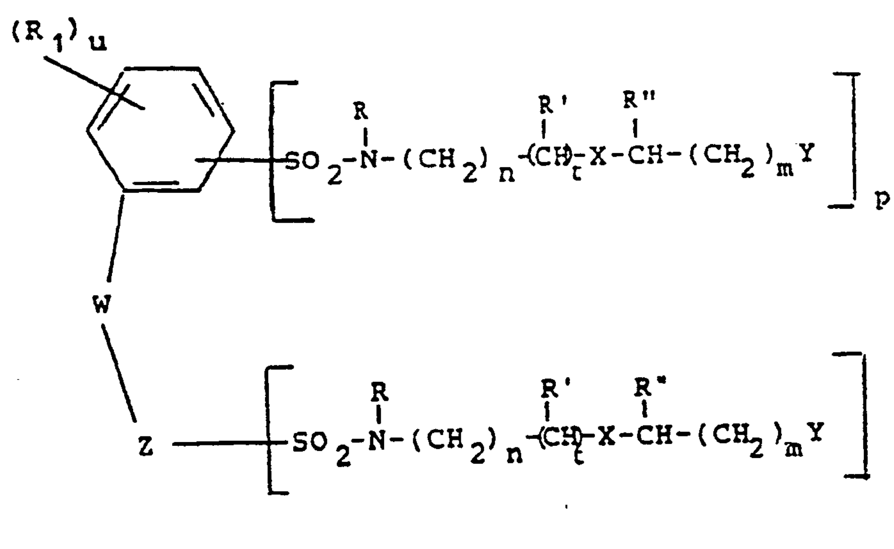

(I)

dans laquelle :
– W représente C = O, $CH_2$ ou CHOH,
– Z représente :

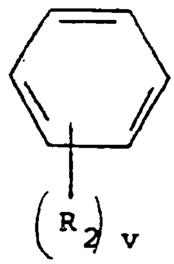

ou

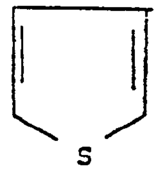

– $R_1$ représente Cl, F, Br, $NO_2$, $NH_2$ , $CF_3$, un radical alcoyle de 1 à 4 atomes de carbone, notamment méthyle ou éthyle, un radical alcoxy de 1 à 4 atomes de carbone, acétamido ou benzamido,
– $R_2$ représente Cl, F, Br, $NO_2$, $NH_2$ , $CF_3$, un radical alcoyle de 1 à 4 atomes de carbone, un radical alcoxy de 1 à 4 atomes de carbone, acétamido ou benzamido,
– R représente H, un radical alcoyle de 1 à 6 atomes de carbone ou un radical benzyle,
– R′ et R″ représente H ou un radical alcoyle de 1 à 4 atomes de carbone,
– X représente $(CH_2)_r$, r valant O ou 1, -CH = CH,

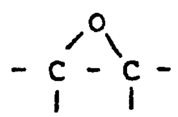 , ou 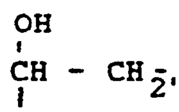

– Y représente $COOR_3$, $R_3$ représentant un hydrogène ou un radical alcoyle de 1 à 4 atomes de carbone,

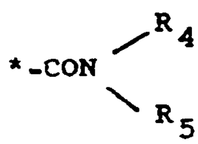 ,

$R_4$ et $R_5$ représentant H ou un radical alcoyle de 1 à 4 atomes de carbone,

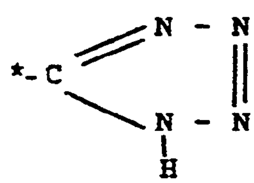 ,

* - C ≡ N,

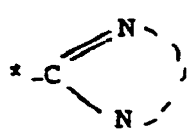 , 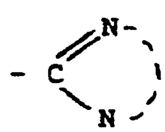

représentant un cycle non aromatique à 5 ou 6 chainons, dans lequel les 2 ou 3 atomes qui entrent dans le cycle sont des atomes de carbone et/ou d'oxygène et/ou d'azote,
* NH2, $NHR_6$, $NR_5R_7$,
$R_6$ et $R_7$ représentant un radical alcoyle de 1 à 4 atomes de carbone, ou susceptible de former avec l'atome d'azote une amine cyclisée non aromatique,
– sous réserve que lorsque Y représente - $NH_2$, X soit différent de

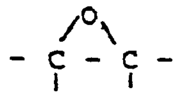

- u est un nombre entier de O à 2
- v est un nombre entier de O à 2
- p vaut O ou 1
- q vaut O ou 1, p + q étant égal ou supérieur à 1
- n est un nombre entier variant de O à 1O
- m est un nombre entier variant de O à 1O
- t vaut O ou 1,
- le nombre total d'atomes de carbone de la chaîne

$$- (CH_2)_n -(CH)_t - X - CH - (CH_2)_m\ Y,$$

variant de 2 à 2O,

ainsi que leurs sels physiologiquement acceptables.

2. Composé selon la revendication 1, répondant à la formule II suivante

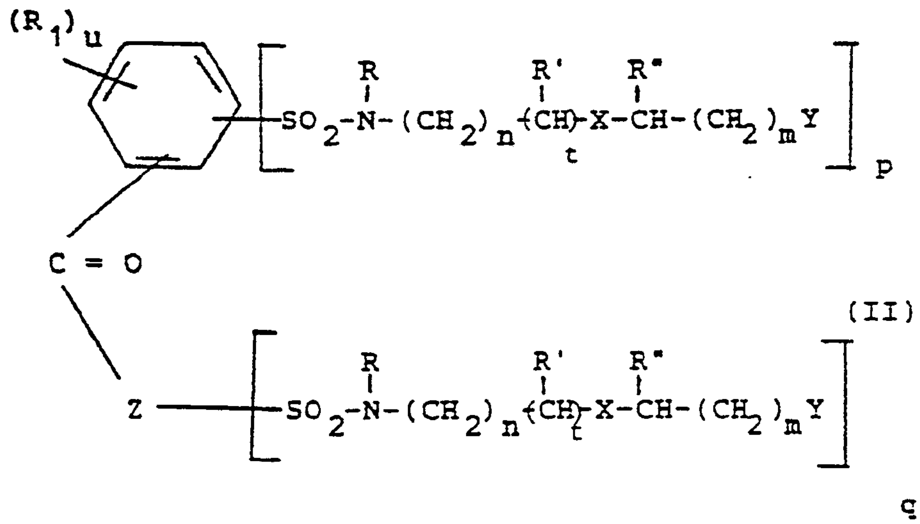

(II)

dans laquelle $R_1$, R, R′, R″, X, Y, Z, m, n, t, p, q et u ont les significations indiquées à la revendication 1.

3. Composé selon la revendication 1, répondant à la formule III suivante

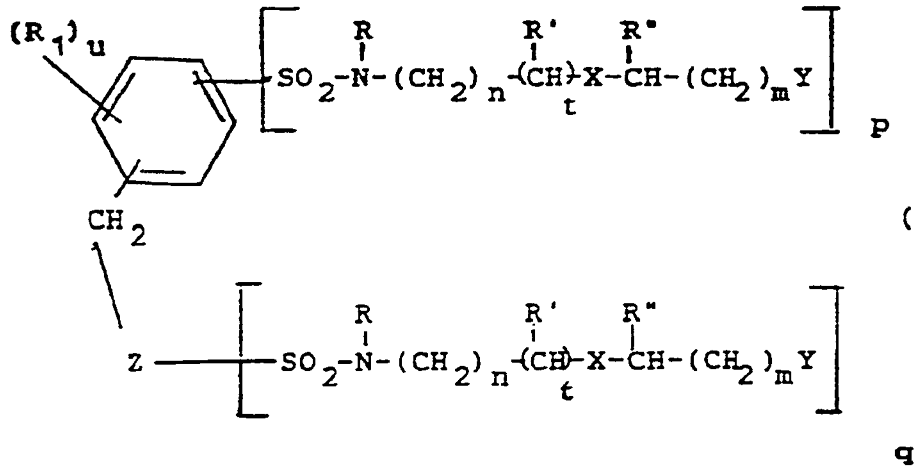

(III)

dans laquelle $R_1$, R, R', R", X, Y, Z, m, n, t, p, q et u ont les significations indiquées à la revendication 1.

4. Composé selon la revendication 1, répondant à la formule IV suivante

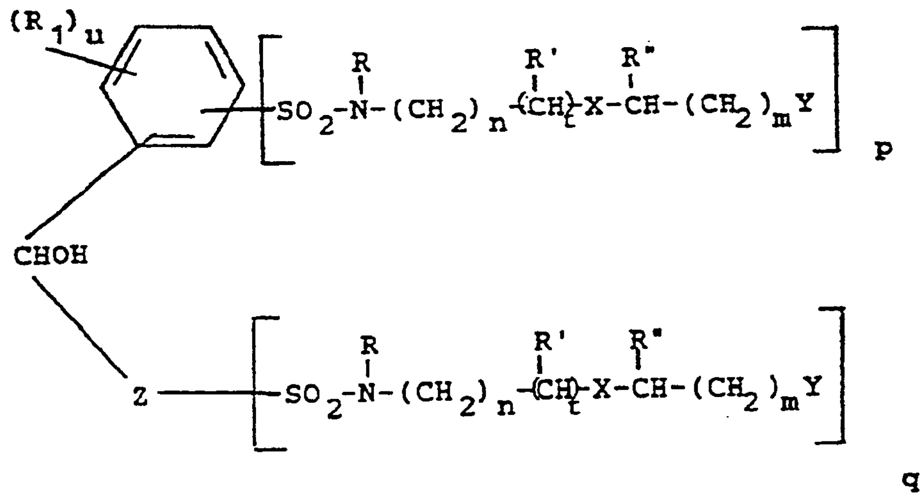

(IV)

dans laquelle $R_1$, R, R', R", X, Y, Z, m, n, t, p, q et u ont les significations indiquées à la revendication 1.

5. Composé selon la revendication 2, répondant à la formule V suivante

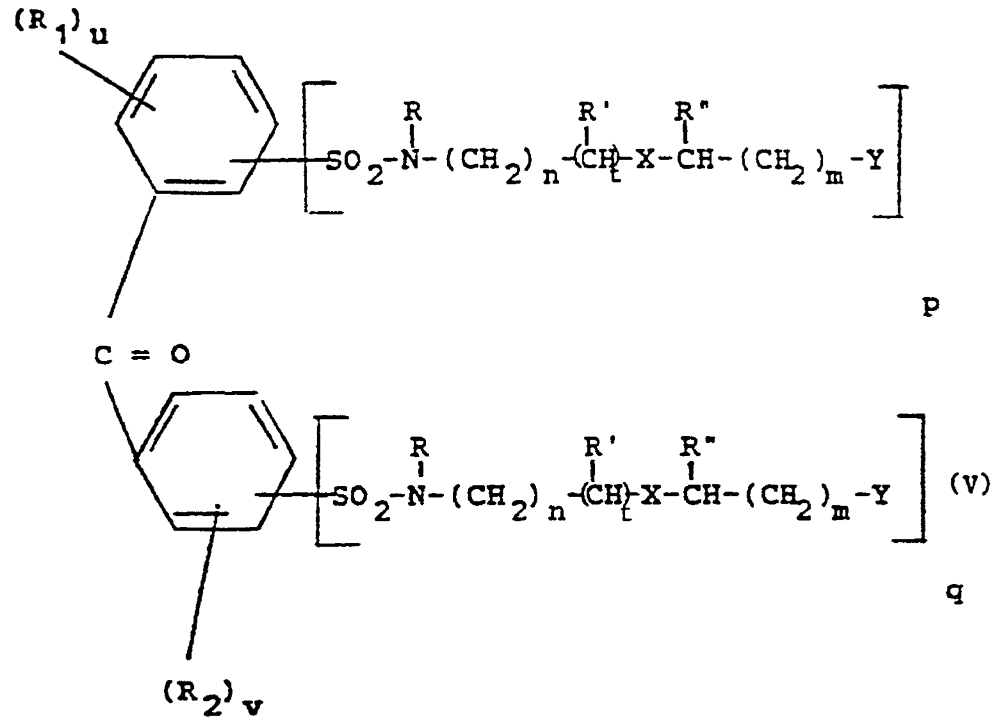

(V)

dans laquelle $R_1$, $R_2$,R, R', R", X, Y, Z, m, n, p, q, t, u et v ont les significations indiquées à la revendication 1.

6. Composé selon la revendication 5, répondant à la formule V dans laquelle

- Y représente $COOR_3$,

$R_3$ représentant H ou un radical alcoyle de 1 à 4 atomes de carbone.

7. Composé selon la revendication 5, répondant à la formule V dans laquelle

- Y représente $NH_2$ , $NHR_6$ ou $NR_6R_7$ , $R_6$ et $R_7$ ayant les significations indiquées à la revendication 1.

8. Composé selon la revendication 5, répondant à la formule V dans laquelle p ou q vaut O.

9. Composé selon la revendication 8, caractérisé en ce que

– u + v supérieur ou égal à 1,

– le nombre total d'atomes de carbone de la chaîne

$$(CH_2)_n - (CH)_t - X - CH - (CH_2)_m - Y$$

est égal ou supérieur à 5.

10. Composé selon la revendication 5, répondant à la formule V, dans laquelle

– p = 1,

– q = 1,

– u + v supérieur ou égal à 1,

– le nombre total d'atomes de carbone de la chaîne

$$(CH_2)_n - (CH)_t - X - CH - (CH_2)_m - Y$$

est égal ou supérieur à 5.

11. Composé selon la revendication 1, répondant à la formule VI suivante

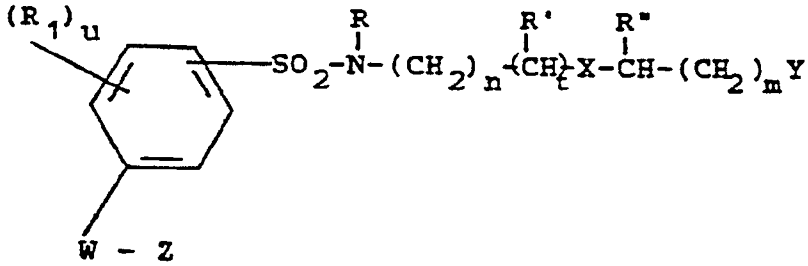

(VI)

dans laquelle $R_1$, R, R′, R″, X, Y, Z, W, m, n, u et t, ont les significations indiquées à la revendication 1.

12. Composé selon la revendication 11, répondant à la formule VII suivante

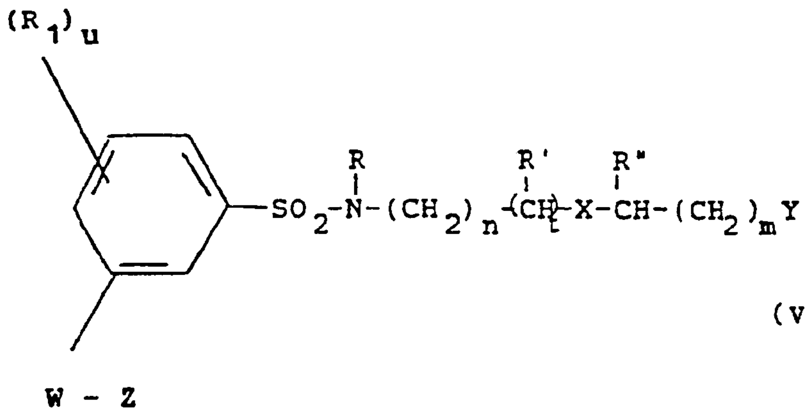

(VII)

dans laquelle $R_1$, R, R′, R″, X, Y, Z, W, m, n, u et t ont les significations indiquées à la revendication 1.

13. Composé selon la revendication 12, répondant à la formule VII
dans laquelle
- Y représente $COOR_3$, $R_3$ représentant H ou un radical alcoyle de 1 à 4 atomes de carbone.

14. Composé selon la revendication 13, répondant à la formule VIII suivante

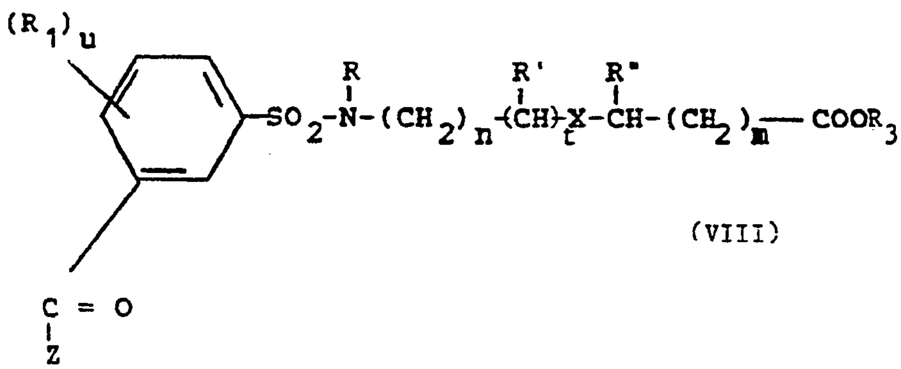

(VIII)

dans laquelle $R_1$, $R_3$, R, R′, R″, X, Z, m, n, u et t ont les significations indiquées à la revendication 1.

15. Composé selon la revendication 13, répondant à la formule IX suivante

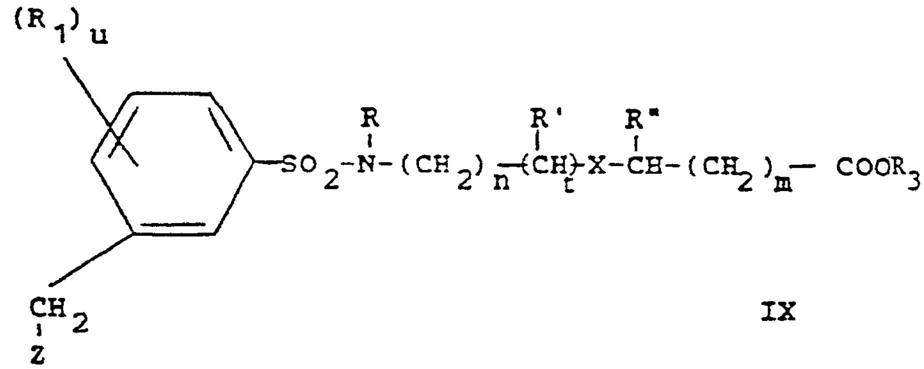

IX

dans laquelle $R_1$, $R_3$, R, R′, R″, X, Z, m, n, u et t ont les significations indiquées à la revendication 1.

16. Composé selon la revendication 13 répondant à la formule X suivante

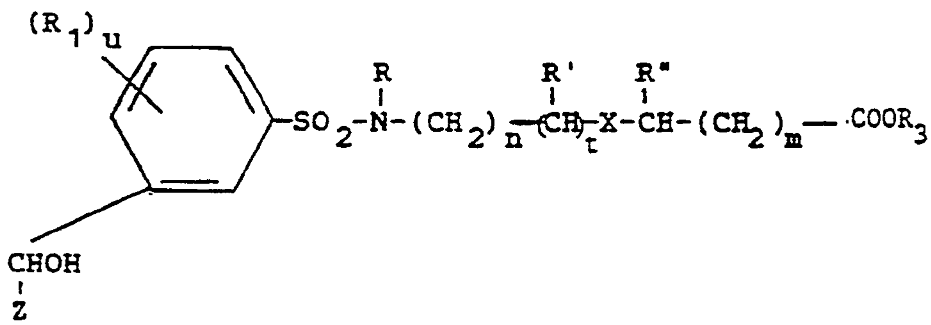

X

dans laquelle $R_1$, $R_3$, R, R′, R″, X, Z, m, n, u et t ont les significations indiquées à la revendication 1.

17. Composé selon la revendication 14, répondant à la formule VIII dans laquelle le nombre total d'atomes de carbone de la chaîne

$$(CH_2)_n - (CH)_t - X - CH - (CH_2)_m - Y$$

est égal ou supérieur à 5.

18. Composé selon la revendication 15, répondant à la formule IX dans laquelle le nombre total d'atomes de carbone de la chaîne

$$(CH_2)_n - (CH)_t - X - CH - (CH_2)_m - Y$$

est égal ou supérieur à 5.

19. Composé selon la revendication 16, répondant à la formule X dans laquelle le nombre total d'atomes de carbone de la chaîne

$$(CH_2)_n - (CH)_t - X - CH - (CH_2)_m - Y$$

est égal ou supérieur à 5.

20. Composé selon la revendication 13, répondant à la formule XI suivante

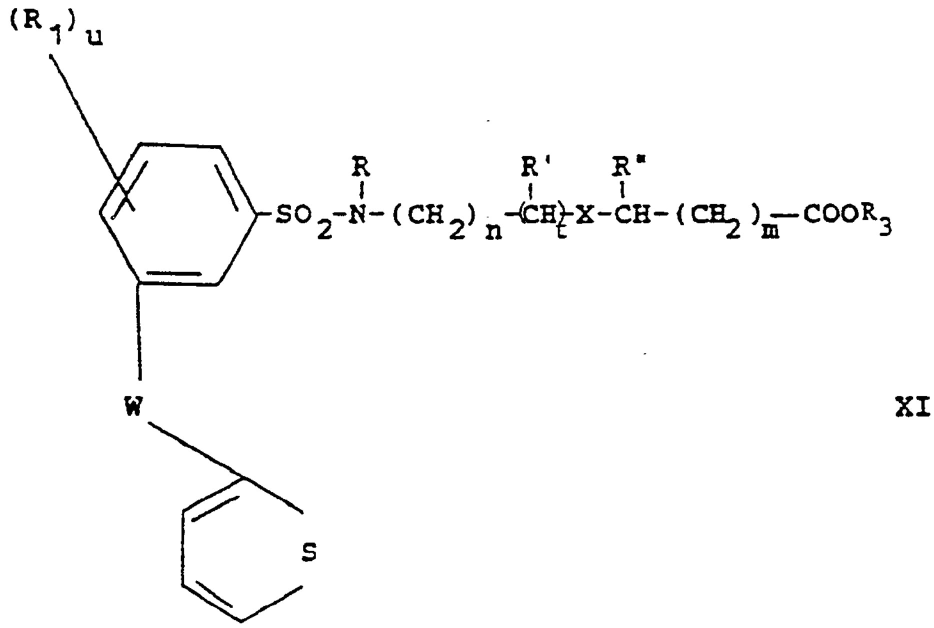

XI

- dans laquelle
le nombre total d'atomes de carbone de la chaîne

$$(CH_2)_n-(CH)_t-X-CH-(CH_2)_m-Y$$

est égal ou supérieur à 5, et dans laquelle
$R_1$, $R_3$, R, R', R'', X, W, m, n, t et u ont les significations indiquées à la revendication 1.

21. Composé selon la revendication 11, répondant à la formule VIbis suivante :

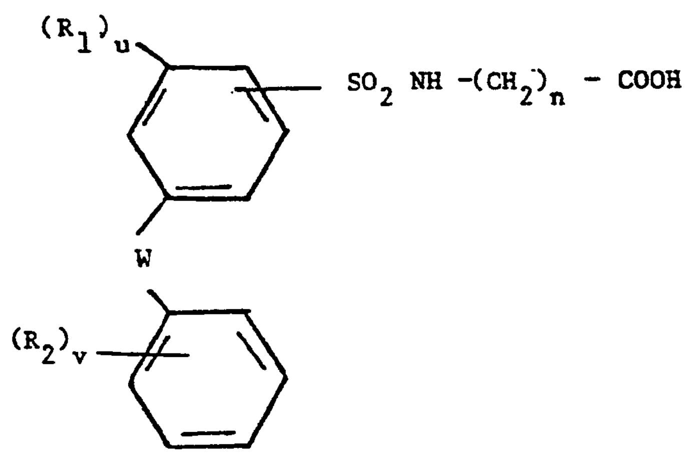

dans laquelle $R_1$ et $R_2$ représentent un halogène
u varie de O à 2, v varie de O à 2,
W représente C = O, CHOH, $CH_2$,
n vaut de 3 à 11

22. Composé selon la revendication 11 répondant à la formule VIter suivante :

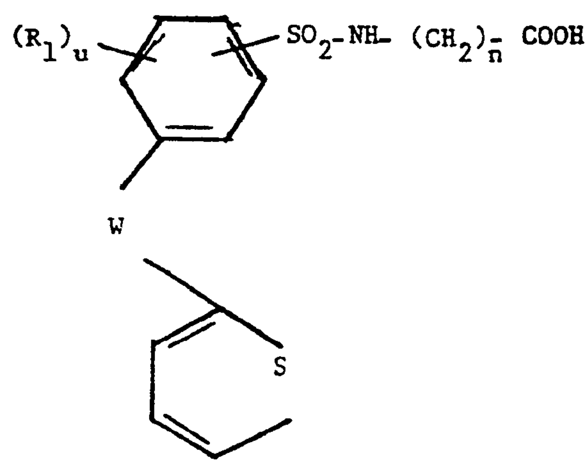

dans laquelle $R_1$ représente un halogène
u vaut O à 2
W représente C=O, CHOH, $CH_2$
n vaut 3 à 11.

23. Composés selon la revendication 1, répondant aux formules suivantes

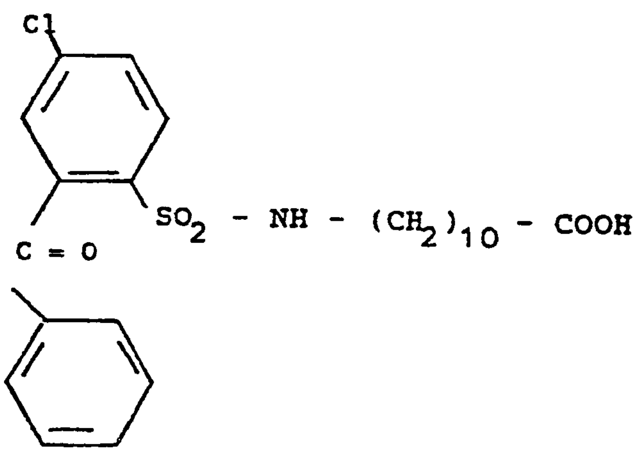

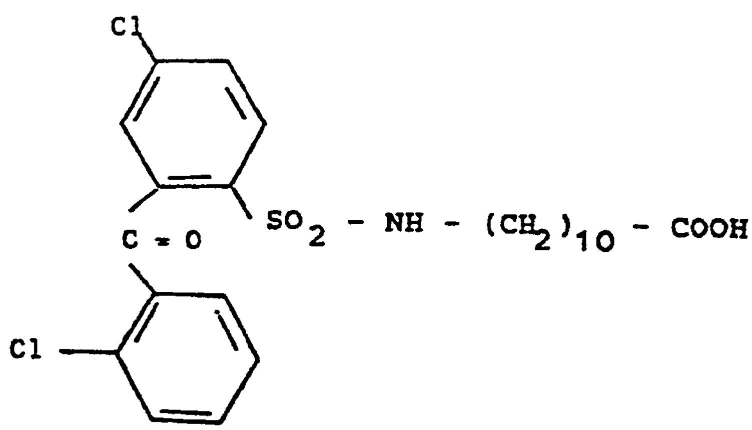

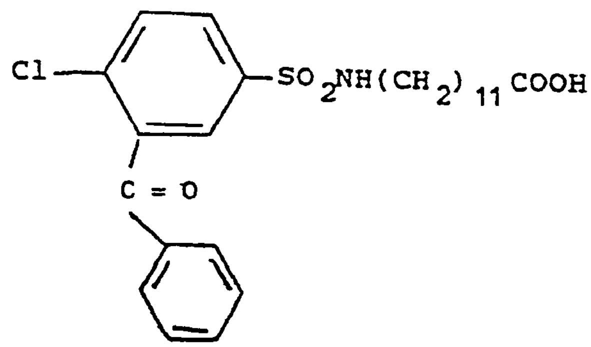

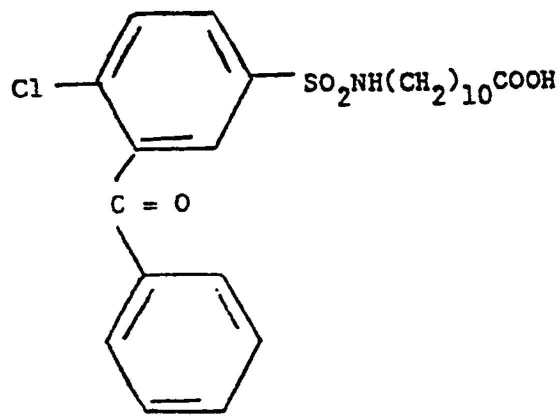

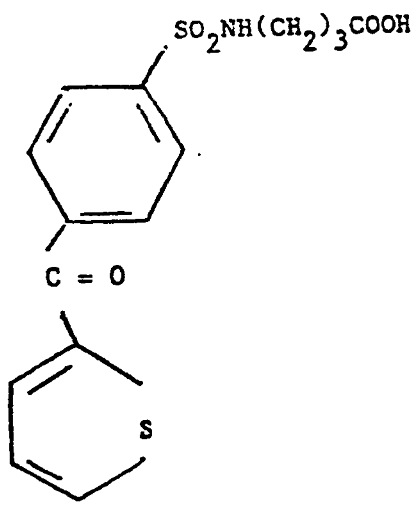

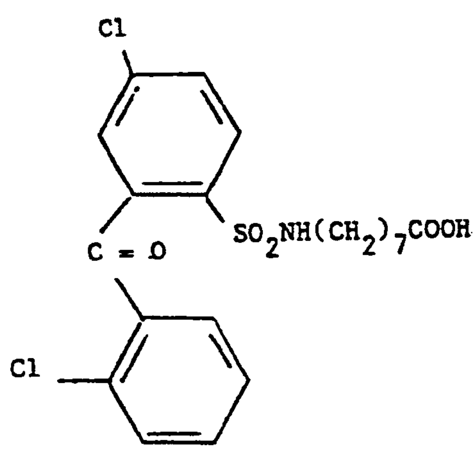

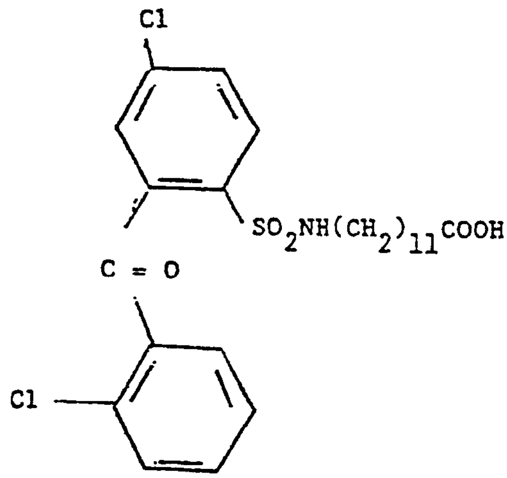

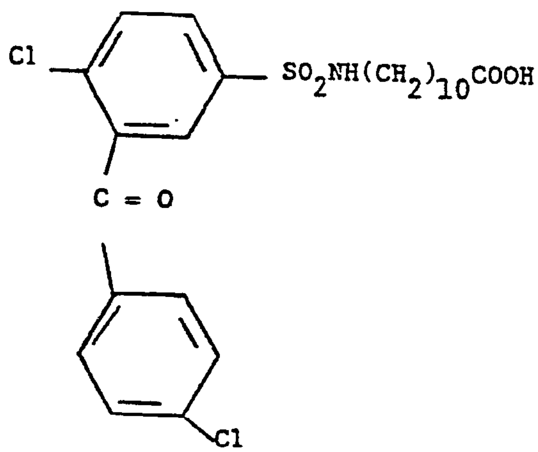

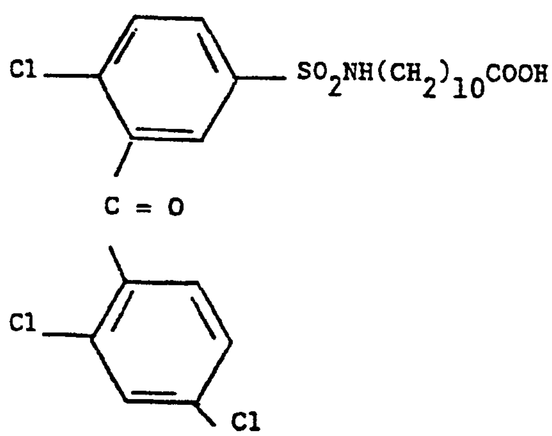

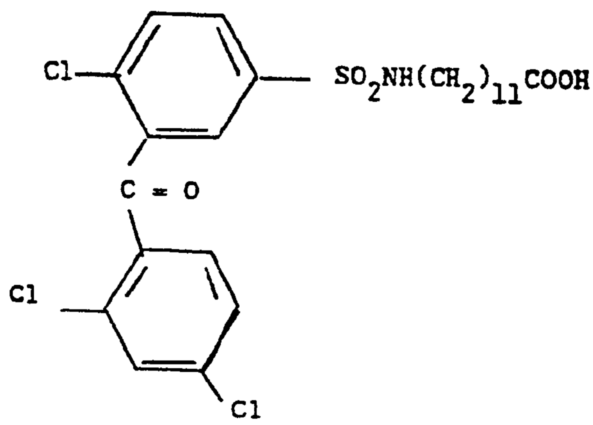

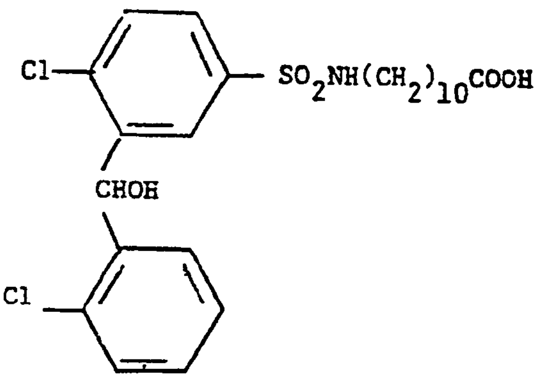

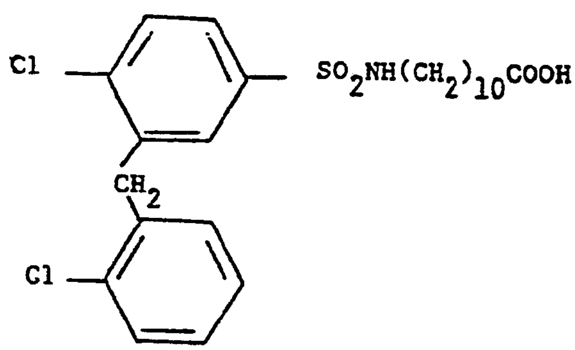

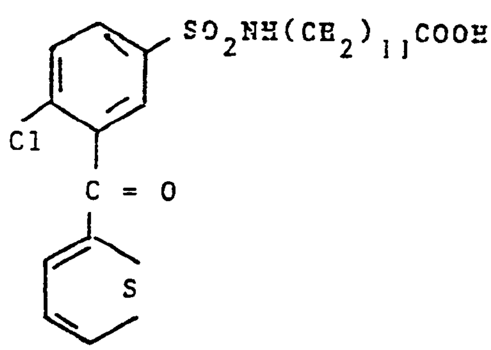

24. Procédé de préparation des composés de formule I, selon la revendication 1, caractérisé en ce que l'on soumet un composé de formule

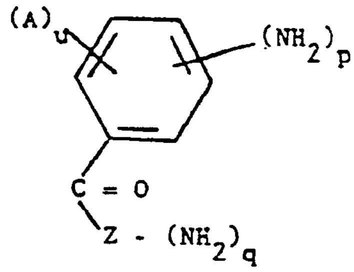

dans laquelle

– A a les significations indiquées pour $R_1$, à l'exception de $NH_2$, c'est-à-dire A représente Cl, F, Br, $NO_2$, $CF_3$, un radical alcoyle de 1 à 4 atomes de carbone, un radical alcoxy de 1 à 4 atomes de carbone, acétamido ou benzamido,

– Z représente

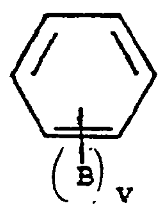

ou

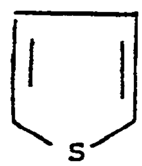

– B a les significations indiquées pour $R_2$, à l'exception de $NH_2$, c'est-à-dire B représente Cl, $NO_2$, $CF_3$, un radical alcoyle de 1 à 4 atomes de carbone, un radical alcoxy de 1 à 4 atomes de carbone, acétamido ou benzamido et

– u représente un nombre entier de O à 2,

– v représente un nombre entier de O à 2,

– p = O ou 1,

– q = O ou 1, p + q étant égal ou supérieur à 1,

. éventuellement à une réduction, notamment à l'aide de chlorure d'aluminium et d'hydrure d'aluminium et de lithium pour réduire - C = O en $CH_2$, et obtenir le composé de formule

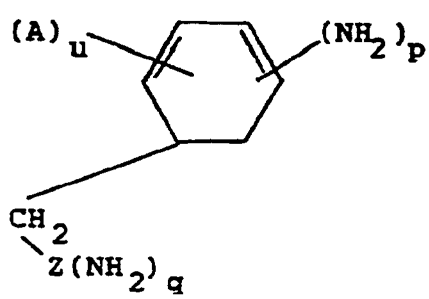

dans laquelle A, Z, u, p et q ont les significations indiquées ci-dessus,

. on soumet l'un des composés aminés de formule indiquée ci-dessus à la réaction de Sandmeyer, pour obtenir un sulfohalogénure de formule :

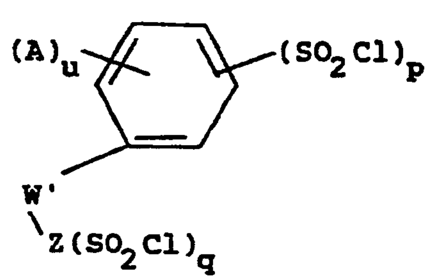

dans laquelle A, Z, u, p et q ont les significations indiquées ci-dessus et W' représente C = O ou $CH_2$,
. on fait réagir le sulfohalogénure, notamment le sulfochlorure, de formule indiquée ci-dessus, sur un ou deux composés de formule

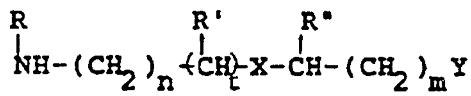

dans laquelle
– R représente H, un radical alcoyle de 1 à 6 atomes de carbone ou un radical benzyle,
– R' et R'' représente H ou un radical alcoyle de 1 à 4 atomes de carbone,
– X représente $(CH_2)_r$, r valant O ou 1 ou -CH = CH,

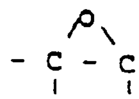 , ou 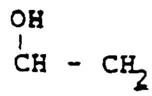 ,

– Y représente $COOR_3$, $R_3$ représentant H ou un radical alcoyle de 1 à 4 atomes de carbone,

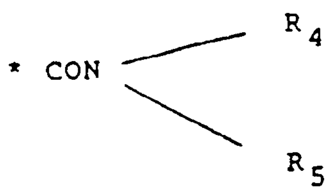

, $R_4$ et $R_5$ représentant H ou un radical alcoyle de 1 à 4 atomes de carbone,

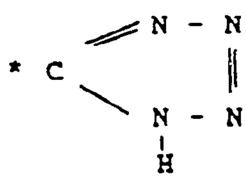 ,

- C ≡ N,

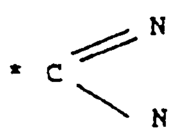 , 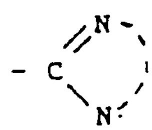

représentant un cycle non aromatique à 5 ou 6 chainons, dans lequel les 2 ou 3 atomes qui entrent dans le cycle sont des atomes de carbone et/ou d'oxygène et/ou d'azote,
* $NH_2$, $NHR_6$, $NR_6R_7$,
$R_6$ et $R_7$ représentant un radical alcoyle de 1 à 4 atomes de carbone, ou susceptible de former avec l'atome d'azote une amine cyclisée non aromatique,
– sous réserve que lorsque Y représente $-NH_2$, X soit différent de

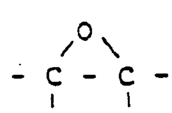

– n est un nombre entier variant de O à 1O

- m est un nombre entier variant de O à 1O
- t vaut O ou 1,
- le nombre total d'atomes de carbone de la chaîne

$$-(CH_2)_n-(\overset{|}{C}H)_t- X - \overset{|}{C}H - (CH_2)_m\ Y,$$

variant de 2 à 2O,
pour obtenir le composé de formule

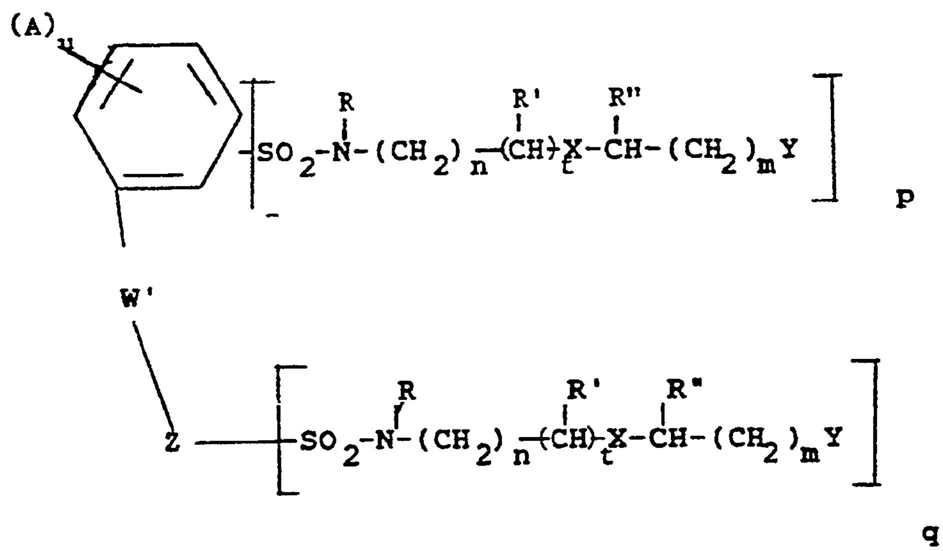

dans laquelle A, Z, W', R, R', R", X, Y, m, n, t, q et u ont les significations indiquées ci-dessus :
. et dans le cas où W' représente C = O, on soumet éventuellement le composé ci-dessus à une réduction, notamment à l'aide de borohydrure alcalin, tel que le borohydrure de sodium, pour réduire C = O en CHOH,
. on effectue ensuite éventuellement une réduction lorsque A et/ou B représente $NO_2$.
25. Procédé selon la revendication 24 de préparation des composés de formule II

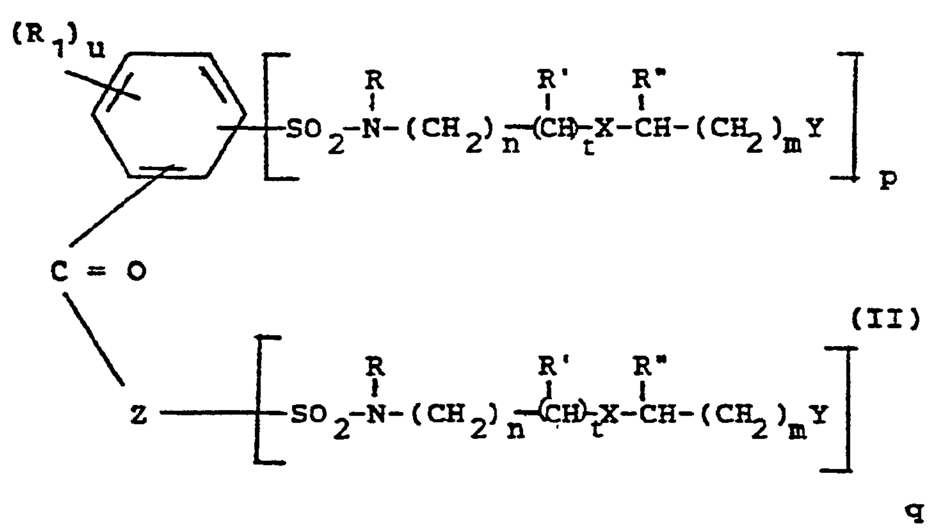

(II)

dans laquelle $R_1$, R, R', R", X, Y, Z, m, n, p, q, u et t ont les significations indiquées à la revendication 2, caractérisé en ce que l'on fait réagir un sulfohalogénure, notamment un sulfochlorure de formule

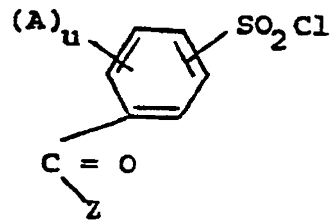

dans laquelle A, Z et u ont les significations indiquées à la revendication 24 ou un disulfohalogénure, notamment un disulfochlorure de formule

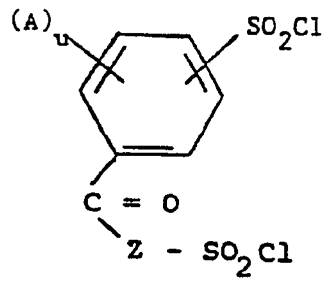

dans laquelle A, u et Z ont les significations précédemment indiquées sur un ou deux composés de formule

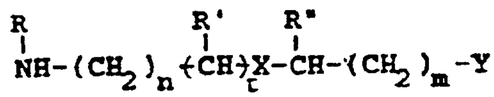

dans laquelle R, R', R'', n, m, t, X et Y ont les significations indiquées à la revendication 24,
-suivi éventuellement d'une réduction lorsque A et/ou B représente $NO_2$.

26. Procédé selon la revendication 25, caractérisé en ce que l'on faite réagir un sulfochlorure de formule

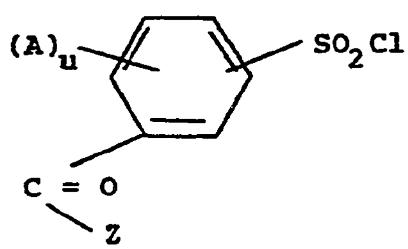

dans laquelle A, u, Z ont les significations indiquées à la revendication 25, ou un disulfochlorure de formule

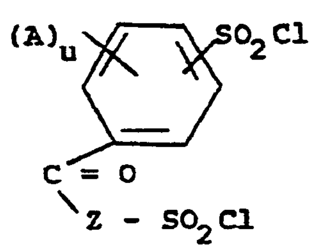

dans lequel A, u, Z ont les significations indiquées à la revendication 25, sur un ou deux amino-acides de formule

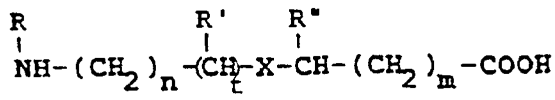

dans laquelle R, R', R'', n, m, t et X ont les significations indiquées ci-dessus à la revendication 25.

27. Procédé selon la revendication 25, caractérisé en ce que

– l'on estérifie un composé tel qu'obtenu selon la revendication 26, et dans lequel Y représente COOH, en un composé de formule II, dans lequel Y représente $COOR_3$, $R_3$ représentant un radical alcoyle de 1 à 4 atomes de carbone

– ou en ce que l'on transforme un composé tel qu'obtenu selon la revendication 26, en un composé de formule II dans lequel Y représente

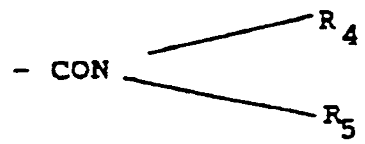

$R_4$, $R_5$ représentant H ou un radical alcoyle de 1 à 4 atomes de carbone,

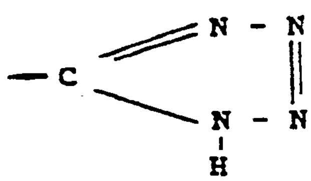

, $- C\equiv N$

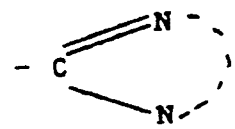

28. Procédé selon la revendication 25, caractérisé en ce que l'on fait réagir un sulfochlorure de formule

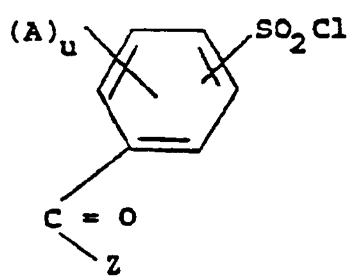

dans laquelle :

– A a les significations indiquées pour $R_1$, à l'exception de $NH_2$, c'est-à-dire Cl, F, Br, $NO_2$, $CF_3$, un radical alcoyle de 1 à 4 atomes de carbone, un radical alcoxy de 1 à 4 atomes de carbone, acétamido ou benzamido,

– Z représente :

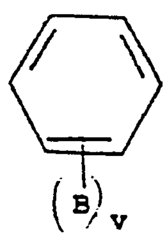

ou

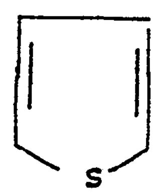

– B a les significations indiquées pour $R_2$, à l'exception de $NH_2$, c'est-à-dire B représente Cl, F, Br, $NO_2$, $CF_3$, un radical alcoyle de 1 à 4 atomes de carbone, un radical alcoxy de 1 à 4 atomes de carbone, acétamido ou benzamido,

– u represente un nombre entier de O à 2

– v représente un nombre entier de O à 2 ou un disulfohalogénure, notamment disulfochlorure de formule

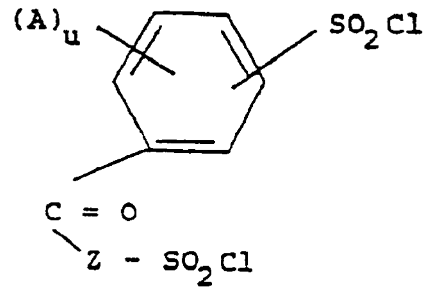

dans laquelle A, u, Z ont les significations indiquées ci-dessus ;
sur une aminoalcoylamine de formule :

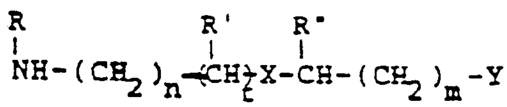

dans laquelle :

– R représente H, un radical alcoyle de 1 à 6 atomes de carbone ou un radical benzyle,
– R′ et R″ représentent H ou un radical alcoyle de 1 à 4 atomes de carbone,
– n représente un nombre entier variant de O à 1O,
– m représente un nombre entier variant de O à 1O,
– t vaut O ou 1
– Y représente $NH_2$, $NHR_6$, $NR_6R_7$, $R_6$ et $R_7$ représentent un radical alcoyle de 1 à 4 atomes de carbone, ou forment avec l'azote une amine cyclisée non aromatique.
– X a la signification donnée dans la formule I

29. Procédé de préparation selon la revendication 25, caractérisé en ce que l'amino-acide utilisé ou la dialcoylaminoalcoylamine utilisée est choisi respectivement parmi :

$$- NH_2 - (CH_2)_n - COOH ,$$

n variant de 1 à 11

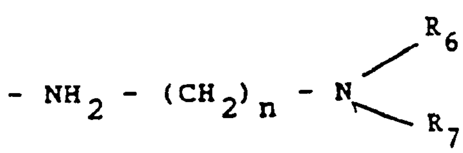

n = 2 ou 3

– $R_6$ et $R_7$ représentant un alcoyle de 1 à 4 atomes de carbone ou un noyau morpholine avec l'atome d'azote et le sulfochlorure utilisé est choisi respectivement parmi :
– le chlorure de benzoyl - 2 nitro - 4 benzènesulfonyle
– le chlorure de benzoyl - 2 chloro - 4 benzènesulfonyle
– le chlorure de chloro - 4(chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

3O. Procédé selon la revendication 24 de préparation des composés de formule III

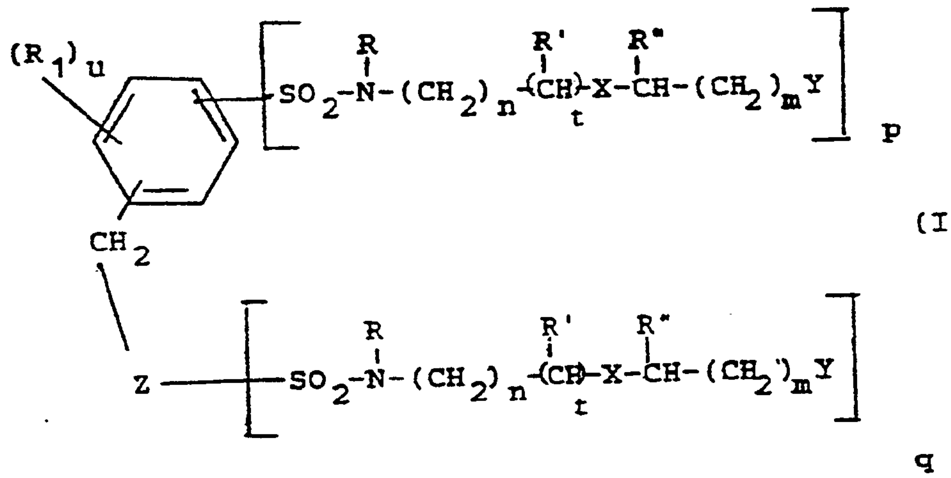

(III)

dans laquelle $R_1$, R, R′, R″, X, Y, Z, m, n, t, p, q et u ont les significations indiquées à la revendication 3 caractérisé en ce que l'on soumet le composé de formule

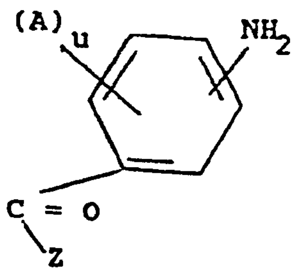

ou de formule

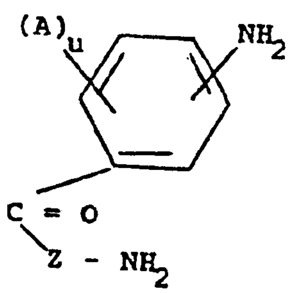

dans laquelle A a la signification indiquée à la revendication 24, à une réduction, pour réduire C = O en $CH_2$ et obtenir le composé de formule

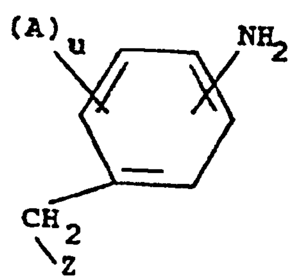

ou

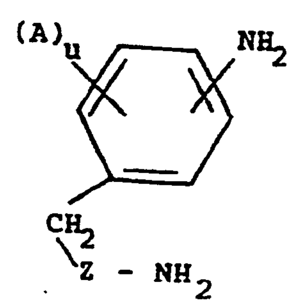

– on soumet l'un de ces composés à la réaction de Sandmeyer, pour obtenir les composés de formule :

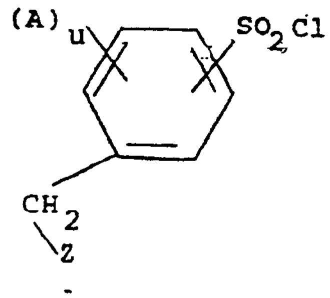

ou

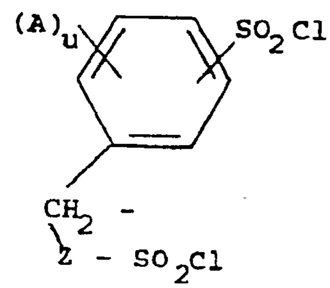

– on condense le sulfohalogénure ou le disulfohalogénure de formule indiquée ci-dessus, sur un composé de formule

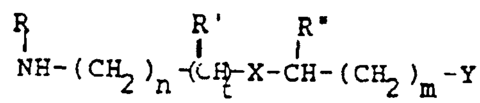

dans laquelle R, R', R", X, Y, n, m et t ont les significations indiquées à la revendication 24 suivi éventuellement d'une réduction lorsque A et/ou B représente $NO_2$.

31. Procédé de préparation selon la revendication 3O, caractérisé en ce que l'amino-acide utilisé ou la dialcoylaminoalcoylamine utilisée est choisie parmi

$$- NH_2 -(CH_2)_n - COOH ,$$

n variant de 1 à 11

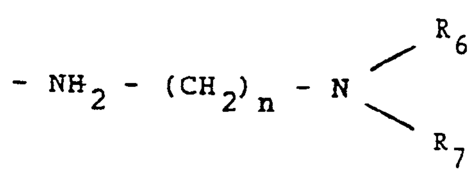 n = 2 ou 3

– $R_6$ et $R_7$ représentant un groupe alcoyle de 1 à 4 atomes de carbone ou un noyau morpholine avec l'atome d'azote et le composé de formule

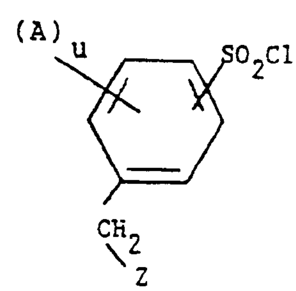

est choisi parmi

– chlorure de (dichloro-2,4 benzyl)-3 chloro-4 benzènesulfonyle
– chlorure de (chloro-4 benzyl)-3 chloro-4 benzènesulfonyle
– chlorure de benzyl-2 chloro-4 benzènesulfonyle
– chlorure de (dichloro-2,4 benzyl)-2 chloro-4 benzènesulfonyle
– chlorure de (chloro-4 benzyl)-2 chloro-4 benzènesulfonyle
– chlorure de benzyl-4 chloro-3 benzènesulfonyle
– chlorure de (dichloro-2,4 benzyl)-4 chloro-3 benzènesulfonyle

- chlorure de (chloro-4 benzyl)-4 chloro-3 benzènesulfonyle
- chlorure de benzyl-3 chloro-4 benzènesulfonyle.

32. Procédé selon la revendication 24, de préparation des composés de formule IV

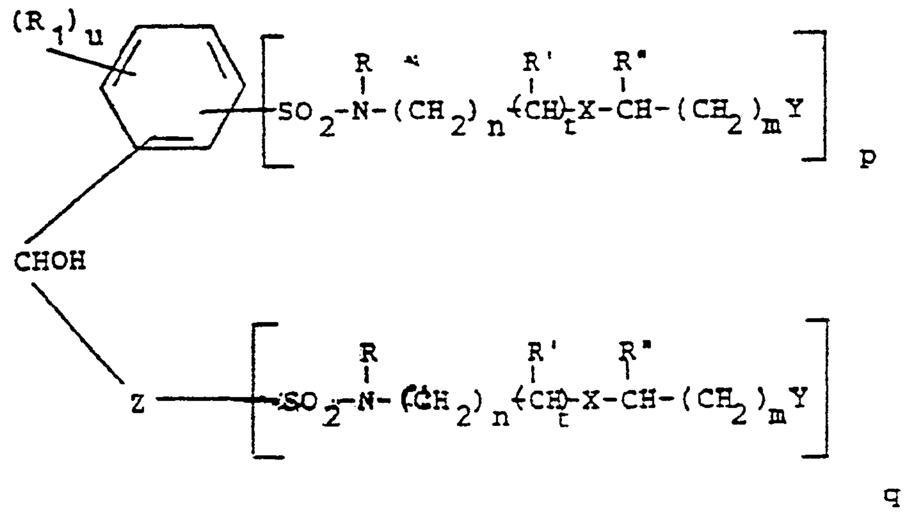

(IV)

dans laquelle $R_1$, R, R', R", X, Y, Z, m, n, t, p, q et u ont les significations indiquées à la revendication 4, caractérisé en ce que l'on soumet le composé de formule

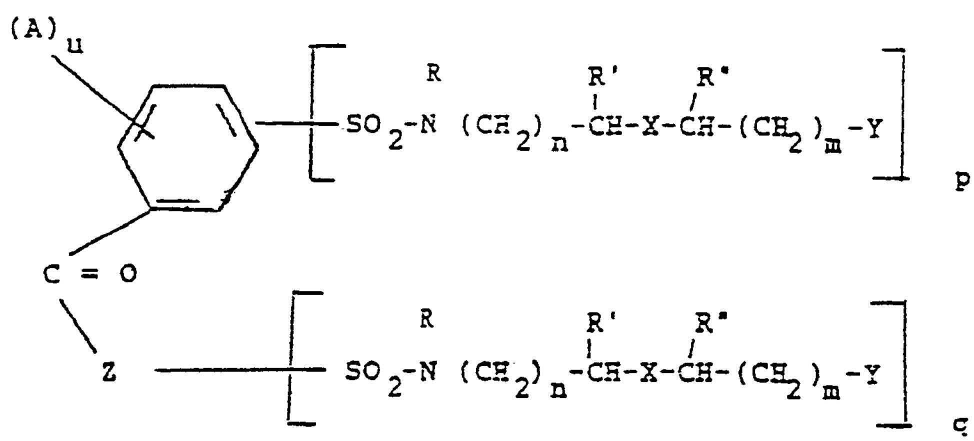

dans laquelle A, X, Y, Z, R, R', R", m, n, p, q t et u les significations indiquées à la revendication 24, et tel qu'obtenu selon la revendication 25, avant réduction éventuelle de A et/ou B lorsqu' représentent $NO_2$, à une réduction pour transformer C = O en CHOH, à l'aide de borohydrure alcalin.

33. Procédé de préparation selon la revendi- cation 32, caractérisé en ce que l'amino-acide utilisé ou la dialcoylaminoalcoylamine utilisée est choisie parmi

$$- NH_2 -(CH_2)_n- COOH ,$$

n variant de 1 à 11

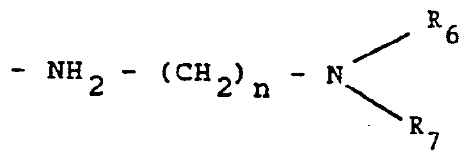

$n = 2$ ou $3$

– $R_6$ et $R_7$ représentant un groupe alcoyle de 1 à 4 atomes de carbone, ou formant un noyau morpholine, avec l'atome d'azote et le sulfochlorure utilisé est choisi parmi
– le chlorure de benzoyl - 2 nitro - 4 benzènesulfonyle
– le chlorure de benzoyl - 2 chloro - 4 benzènesulfonyle
– le chlorure de chloro - 4(chloro - 2 benzoyl) - 2 benzènesulfonyle
– le chlorure de benzoyl - 2 benzènesulfonyle
– le chlorure de (chloro - 4 benzoyl) - 2 benzènesulfonyle
– le chlorure de benzoyl - 4 benzènesulfonyle
– le chlorure de benzoyl - 3 benzènesulfonyle
– le chlorure de (dichloro-2,4 benzoyl)-3 chloro-4 benzènesulfonyle
– le chlorure de benzoyl - 3 chloro - 4 benzènesulfonyle
– le chlorure de (chloro-4 benzoyl)-3 chloro-4 benzènesulfonyle
– le chlorure de benzoyl - 4 chloro - 3 benzènesulfonyle
– le chlorure de chloro-4 (thenoyl-2)-3 benzènesulfonyle.

**Revendications pour l'Etat contractant suivant : ES**

1. Procédé de préparation de composés répondant à la formule I

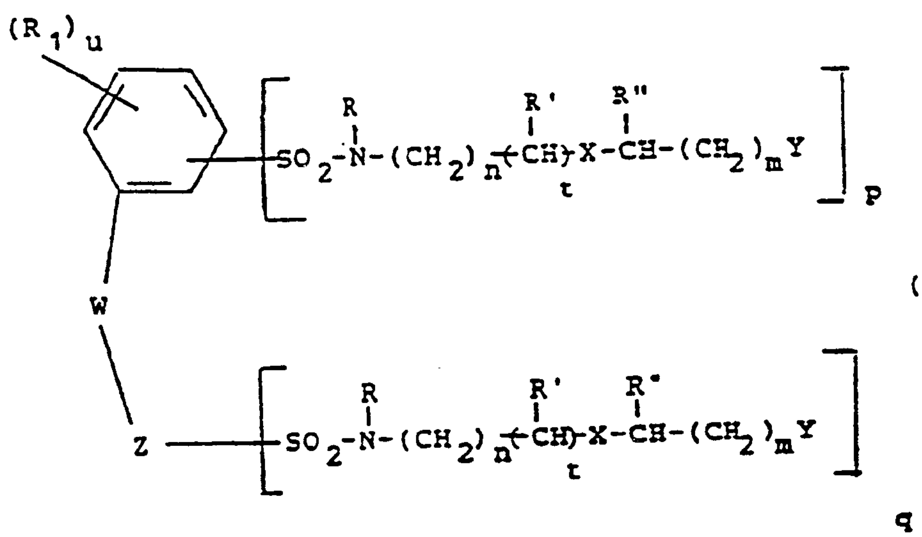

(I)

dans laquelle :
– W représente C = O, $CH_2$ ou CHOH,
– Z représente :

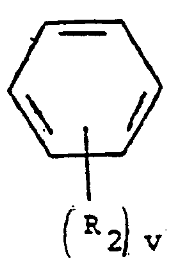

ou

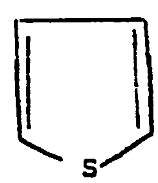

– $R_1$ représente Cl, F, Br, $NO_2$, $NH_2$, $CF_3$, un radical alcoyle de 1 à 4 atomes de carbone, notamment méthyle ou éthyle, un radical alcoxy de 1 à 4 atomes de carbone, acétamido ou benzamido,
– $R_2$ représente Cl, F, Br, $NO_2$, $NH_2$, $CF_3$, un radical alcoyle de 1 à 4 atomes de carbone, un radical alcoxy de 1 à 4 atomes de carbone, acétamido ou benzamido,
– R représente H, un radical alcoyle de 1 à 6 atomes de carbone ou un radical benzyle,
– R' et R" représentent H ou un radical alcoyle de 1 à 4 atomes de carbone,
– X représente $(CH_2)_r$, r valant O ou 1, -CH = CH,

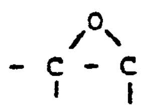 , ou 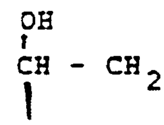

– Y représente $COOR_3$, $R_3$ représentant un hydrogène ou un radical alcoyle de 1 à 4 atomes de carbone,

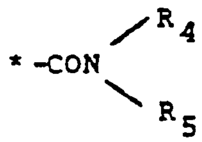 ,

$R_4$ et $R_5$ représentant H ou un radical alcoyle de 1 à 4 atomes de carbone,

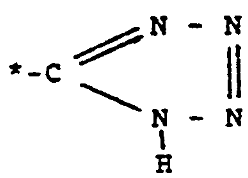 ,

- C ≡ N,

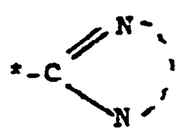 , 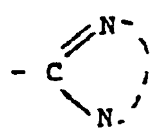

représentant un cycle non aromatique à 5 ou 6 chainons, dans lequel les 2 ou 3 atomes qui entrent dans le cycle sont des atomes de carbone et/ou d'oxygène et/ou d'azote,
* $NH_2$, $NHR_6$, $NR_6R_7$,
$R_6$ et $R_7$ représentant un radical alcoyle de 1 à 4 atomes de carbone, ou susceptible de former avec l'atome d'azote une amine cyclisée non aromatique,
– sous réserve que lorsque Y représente $-NH_2$, X soit différent de

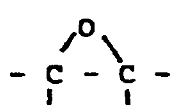

– u est un nombre entier de O à 2
– v est un nombre entier de O à 2
– p vaut O ou 1
– q vaut O ou 1, p + q étant égal ou supérieur à 1
– n est un nombre entier variant de O à 1O
– m est un nombre entier variant de O à 1O
– t vaut O ou 1,
– le nombre total d'atomes de carbone de la chaîne

$$-(CH_2)_n-(CH)_t-X-CH-(CH_2)_m\ Y,$$

variant de 2 à 20,
caractérisé en ce que l'on soumet un composé de formule

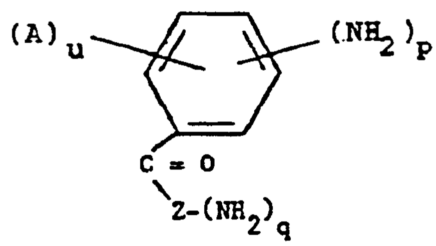

dans laquelle
– A a les significations indiquées pour $R_1$, à l'exception de $NH_2$, c'est-à-dire A représente Cl, F, Br, $NO_2$, $CF_3$, un radical alcoyle de 1 à 4 atomes de carbone, un radical alcoxy de 1 à 4 atomes de carbone, acétamido ou benzamido, et de préférence Cl ou $NO_2$,
– Z représente

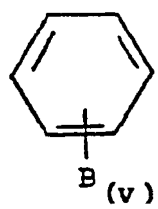

ou

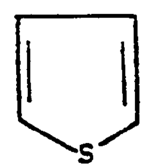

– B a les significations indiquées pour $R_2$, à l'exception de $NH_2$, c'est-à-dire B représente Cl, Br, F, $NO_2$, $CF_3$, un radical alcoyle de 1 à 4 atomes de carbone, un radical alcoxy de 1 à 4 atomes de carbone, acétamido ou benzamido et de préférence Cl ou $NO_2$,
– u, v, p et q ont les significations indiquées ci-dessus,
éventuellement à une réduction, notamment à l'aide de chlorure d'aluminium et d'hydrure d'aluminium et de lithium pour réduire - C = O en $CH_2$, et obtenir le composé de formule

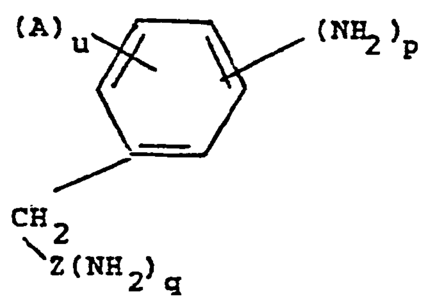

dans laquelle A, Z, u, p et q ont les significations indiquées ci-dessus,
. on soumet l'un des composés aminés de formule indiquée ci-dessus à la réaction de Sandmeyer, pour obtenir un sulfohalogénure de formule :

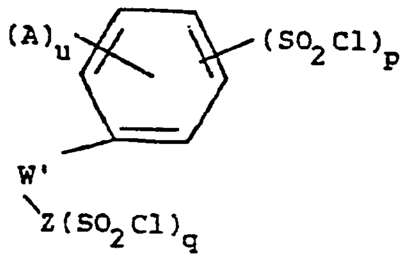

dans laquelle A, Z, u, p et q ont les significations indiquées ci-dessus et W′ représente C = O ou $CH_2$,
. on fait réagir le sulfohalogénure, de formule indiquée ci-dessus, sur un composé de formule

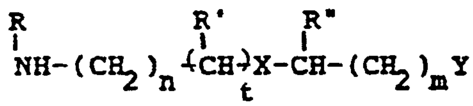

dans laquelle
- R, R′, R″, m, n, t, X et Y ont les significations indiquées ci-dessus,

pour obtenir le composé de formule

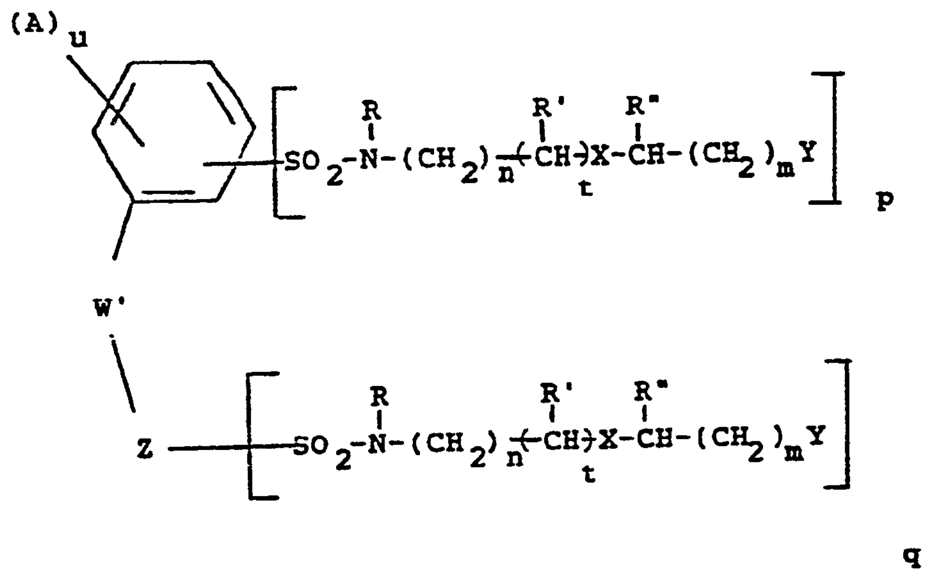

dans laquelle $R_1$, R′, R″, X, Y,A, Z, W′, m, n, t, p et q ont les signification indiquées ci-dessus et dans le cas où W′ représente C = O, on soumet éventuellement le composé ci-dessus à une réduction, notamment à l'aide de borohydrure alcalin, pour réduire C = O en CHOH,
. on effectue ensuite éventuellement une réduction lorsque A et/ou B représente $NO_2$.
2. Procédé de préparation selon la revendication 1 des composés de formule II

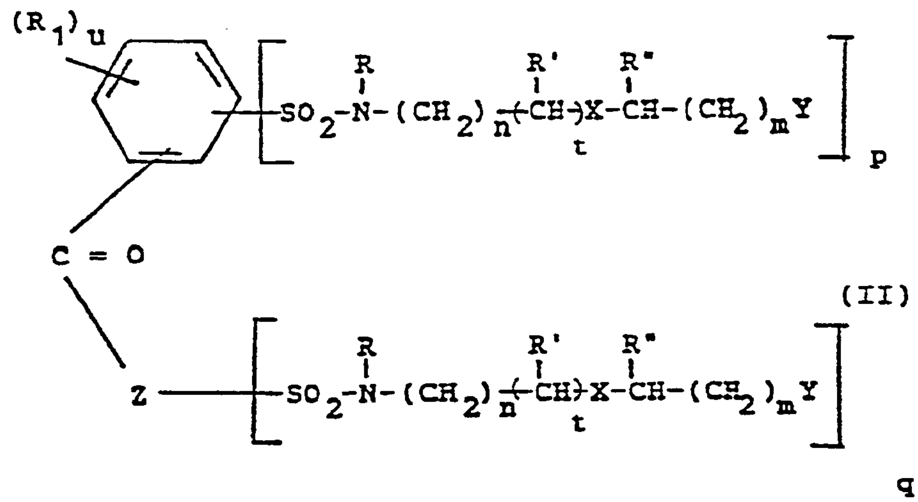

(II)

dans laquelle $R_1$, R, R', R", X, Y, z, m, n, t, p, q et u ont les significations indiquées à la revendication 1, caractérisé en ce que l'on fait réagir un sulfohalogénure, de formule :

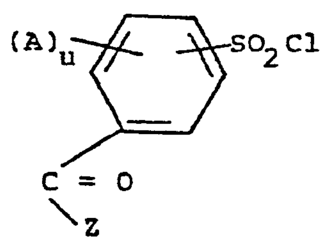

dans laquelle A, Z et u ont les significations indiquées à la revendication 1 ou un disulfohalogénure, de formule:

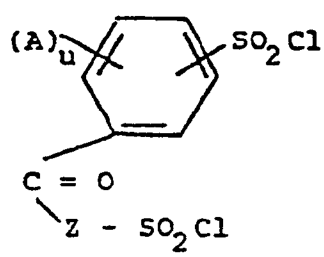

dans laquelle A, u et Z ont les significations précédemment indiquées sur un composé de formule :

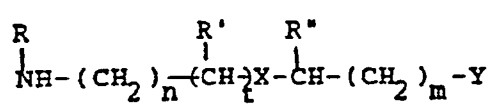

dans laquelle R, R', R", m, n, t, X et Y ont les significations indiquées à la revendication 1,
- suivi éventuellement d'une réduction lorsque A et/ou B représente $NO_2$.

3. Procédé de préparation selon la revendication 2 des composés de formule V suivante

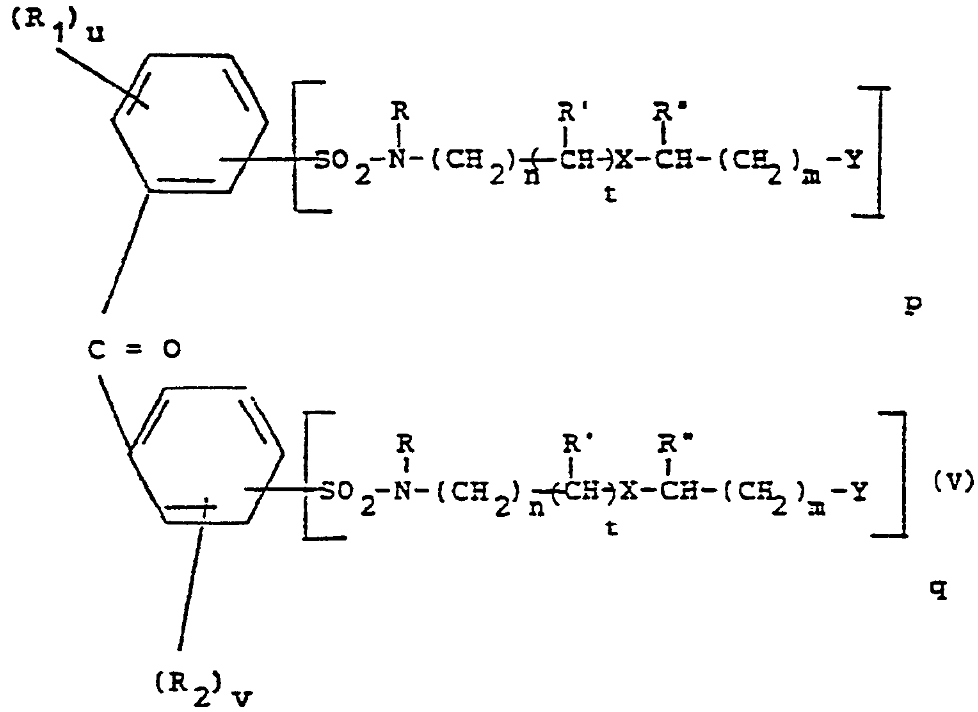

(V)

dans laquelle $R_1$, R, R', R", X, Y,Z, m, n, t, p, q et u ont les significations indiquées à la revendication 1, caractérisé en ce que l'on fait réagir un sulfohalogénure, de formule :

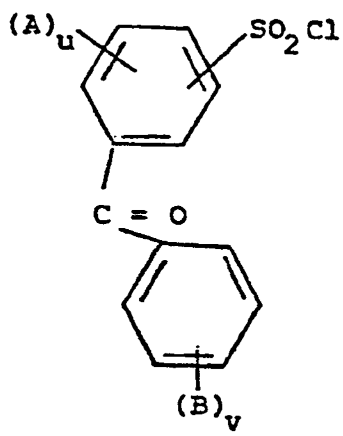

dans laquelle A, B, u et v ont les significations indiquées à la revendication 2, ou un disulfohalogénure, de formule :

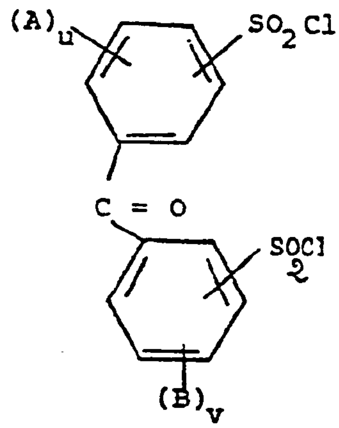

dans laquelle A, B, u et v ont les significations précédemment indiquées sur un ou deux composés de formule

$$\overset{R}{\overset{|}{NH}}-(CH_2)_n-(\overset{R'}{\overset{|}{CH}})_t-X-\overset{R''}{\overset{|}{CH}}-(CH_2)_m-Y$$

dans laquelle R, R′, R″, m, n, t, X et Y ont les significations indiquées à la revendication 2, suivi éventuellement d'une réduction lorsque A et/ou B représente $NO_2$.

4. Procédé de préparation, selon la revendication 3, des composés de formule V dans laquelle
- Y représente $COOR_3$,
$R_3$ représentant l'hydrogène ou un radical alcoyle de 1 à 4 atomes de carbone.

5. Procédé de préparation selon la revendication 3 des composés de formule V dans laquelle
- Y représente $NH_2$, $NHR_6$ ou $NR_6R_7$, $R_6$ et $R_7$ ayant les significations indiquées à la revendication 1.

6. Procédé de préparation selon la revendication 3, des composés de formule V, dans laquelle p ou q vaut O.

7. Procédé de préparation selon la revendication 3, des composés de formule V, dans laquelle
– p ou q vaut O
– u + v supérieur ou égal à 1,
– le nombre total d'atomes de carbone de la chaîne _

$$(CH_2)_n-(\overset{|}{CH})_t X-\overset{|}{CH}-(CH_2)_m-Y$$

est égal ou supérieur à 5.

8. Procédé de préparation, selon la revendication 3, des composés de formule V, dans laquelle
– p = 1,
– q = 1,
– u + v supérieur ou égal à 1,
– le nombre total d'atomes de carbone de la chaîne

$$(CH_2)_n-(\overset{|}{CH})_t X-\overset{|}{CH}-(CH_2)_m-Y$$

est égal ou supérieur à 5.

9. Procédé de préparation, selon la revendication 2, des composés de formule II, caractérisé en ce que l'on fait réagir un sulfochlorure de formule

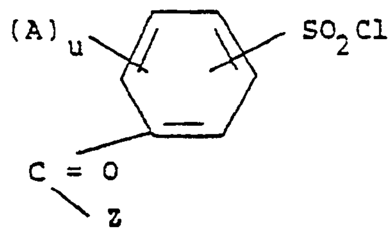

dans laquelle A, u, Z ont les significations indiquées à la revendication 2, ou un disulfochlorure de formule

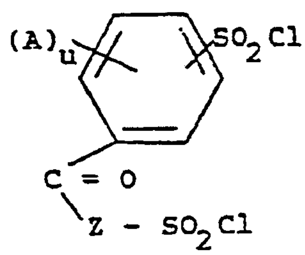

dans lequel A, u, Z ont les significations indiquées à la revendication 2, sur un amino-acide formule

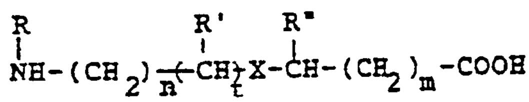

dans laquelle R, R', R", n, m, t et X ont les significations indiquées ci-dessus à la revendication 2.

1O. Procédé de préparation, selon la revendication 2, des composés de formule II, caractérisé en ce que
– l'on estérifie un composé tel qu'obtenu selon la revendication 9, et dans lequel Y représente COOH, en un composé de formule II, dans lequel Y représente $COOR_3$, $R_3$ représentant un radical alcoyle de 1 à 4 atomes de carbone
– ou en ce que l'on transforme un composé tel qu'obtenu selon la revendication 9, en un composé de formule II dans lequel Y représente

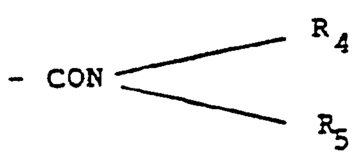

$R_4$, $R_5$ représentant H ou un radical alcoyle de 1 à 4 atomes de carbone,

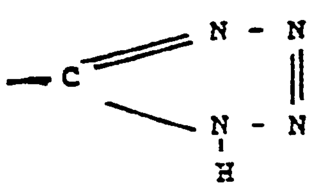

- C≡N

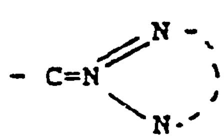

11. Procédé de préparation, selon la revendication 2, des composés de formule II, caractérisé en ce que l'on fait réagir un sulfochlorure de formule

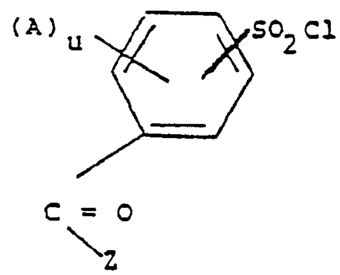

dans laquelle :

– A a les significations indiquées pour $R_1$, à l'exception de $NH_2$, c'est-à-dire Cl, F, Br, $NO_2$, $CF_3$ un radical alcoyle de 1 à 4 atomes de carbone, un radical alcoxy de 1 à 4 atomes de carbone, acétamido ou benzamido,

– Z représente :

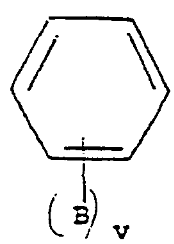

ou

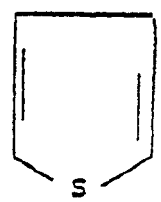

– B a les significations indiquées pour $R_2$, à l'exception de $NH_2$, c'est-à-dire B représente Cl, F, Br, $NO_2$, $CF_3$, un radical alcoyle de 1 à 4 atomes de carbone, un radical alcoxy de 1 à 4 atomes de carbone, acétamido ou benzamido et de préférence Cl, ou $NO_2$,

– u représente un nombre entier de O à 2

– v représente un nombre entier de O à 2 ou un disulfohalogénure, de formule :

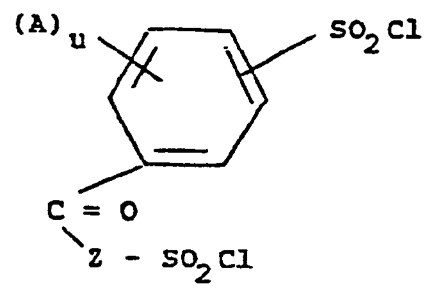

dans laquelle A, u, Z ont les significations indiquées ci-dessus ;

sur une aminoalcoylamine de formule :

$$\overset{R}{N}H-(CH_2)_n(-\overset{R'}{C}H-X)_t-\overset{R''}{C}H-(CH_2)_m-Y$$

dans laquelle :

– R représente H, un radical alcoyle de 1 à 6 atomes de carbone ou un radical benzyle,

– R' et R'' représentent H ou un radical alcoyle de 1 à 4 atomes de carbone,

– n représente un nombre entier variant de O à 1O,

– m représente un nombre entier variant de O à 1O,

– t vaut O ou 1,

– Y représente $NH_2$, $NHR_6$, $NR_6R_7$, $R_6$ et $R_7$ représentent un radical alcoyle de 1 à 4 atomes de carbone, ou forme avec l'azote une amine cyclisée non aromatique.

12. Procédé de préparation, selon la revendication 2, des composés de formule II, caractérisé en ce que l'amino-acide utilisé ou la dialcoylaminoalcoylamine utilisée est choisi respectivement parmi :

$$- NH_2 -(CH_2)_n- COOH$$

n variant de 1 à 11,

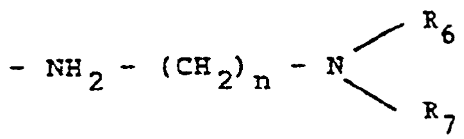

n = 2 ou 3

$R_6$ et $R_7$ représentant un groupe alcoyle de 1 à 4 atomes de carbone ou formant avec l'azote un cycle morpholine, et le sulfochlorure utilisé est choisi respectivement parmi :

- le chlorure de benzoyl - 2 nitro - 4 benzènesulfonyle
- le chlorure de benzoyl - 2 chloro - 4 benzènesulfonyle
- le chlorure de chloro - 4(chloro - 2 benzoyl) - 2 benzènesulfonyle
- le chlorure de benzoyl - 2 benzènesulfonyle
- le chlorure de (chloro - 4 benzoyl) - 2 benzènesulfonyle
- le chlorure de benzoyl - 4 benzènesulfonyle
- le chlorure de benzoyl - 3 benzènesulfonyle
- le chlorure de (dichloro-2,4 benzoyl)-3 chloro-4 benzènesulfonyle
- le chlorure de benzoyl - 3 chloro - 4 benzènesulfonyle
- le chlorure de (chloro-4 benzoyl)-3 chloro-4 benzènesulfonyle
- le chlorure de benzoyl - 4 chloro - 3 benzènesulfonyle
- le chlorure de chloro-4 (thenoyl-2)-3 benzènesulfonyle.

13. Procédé selon la revendication 1, de préparation des composés de formule III

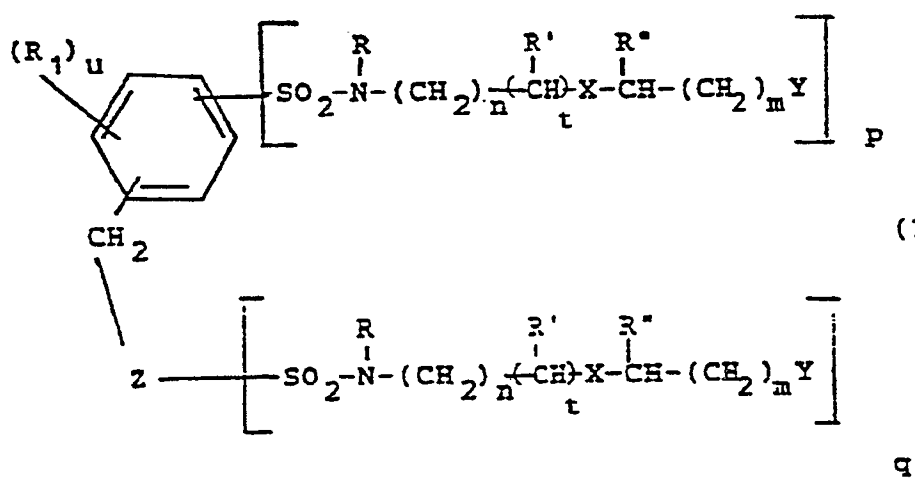

(III)

dans laquelle $R_1$, R, R', R'', X, Y, Z, m, n, t, p, q et u ont les significations indiquées à la revendication 3 caractérisé en ce que l'on soumet le composé de formule

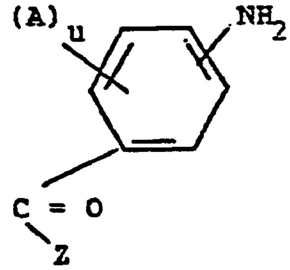

ou de formule

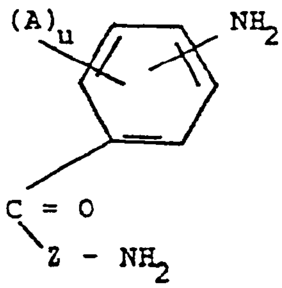

à une réduction, notamment à l'aide de chlorure d'aluminium et d'hydrure de lithium et d'aluminium, pour réduire C = O en $CH_2$ et obtenir le composé de formule

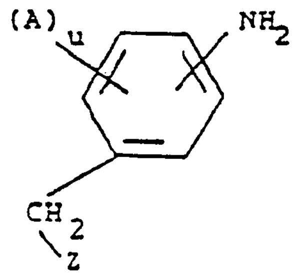

ou

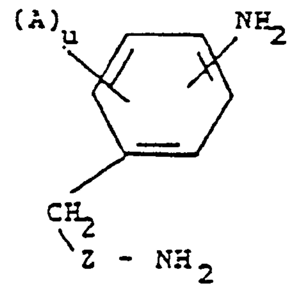

– on soumet l'un de ces composés à la réaction de Sandmeyer, pour obtenir les composés de formule :

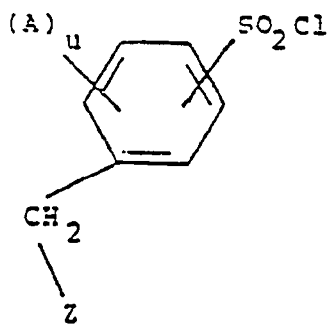

ou

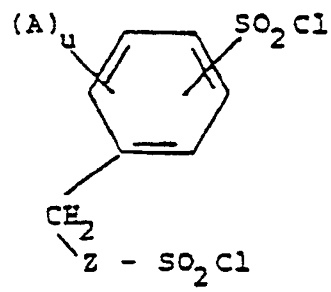

– on condense le sulfohalogénure ou le disulfohalogénure de formule indiquée ci-dessus, sur un composé de formule

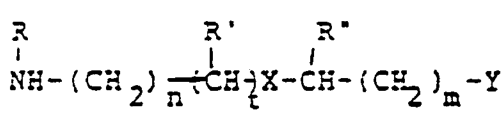

dans laquelle R, R', R", X, Y, m, n et t ont les significations indiquées à la revendication 1, suivi éventuellement d'une réduction lorsque A et/ou B représente $NO_2$.

14. Procédé selon la revendication 13, de préparation de composés de formule III, caractérisé en ce que l'amino-acide utilisé ou la dialcoylaminoalcoylamine utilisée est choisie parmi

$$- NH_2 -( CH_2 )_n- COOH$$

n variant de 1 à 11,

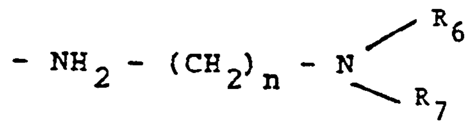

n = 2 ou 3

$R_6$ et $R_7$ représentant un groupe alcoyle de 1 à 4 atomes de carbone ou formant avec l'azote un cycle morpholine, et le composé de formule

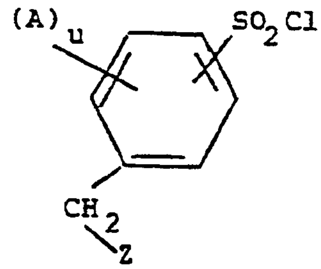

est choisi parmi
- chlorure de (dichloro-2,4 benzyl)-3 chloro-4 benzènesulfonyle
- chlorure de (chloro-4 benzyl)-3 chloro-4 benzènesulfonyle
- benzyl-2 chloro-4 benzènesulfonyle
- chlorure de (dichloro-2,4 benzyl)-2 chloro-4 benzènesulfonyle
- chlorure de (chloro-4 benzyl)-2 chloro-4 benzènesulfonyle
- chlorure de benzyl-4 chloro-3 benzènesulfonyle
- chlorure de (dichloro-2,4 benzyl)-4 chloro-3 benzènesulfonyle
- chlorure de (chloro-4 benzyl)-4 chloro-3 benzènesulfonyle
- chlorure de benzyl-3 chloro-4 benzènesulfonyle.

15. Procédé selon la revendication 1, de préparation de composés de formule IV

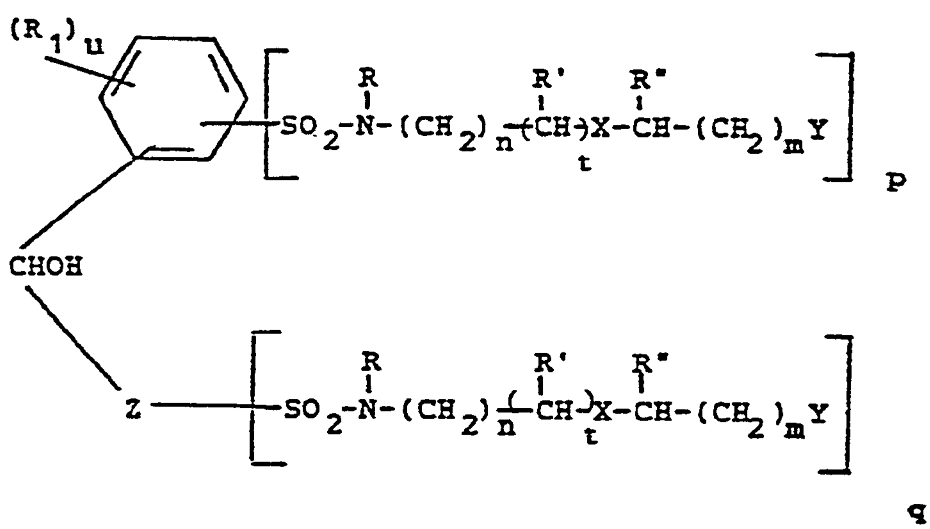

(IV)

dans laquelle $R_1$, R, R', R'', X, Y, Z, m, n, t, p, q et u ont les significations indiquées à la revendication 1, caractérisé en ce que l'on soumet le composé de formule

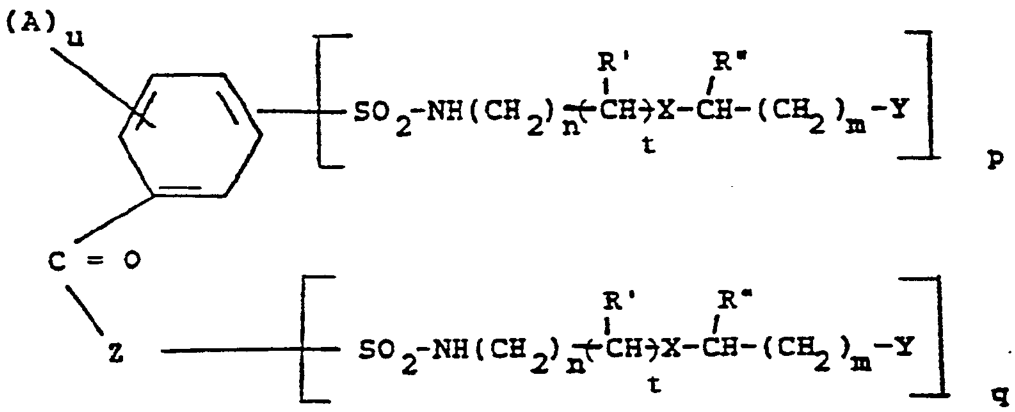

dans laquelle A, X, Y, Z, R, R', R'', m, n, t, p, q et u ont les significations indiquées à la revendication 1, et tel qu'obtenu selon la revendication 2, avant la réduction éventuelle de A et/ou B lorsqu'ils représentent $NO_2$, à une réduction pour transformer C = O en CHOH, à l'aide de borohydrure alcalin.

16. Procédé selon la revendication 15, de préparation de composés de formule IV, caractérisé en ce que l'amino-acide utilisé ou la dialcoylaminoalcoylamine utilisée est choisie parmi

$$-\ NH_2-(CH_2)_n-\ COOH$$

n variant de 1 à 11,

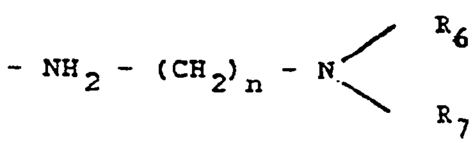 n = 2 ou 3

$R_6$ et $R_7$ représentant un groupe alcoyle de 1 à 4 atomes de carbone ou formant avec l'azote un cycle morpholine, et le sulfochlorure utilisé est choisi parmi

- le chlorure de benzoyl - 2 nitro - 4 benzènesulfonyle
- le chlorure de benzoyl - 2 chloro - 4 benzènesulfonyle
- le chlorure de chloro - 4(chloro - 2 benzoyl) - 2 benzènesulfonyle
- le chlorure de benzoyl - 2 benzènesulfonyle
- le chlorure de (chloro - 4 benzoyl) - 2 benzènesulfonyle
- le chlorure de benzoyl - 4 benzènesulfonyle
- le chlorure de benzoyl - 3 benzènesulfonyle
- le chlorure de (dichloro-2,4 benzoyl)-3 chloro-4 benzènesulfonyle
- le chlorure de benzoyl - 3 chloro - 4 benzènesulfonyle
- le chlorure de (chloro-4 benzoyl)-3 chloro-4 benzènesulfonyle
- le chlorure de benzoyl - 4 chloro - 3 benzènesulfonyle
- le chlorure de chloro-4 (thenoyl-2)-3 benzènesulfonyle.

17. Procédé selon la revendication 1, de préparation de composés de formule VI,

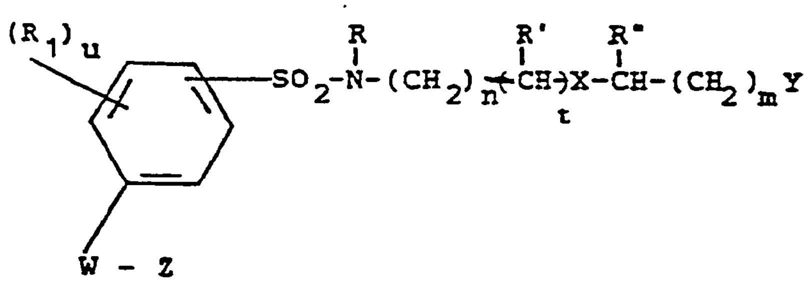

(VI)

dans laquelle $R_1$, R, R', R", X, Y, Z, W, m, r, t et u ont les significations indiquées à la revendication 1, caractérisé en ce que l'on soumet un composé de formule

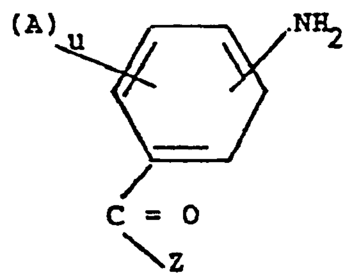

dans laquelle A, Z et u ont les significations indiquées dans la revendication 1,
éventuellement à une réduction, notamment à l'aide de chlorure d'aluminium et d'hydrure d'aluminium et de lithium pour réduire - C = O en $CH_2$, et obtenir le composé de formule

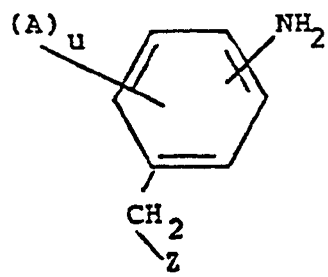

dans laquelle A, Z, u ont les significations indiquées ci-dessus,
. on soumet l'un des composés aminés de formule indiquée ci-dessus à la réaction de Sandmeyer, pour obtenir un sulfohalogénure de formule :

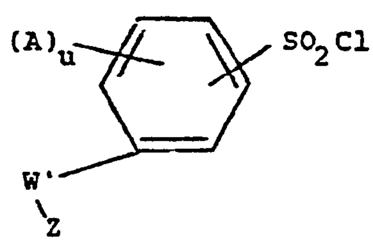

dans laquelle A, Z et u ont les significations indiquées ci-dessus et W' représente C = O ou $CH_2$,
. on fait réagir le sulfohalogénure, de formule indiquée ci-dessus, sur un composé de formule

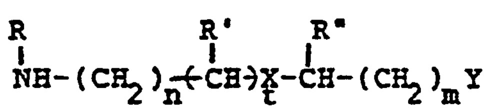

dans laquelle R, R', R", m, n, t, X et Y ont les significations indiquées à la revendication 1,
pour obtenir le composé de formule

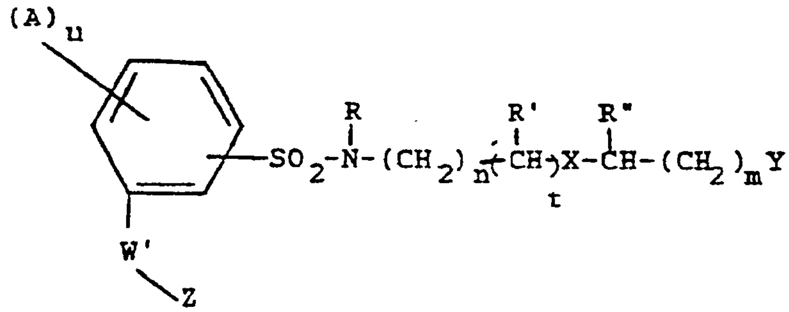

. et dans le cas où W' représente C = O, on soumet éventuellement le composé ci-dessus à une réduction, notamment à l'aide de borohydrure alcalin, pour réduire C = O en CHOH,

. on effectue ensuite éventuellement une réduction lorsque A et/ou B représente $NO_2$.

18. Procédé selon la revendication 17, de préparation des composés de formule VII,

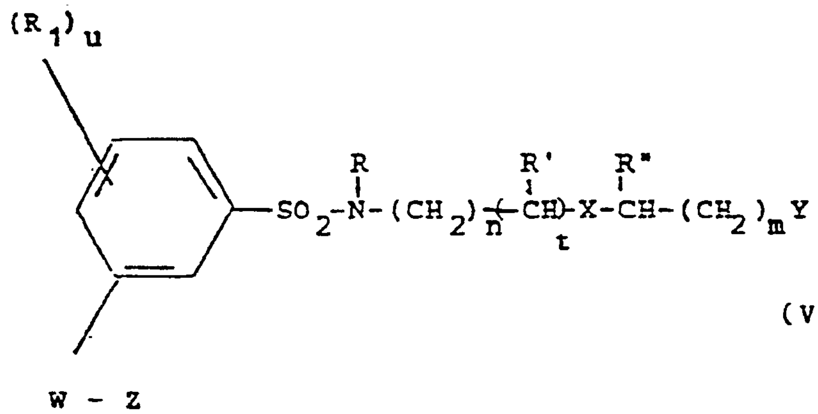

(VII)

dans laquelle $R_1$, R, R', R'', X, Y, Z, W, m, n, t et u ont les significations indiquées à la revendication 1, caractérisé en ce que l'on soumet un composé de formule

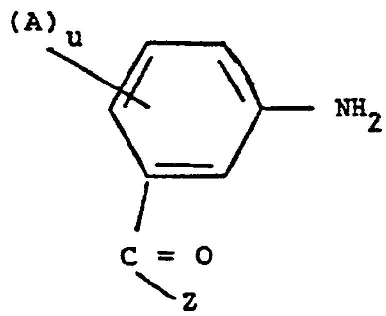

dans laquelle A, Z et u ont les significations indiquées à la revendication 1,

éventuellement à une réduction, notamment à l'aide de chlorure d'aluminium et d'hydrure d'aluminium et de lithium pour réduire - C = O en $CH_2$, et obtenir le composé de formule

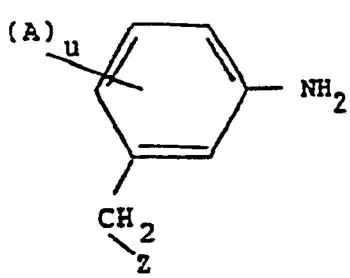

dans laquelle A, Z et u ont les significations indiquées ci-dessus,
. on soumet l'un des composés aminés de formule indiquée ci-dessus à la réaction de Sandmeyer, pour obtenir un sulfohalogénure de formule :

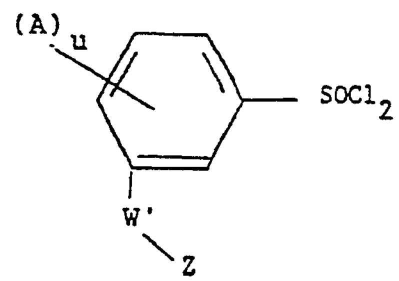

dans laquelle A, Z et u ont les significations indiquées ci-dessus et W' représente C = O ou $CH_2$,
. on fait réagir le sulfohalogénure, de formule indiquée ci-dessus, sur un composé de formule

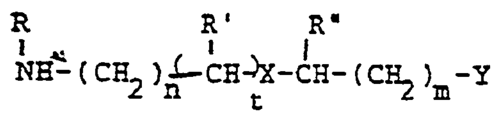

dans laquelle R, R', R", X, Y, m, n et t ont les significations indiquées ci-dessus, pour obtenir le composé de formule

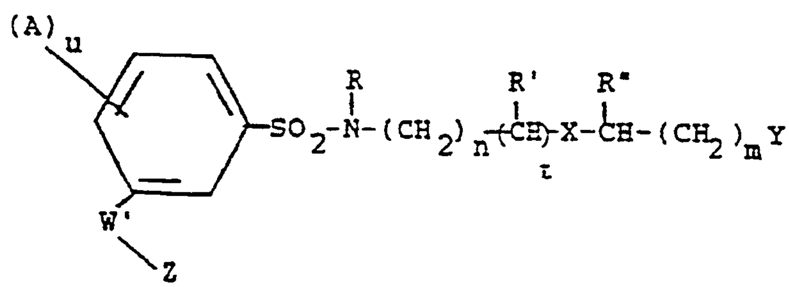

. et dans le cas où W' représente C = O, on soumet éventuellement le composé ci-dessus à une réduction, notamment à l'aide de borohydrure alcalin, pour réduire C = O en CHOH,
. on effectue ensuite éventuellement une réduction lorsque A et/ou B représente $NO_2$.

19. Procédé selon la revendication 18, de préparation des composés de formule VII, dans laquelle
- Y représente $COOR_3$, $R_3$ représentant H ou un radical alcoyle de 1 à 4 atomes de carbone,
caractérisé en ce que l'on soumet un composé de formule

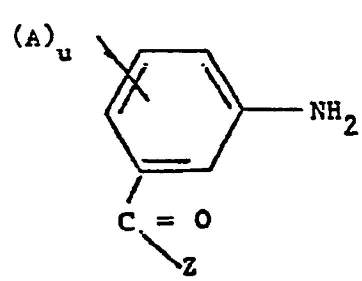

dans laquelle A, Z et u ont les significations indiquées à la revendication 1,
éventuellement à une réduction, notamment à l'aide de chlorure d'aluminium et d'hydrure d'aluminium et de lithium pour réduire - C = O en $CH_2$, et obtenir le composé de formule

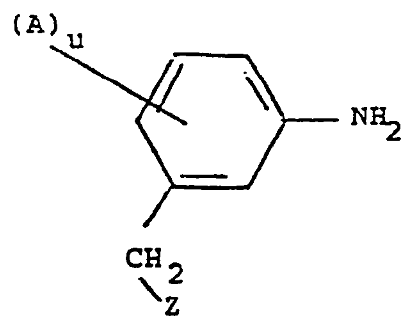

dans laquelle A, Z, et u ont les significations indiquées ci-dessus,

. on soumet l'un des composés aminés de formule indiquée ci-dessus à la réaction de Sandmeyer, pour obtenir un sulfohalogénure de formule :

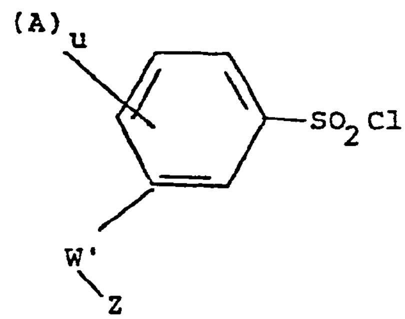

dans laquelle A, Z et u ont les significations indiquées ci-dessus et W' représente C = O ou $CH_2$,

. on fait réagir le sulfohalogénure, notamment le sulfochlorure, de formule indiquée ci-dessus, sur un ou deux composés de formule

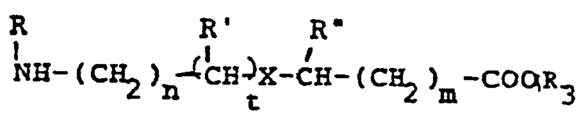

dans laquelle R, R', R", X, $R_3$,m, n et t ont les significations indiquées à la revendication 1,
pour obtenir le composé de formule

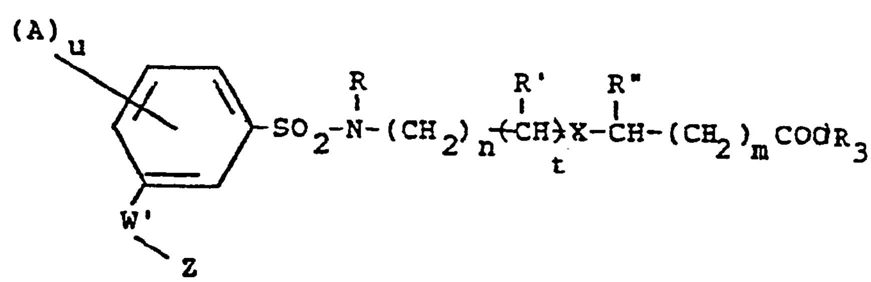

. et dans le cas où W' représente C = O, on soumet éventuellement le composé ci-dessus à une réduction, notamment à l'aide de borohydrure alcalin, tel que le borohydrure de sodium, pour réduire C = O en CHOH,

. on effectue ensuite éventuellement une réduction lorsque A et/ou B représente $NO_2$.

2O. Procédé de préparation selon la revendication 19, de préparation de composés de formule VIII

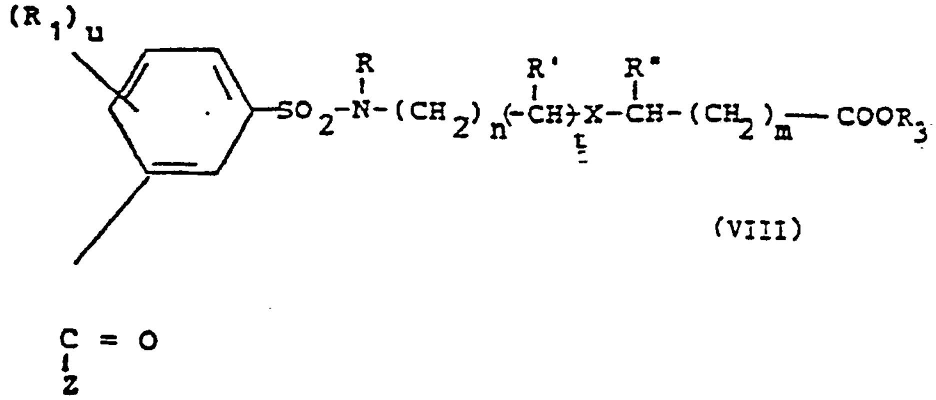

(VIII)

dans laquelle $R_1$, $R_3$, R, R′, R″, X, Z, m, n, t et u ont les significations indiquées à la revendication 1.

21. Procédé de préparation selon la revendication 19, de préparation de composés de formule IX

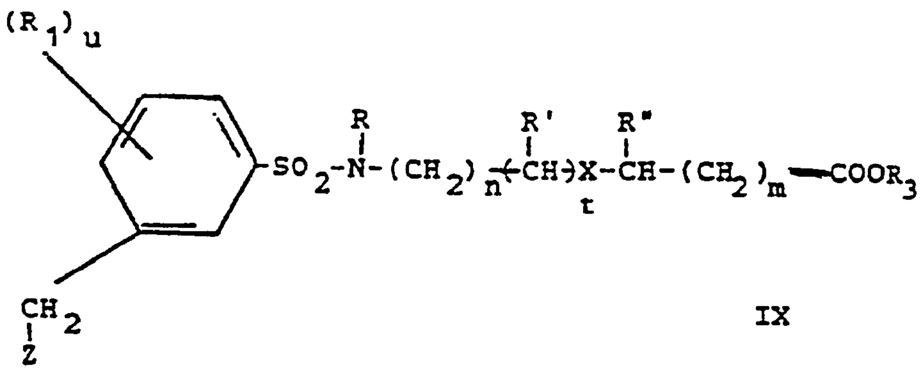

IX

dans laquelle $R_1$, $R_3$, R, R′, R″, X, Z, w, m, n, t et u ont les significations indiquées à la revendication 1.

22. Procédé de préparation selon la revendication 19, de préparation de composés de formule X

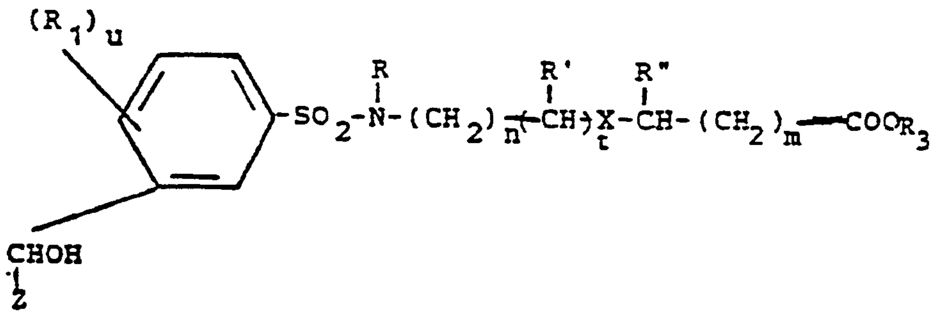

X

dans laquelle $R_1$, $R_3$, R, R′, R″, X, Z, W, m, n, t et u ont les significations indiquées à la revendication 1.

23. Procédé de préparation selon la revendication 20, de préparation de composés de formule VIII dans laquelle le nombre total d'atomes de carbone de la chaîne

$$(CH_2)_n -(CH)_t- X - CH - (CH_2)_m - Y$$

est égal ou supérieur à 5.

24. Procédé de préparation selon la revendication 21, de préparation de composés de formule IX dans laquelle le nombre total d'atomes de carbone de la chaîne

$$(CH_2)_n -(CH)_t- X - CH - (CH_2)_m - Y$$

est égal ou supérieur à 5.

25. Procédé de préparation selon la revendication 22, de préparation de composés de formule X dans laquelle le nombre total d'atomes de carbone de la chaîne

$$(CH_2)_n -( CH )_t- X - CH - (CH_2)_m - Y$$

est égal ou supérieur à 5.

26. Procédé de préparation selon la revendication 1, de préparation de composés de formule XI

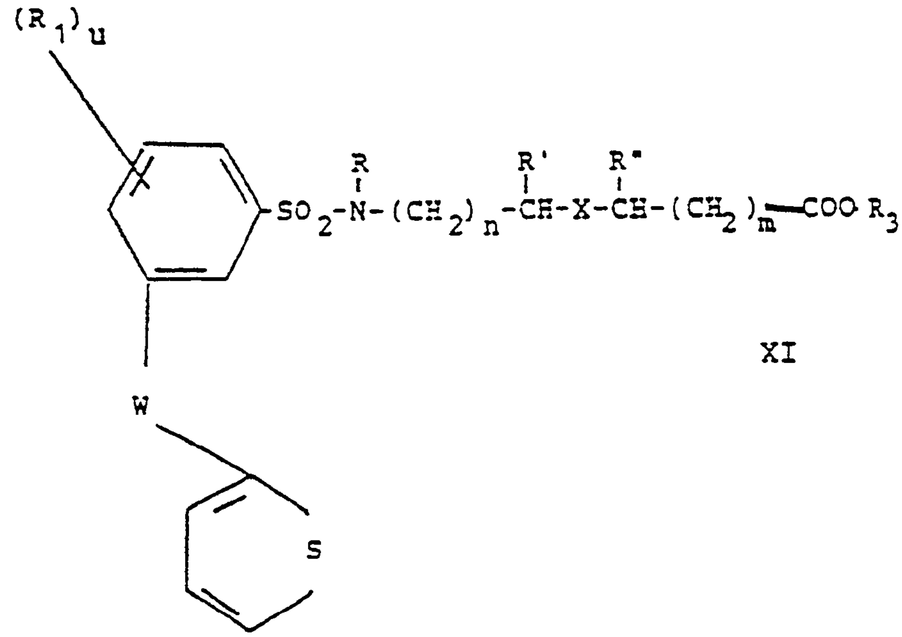

XI

- dans laquelle

le nombre total d'atomes de carbone de la chaîne

$$(CH_2)_n -( CH )_t- X - CH - (CH_2)_m - Y$$

est égal ou supérieur à 5,

et dans laquelle

$R_1$, $R_3$, R, R′, R″, X, W, m, n, t et u ont les significations indiquées à la revendication 1, caractérisé en ce que l'on soumet le composé de formule

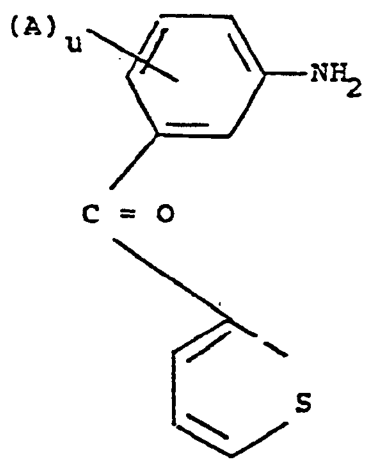

dans laquelle

A et u ont les significations indiquées à la revendication 1,

éventuellement à une réduction, notamment à l'aide de chlorure d'aluminium et d'hydrure d'aluminium et de lithium pour réduire - C = O en $CH_2$, et obtenir le composé de formule

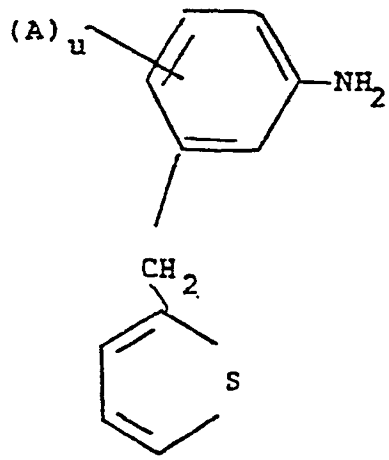

dans laquelle A, et u ont les significations indiquées ci-dessus,
. on soumet l'un des composés aminés de formule indiquée ci-dessus à la réaction de Sandmeyer, pour obtenir un sulfohalogénure de formule :

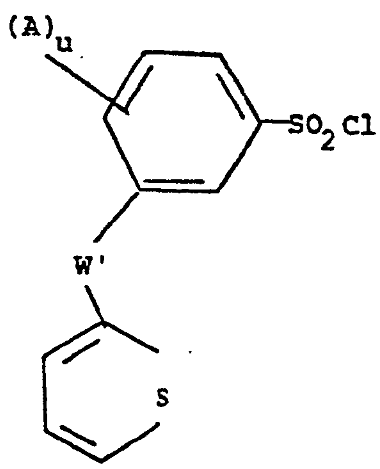

dans laquelle A, et u ont les significations indiquées ci-dessus et W' représente C = O ou $CH_2$,
. on fait réagir le sulfohalogénure, de formule indiquée ci-dessus, sur un ou deux composés de formule

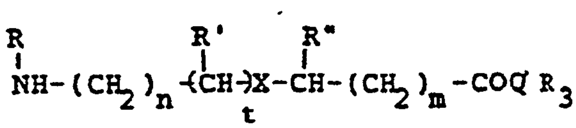

dans laquelle R, R', R", X, m, n, t et $R_3$ ont les significations indiquées à la revendication 1, pour obtenir le composé de formule

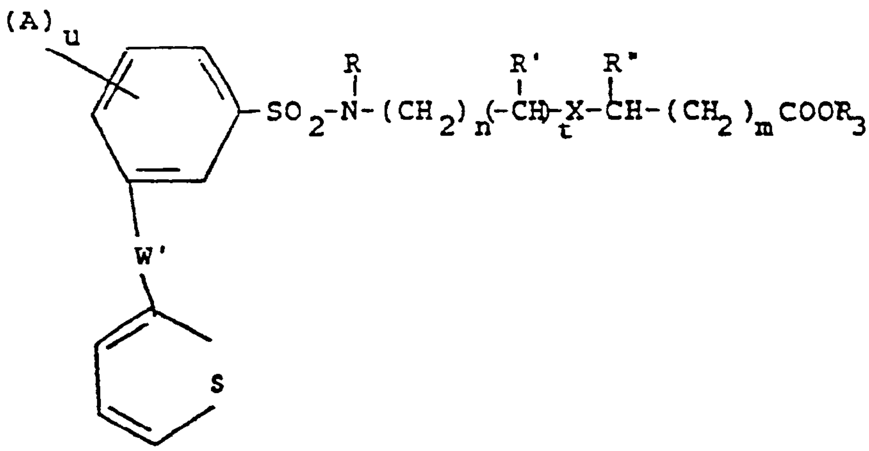

. et dans le cas ou W' représente C = O, on soumet éventuellement le composé ci-dessus à une réduction, notamment à l'aide de borohydrure alcalin, pour réduire C = O en CHOH,

. on effectue ensuite éventuellement une réduction lorsque A et/ou B représente $NO_2$.

27. Procédé de préparation d'une composition pharmaceutique, caractérisé en ce que l'on associe l'un quelconque des composés obtenus à l'issue du procédé selon l'une quelconque des revendications 1 à 26, avec un véhicule pharmaceutiquement acceptable dans des conditions permettant la production de ladite composition pharmaceutique.

## Claims

### Claims for the Contracting States : AT, BE, CH, DE, FR, GB, IT, LI, LU, NL, SE

1. Compound of the formula I

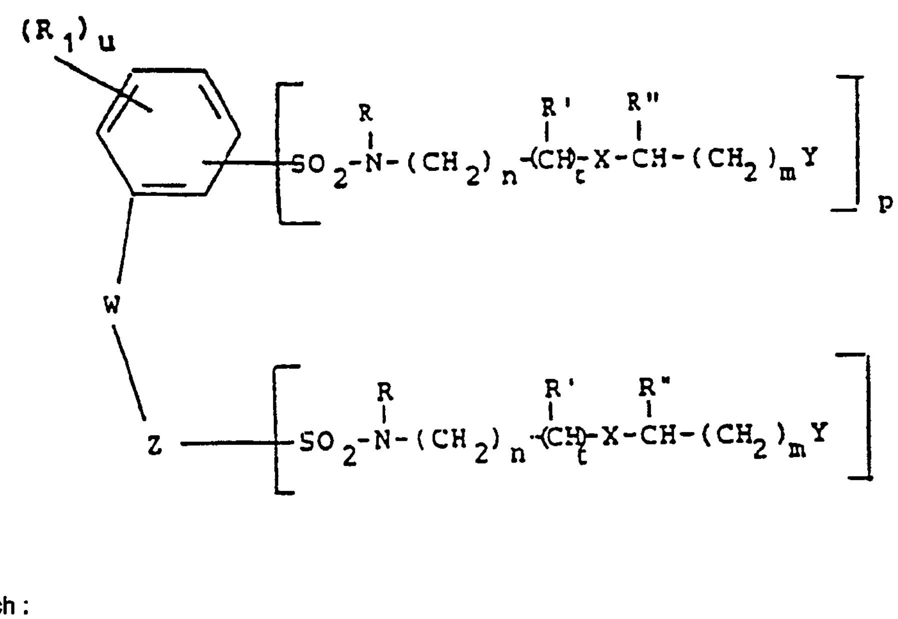

(I)

in which :

– W represents C = O, $CH_2$ or CHOH

– Z represents :

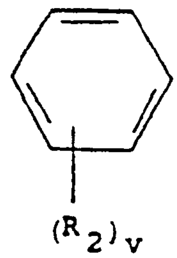 or 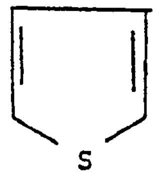

– $R_1$ represents Cl, F, Br, $NO_2$, $NH_2$, $CF_3$, an alkyl radical having from 1 to 4 carbon atoms, notably methyl or ethyl, an alkoxy radical having from 1 to 4 carbon atoms, acetamido or benzamido,
– $R_2$ represents Cl, F, Br, $NO_2$, $NH_2$, $CF_3$, an alkyl radical having from 1 to 4 carbon atoms, an alkoxy radical having from 1 to 4 carbon atoms, acetamido or benzamido,
– R represents H, an alkyl radical having from 1 to 6 carbon atoms or a benzyl radical,
– R′ and R″ represent H or an alkyl radical having from 1 to 4 carbon atoms,
– X represents $(CH_2)_r$, r being O or 1, -CH = CH

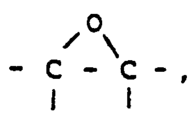, or 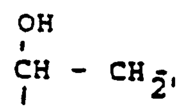,

– Y represents $COOR_3$, $R_3$ representing a hydrogen or an alkyl radical having from 1 to 4 carbon atoms,

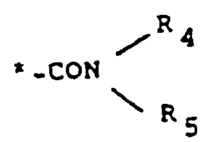 ,

$R_4$ and $R_5$ representing H or an alkyl radical having from 1 to 4 carbon atoms

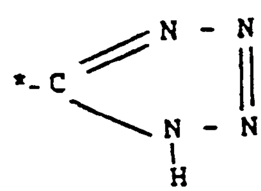 ,

* - C ≡ N,

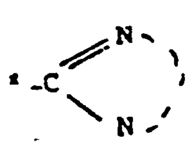 , 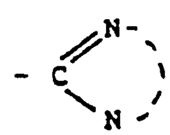

representing a non-aromatic cycle of 5 or 6 links, in which the 2 or 3 atoms which enter into the cycle are atoms of carbon and/or of oxygen and/or of nitrogen,

* $NH_2$, $NHR_6$, $NR_6R_7$,

$R_6$ and $R_7$ representing an alkyl radical having from 1 to 4 carbon atoms, or capable of forming a non-aromatic cyclised amine with the nitrogen atom,

– provided that when Y represents $-NH_2$, X is different from

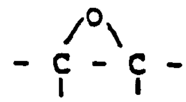

- u is a whole number from O to 2,
- v is a whole number from O to 2,
- p is equal to O or 1,
- q is equal to O or 1, p + q being equal to or greater than 1,
- n is a whole number varying from O to 1O,
- m is a whole number varying from O to 1O,
- t is equal to O or 1,
- the total number of carbon atoms in the chain

$$-(CH_2)_n - (\overset{|}{C}H)_t - X - \overset{|}{C}H - (CH_2)_m Y$$

varying from 2 to 20,
as well as their physiologically acceptable salts.

2. Compound as claimed in claim 1 complying with the following formula II

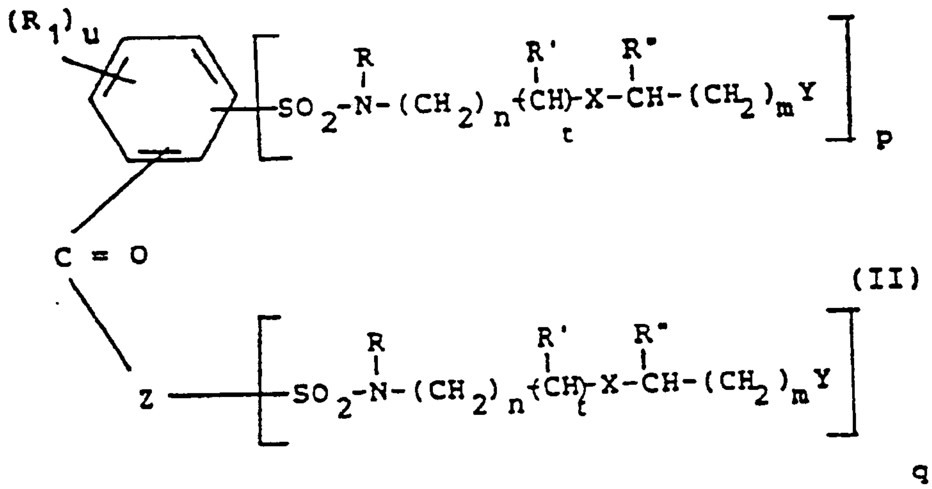

(II)

in which $R_1$, R, R', R", X, Y, Z, m, n, t, p, q and u have the meanings indicated in claim 1.

3. Compound as claimed in claim 1, complying with the following formula III

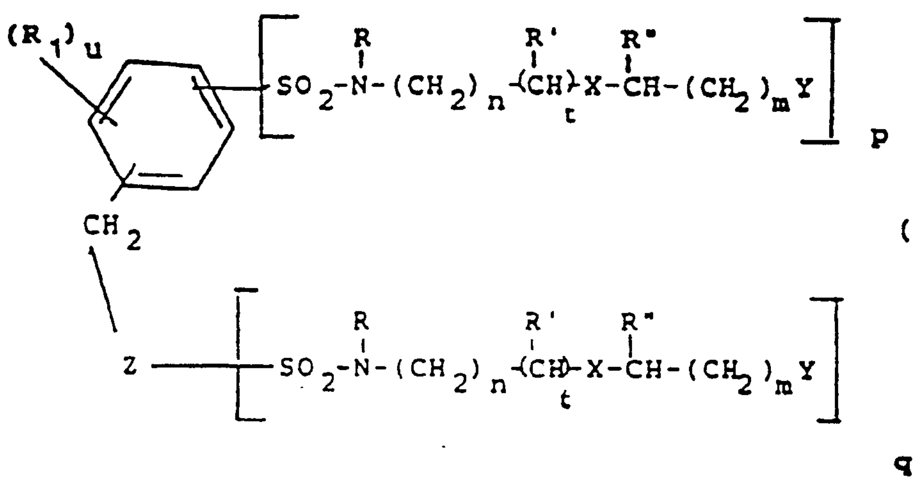

(III)

in which $R_1$, R, R′, R″, X, Y, Z, m, n, t, p, q and u have the meanings indicated in claim 1.

4. Compound as claimed in claim 1, complying with the following formula IV

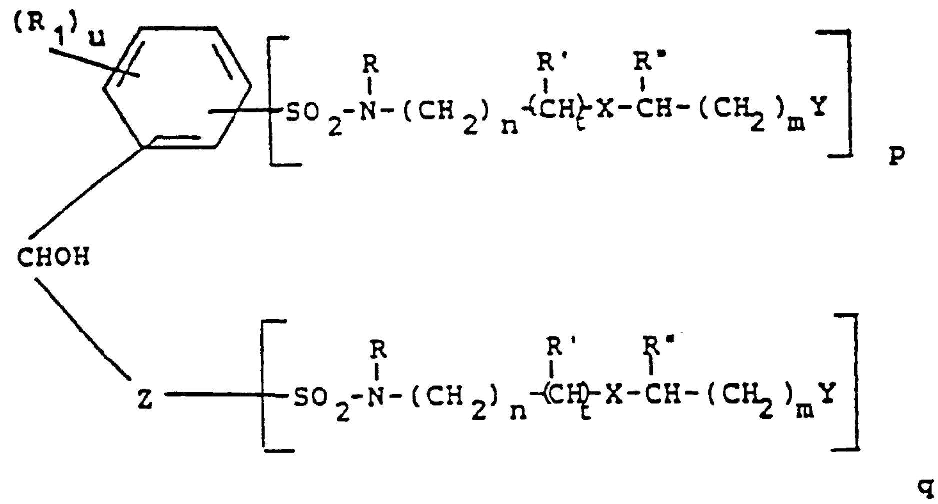

in which $R_1$, R, R′, R″, X, Y, Z, m, n, t, p, q and u have the meanings indicated in claim 1.

5. Compound as claimed in claim 2, complying with the following formula V

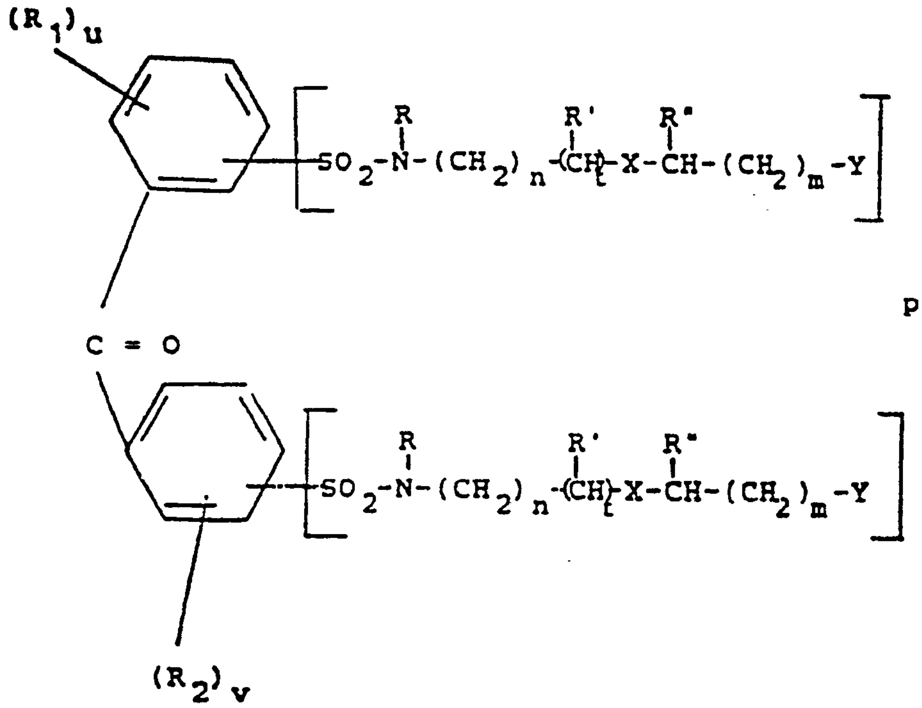

(V)

in which $R_1$, $R_2$. R, R′, R″, X, Y, Z, m, n, p, q, t, u and v the meanings indicated in claim 1.

6. Compound as claimed in claim 5, complying with formula V, in which -Y represents $COOR_3$, $R_3$ representing H or an alkyl radical having from 1 to 4 carbon atoms.

7. Compound as claimed in claim 5, complying with formula V, in which -Y represents $NH_2$, $NHR_6$ or $NR_6R_7$, $R_6$ and $R_7$ having the meanings indicated in claim 1.

8. Compound as claimed in claim 5, complying with formula V, in which p or q is equal to O.

9. Compound as claimed in claim 8, characterised in that

– u + v is greater than or equal to 1,

– the total number of carbon atoms in the chain

$$(CH_2)_n - (CH)_t - X - CH - (CH_2)_m - Y$$

is equal to or greater than 5.

10. Compound as claimed in claim 5, conplying with formula V, in which

– p = 1,

– q = 1,

– u + v is greater than or equal to 1,

– the total number of carbon atoms in the chain

$$(CH_2)_n - (CH)_t - X - CH - (CH_2)_m - Y$$

is equal to or greater than 5.

11. Compound as claimed in claim 1, complying with the following formula VI

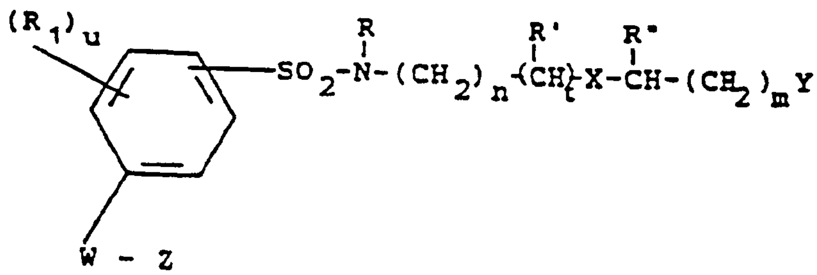

(VI)

in which $R_1$, R, R′, R″, X, Y, Z, W, m, n, u and t have the meanings indicated in claim 1.

12. Compound as claimed in claim 11, complying with the following formula VII

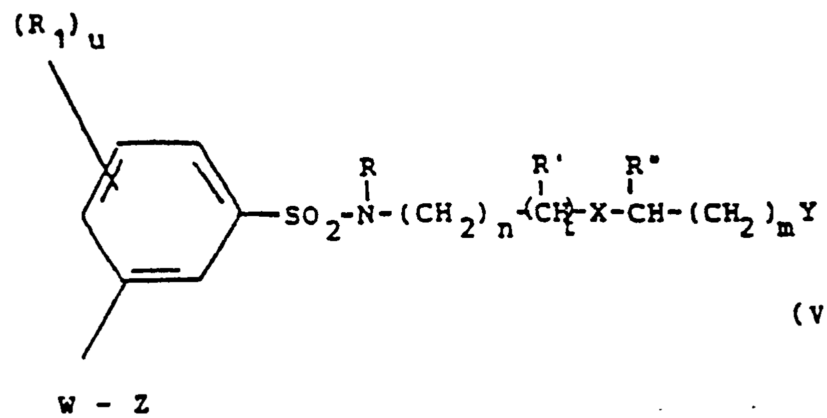

(VII)

in which $R_1$, R, R′, R″, X, Y, Z, W, m, n, u and t have the meanings indicated in claim 1.

13. Compound as claimed in claim 12, complying with formula VII, in which -Y represents $COOR_3$, $R_3$ representing H or an alkyl radical having from 1 to 4 carbon atom.

14. Compound as claimed in claim 13, complying with the following formula VIII

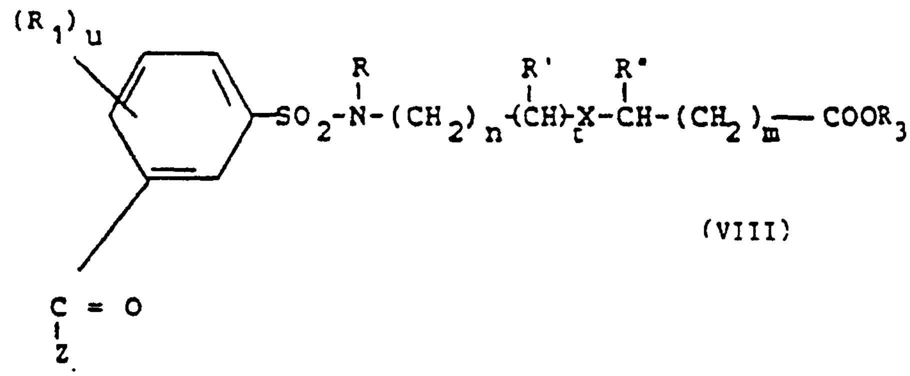

(VIII)

in which $R_1$, $R_3$, R, R′, R″, X, Z, m, n, u and t have the meanings indicated in claim 1.

15. Compound as claimed in claim 13, complying with the following formula IX

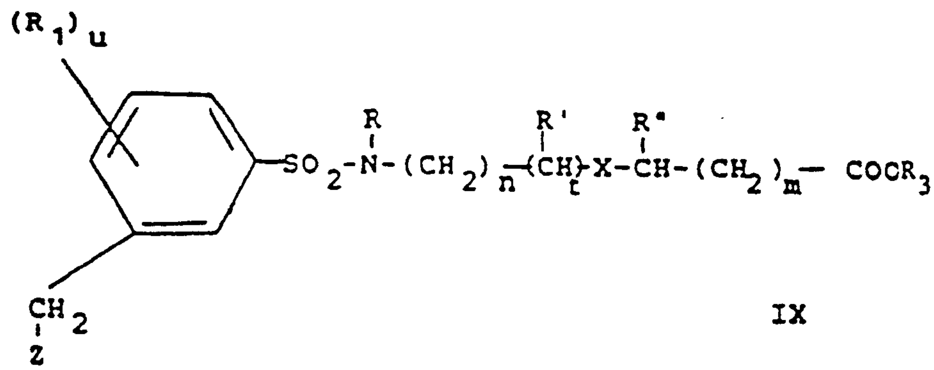

IX

in which $R_1$, $R_3$, R, R′, R″, X, Z, m, n, u and t have the meanings indicated in claim 1.

16. Compound as claimed in claim 13, complying with the following formula X

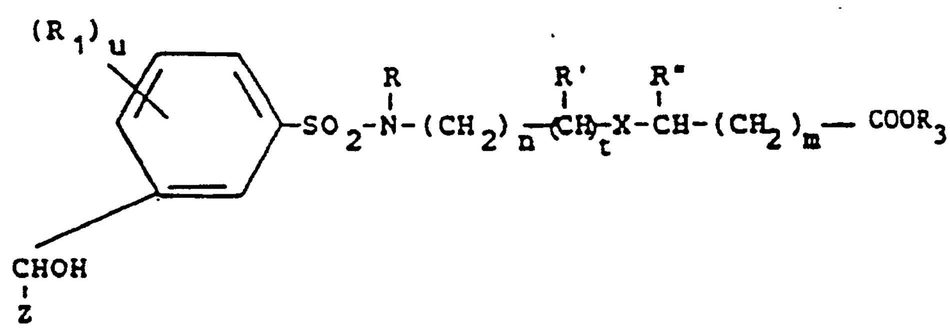

X

in which $R_1$, $R_3$, R, R′, R″, X, Z, m, n, u and t have the meanings indicated in claim 1.

17. Compound as claimed in claim 14, complying with formula VIII, in which the total number of carbon atoms of the chain

$$(CH_2)_n - (CH)_t - X - CH - (CH_2)_m - Y$$

is equal to or greater than 5.

18. Compound as claimed in claim 15, complying with formula IX, in which the total number of carbon atoms of the chain

$$(CH_2)_n - (CH)_t - X - CH - (CH_2)_m - Y$$

is equal to or greater than 5.

19. Compound as claimed in claim 16, complying with formula X, in which the total number of carbon atoms of

the chain

$$(CH_2)_n - (CH)_t - X - CH - (CH_2)_m - Y$$

is equal to or greater than 5.

20. Compound as claimed in claim 13, complying with the following formula XI,

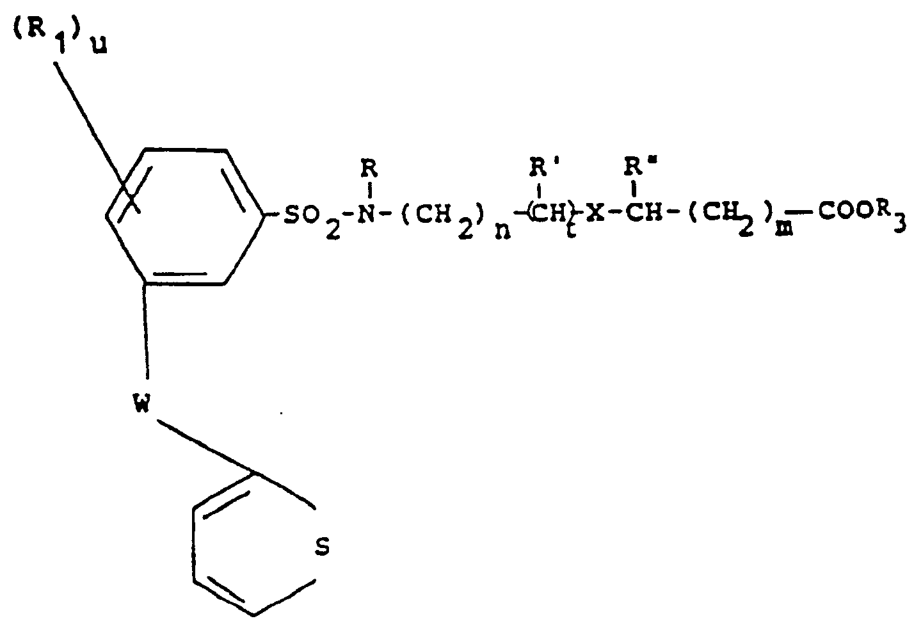

XI

in which the total number of carbon atoms of the chain

$$(CH_2)_n - (CH)_t - X - CH - (CH_2)_m - Y$$

is equal to or greater than 5, and in which $R_1$, $R_3$, R, R′, R″, X, W, m, n and t have the meanings indicated in claim 1.

21. Compound as claimed in claim 11, complying with the following formula VIbis

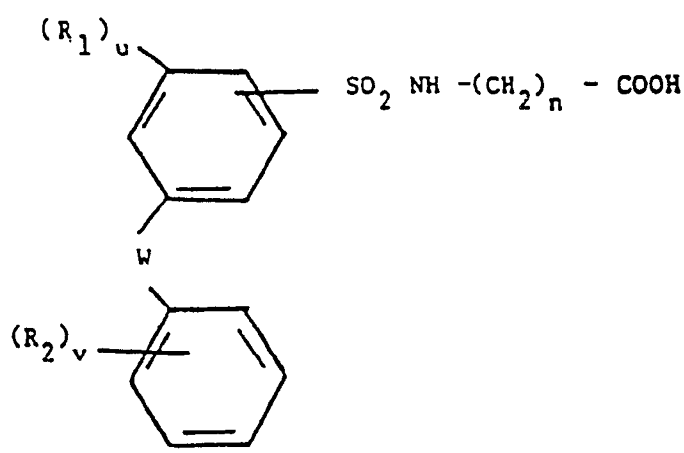

in which :

- $R_1$ and $R_2$ represent a halogen,
- u varies from O to 2
- v varies from O to 2,
- W represents C = O, CHOH, $CH_2$
- n is equal to 3 to 11.

22. Compound as claimed in claim 11, complying with the following formula VIter :

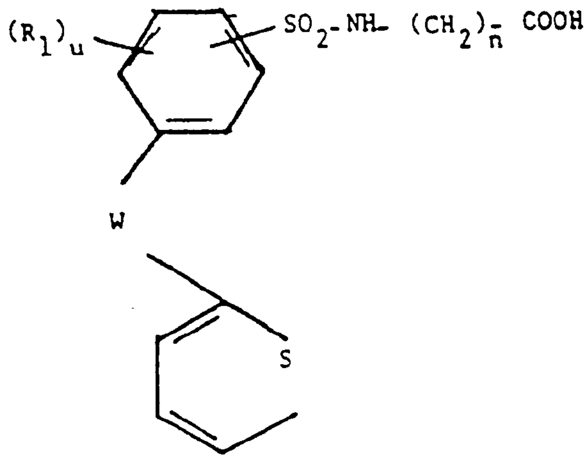

in which :

– $R_1$ represents a halogen,

– u is equal to 2,

– W represents C = O, CHOH, $CH_2$

– n is equal to 3 to 11.

23. Compounds as claimed in claim 1, complying with the following formulae :

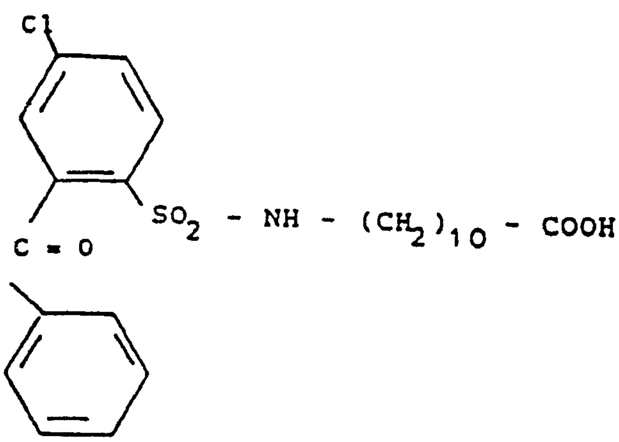

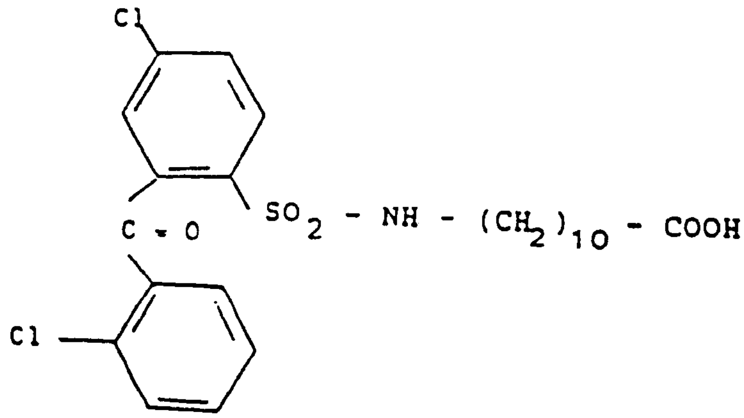

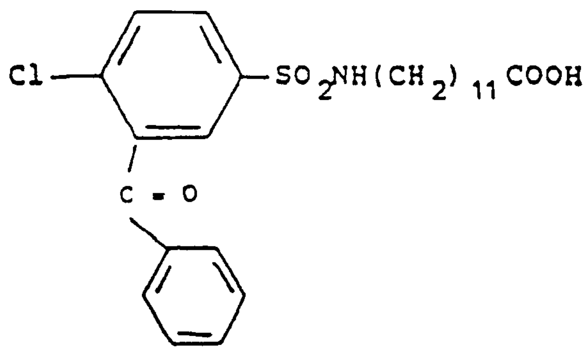

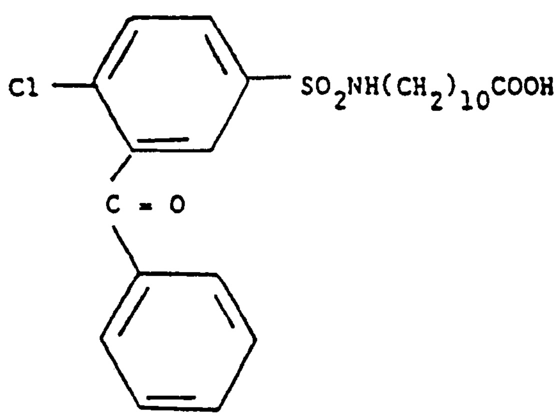

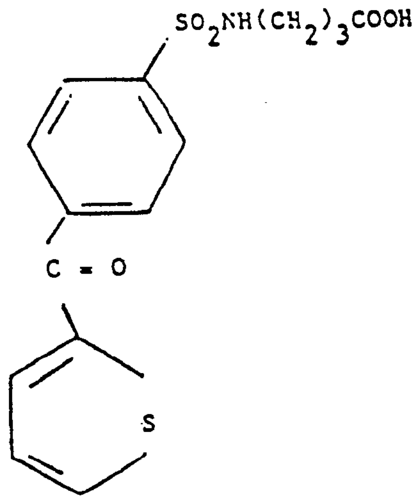

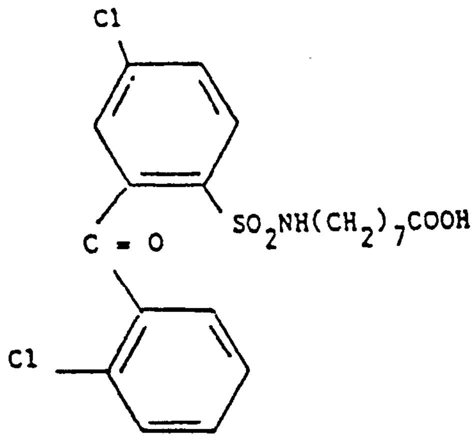

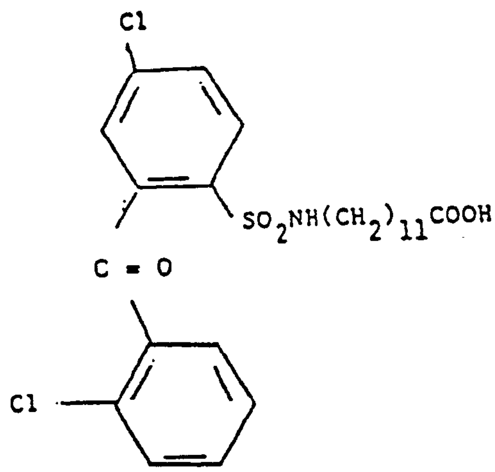

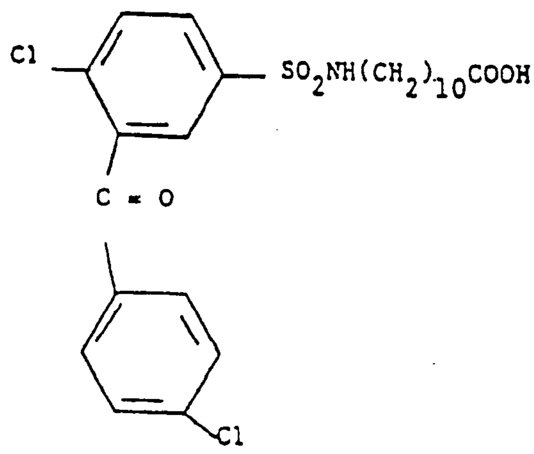

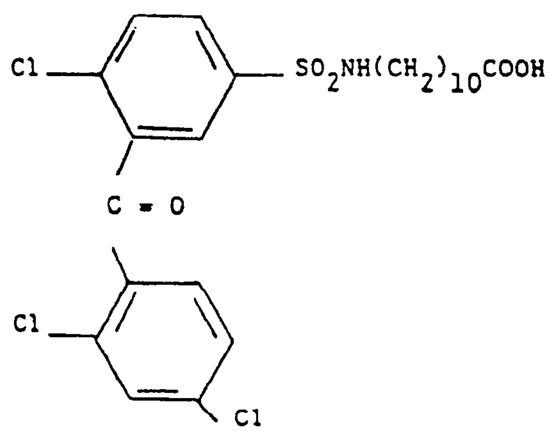

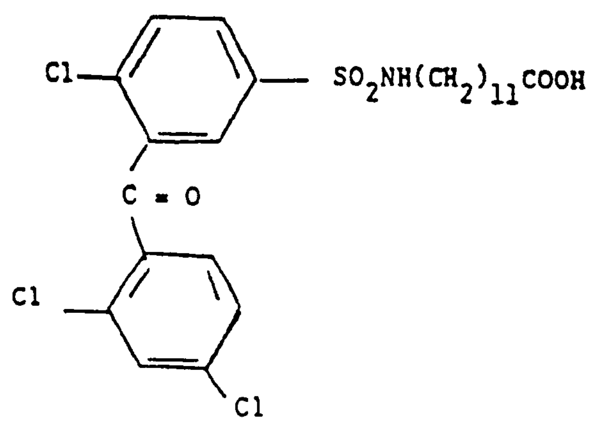

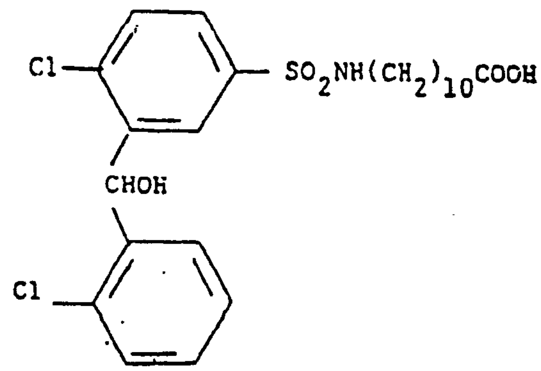

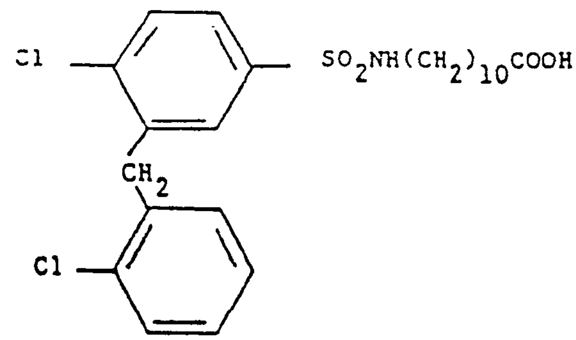

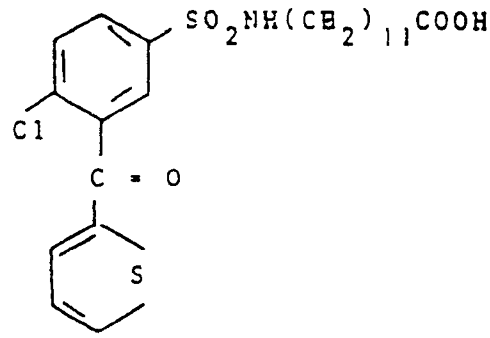

24. Process for preparation of the compounds of formula I, as claimed in claim 1, characterised in that a compound of formula

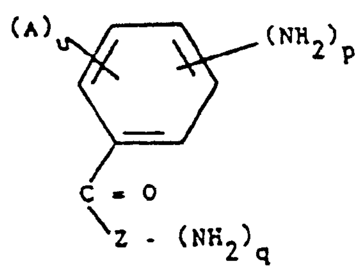

in which :
- A has the meanings indicated for $R_1$, with the exception of $NH_2$, that is to say A represents Cl, F, Br, $NO_2$, $CF_3$, an alkyl radical having from 1 to 4 carbon atoms, an alkoxy radical having from 1 to 4 carbon atoms, acetamido or benzamido,
- Z represents

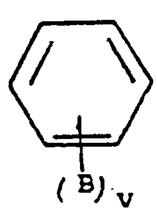

or

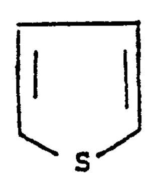

- B has the meanings indicated for $R_2$, with the exception of $NH_2$, that is to say B represents Cl, $NO_2$, $CF_3$, an alkyl radical having from 1 to 4 carbon atoms, an alkoxy radical having from 1 to 4 carbon atom, acetamido or benzamido and
- u represents a whole number from O to 2,
- v represents a whole number from O to 2,
- p = O or 1,
- q = O or 1, p+q being equal to or greater than 1,
. is possibly subjected to a reduction, notably with the aid of aluminium chloride, aluminium hydride and lithium

hydride to reduce -C=O to $CH_2$ and to obtain the compound of formula

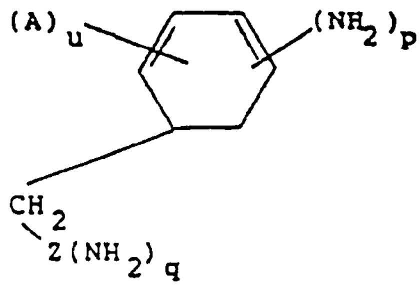

in which A, Z, u, p and q have the meanings indicated above,
. one of the amino compounds of the formula indicated above is subjected to the Sandmeyer reaction in order to obtain a sulphohalide of formula :

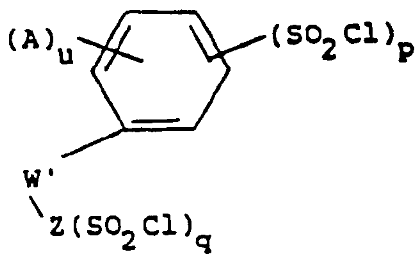

in which A, Z, u, p and q have the meanings indicated above and W′ represents C = O or $CH_2$,
. the sulphohalide, notably sulphochloride, of the formula indicated above is made to react on one or two compounds of formula

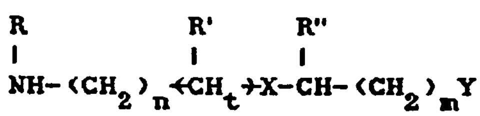

in which
– R represents H, an alkyl radical having from 1 to 6 carbon atoms or a benzyl radical,
– R′ and R″ represent H or an alkyl radical having from 1 to 4 carbon atoms,
– X represents $(CH_2)_r$, r being equal to O or 1 or -CH=CH,

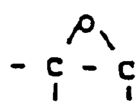 , or 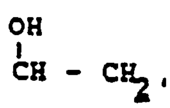 ,

– Y represents $COOR_3$, $R_3$ representing a hydrogen or an alkyl radical having from 1 to 4 carbon atoms,

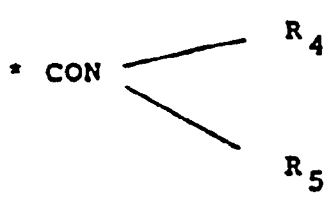

$R_4$ and $R_5$ representing H or an alkyl radical having from 1 to 4 carbon atoms

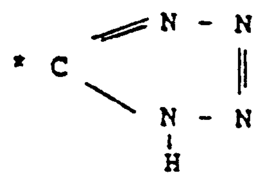

- C ≡ N,

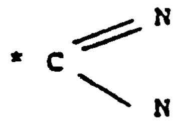 , 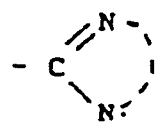

representing a non-aromatic cycle of 5 or 6 links, in which the 2 or 3 atoms which enter into the cycle are atoms of carbon and/or of oxygen and/or of nitrogen,

* $NH_2$, $NHR_6$, $NR_6R_7$,

$R_6$ and $R_7$ representing an alkyl radical having from 1 to 4 carbon atoms, or capable of forming a non-aromatic cyclised amine with the nitrogen atom,

– provided that when Y represents -$NH_2$, X is different from

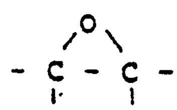

– n is a whole number varying from O to 1O,
– m is a whole number varying from O to 1O,
– t is equal to O or 1,
– the total number of carbon atoms in the chain

$$-(CH_2)_n-(CH)_t-X-CH-(CH_2)_m Y$$

varying from 2 to 2O,
in order to obtain the compound of formula

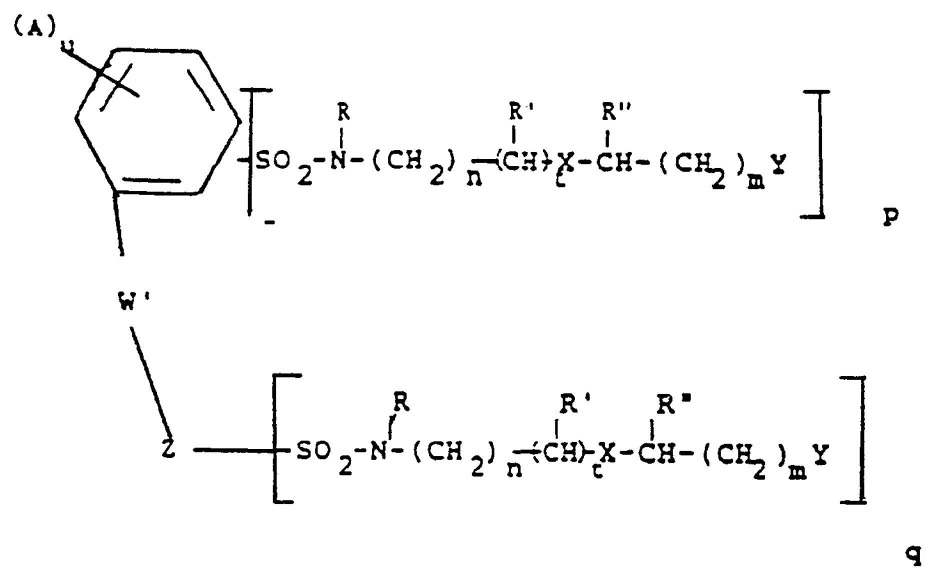

in which A, Z, W', R, R', R", X, Y, m, n, t, q and u have the meanings indicated above,
. and in the case where W' represents C=O the above compound is possibly subjected to a reduction, notably

with the aid of alkaline borohydride, such as sodium borohydride, to reduce C=O to CHOH,
. then a reduction is possibly carried out when A and/or B represents $NO_2$.

25. Process as claimed in claim 24 for preparation of compounds of formula II

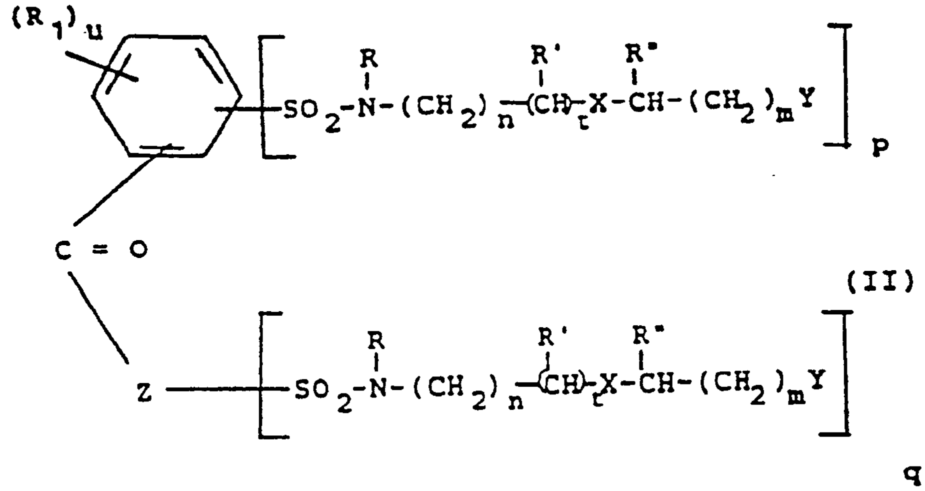

(II)

in which $R_1$, R, R', R'', X, Y, Z, m, n, p, q, u and t have the meanings indicated in claim 2, characterised in that a sulphohalide, notably a sulphochloride of formula

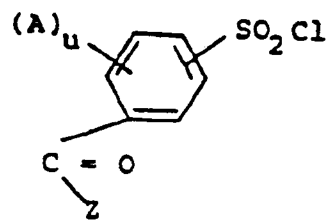

in which A, Z and u have the meanings indicated in claim 24, or a disulphohalide, notably a disulphochloride of formula

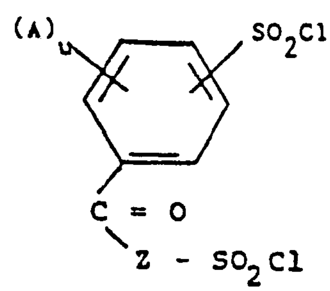

in which A, u and Z have the meanings indicated previously, is made to react on one or two compounds of formula

R R' R''
I I I
NH-$(CH_2)_n$-$(CH)_t$-X-CH-$(CH_2)_m$-Y

in which R, R', R'', n, m, t, X and Y have the meanings indicated in claim 24,
- possibly followed by a reduction when A and/or B represents $NO_2$.

26. Process as claimed in claim 25, characterised in that a sulphochloride of formula

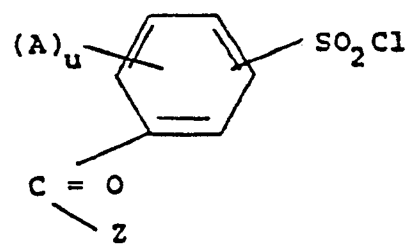

in which A, u and Z have the meanings indicated in claim 25, or a disulphochloride of formula

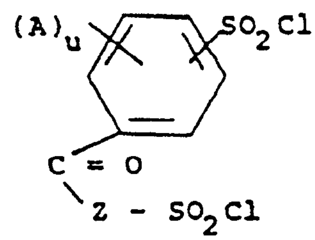

in which A, u, A have the meanings indicated in claim 25, is made to react on one or two amino acids of formula

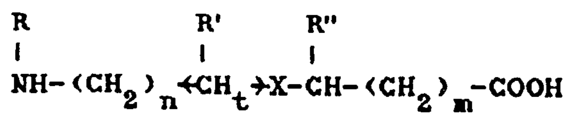

in which R, R′, R″, n, m, t, and X have the meanings indicated above in claim 25.

27. Process as claimed in claim 25, characterized in that

– a compound such as that obtained according to claim 26, and in which Y represents COOH, is esterified in a compound of formula II, in which Y represents $COOR_3$, $R_3$ representing an alkyl radical having from 1 to 4 carbon atoms,

– or a compound such as that obtained according to claim 26 is transformed into a compound of formula II in which Y represents

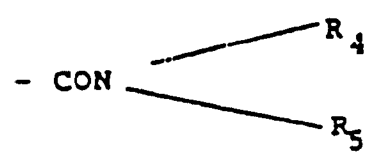

$R_4$, $R_5$ representing H or an alkyl radical having from 1 to 4 carbon atoms,

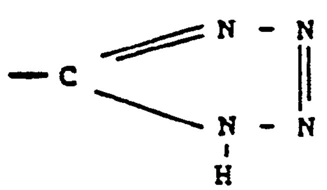 , - C≡N 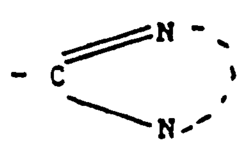

28. Process as claimed in claim 25, characterised in that a sulphochloride of formula

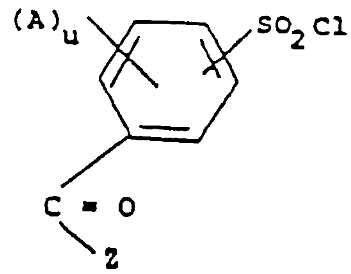

in which :

– A has the meanings indicated for $R_1$, with the exception of $NH_2$, that is to say Cl, F, Br, $NO_2$, $CF_3$, an alkyl radical having from 1 to 4 carbon atoms, an alkoxy radical having from 1 to 4 carbon atom, acetamido or benzamido,

– Z represents :

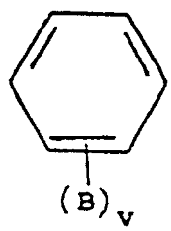

or

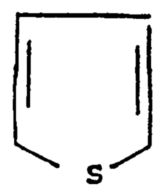

– B has the meanings indicated for $R_2$, with the exception of $NH_2$, that is to say B represents Cl, F, Br, $NO_2$, $CF_3$, an alkyl radical having from 1 to 4 carbon atoms, an alkoxy radical having from 1 to 4 carbon atoms, acetamido or benzamido,

– u represents a whole number from O to 2,

– v represents a whole number from O to 2, or a disulphohalide, notably disulphochloride of formula

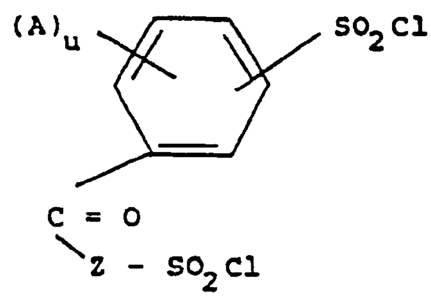

in which A, u, Z have the meanings indicated above, is made to react on an aninoalkylamine of formula

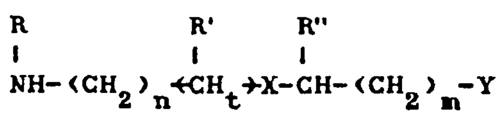

in which

– R represents H, an alkyl radical having from 1 to 6 carbon atoms or a benzyl radical,

– R′ and R″ represent H or an alkyl radical having from 1 to 4 carbon atoms,

– n represents a whole number varying from O to 1O,

– m represents a whole number varying from O to 1O,

– t is equal to O or 1,

– Y represents $NH_2$, $NHR_6$, $NR_6R_7$, $R_6$ and $R_7$ representing an alkyl radical having from 1 to 4 carbon atoms, or form a non-aromatic cyclised amine with the nitrogen.

29. Process for preparation as claimed in claim 25, characterised in that the amino acid used or the dialkylaminoalkylamine used is chosen respectively from amongst :

$$- NH_2 - (CH_2)_n - COOH,$$

n varying from 1 to 11,

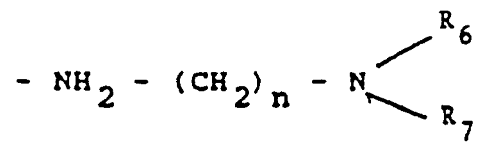 n = 2 or 3

– $R_6$ and $R_7$ representing an alkyl having from 1 to 4 atoms of carbon or a morpholine nucleus with the nitrogen atom,
and the sulphochloride used is chosen respectively from amongst :
– 2-benzoyl-4-nitro-benzenesulphonyl chloride,
– 2-benzoyl-4-chloro-benzenesulphonyl chloride,
– 4-chloro-2-(2-chlorobenzoyl) benzenesulphonyl chloride,
– 2-benzoyl benzenesulphonyl chloride,
– 2-(4-chlorobenzoyl) benzenesulphonyl chloride,
– 4-benzoyl benzenesulphonyl chloride,
– 3-(2,4-dichlorobenzoyl)-4-chloro benzenesulphonyl chloride,
– 3-benzoyl-4-chloro benzenesulphonyl chloride,
– 3-(4-chlorobenzoyl)-4-chloro benzenesulphonyl chloride,
– 4-benzoyl-3-chloro benzenesulphonyl chloride,
– 4-chloro-3-(2-thenoyl) benzenesulphonyl chloride.

30. Process as claimed in claim 24 for preparation of compounds of formula III

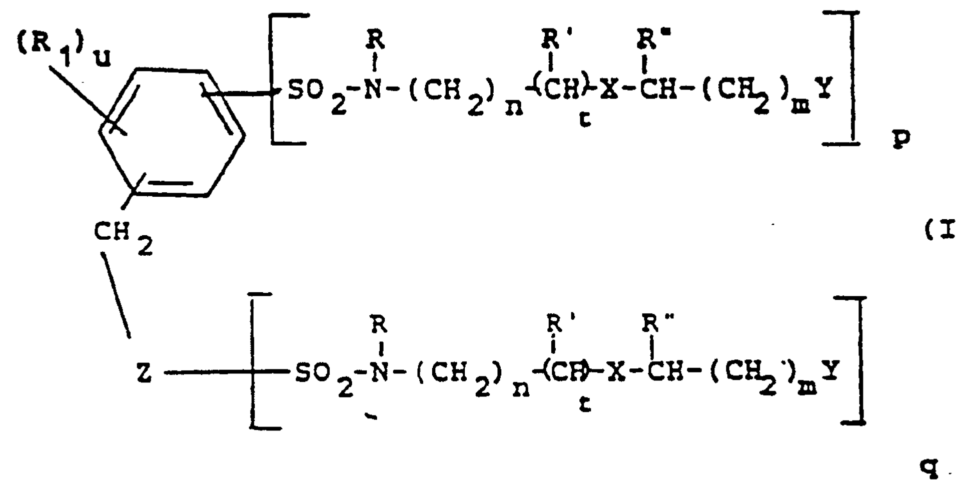

(III)

in which $R_1$, R, R′, R″, X, Y, Z, m, n, t, p, q and u have the meanings indicated in claim 3, characterised in that the compound of formula

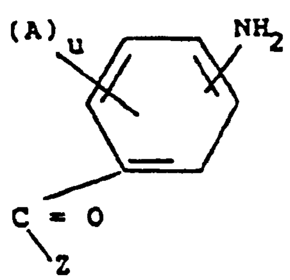

or of formula

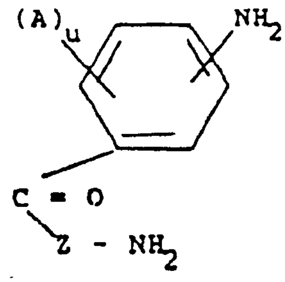

in which A has the meaning indicated claimed in 24, is subjected to a reduction to reduce C=O to $CH_2$ and to obtain the compound of formula

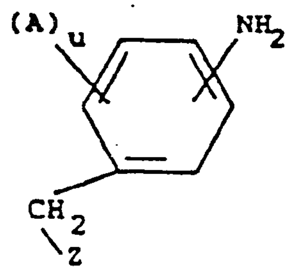

or

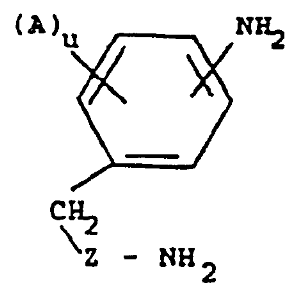

– one of these compounds is subjected to the Sandmeyer reaction in order to obtain compounds of formula

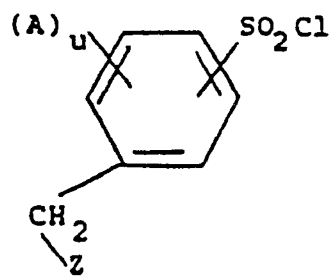

or

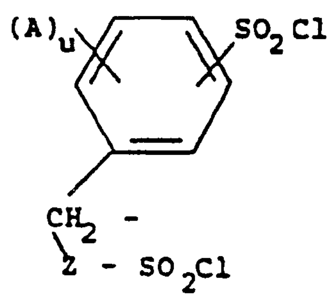

– the sulphohalide or the disulphohalide of formula indicated above is condensed on a compound of formula

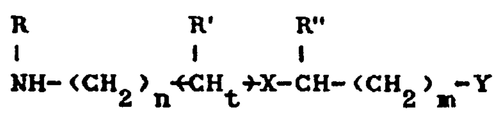

in which R, R', R", X, Y, n, m and t have the meanings indicated in claim 24, followed possibly by a reduction when A and/or B represents $NO_2$.

31. Process for preparation as claimed in claim 30, characterised in that the amino acid used or the dialkylaminoalkylamine used is chosen respectively from amongst :

$$- NH_2 - (CH_2)_n - COOH,$$

n varying from 1 to 11,

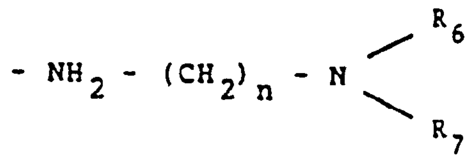

n = 2 or 3

– $R_6$ and $R_7$ representing an alkyl group having from 1 to 4 atoms of carbon or a morpholine nucleus with the nitrogen atom,
and the compound of formula :

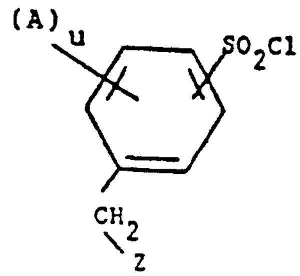

– 3-(2,4-dichlorobenzyl)-4-chloro benzenesulphonyl chloride,
– 3-(4-chlorobenzyl)-4-chloro benzenesulphonyl chloride,
– 2-benzyl-4-chloro benzenesulphonyl chloride,
– 2-(2,4-dichlorobenzyl)-4-chloro benzenesulphonyl chloride,
– 2-(4-chlorobenzyl)-4-chloro benzenesulphonyl chloride,
– 4-benzyl-3-chloro benzenesulphonyl chloride,
– 4-(2,4-dichlorobenzyl)-3-chloro benzenesulphonyl chloride,
– 4-(4-chlorobenzyl)-3-chloro benzenesulphonyl chloride,
– 3-benzyl-4-chloro benzenesulphonyl chloride.

32. Process according to claim 24, for preparation of compounds of formula IV,

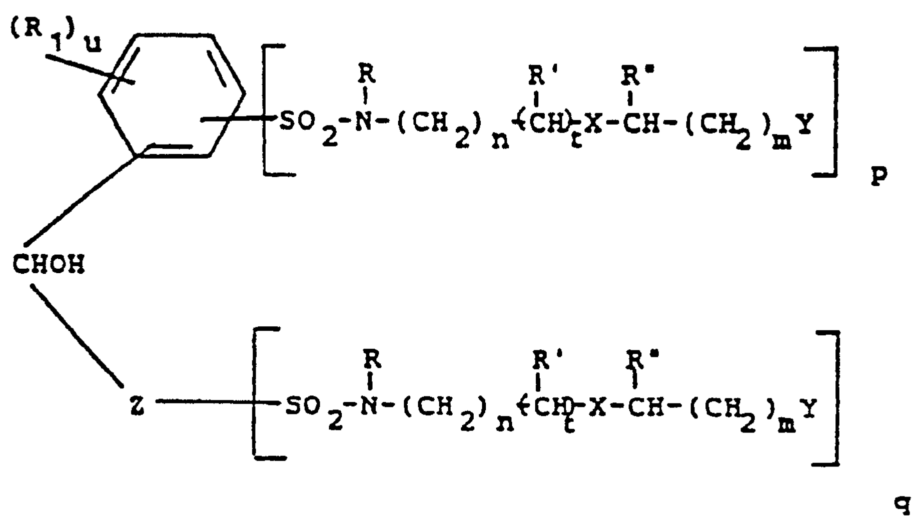

in which $R_1$, R, R', R", X, Y, Z, m, n, t, p, q and u have the meanings indicated in claim 4, characterised in that the compound of formula

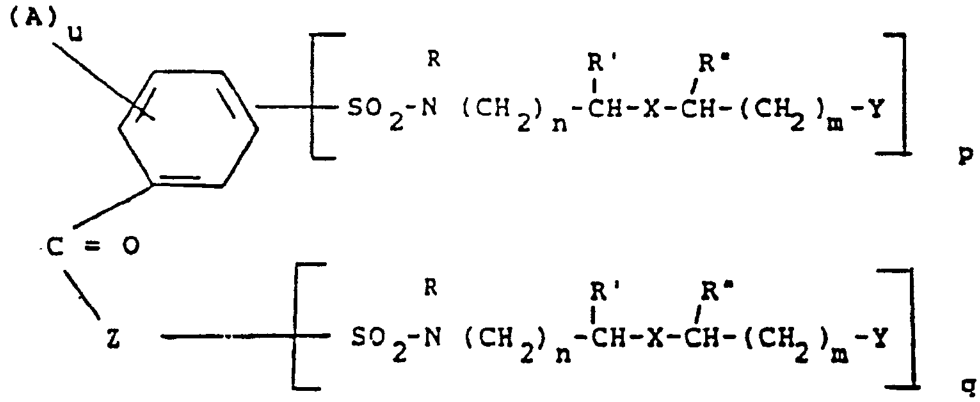

in which A, X, Y, Z, R', R", m, n, p, q, t and u have the meanings indicated in claim 24, and such as is obtained according to claim 25, is subjected to a reduction to transform C=O into CHOH with the aid of alkaline borohydride, before the possible reduction of A and/or B when they represent $NO_2$.

33. Process for preparation as claimed in claim 32, characterised in that the amino acid used or the dialkylaminoalkylamine used is chosen from amongst :

$$- NH_2 - (CH_2)_n - COOH,$$

n varying from 1 to 11,

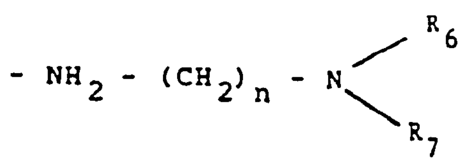

n = 2 or 3

– $R_6$ and $R_7$ representing an alkyl having from 1 to 4 atoms of carbon or a morpholine nucleus with the nitrogen atom,

and the sulphochloride used is chosen from amongst :

– 2-benzoyl-4-nitro-benzenesulphonyl chloride,
– 2-benzoyl-4-chloro-benzenesulphonyl chloride,
– 4-chloro-2-(2-chlorobenzoyl) benzenesulphonyl chloride,
– 2-benzoyl benzenesulphonyl chloride,
– 2-(4-chlorobenzoyl) benzenesulphonyl chloride,
– 4-benzoyl benzenesulphonyl chloride,
– 3-(2,4-dichlorobenzoyl)-4-chloro benzenesulphonyl chloride,
– 3-benzoyl-4-chloro benzenesulphonyl chloride,
– 3-(4-chlorobenzoyl)-4-chloro benzenesulphonyl chloride,
– 4-benzoyl-3-chloro benzenesulphonyl chloride,
– 4-chloro-3-(2-thenoyl) benzenesulphonyl chloride.

34. The compounds as claimed in one of claims 1 to 23 as a therapeutic substance.

35. Pharmaceutical substance characterised in that it contains by way of active substance one of the compounds as claimed in any one of claim 1 to 23 in association with a pharmaceutically acceptable vehicle.

36. Pharmaceutical composition intended for the treatment of inflammatory, allergic or asthmatic conditions, characterised in that it contains as active substance one of the compounds as claimed in one of claim 1 to 23.

37. Pharmaceutical composition intended for the treatment of renal failure and of cardiovascular pathologies, characterised in that it contains as active substance one of the compounds as claimed in one of claims 1 to 23.

38. Pharmaceutical compositions including by way of active substance at least one of the compounds as claimed in one of claims 1 to 23 associated with a pharmaceutically acceptable vehicle, in the form of indictable preparations containing 1O to 1OO mg of active substance per unit dose.

39. Pharmaceutical compositions including by way of active substance at least one of the compounds as

claimed in one of claims 1 to 23, associated with a pharmaceutically acceptable vehicle, in the form of presentations intended for oral administration, containing from 1O to 5OO mg per unit dose.

**Claims for the following Contracting State : GR**

1. Compound of the formula I

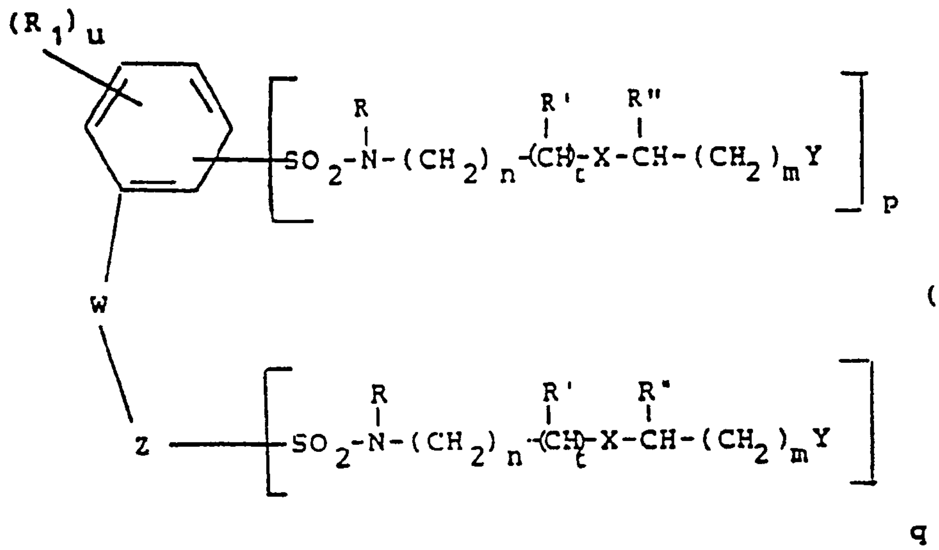

(I)

in which :

– W represents C = O, $CH_2$ or CHOH

– Z represents :

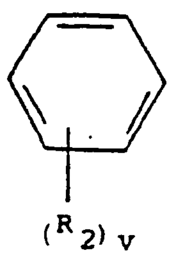

or

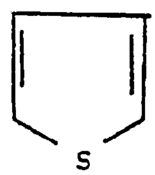

– $R_1$ represents Cl, F, Br, $NO_2$, $NH_2$, $CF_3$, an alkyl radical having from 1 to 4 carbon atom, notably methyl or ethyl, an alkoxy radical having from 1 to 4 carbon atoms, acetamido or benzamido,

– $R_2$ represents Cl, F, Br, $NO_2$, $NH_2$, $CF_3$, an alkyl radical having from 1 to 4 carbon atoms, an alkoxy radical having from 1 to 4 carbon atom, acetamido or benzamido,

– R represents H, an alkyl radical having from 1 to 6 carbon atoms or a benzyl radical,

– R′ and R″ represent H or an alkyl radical having from 1 to 4 carbon atoms,

– X represents $(CH_2)_r$, r being O or 1, -CH = CH

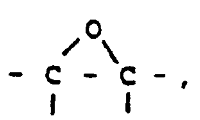

or

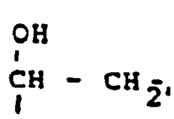

– Y represents $COOR_3$, $R_3$ representing a hydrogen or an alkyl radical having from 1 to 4 carbon atoms,

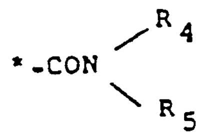 ,

$R_4$ and $R_5$ representing H or an alkyl radical having from 1 to 4 carbon atoms

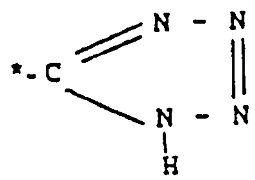 ,

* - C ≡ N,

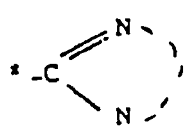 , 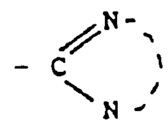

representing a non-aromatic cycle of 5 or 6 links, in which the 2 or 3 atom which enter into the cycle are atoms of carbon and/or of oxygen and/or of nitrogen,

* $NH_2$, $NHR_6$, $NR_6R_7$,

$R_6$ and $R_7$ representing an alkyl radical having from 1 to 4 carbon atoms, or capable of forming a non-aromatic cyclised amine with the nitrogen atom,

– provided that when Y represents $-NH_2$, X is different from

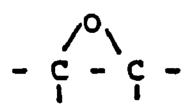

– u is a whole number from O to 2,
– v is a whole number from O to 2,
– p is equal to O or 1,
– q is equal to O or 1, p + q being equal to or greater than 1,
– n is a whole number varying from O to 1O,
– m is a whole number varying from O to 1O,
– t is equal to O or 1,
– the total number of carbon atoms in the chain

$$-(CH_2)_n - (CH)_t - X - CH - (CH_2)_m Y$$

varying from 2 to 20, as well as their physiologically acceptable salts.

2. Compound as claimed in claim 1 complying with the following formula II

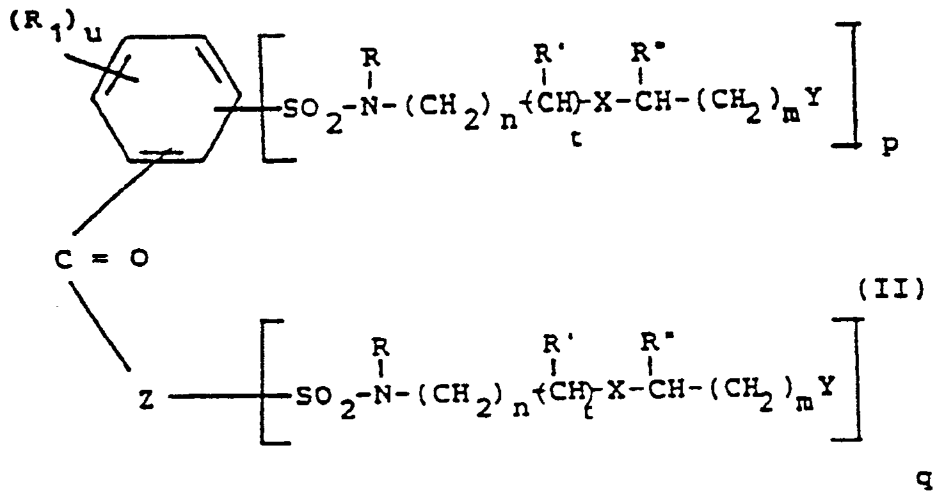

(II)

in which $R_1$, R, R′, R″, X, Y, Z, m, n, t, p, q and u have the meanings indicated in claim 1.
3. Compound as claimed in claim 1, complying with the following formula III

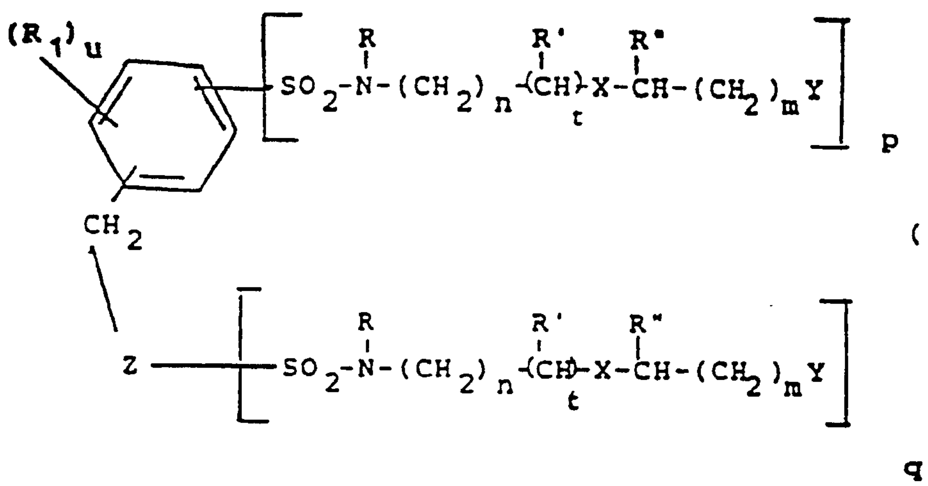

(III)

in which $R_1$, R, R′, R″, X, Y, Z, m, n, t, p, q and u have the meanings indicated in claim 1.
4. Compound as claimed in claim 1, complying with the following formula IV

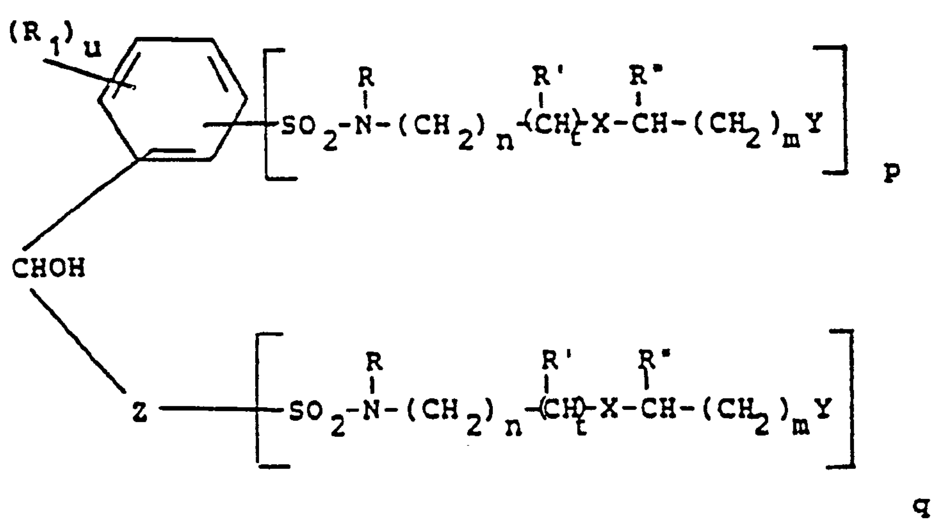

in which $R_1$, R, R', R", X, Y, Z, m, n, t, p, q and u have the meanings indicated in claim 1.

5. Compound as claimed in claim 2, complying with the following formula V

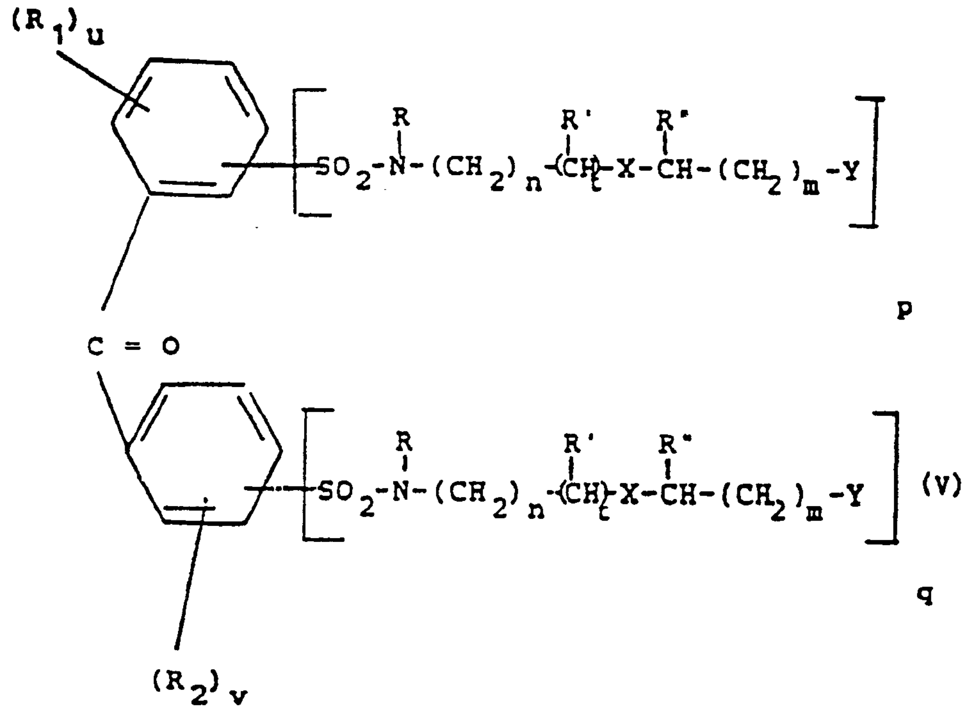

(V)

in which $R_1$, $R_2$. R, R', R", X, Y, Z, m, n, p, q, t, u and v the meanings indicated in claim 1.

6. Compound as claimed in claim 5, complying with formula V, in which -Y represents $COOR_3$, $R_3$ representing H or an alkyl radical having from 1 to 4 carbon atoms.

7. Compound as claimed in claim 5, complying with formula V, in which -Y represents $NH_2$, $NHR_6$ or $NR_6R_7$, $R_6$ and $R_7$ having the meanings indicated in claim 1.

8. Compound as claimed in claim 5, complying with formula V, in which p or q is equal to O.

9. Compound as claimed in claim 8, characterised in that

- u + v is greater than or equal to 1,
- the total number of carbon atoms in the chain

$$(CH_2)_n - (CH)_t - X - CH - (CH_2)_m - Y$$

is equal to or greater than 5.

10. Compound as claimed in claim 5, complying with formula V, in which

- p = 1,
- q = 1,
- u + v is greater than or equal to 1,
- the total number of carbon atoms in the chain

$$(CH_2)_n - (CH)_t - X - CH - (CH_2)_m - Y$$

is equal to or greater than 5.

11. Compound as claimed in claim 1, complying with the following formula VI

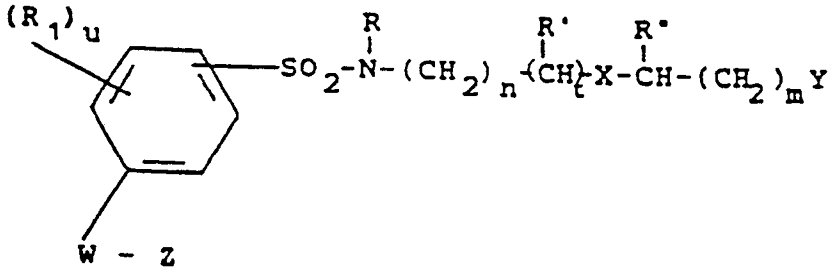

(VI)

in which $R_1$, R, R′, R″, X, Y, Z, W, m, n, u and t have the meanings indicated in claim 1.

12. Compound as claimed in claim 11, complying with the following formula VII

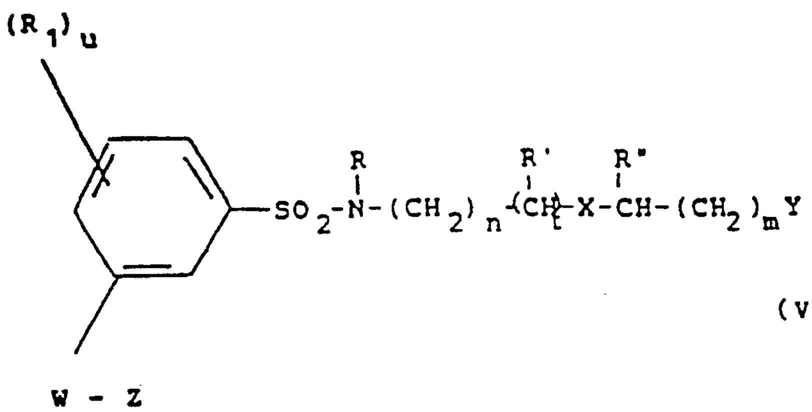

(VII)

in which $R_1$, R, R′, R″, X, Y, Z, W, m, n, u and t have the meanings indicated in claim 1.

13. Compound as claimed in claim 12, complying with formula VII, in which -Y represents $COOR_3$, $R_3$ representing H or an alkyl radical having from 1 to 4 carbon atoms.

14. Compound as claimed in claim 13, complying with the following formula VIII

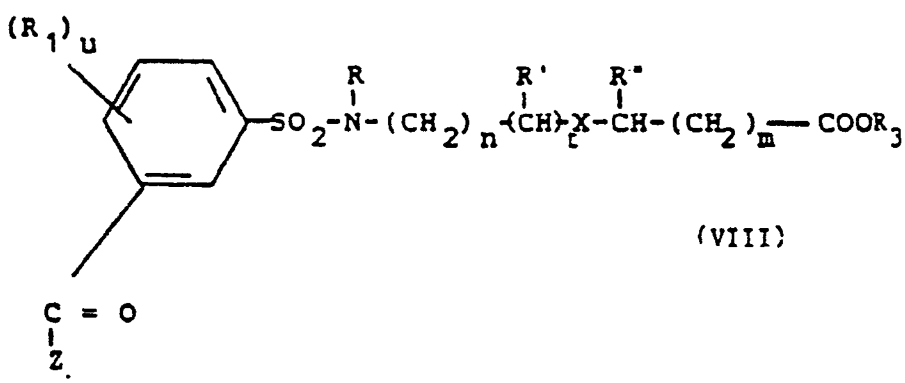

(VIII)

in which $R_1$, $R_3$, R, R′, R″, X, Z, m, n, u and t have the meanings indicated in claim 1.

15. Compound as claimed in claim 13, complying with the following formula IX

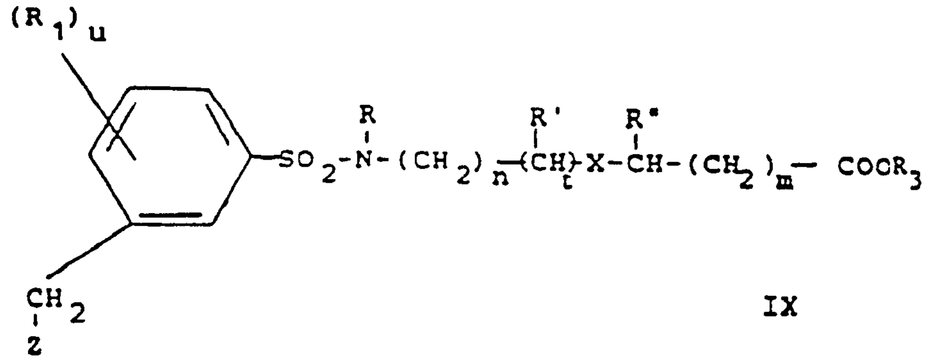

IX

in which $R_1$, $R_3$, R, R', R", X, Z, m, n, u and t have the meanings indicated in claim 1.

16. Compound as claimed in claim 13, complying with the following formula X

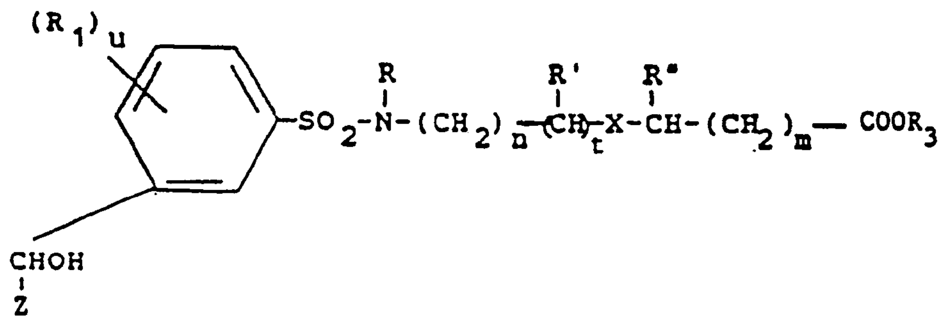

X

in which $R_1$, $R_3$, R, R', R", X, Z, m, n, u and t have the meanings indicated in claim 1.

17. Compound as claimed in claim 14, complying with formula VIII, in which the total number of carbon atoms of the chain

$$(CH_2)_n - (\overset{|}{C}H)_t - X - \overset{|}{C}H - (CH_2)_m - Y$$

is equal to or greater than 5.

18. Compound as claimed in claim 15, complying with formula IX, in which the total number of carbon atoms of the chain

$$(CH_2)_n - (\overset{|}{C}H)_t - X - \overset{|}{C}H - (CH_2)_m - Y$$

is equal to or greater than 5.

19. Compound as claimed in claim 16, complying with formula X, in which the total number of carbon atoms of the chain

$$(CH_2)_n - (\overset{|}{C}H)_t - X - \overset{|}{C}H - (CH_2)_m - Y$$

is equal to or greater than 5.

20. Compound as claimed in claim 13, complying with the following formula XI,

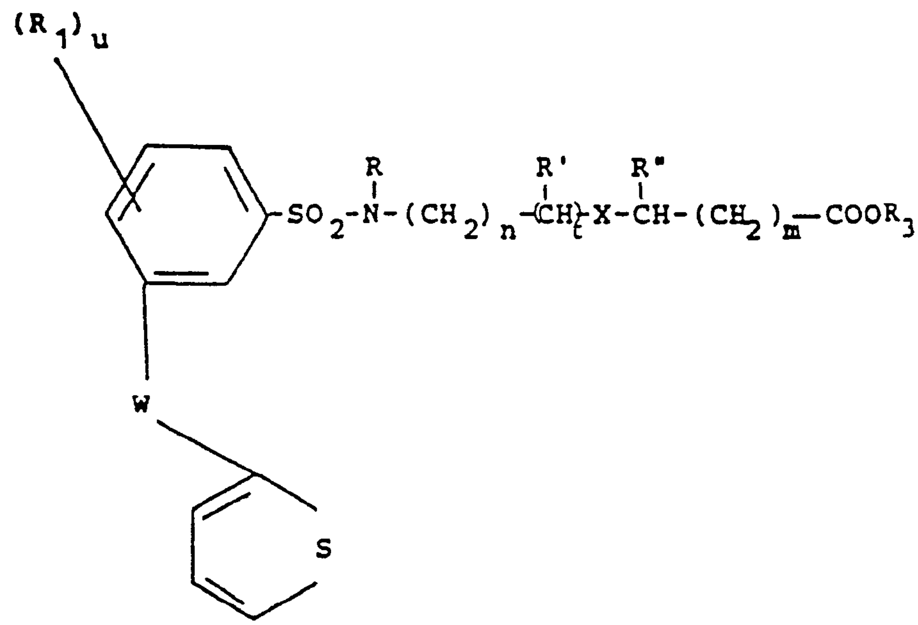

XI

in which the total number of carbon atoms of the chain

$$(CH_2)_n - (\overset{|}{C}H)_t - X - \overset{|}{C}H - (CH_2)_m - Y$$

is equal to or greater than 5, and in which $R_1$, $R_3$, R, R', R'', X, W, m, n and t have the meanings indicated in claim 1.

21. Compound as claimed in claim 11, complying with the following formula VIbis

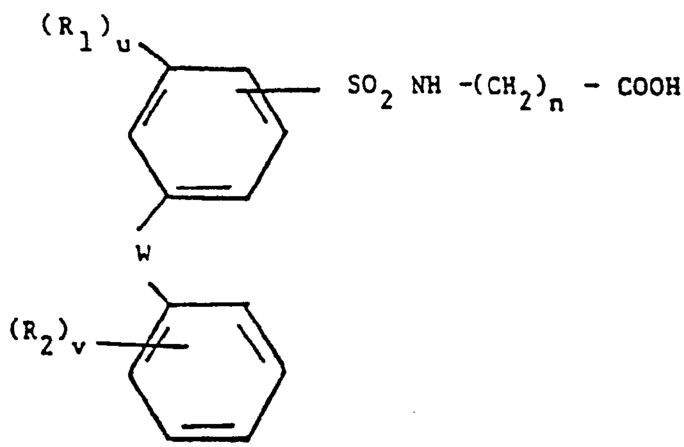

in which :

- $R_1$ and $R_2$ represent a halogen,
- u varies from O to 2
- v varies from O to 2,
- W represents C = O, CHOH, $CH_2$
- n is equal to 3 to 11.

22. Compound as claimed in claim 11, complying with the following formula VIter :

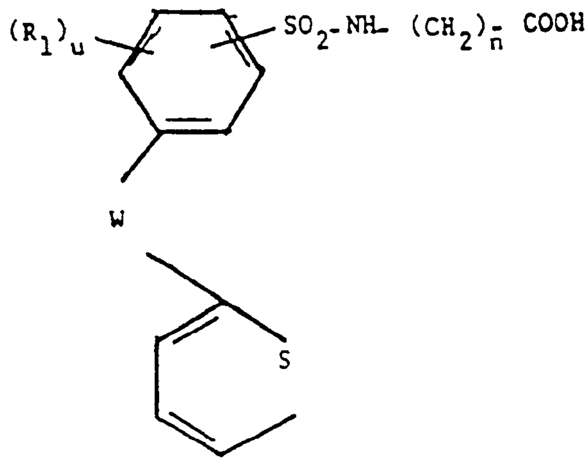

in which :

- $R_1$ represents a halogen,
- u is equal to 2,
- W represents C = O, CHOH, $CH_2$
- n is equal to 3 to 11.

23. Compounds as claimed in claim 1, complying with the following formulae :

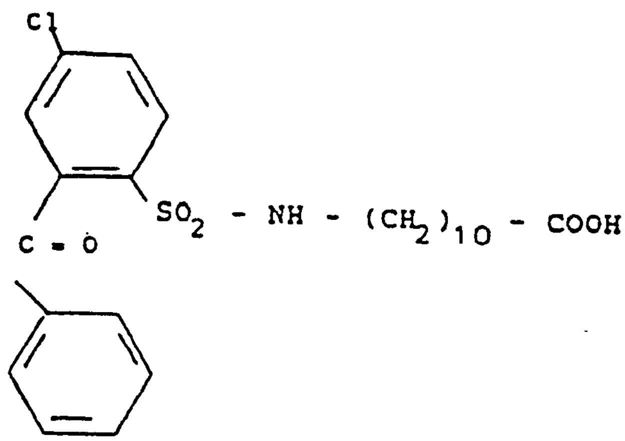

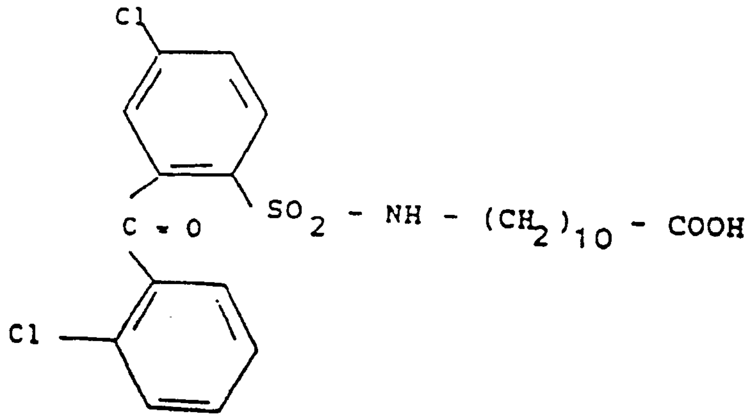

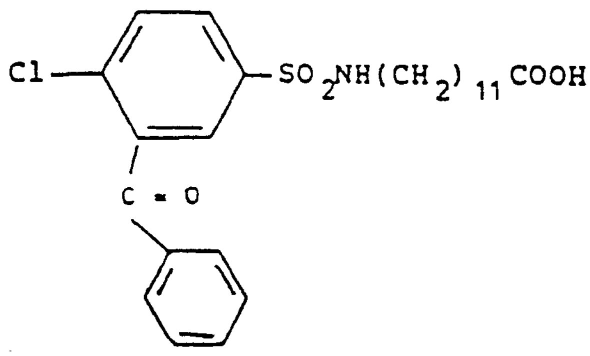

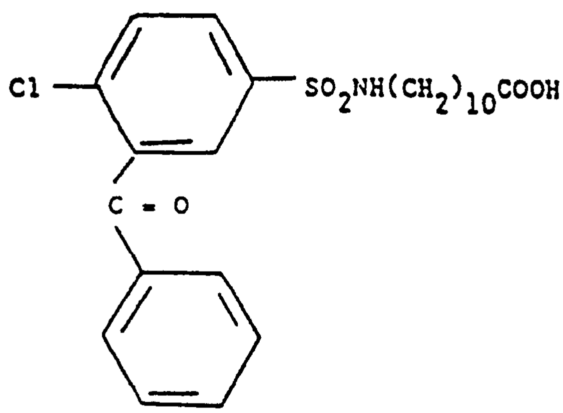

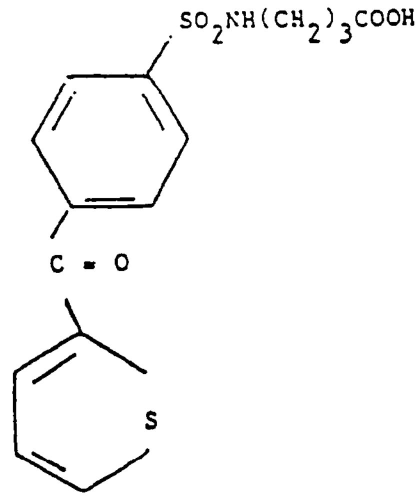

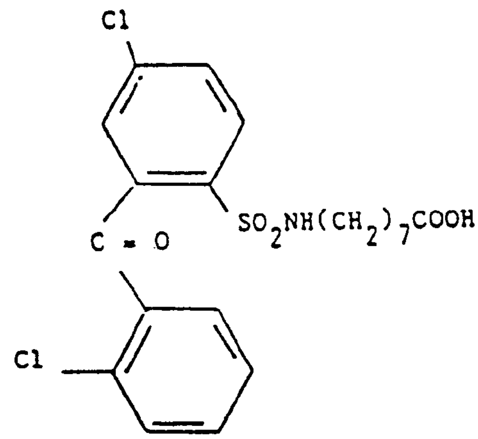

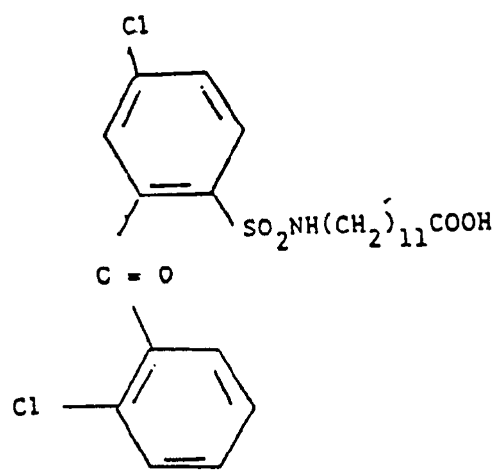

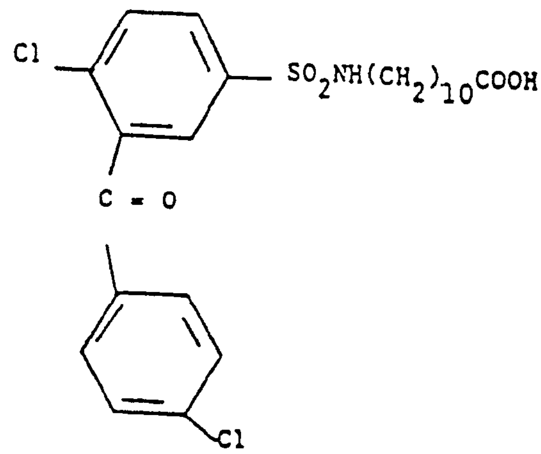

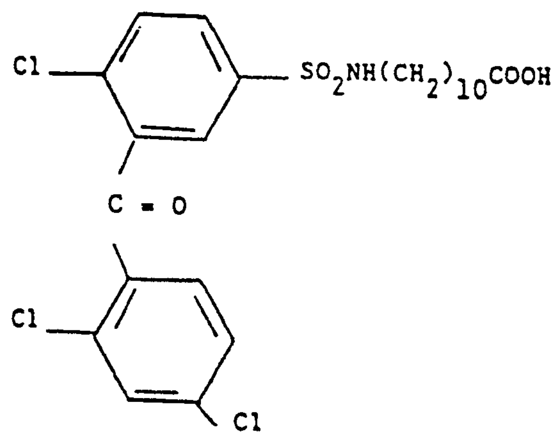

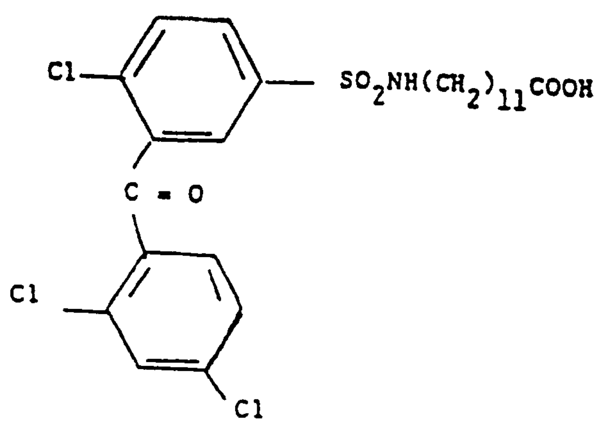

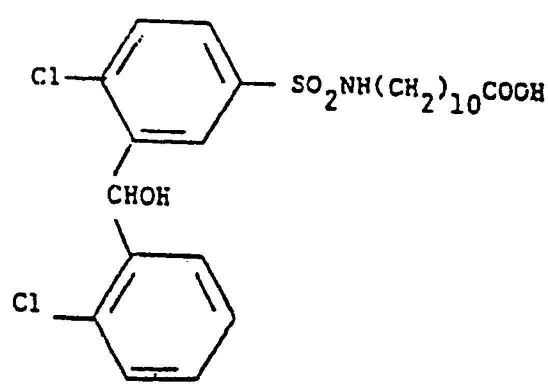

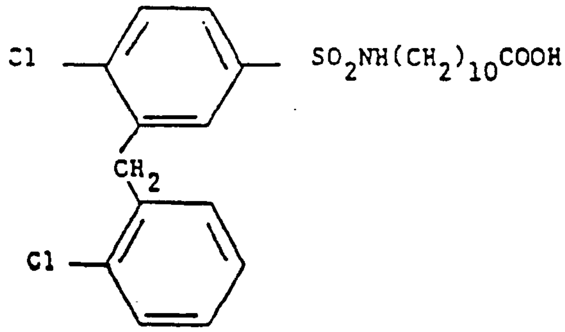

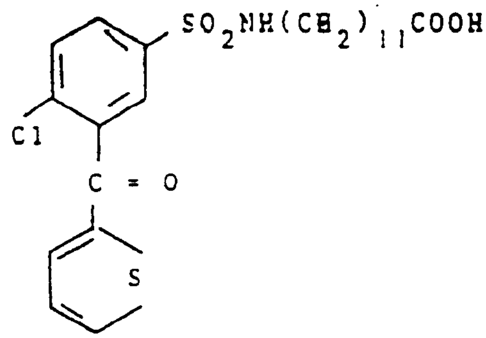

24. Process for preparation of the compounds of formula I, as claimed in claim 1, characterised in that a compound of formula

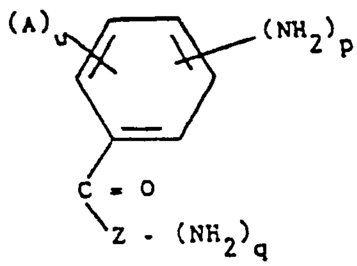

in which :
– A has the meanings indicated for $R_1$, with the exception of $NH_2$, that is to say A represents Cl, F, Br, $NO_2$, $CF_3$, an alkyl radical having from 1 to 4 carbon atoms, an alkoxy radical having from 1 to 4 carbon atoms, acetamido or benzamido,
– Z represents

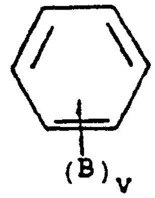

or

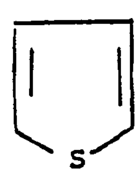

– B has the meanings indicated for $R_2$, with the exception of $NH_2$, that is to say B represents Cl, $NO_2$, $CF_3$, an alkyl radical having from 1 to 4 carbon atoms, an alkoxy radical having from 1 to 4 carbon atoms, acetamido or benzamido and
– u represents a whole number from O to 2,
– v represents a whole number from O to 2,
– p = O or 1,
– q = O or 1, p+q being equal to or greater than 1,
. is possibly subjected to a reduction, notably with the aid of aluminium chloride, aluminium hydride and lithium

hydride to reduce -C=O to $CH_2$ and to obtain the compound of formula

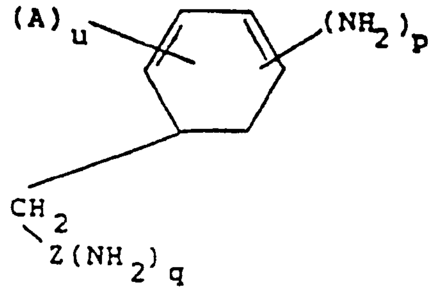

in which A, Z, u, p and q have the meanings indicated above,
. one of the amino compounds of the formula indicated above is subjected to the Sandmeyer reaction in order to obtain a sulphohalide of formula :

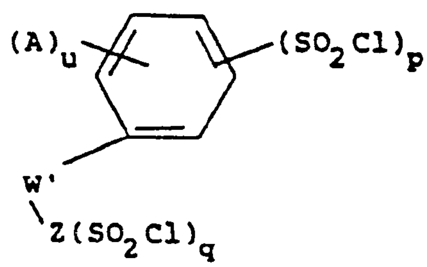

in which A, Z, u, p and q have the meanings indicated above and W′ represents C = O or $CH_2$,
. the sulphohalide, notably sulphochloride, of the formula indicated above is made to react on one or two compounds of formula

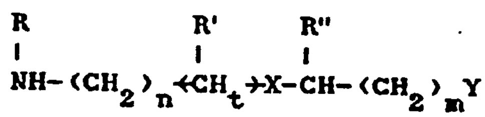

in which
– R represents H, an alkyl radical having from 1 to 6 carbon atoms or a benzyl radical,
• R′ and R″ represent H or an alkyl radical having from 1 to 4 carbon atoms,
– X represents $(CH_2)_r$, r being equal to O or 1 or -CH=CH,

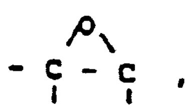

, or

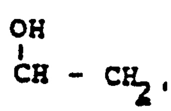

– Y represents $COOR_3$, $R_3$ representing a hydrogen or an alkyl radical having from 1 to 4 carbon atoms,

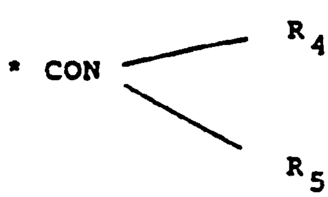

$R_4$ and $R_5$ representing H or an alkyl radical having from 1 to 4 carbon atoms

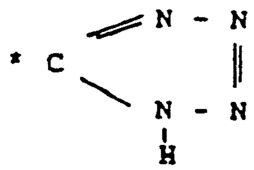 ,

- C ≡ N,

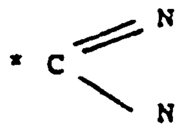 , 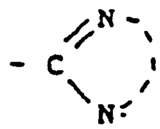

representing a non-aromatic cycle of 5 or 6 links, in which the 2 or 3 atoms which enter into the cycle are atoms of carbon and/or of oxygen and/or of nitrogen,

* $NH_2$, $NHR_6$, $NR_6R_7$,

$R_6$ and $R_7$ representing an alkyl radical having from 1 to 4 carbon atoms, or capable of forming a non-aromatic cyclised amine with the nitrogen atom,

– provided that when Y represents $-NH_2$, X is different from

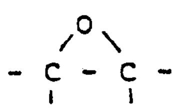

– n is a whole number varying from O to 1O,
– m is a whole number varying from O to 1O,
– t is equal to O or 1,
– the total number of carbon atoms in the chain

$$-(CH_2)_n - (CH)_t - X - CH - (CH_2)_m Y$$

varying from 2 to 2O,

in order to obtain the compound of formula

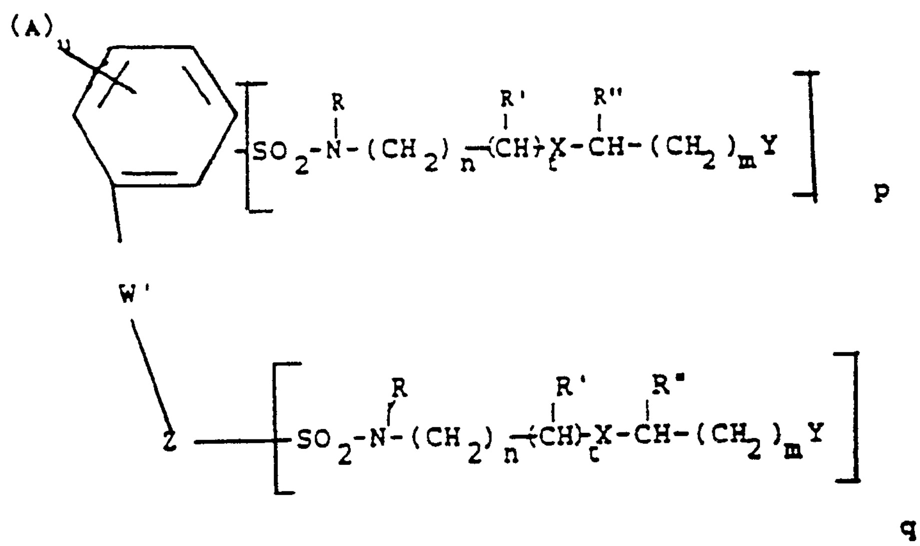

in which A, Z, W', R, R', R", X, Y, m. n. t. q and u have the meanings indicated above,

. and in the case where W' represents C=O the above compound is possibly subjected to a reduction, notably

with the aid of alkaline borohydride, such as sodium borohydride, to reduce C=O to CHOH,
. then a reduction is possibly carried out when A and/or B represents $NO_2$.

25. Process as claimed in claim 24 for preparation of compounds of formula II

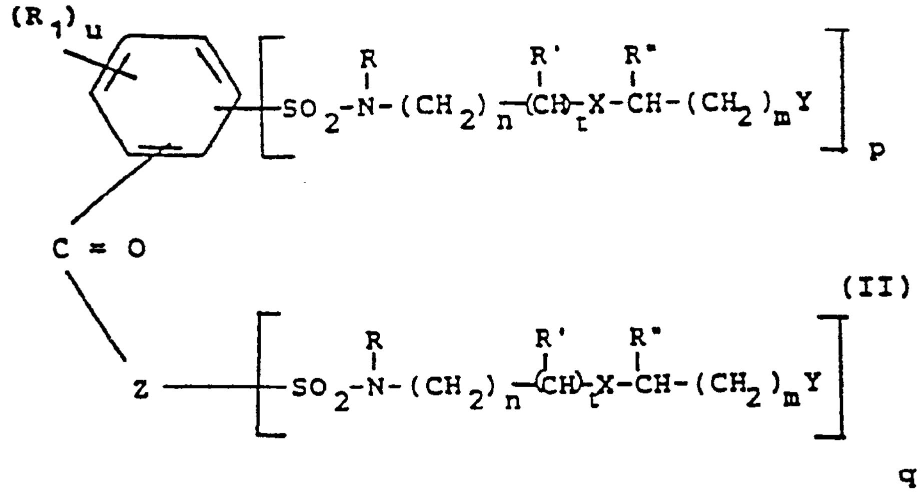

(II)

in which $R_1$, R, R', R", X, Y, Z, m, n, p, q, u and t have the meanings indicated in claim 2, characterised in that a sulphohalide, notably a sulphochloride of formula

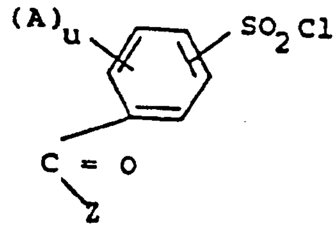

in which A, Z and u have the meanings indicated in claim 24, or a disulphohalide, notably a disulphochloride of formula

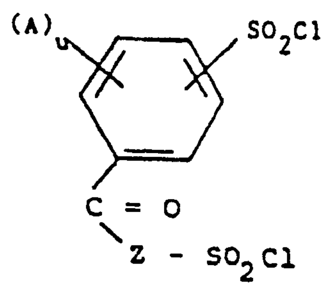

in which A, u and Z have the meanings indicated previously, is made to react on one or two compounds of formula

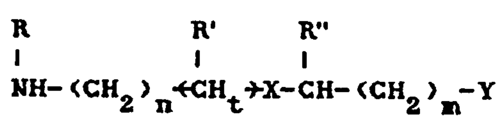

in which R, R', R", n, m, t, X and Y have the meanings indicated in claim 24,
- possibly followed by a reduction when A and/or B represents $NO_2$.

26. Process as claimed in claim 25, characterised in that a sulphochloride of formula

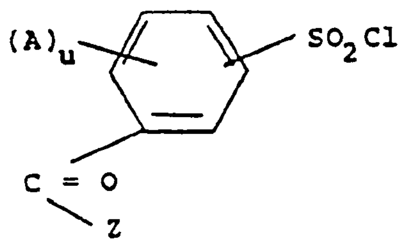

in which A, u and Z have the meanings indicated in claim 25, or a disulphochloride of formula

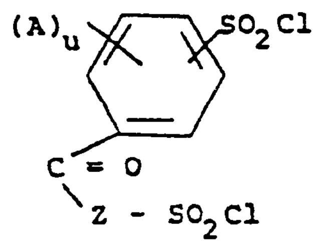

in which A, u, A have the meanings indicated in claim 25, is made to react on one or two amino acids of formula

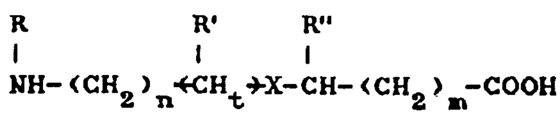

in which R, R′, R″, n, m, t, and X have the meanings indicated above in claim 25.

27. Process as claimed in claim 25, characterised in that

– a compound such as that obtained according to claim 26, and in which Y represents COOH, is esterified in a compound of formula II, in which Y represents $COOR_3$, $R_3$ representing an alkyl radical having from 1 to 4 carbon atoms,

– or a compound such as that obtained according to claim 26 is transformed into a compound of formula II in which Y represents

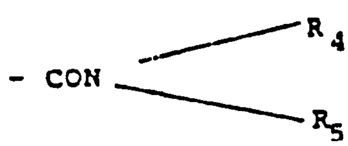

$R_4$, $R_5$ representing H or an alkyl radical having from 1 to 4 carbon atoms,

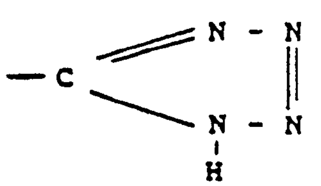 , - C≡N 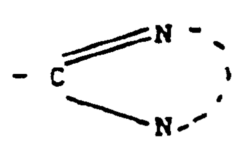

28. Process as claimed in claim 25, characterised in that a sulphochloride of formula

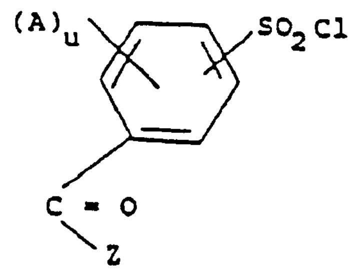

in which :

– A has the meanings indicated for $R_1$, with the exception of $NH_2$, that is to say Cl, F, Br, $NO_2$, $CF_3$, an alkyl radical having fron 1 to 4 carbon atoms, an alkoxy radical having from 1 to 4 carbon atoms, acetamido or benzamido,

– Z represents :

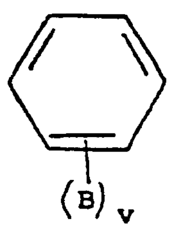

or

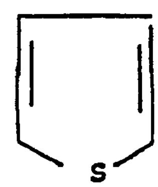

– B has the meanings indicated for $R_2$, with the exception of $NH_2$, that is to say B represents Cl, F, Br, $NO_2$, $CF_3$, an alkyl radical having from 1 to 4 carbon atoms, an alkoxy radical having from 1 to 4 carbon atoms, acetamido or benzamido,

– u represents a whole number from O to 2,

– v represents a whole number from O to 2,

or a disulphohalide, notably disulphochloride of formula

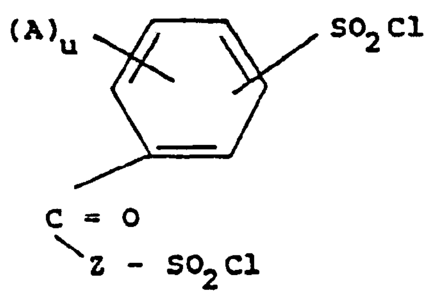

in which A, u, Z have the meanings indicated above, is made to react on an aminoalkylamine of formula

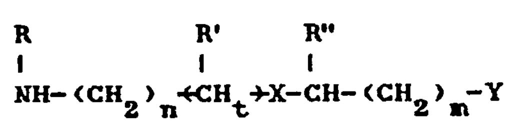

in which

– R represents H, an alkyl radical having from 1 to 6 carbon atoms or a benzyl radical,

– R′ and R″ represent H or an alkyl radical having from 1 to 4 carbon atoms,

– n represents a whole number varying from O to 1O,

– m represents a whole number varying from O to 1O,

– t is equal to O or 1,

– Y represents $NH_2$, $NHR_6$, $NR_6R_7$, $R_6$ and $R_7$ representing an alkyl radical having from 1 to 4 carbon atoms, or form a non-aromatic cyclised amine with the nitrogen.

29. Process for preparation as claimed in claim 25, characterised in that the amino acid used or the dialkylaminoalkylamine used is chosen respectively from amongst :

$$-NH_2-(CH_2)_n-COOH,$$

n varying from 1 to 11,

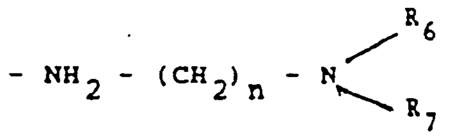

n = 2 or 3

– $R_6$ and $R_7$ representing an alkyl having from 1 to 4 atoms of carbon or a morpholine nucleus with the nitrogen atom,
and the sulphochloride used is chosen respectively from amongst:
– 2-benzoyl-4-nitro-benzenesulphonyl chloride,
– 2-benzoyl-4-chloro-benzenesulphonyl chloride,
– 4-chloro-2-(2-chlorobenzoyl) benzenesulphonyl chloride,
– 2-benzoyl benzenesulphonyl chloride,
– 2-(4-chlorobenzoyl) benzenesulphonyl chloride,
– 4-benzoyl benzenesulphonyl chloride,
– 3-(2,4-dichlorobenzoyl)-4-chloro benzenesulphonyl chloride,
– 3-benzoyl-4-chloro benzenesulphonyl chloride,
– 3-(4-chlorobenzoyl)-4-chloro benzenesulphonyl chloride,
– 4-benzoyl-3-chloro benzenesulphonyl chloride,
– 4-chloro-3-(2-thenoyl) benzenesulphonyl chloride.

3O. Process as claimed in claim 24 for preparation of compounds of formula III

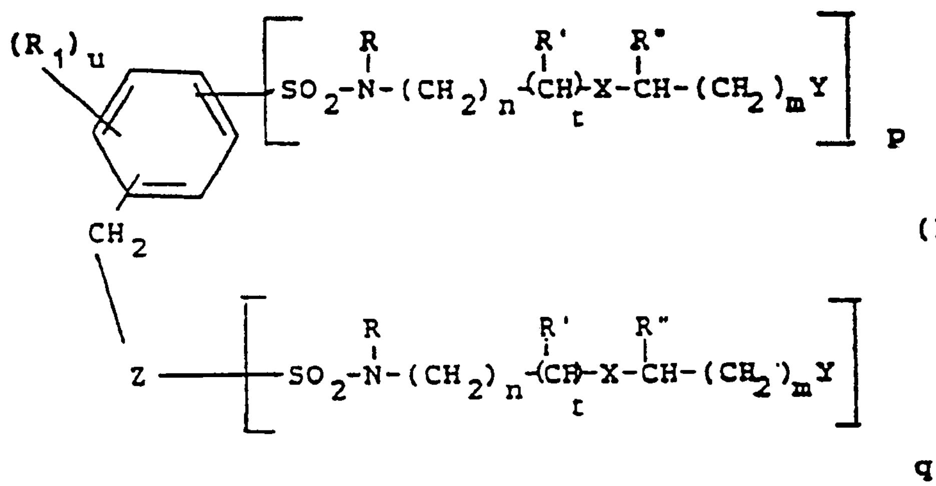

(III)

in which $R_1$, R, R′, R″, X, Y, Z, m, n, t, p, q and u have the meanings indicated in claim 3, characterised in that the compound of formula

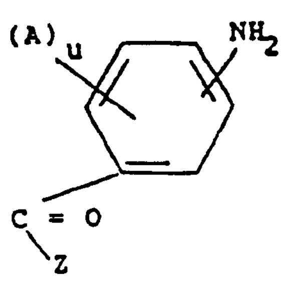

or of formula

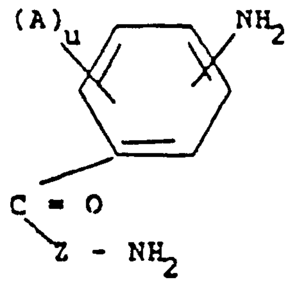

in which A has the meaning indicated claimed in 24, is subjected to a reduction to reduce C=O to $CH_2$ and to obtain the compound of formula

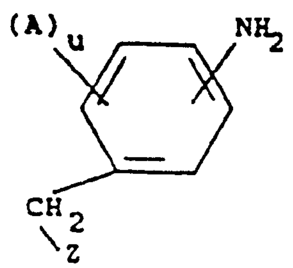

or

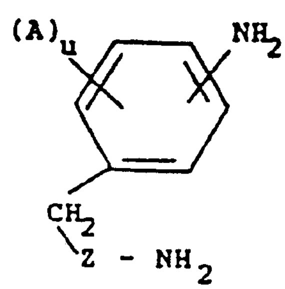

– one of these compounds is subjected to the Sandmeyer reaction in order to obtain compounds of formula

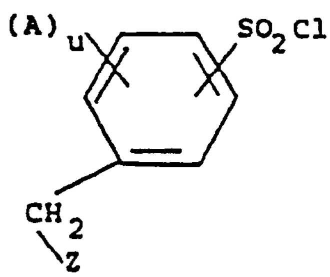

or

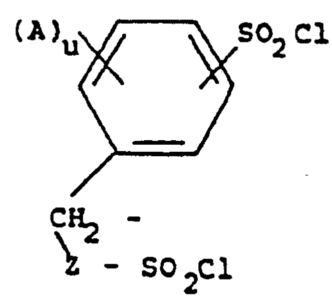

– the sulphohalide or the disulphohalide of formula indicated above is condensed on a compound of formula

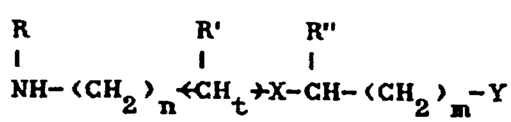

in which R, R′, R″, X, Y, n, m and t have the meanings indicated in claim 24, followed possibly by a reduction when A and/or B represents $NO_2$.

31. Process for preparation as claimed in claim 30, characterised in that the amino acid used or the dialkylaminoalkylamine used is chosen respectively from amongst :

$$-\ NH_2-\ (CH_2)_n\ -\ COOH,$$

n varying from 1 to 11,

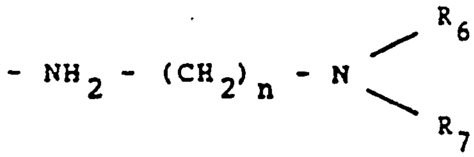

n = 2 or 3

– $R_6$ and $R_7$ representing an alkyl group having from 1 to 4 atoms of carbon or a morpholine nucleus with the nitrogen atom,
and the compound of formula

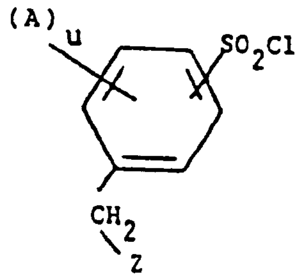

– 3-(2,4-dichlorobenzyl)-4-chloro benzenesulphonyl chloride,
– 3-(4-chlorobenzyl)-4-chloro benzenesulphonyl chloride,
– 2-benzyl-4-chloro benzenesulphonyl chloride,
– 2-(2,4-dichlorobenzyl)-4-chloro benzenesulphonyl chloride,
– 2-(4-chlorobenzyl)-4-chloro benzenesulphonyl chloride,
– 4-benzyl-3-chloro benzenesulphonyl chloride,
– 4-(2,4-dichlorobenzyl)-3-chloro benzenesulphonyl chloride,
– 4-(4-chlorobenzyl)-3-chloro benzenesulphonyl chloride,
– 3-benzyl-4-chloro benzenesulphonyl chloride.

32. Process according to claim 24, for preparation of compounds of formula IV,

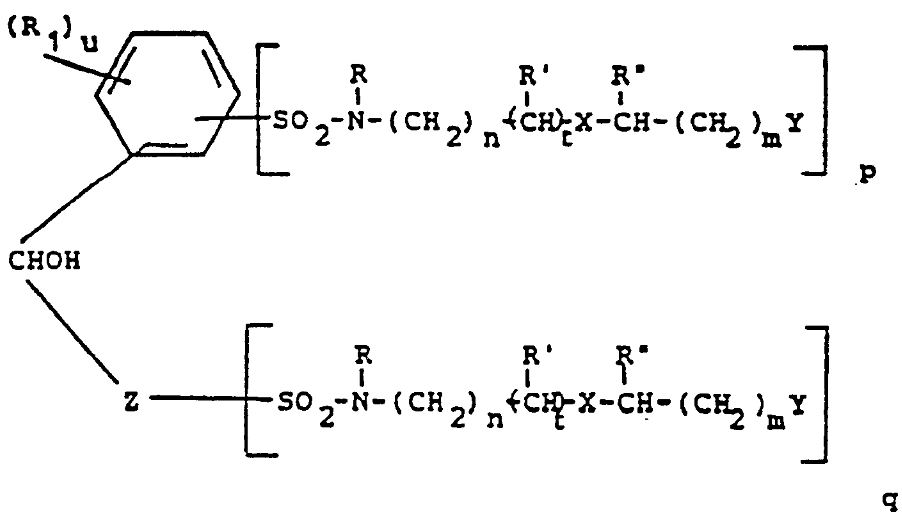

in which $R_1$, R, R′, R″, X, Y, Z, m, n, t, p, q and u have the meanings indicated in claim 4, characterised in that the compound of formula

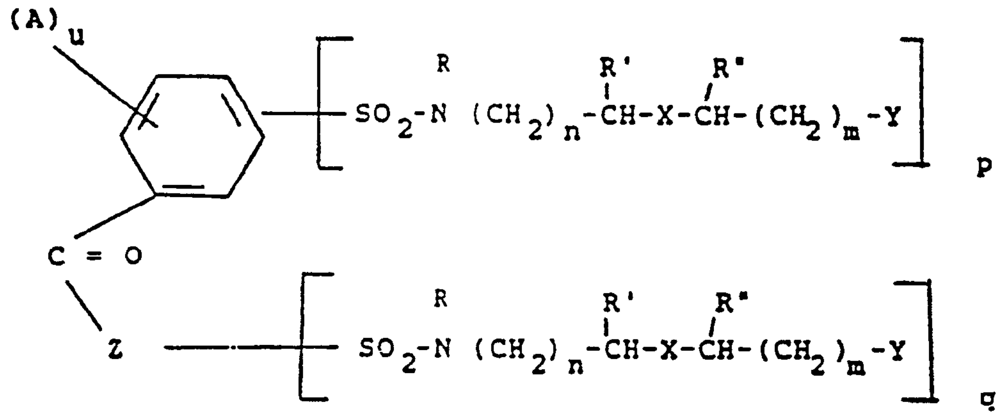

in which A, X, Y, Z, R', R", m, n, p, q, t and u have the meanings indicated in claim 24, and such as is obtained according to claim 25, is subjected to a reduction to transform C=O into CHOH with the aid of alkaline borohydride, before the possible reduction of A and/or B when they represent $NO_2$.

33. Process for preparation as claimed in claim 32, characterised in that the amino acid used or the dialkylaminoalkylamine used is chosen from amongst:

$$-NH_2-(CH_2)_n-COOH,$$

n varying from 1 to 11,

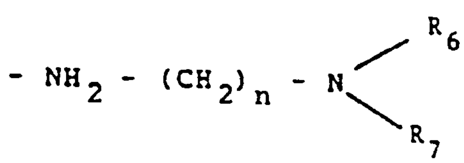

n = 2 or 3

– $R_6$ and $R_7$ representing an alkyl having from 1 to 4 atoms of carbon or a morpholine nucleus with the nitrogen atom,

and the sulphochloride used is chosen from amongst:

- 2-benzoyl-4-nitro-benzenesulphonyl chloride,
- 2-benzoyl-4-chloro-benzenesulphonyl chloride,
- 4-chloro-2-(2-chlorobenzoyl) benzenesulphonyl chloride,
- 2-benzoyl benzenesulphonyl chloride,
- 2-(4-chlorobenzoyl) benzenesulphonyl chloride,
- 4-benzoyl benzenesulphonyl chloride,
- 3-(2,4-dichlorobenzoyl)-4-chloro benzenesulphonyl chloride,
- 3-benzoyl-4-chloro benzenesulphonyl chloride,
- 3-(4-chlorobenzoyl)-4-chloro benzenesulphonyl chloride,
- 4-benzoyl-3-chloro benzenesulphonyl chloride,
- 4-chloro-3-(2-thenoyl) benzenesulphonyl chloride.

**Claims for the following Contracting State : ES**

1. Process for preparation of compounds of formula I

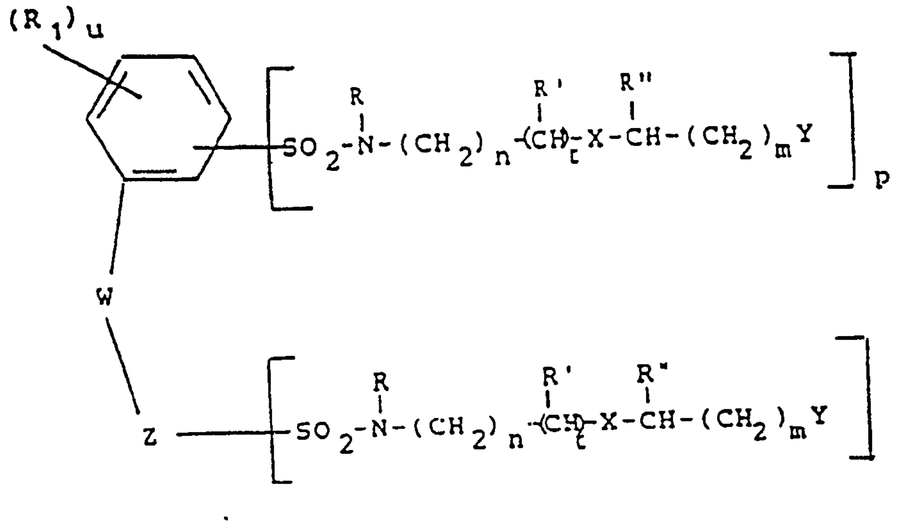

(I)

in which :

– W represents C = O, $CH_2$ or CHOH

– Z represents :

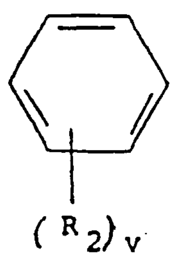

or

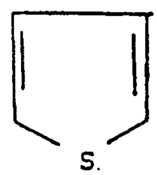

– $R_1$ represents Cl, F, Br, $NO_2$, $NH_2$, $CF_3$, an alkyl radical having from 1 to 4 carbon atoms, notably methyl or ethyl, an alkoxy radical having from 1 to 4 carbon atoms, acetamido or benzamido,

– $R_2$ represents Cl, F, Br, $NO_2$, $NH_2$, $CF_3$, an alkyl radical having from 1 to 4 carbon atoms, an alkoxy radical having from 1 to 4 carbon atoms, acetamido or benzamido,

– R represents H, an alkyl radical having from 1 to 6 carbon atoms or a benzyl radical,

– R′ and R″ represent H or an alkyl radical having from 1 to 4 carbon atoms,

– X represents $(CH_2)_r$, r being O or 1, -CH = CH

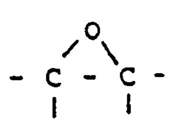

or

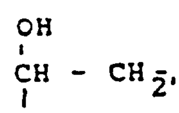

– Y represents $COOR_3$, $R_3$ representing a hydrogen or an alkyl radical having from 1 to 4 carbon atoms,

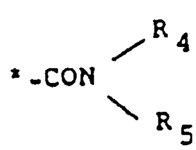

,

$R_4$ and $R_5$ representing H or an alkyl radical having from 1 to 4 carbon atoms

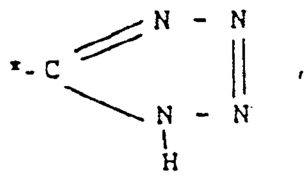

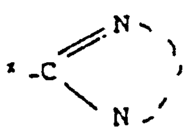 , 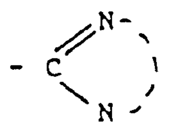

representing a non-aromatic cycle of 5 or 6 links, in which the 2 or 3 atoms which enter into the cycle are atoms of carbon and/or of oxygen and/or of nitrogen,

* $NH_2$, $NHR_6$, $NR_6R_7$,

$R_6$ and $R_7$ representing an alkyl radical having from 1 to 4 carbon atoms, or capable of forming a non-aromatic cyclised amine with the nitrogen atom,

– provided that when Y represents $-NH_2$, X is different from
– u is a whole number from O to 2,
– v is a whole number from O to 2,
– p is equal to O or 1,
– q is equal to O or 1, p + q being equal to or greater than 1,
– n is a whole number varying from O to 1O,
– m is a whole number varying from O to 1O,
– t is equal to O or 1,
– the total number of carbon atoms in the chain

$$-(CH_2)_n - (\overset{|}{C}H)_t - X - \overset{|}{C}H - (CH_2)_m\ Y$$

varying from 2 to 2O,

characterised in that a compound of formula

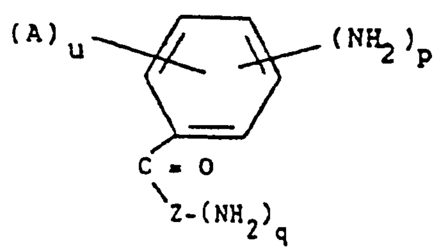

in which :
– A has the meanings indicated for $R_1$, with the exception of $NH_2$, that is to say A represents Cl, F, Br, $NO_2$, $CF_3$, an alkyl radical having from 1 to 4 carbon atoms, an alkoxy radical having from 1 to 4 carbon atoms, acetamido or benzamido,
– Z represents

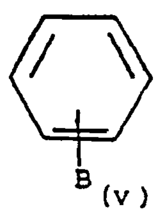 or 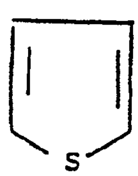

– B has the meanings indicated for $R_2$, with the exception of $NH_2$, that is to say B represents Cl, $NO_2$, $CF_3$, an alkyl radical having from 1 to 4 carbon atoms, an alkoxy radical having from 1 to 4 carbon atoms, acetamido

or benzamido and preferably Cl or $NO_2$,
– u, v, p and q have the meanings indicated above, is possibly subjected to a reduction, notably with the aid of aluminium chloride, aluminium hydride and lithium hydride to reduce -C=O to $CH_2$ and to obtain the compound of formula

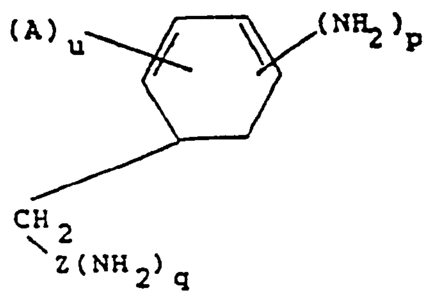

in which A, Z, u, p and q have the meanings indicated above,
. one of the amino compounds of the formula indicated above is subjected to the Sandmeyer reaction in order to obtain a sulphohalide of formula :

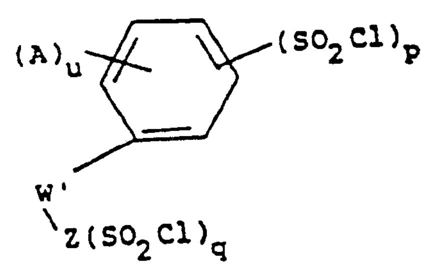

In which A, Z, u, p and q have the meanings indicated above and W′ represents C = O or $CH_2$,
. the sulphohalide, of the formula indicated above, is made to react on a compound of formula

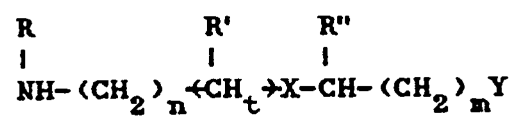

in which R, R′, R″, m, n, t, X and Y have the meanings indicated above,
in order to obtain the compound of formula

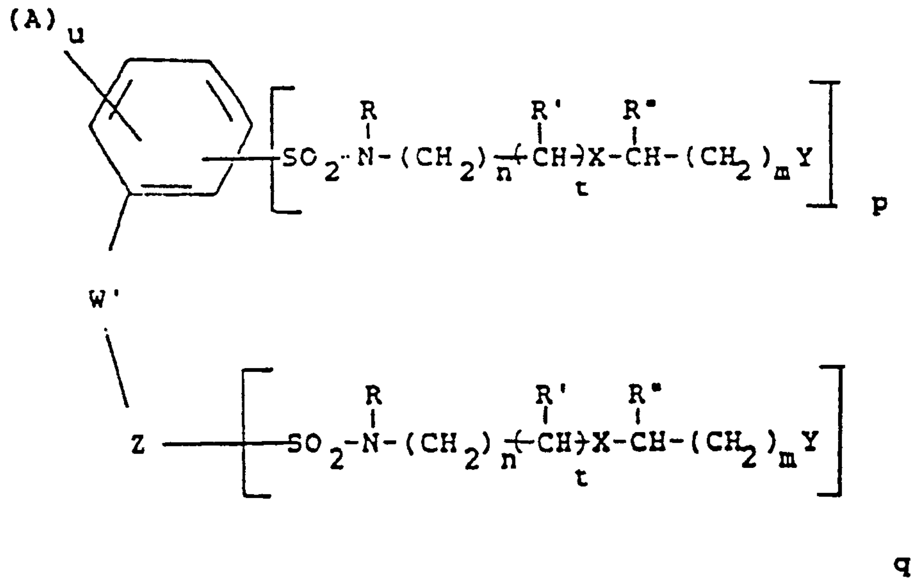

in which $R_3$, R', R", X, Y, A, Z, W', m, n, t, p and q have the meanings indicated above,
. and in the case where W' represents C=O the above compound is possibly subjected to a reduction, notably with the aid of alkaline borohydride to reduce C=O to CHOH,
. then a reduction is possibly carried out when A and/or B represents $NO_2$.
2. Process as claimed in claim 1 for preparation of compounds of formula II

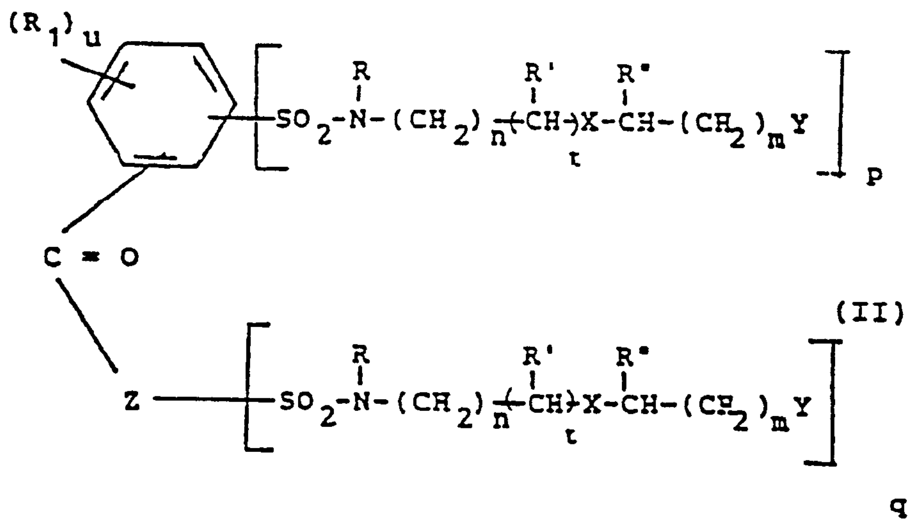

(II)

in which $R_1$, R, R', R", X, Y, Z, m, n, p, q and u have the meanings indicated in claim 1, characterised in that a sulphohalide of formula

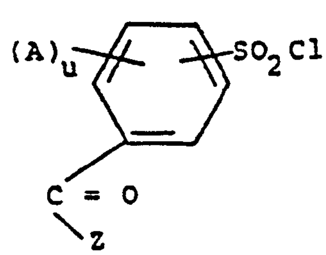

in which A, Z and u have the meanings indicated in claim 1, or a disulphohalide of formula

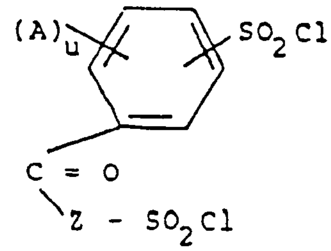

in which A, u and Z have the meanings indicated previously, is made to react on a compound of formula

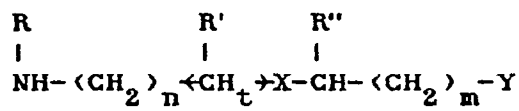

in which R, R′, R″, m, n, t, X and Y have the meanings indicated in claim 1,
- possibly followed by a reduction when A and/or B represents $NO_2$.

3. Process as claimed in claim 2 for preparation of compounds of the following formula V :

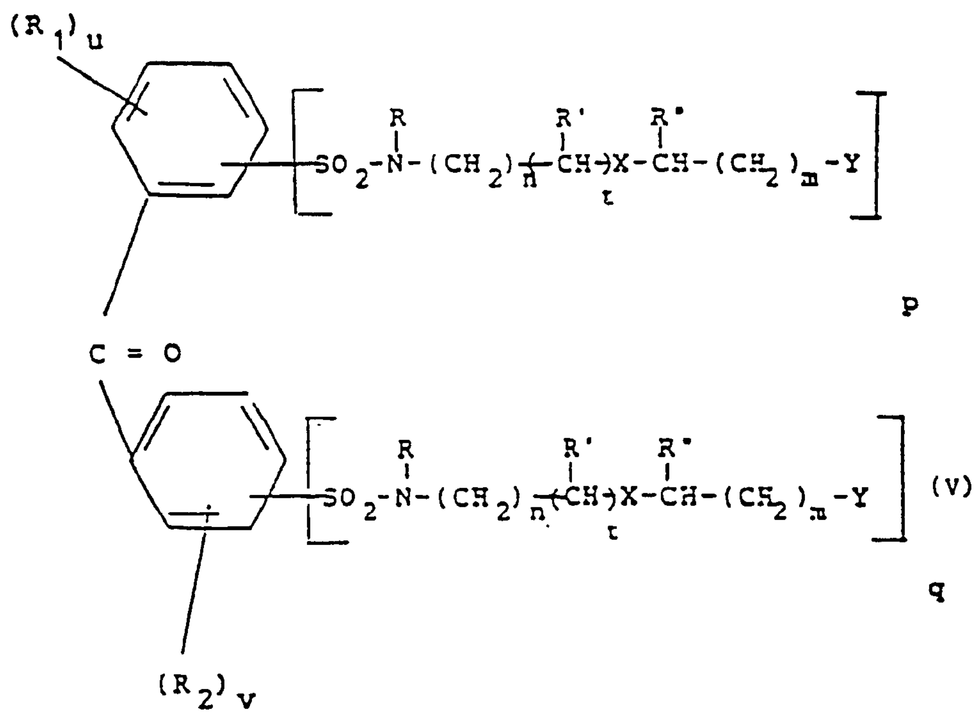

(V)

in which $R_1$, R, R′, R″, X, Y, Z, m, n, t, p, q and u have the meanings indicated in claim 1, characterised in that a sulphohalide of formula :

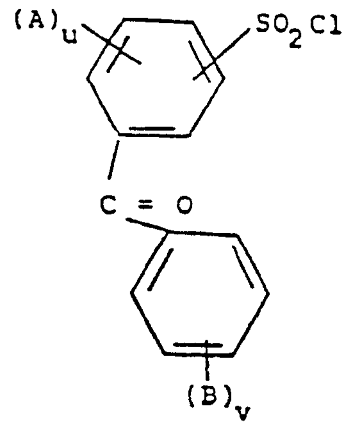

in which A, B, u and v have the meanings indicated in claim 2, or a disuphohalide of formula

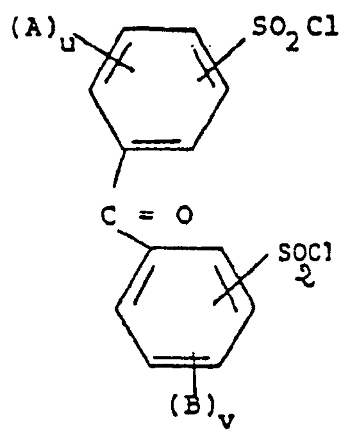

in which A, B, u and v have the meanings previously indicated is made to react on one or two compounds of formula

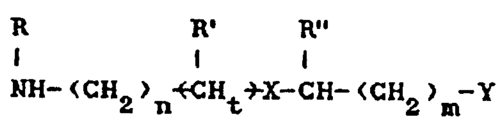

in which R, R', R", m, n, t, X and Y have the meanings indicated in claim 2, possibly followed by a reduction when A and/or B represents $NO_2$.

4. Process as claimed in claim 3 for preparation of compounds of formula V in which -Y represents $COOR_3$, $R_3$ representing hydrogen or an alkyl radical having from 1 to 4 carbon atoms.

5. Process as claimed in claim 3 for preparation of compounds of formula V in which -Y represents $NH_2$, $NHR_6$ or $NR_6R_7$, $R_6$ and $R_7$ having the meanings indicated in claim 1.

6. Process as claimed in claim 3 for preparation of compounds of formula V, in which p or q is equal to O.

7. Process as claimed in claim 3 for preparation of compounds of formula V, in which
- p or q is equal to O,
- u + v is greater than or equal to 1,
- the total number of carbon atoms in the chain

$$(CH_2)_n - (CH)_t - X - CH - (CH_2)_m - Y$$

is equal to or greater than 5.

8. Process for preparation as claimed in claim 3 of compounds of formula V, in which

- $p = 1$,
- $q = 1$,
- $u + v$ is greater than or equal to 1,
- the total number of carbon atoms in the chain

$$(CH_2)_n - (CH)_t - X - CH - (CH_2)_m - Y$$

is equal to or greater than 5.

9. Process as claimed in claim 2 for preparation of compounds of formula II, characterised in that a sulphochloride of formula

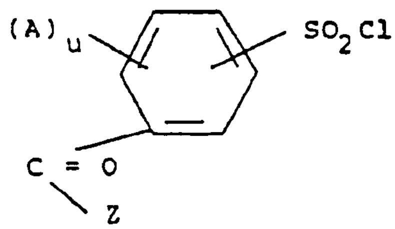

in which A, u, Z have the meanings indicated in claim 2, or a disulphochloride of formula

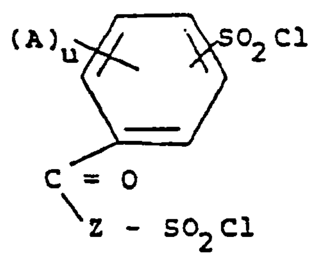

in which A, u, Z have the meanings indicated in claim 2, is made to react on an amino acid of formula

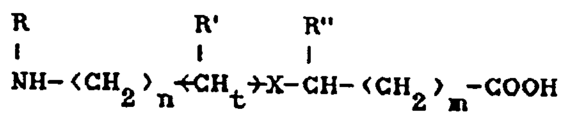

in which R, R′, R″, n, m, t, and X have the meanings indicated above in claim 2.

10. Process as claimed in claim 2 for preparation of compounds of formula II, characterised in that

- a compound such as that obtained according to claim 9, and in which Y represents COOH, is esterified in a compound of formula II, in which Y represents $COOR_3$, $R_3$ representing an alkyl radical having from 1 to 4 carbon atoms,
- or a compound such as that obtained according to claim 9 is transformed into a compound of formula II in which Y represents

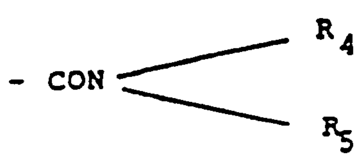

$R_4$, $R_5$ representing H or an alkyl radical having from 1 to 4 carbon atoms,

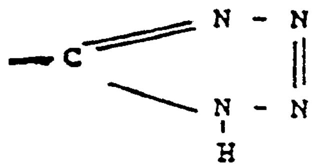

$- C \equiv N$

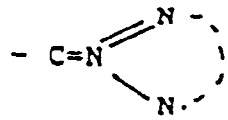

11. Process as claimed in claim 2 for preparation of compounds of formula II, characterised in that a sulphochloride of formula

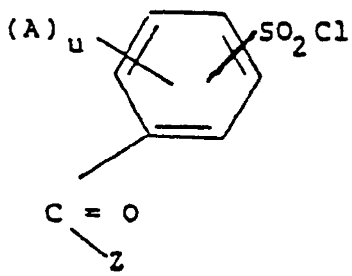

in which :

– A has the meanings indicated for $R_1$, with the exception of $NH_2$, that is to say Cl, F, Br, $NO_2$, $CF_3$, an alkyl radical having from 1 to 4 carbon atoms, an alkoxy radical having from 1 to 4 carbon atoms, acetamido or benzamido,

– Z represents :

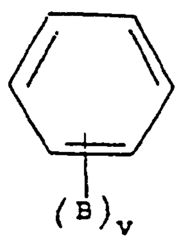

or

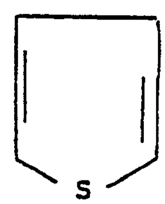

– B has the meanings indicated for $R_2$, with the exception of $NH_2$, that is to say B represents Cl, F, Br, $NO_2$, $CF_3$, an alkyl radical having from 1 to 4 carbon atoms, an alkoxy radical having from 1 to 4 carbon atoms, acetamido or benzamido and preferably Cl or $NO_2$,

– u represents a whole number from O to 2,

– v represents a whole number from O to 2, or a disulphohalide of formula

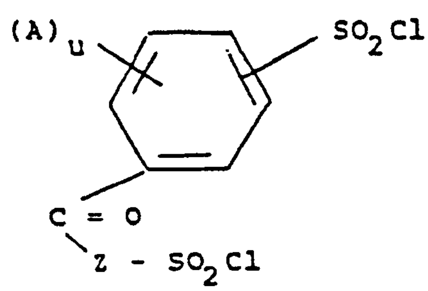

in which A, u, Z have the meanings indicated above, is made to react on an aminoalkylamine of formula

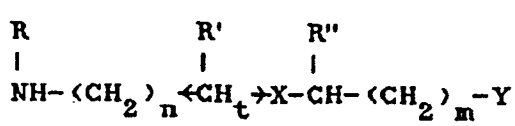

in which

– R represents H, an alkyl radical having from 1 to 6 carbon atoms or a benzyl radical,
– R′ and R″ represent H or an alkyl radical having from 1 to 4 carbon atoms,
– n represents a whole number varying from O to 1O,
– m represents a whole number varying from O to 1O,
– t is equal to O or 1,
– Y represents $NH_2$, $NHR_6$, $NR_6R_7$, $R_6$ and $R_7$ representing an alkyl radical having from 1 to 4 carbon atoms, or forms a non-aromatic cyclised amine with the nitrogen.

12. Process as claimed in claim 2 for preparation of compounds of formula II, characterised in that the amino acid used or the dialkylaminoalkylamine used is chosen respectively from amongst:

$$- NH_2 - (CH_2)_n - COOH,$$

n varying from 1 to 11,

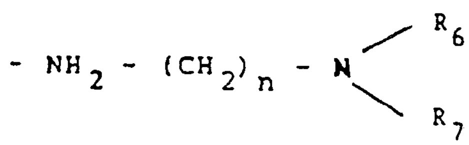

n = 2 ou 3

– $R_6$ and $R_7$ representing an alkyl having from 1 to 4 atoms of carbon or forming a morpholine cycle with the nitrogen atom,

and the sulphochloride used is chosen respectively from amongst:

– 2-benzoyl-4-nitro-benzenesulphonyl chloride,
– 2-benzoyl-4-chloro-benzenesulphonyl chloride,
– 4-chloro-2-(2-chlorobenzoyl) benzenesulphonyl chloride,
– 2-benzoyl benzenesulphonyl chloride,
– 2-(4-chlorobenzoyl) benzenesulphonyl chloride,
– 4-benzoyl benzenesulphonyl chloride,
– 3-(2,4-dichlorobenzoyl)-4-chloro benzenesulphonyl chloride,
– 3-benzoyl-4-chloro benzenesulphonyl chloride,
– 3-(4-chlorobenzoyl)-4-chloro benzenesulphonyl chloride,
– 4-benzoyl-3-chloro benzenesulphonyl chloride,
– 4-chloro-3-(2-thenoyl) benzenesulphonyl chloride.

13. Process as claimed in claim 1 for preparation of compounds of formula III

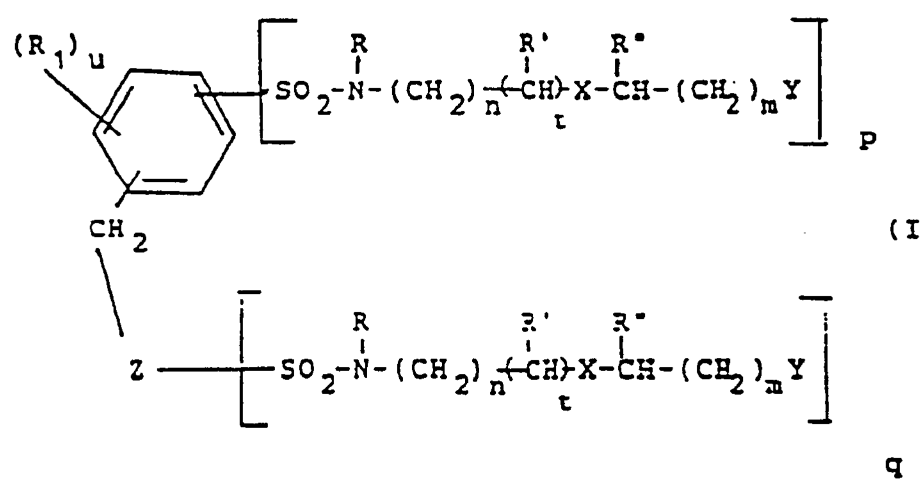

(III)

in which $R_1$, R, R′, R″, X, Y, Z, m, n, t, p, q and u have the meanings indicated in claim 3, characterised in that the compound of formula

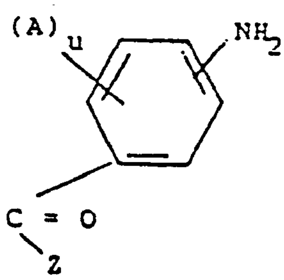

or of formula

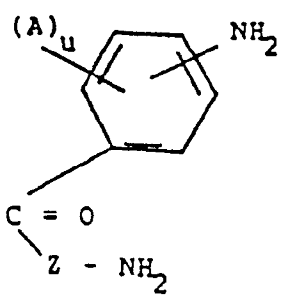

is subjected to a reduction to reduce C=O to $CH_2$ and to obtain the compound of formula

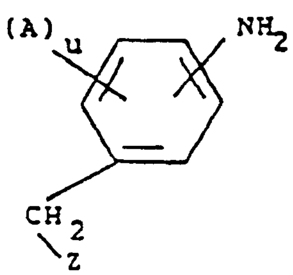

or

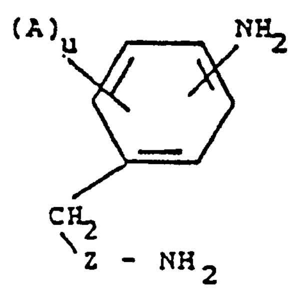

– one of these compounds is subjected to the Sandmeyer reaction in order to obtain compounds of formula

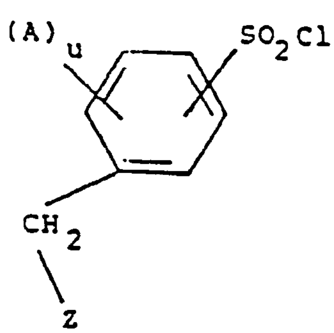

or

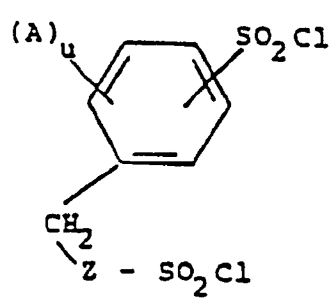

– the sulphohalide or the disulphohalide of formula indicated above is condensed with compound of formula

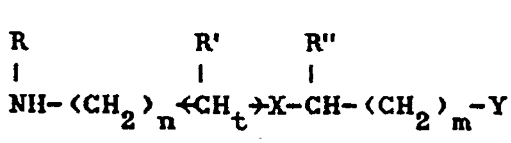

in which R, R′, R″, X, Y, n, m and t have the meanings indicated in claim 1, followed possibly by a reduction when A and/or B represents $NO_2$.

14. Process as claimed in claim 13 for preparation of compounds of formula III, characterised in that the amino acid used or the dialkylaminoalkylamine used is chosen from amongst :

$$- NH_2 - (CH_2)_n - COOH,$$

n varying from 1 to 11,

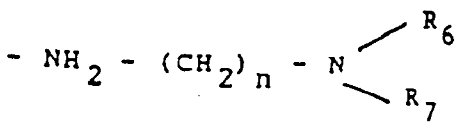

$n = 2$ ou 3

$R_6$ and $R_7$ representing an allyl group having from 1 to 4 atoms of carbon or forming a morpholine cycle with the nitrogen atom,
and the compound of formula :

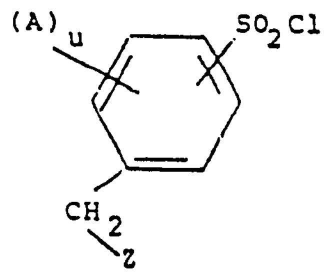

is chosen from amongst :
- 3-(2,4-dichlorobenzyl)-4-chloro benzenesulphonyl chloride,
- 3-(4-chlorobenzyl)-4-chloro benzenesulphonyl chloride,
- 2-benzyl-4-chloro benzenesulphonyl chloride,
- 2-(2,4-dichlorobenzyl)-4-chloro benzenesulphonyl chloride,
- 2-(4-chorobenzyl)-4-chloro benzenesulphonyl chloride,
- 4-benzyl-3-chloro benzenesulphonyl chloride,
- 4-(2,4-dichlorobenzyl)-3-chloro benzenesulphonyl chloride,
- 4-(4-chlorobenzyl)-3-chloro benzenesulphonyl chloride,
- 3-benzyl-4-chloro benzenesulphonyl chloride.

15. Process as claimed in claim 1 for preparation of compounds of formula IV,

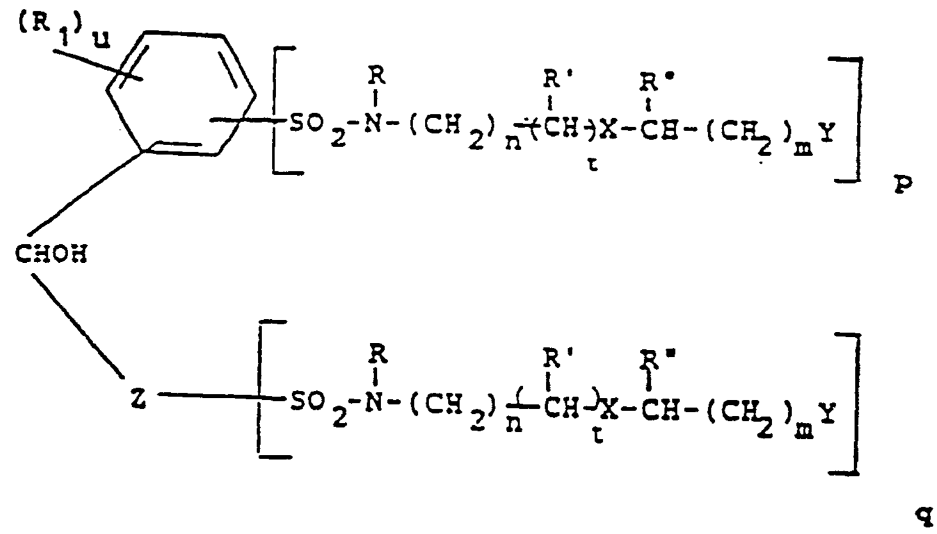

(IV)

in which $R_1$, R, R′, R″, X, Y, Z, m, n, t, p, q and u have the meanings indicated in claim 1, characterised in that the compound of formula

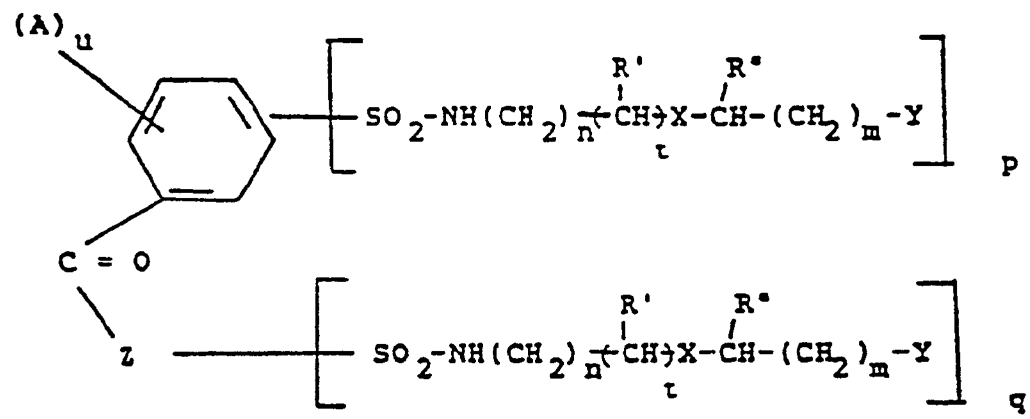

in which A, X, Y, Z, R, R′, R″, m, n, t, p, q and u have the meanings indicated in claim 1, and such as is obtained according to claim 2, is subjected to a reduction to transform C=O into CHOH with the aid of alkaline borohydride, before the possible reduction of A and/or B when they represent $NO_2$.

16. Process as claimed in claim 15 for preparation of compounds of formula IV, characterised in that the amino acid used or the dialkylaminoalkylamine used is chosen from amongst :

$$- NH_2 - (CH_2)_n - COOH,$$

n varying from 1 to 11,

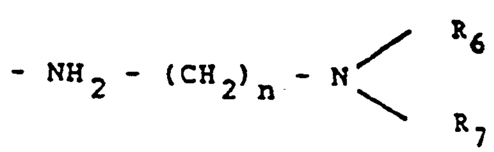 n = 2 ou 3

$R_6$ and $R_7$ representing an alkyl group having from 1 to 4 carbon atoms or forming a morpholine cycle with the nitrogen atom,
and the sulphochloride used is chosen from amongst :

- 2-benzoyl-4-nitro-benzenesulphonyl chloride,
- 2-benzoyl-4-chloro-benzenesulphonyl chloride,
- 4-chloro-2-(2-chlorobenzoyl) benzenesulphonyl chloride,
- 2-benzoyl benzenesulphonyl chloride,
- 2-(4-chlorobenzoyl) benzenesulphonyl chloride,
- 4-benzoyl benzenesulphonyl chloride,
- 3-benzoyl benzenesulphonyl chloride,
- 3-(2,4-dichlorobenzoyl)-4-chloro benzenesulphonyl chloride,
- 3-benzoyl-4-chloro benzenesulphonyl chloride,
- 3-(4-chlorobenzoyl)-4-chloro benzenesulphonyl chloride,
- 4-benzoyl-3-chloro benzenesulphonyl chloride,
- 4-chloro-3-(2-thenoyl) benzenesulphonyl chloride.

17. Process as claimed in claim 1 for preparation of compounds of formula VI,

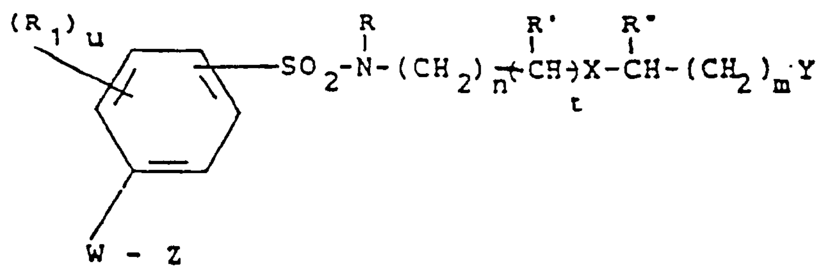

(VI)

in which $R_1$, R, R', R", X, Y, Z, W, m, n, t and u have the meanings indicated in claim 1, characterised in that a compound of formula

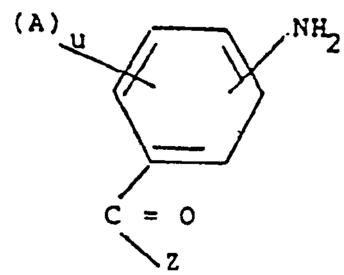

in which A, Z and u have the meanings indicated in claim 1, is possibly subjected to a reduction, notably with the aid of aluminium chloride, aluminium hydride and lithium hydride to reduce -C=O to $CH_2$ and to obtain the compound of formula

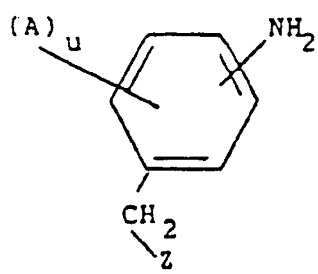

in which A, Z, u have the meanings indicated above,

. one of the amino compounds of the formula indicated above is subjected to the Sandmeyer reaction in order to obtain a sulphohalide of formula :

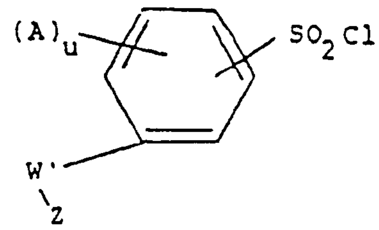

in which A, Z and u have the meanings indicated above and W′ represents C = O or $CH_2$,
. the sulphohalide of the formula indicated above is made to react on a compound of formula

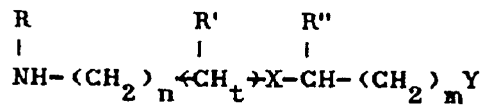

in which R, R′, R″, m, n, t, X and Y have the meanings indicated in claim 1,
in order to obtain the compound of formula

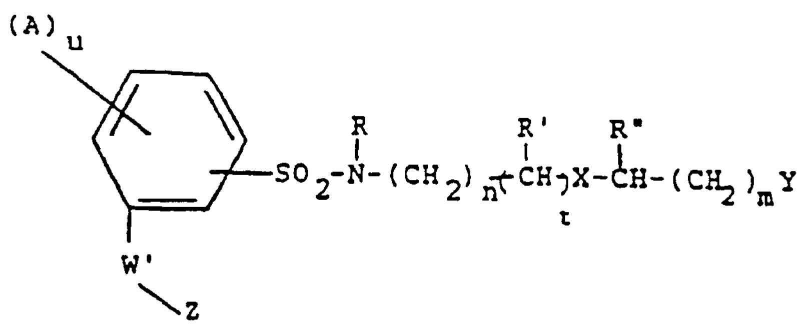

. and in the case where W′ represents C=O the above compound is possibly subjected to a reduction, notably with the aid of alkaline borohydride to reduce C=O to CHOH,
. then a reduction is possibly carried out when A and/or B represents $NO_2$.
18. Process as claimed in claim 17 for preparation of the compounds of formula VII,

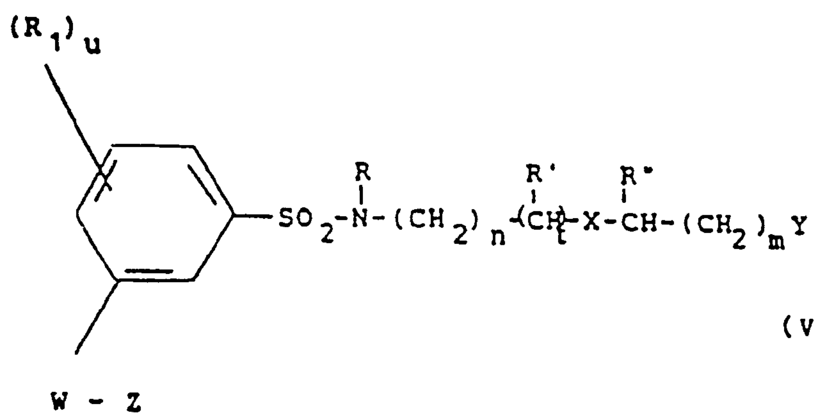

(VII)

in which $R_1$, R, R′, R″, X, Y, Z, W, m, n, t and u have the meanings indicated in claim 1, characterised in that a compound of formula

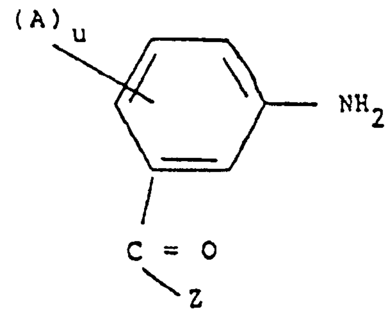

in which A, Z and u have the meanings indicated in claim 1, is possibly subjected to a reduction, notably with the aid of aluminium chloride, aluminium hydride and lithium hydride to reduce -C=O to $CH_2$ and to obtain the compound of formula

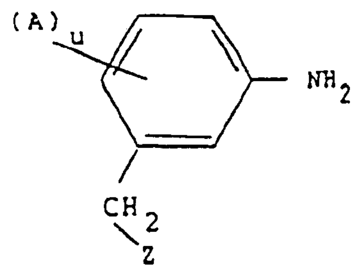

in which A, Z and u have the meanings indicated above,
. one of the amino compounds of the formula indicated above is subjected to the Sandmeyer reaction in order to obtain a sulphohalide of formula :

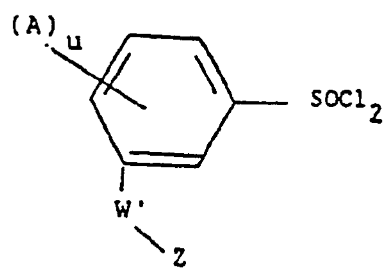

in which A, Z and u have the meanings indicated above and W′ represents C = O or $CH_2$,
. the sulphohalide of the formula indicated above is made to react on a compound of formula

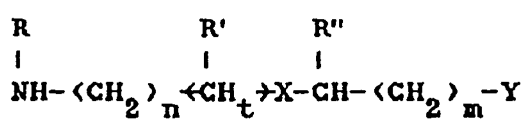

in which R, R′, R″, m, n, and t have the meanings indicated above,
in order to obtain the compound of formula

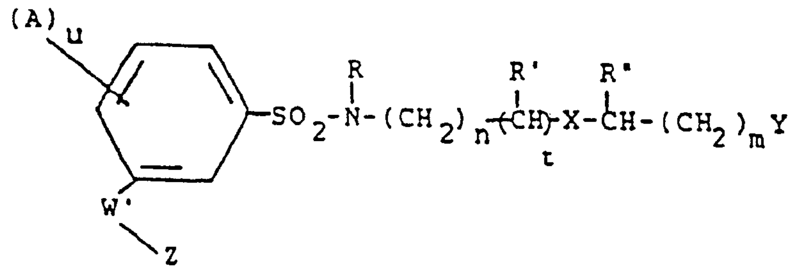

. and in the case where W′ represents C=O the above compound is possibly subjected to a reduction, notably with the aid of alkaline borohydride to reduce C=O to CHOH,

. then a reduction is possibly carried out when A and/or B represents $NO_2$.

19. Process as claimed in claim 18 for preparation of the compounds of formula VII, in which -Y represents $COOR_3$, $R_3$ representing H or an alkyl radical having from 1 to 4 carbon atoms, characterised in that a compound of formula

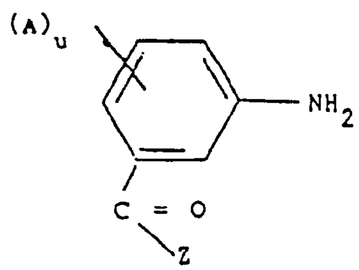

in which A, Z and u have the meanings indicated in claim 1, is possibly subjected to a reduction, notably with the aid of aluminium chloride, aluminiun hydride and lithium hydride to reduce -C=O to $CH_2$ and to obtain the compound of formula

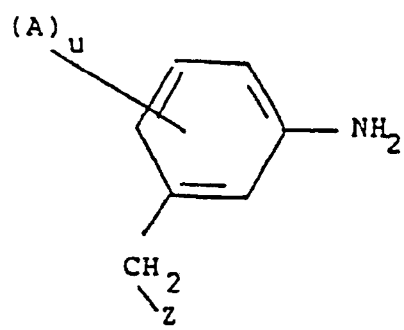

in which A, Z, and u have the meanings indicated above,

. one of the amino compounds of the formula indicated above is subjected to the Sandmeyer reaction in order to obtain a sulphohalide of formula :

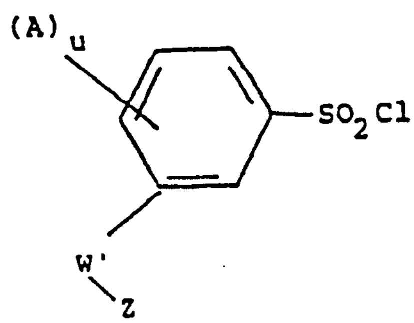

in which A, Z and u have the meanings indicated above and W′ represents C = O or $CH_2$,
. the sulphohalide, notably sulphochloride, of the formula Indicated above is made to react on a compound of formula

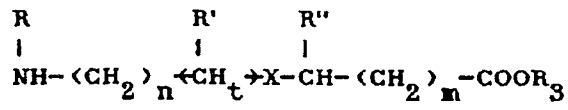

in which R, R′, R″, X, $R_3$, m, n and t have the meanings indicated in claim 1,
in order to obtain the compound of formula

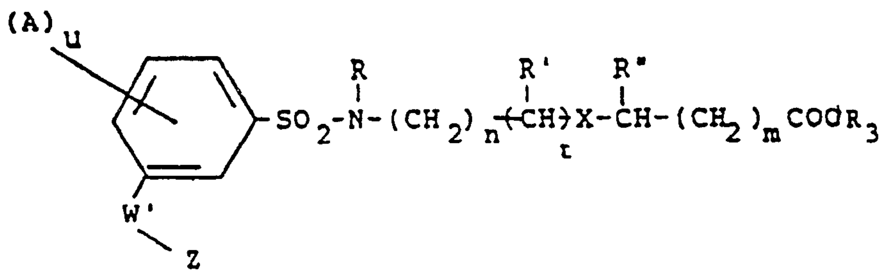

. and In the case where W′ represents C=O the above compound is possibly subjected to a reduction, notably with the aid of alkaline borohydride, such as sodium borohydride, to reduce C=O to CHOH,
. then a reduction is possibly carried out when A and/or B represents $NO_2$.
20. Process as claimed in claim 19 for preparation of the compounds of formula VIII,

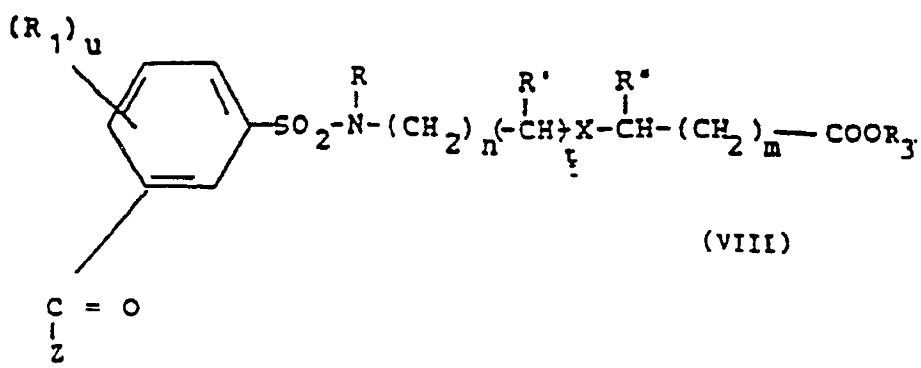

(VIII)

in which $R_1$, $R_3$, R, R′, R″, X, Z, m, n, t and u have the meanings indicated in claim 1.
21. Process as claimed in claim 19 for preparation of the compounds of formula IX,

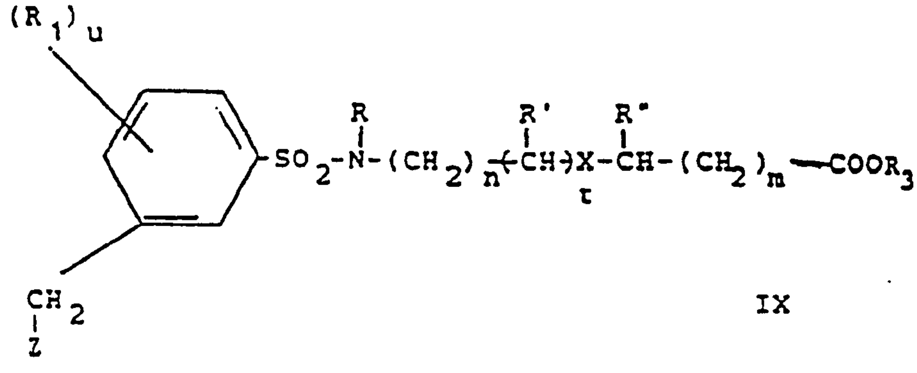

IX

in which $R_1$, $R_3$, R, R′, R″, X, Z, w, m, n, t and u have the meanings indicated in claim 1.
22. Process as claimed in claim 19 for preparation of the compounds of formula X,

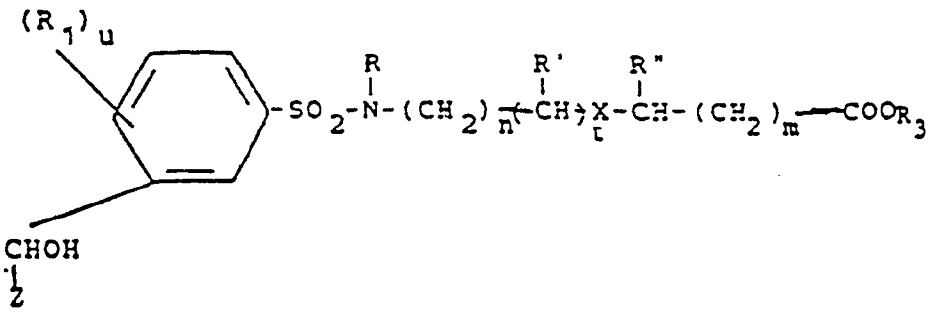

X

in which $R_1$, $R_3$, R, R', R", X, Z, W, m, n, t and u have the meanings indicated in claim 1.

23. Process as claimed in claim 20 for preparation of compounds of formula VIII in which the total number of carbon atoms in the chain

$$(CH_2)_n - (CH)_t - X - CH - (CH_2)_m - Y$$

is equal to or greater than 5.

24. Process as claimed in claim 21 for preparation of compounds of formula IX in which the total number of carbon atoms in the chain

$$(CH_2)_n - (CH)_t - X - CH - (CH_2)_m - Y$$

is equal to or greater than 5.

25. Process as claimed in claim 22 for preparation of compounds of formula X in which the total number of carbon atoms in the chain

$$(CH_2)_n - (CH)_t - X - CH - (CH_2)_m - Y$$

is equal to or greater than 5.

26. Process as claimed in claim 1 for preparation of compounds of formula XI

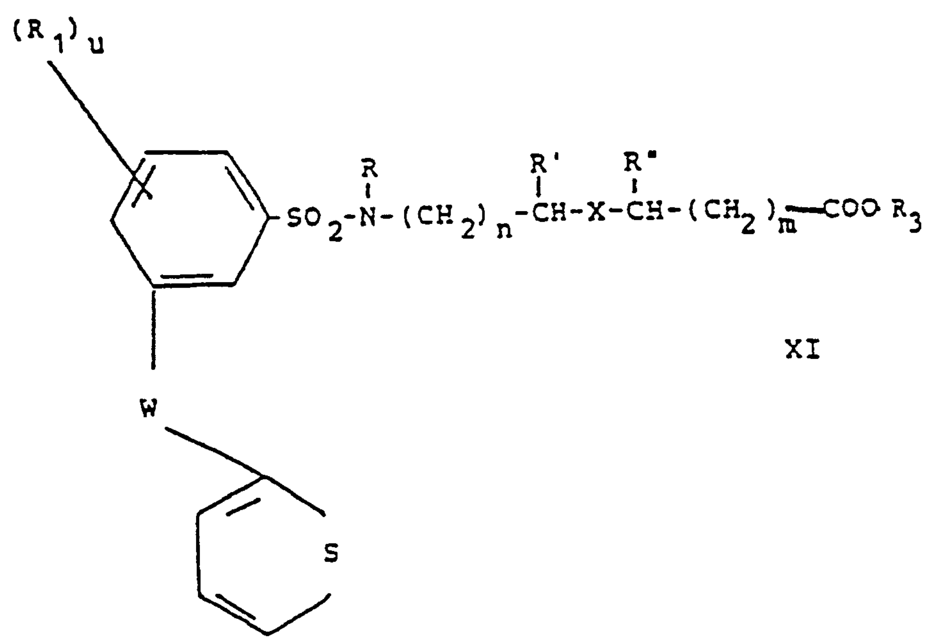

XI

in which the total number of carbon atoms in the chain

$$(CH_2)_n - (CH)_t - X - CH - (CH_2)_m - Y$$

is equal to or greater than 5, and in which $R_1$, $R_3$, R, R', R", X, W, m, n, t and u have the meanings indicated in claim 1, characterised in that a compound of formula

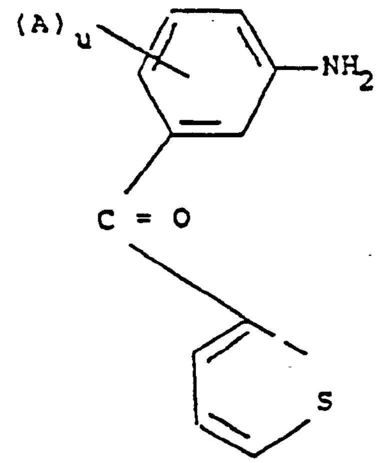

in which A and u have the meanings indicated in claim 1, is possibly subjected to a reduction, notably with the aid of aluminium chloride, aluminium hydride and lithium hydride to reduce -C=O to $CH_2$ and obtain the compound of formula

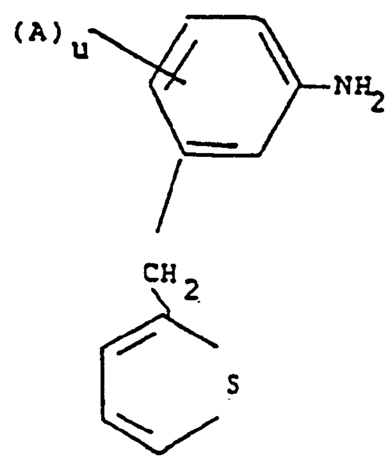

in which A and u have the meanings indicated above,
. one of the amino compounds of the formula indicated above is subjected to the Sandmeyer reaction in order to obtain a sulphohalide of formula :

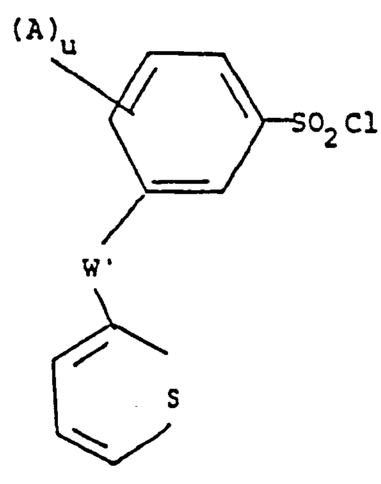

in which A and u have the meanings indicated above and W′ represents C = O or $CH_2$,

. the sulphohalide of the formula indicated above is made to react on one or two compounds of formula

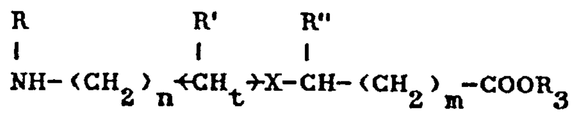

in which R, R′, R″, X, m, n, t and $R_3$ have the meanings indicated in claim 1, in order to obtain the compound of formula

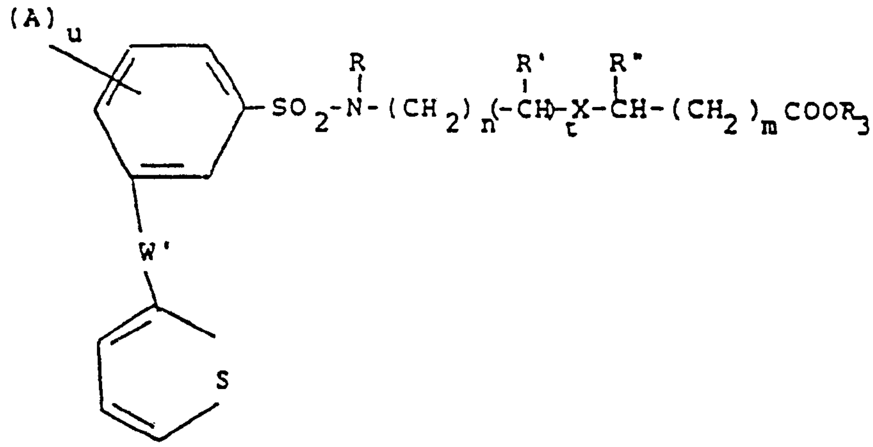

and in the case where W′ represents C=O the above compound is possibly subjected to a reduction, notably with the aid of alkaline borohydride, to reduce C=O to CHOH,

. then a reduction is possibly carried out when A and/or B represents $NO_2$.

27. Process for preparation of a pharmaceutical composition, characterised in that any one of the compounds obtained using the process according to any one of claims 1 to 26 is associated with a pharmaceutically acceptable vehicle in conditions permitting the production of the said pharmaceutical composition.

## Patentansprüche

### Patentanspruche für folgende Vertragsstaaten : AT, BE, CH, DE, FR, GB, IT, LI, LU, NL, SE

1. Verbindungen der Formel I,

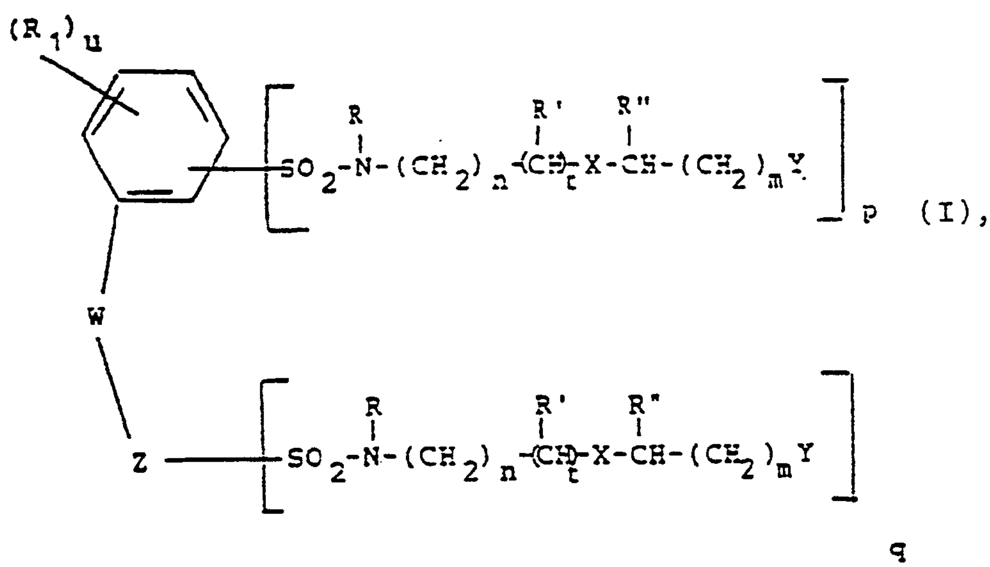

wobei bedeuten :

– W C=O, $CH_2$ oder CHOH,

– Z

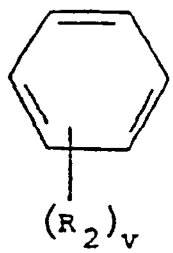

oder

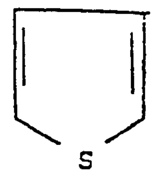

,

– $R_1$ Cl, F, Br, $NO_2$, $NH_2$, $CF_3$, $C_{1-4}$-Alkyl, insbesondere Methyl oder Ethyl, $C_{1-4}$-Alkoxy, Acetamido oder Benzamido,

– $R_2$ Cl, F, Br, $NO_2$, $NH_2$, $CF_3$, $C_{1-4}$-Alkyl, $C_{1-4}$-Alkoxy, Acetamido oder Benzamido,

– R H, $C_{1-6}$-Alkyl oder Benzyl,

– R' und R" H oder $C_{1-4}$-Alkyl,

– X $(CH_2)_r$, wobei r O oder 1 bedeutet, -CH=CH,

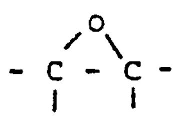

oder

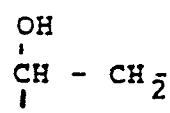

,

– Y COOR$_3$, wobei $R_3$ H oder $C_{1-4}$-Alkyl darstellt,

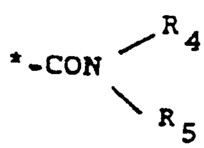

,

worin $R_4$ und $R_5$ H oder $C_{1-4}$-Alkyl darstellen,

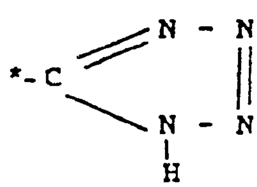

,

* -C≡N oder

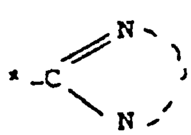

,

wobei

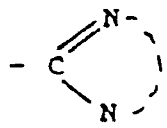

einen nichtaromatischen 5- oder 6-gliedrigen Ring darstellt, in dem die zwei oder drei Atome, mit denen der Ring vervollständigt wird, Kohlenstoffatome und/oder Sauerstoffatome und/oder Stickstoffatome sind, oder

* $NH_2$, $NHR_6$ oder $NR_6R_7$,
wobei $R_6$ und $R_7$ $C_{1-4}$-Alkyl darstellen oder mit dem Stickstoffatom ein nichtaromatisches cyclisches Amin bilden können,

– mit der Maßgabe, daß X nicht

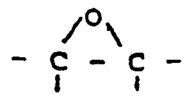

bedeutet, wenn Y $NH_2$ darstellt,
– u eine ganze Zahl von O bis 2,
– v eine ganze Zahl von O bis 2,
– p O oder 1,
– q O oder 1, wobei p + q $\geqq$ 1 ist,
– n eine ganze Zahl von O bis 1O,
– m eine ganze Zahl von O bis 1O
und
– t O oder 1,
– wobei die Gesamtzahl der Kohlenstoffatome der Kette

$$-(CH_2)_n-(CH)_t-X-CH-(CH_2)_m\ Y$$

2 bis 2O beträgt,
sowie ihre physiologisch akzeptablen Salze.

2. Verbindungen nach Anspruch 1 der Formel II,

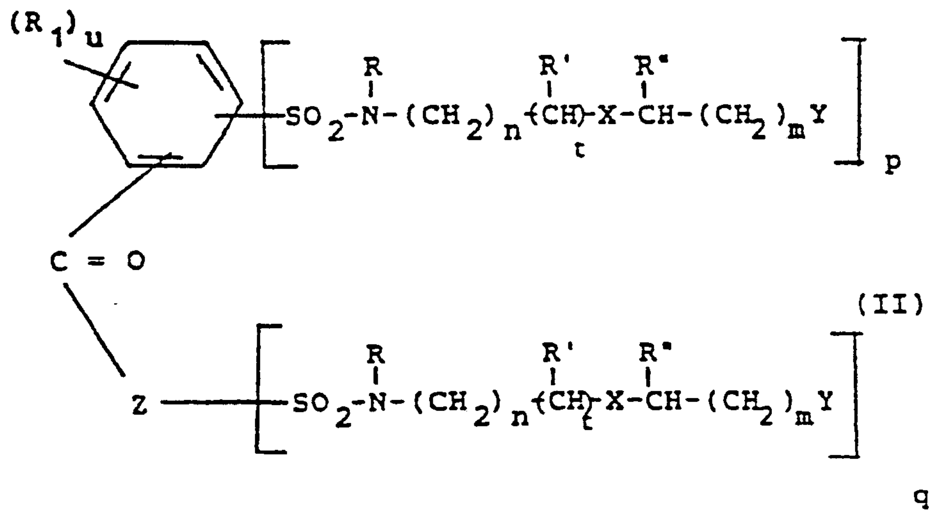

(II) ,

in der $R_1$, R, R′, R″, X, Y, Z, m, n, t, p, q und u dasselbe wie in Anspruch 1 bedeuten.

3. Verbindungen nach Anspruch 1 der Formel III,

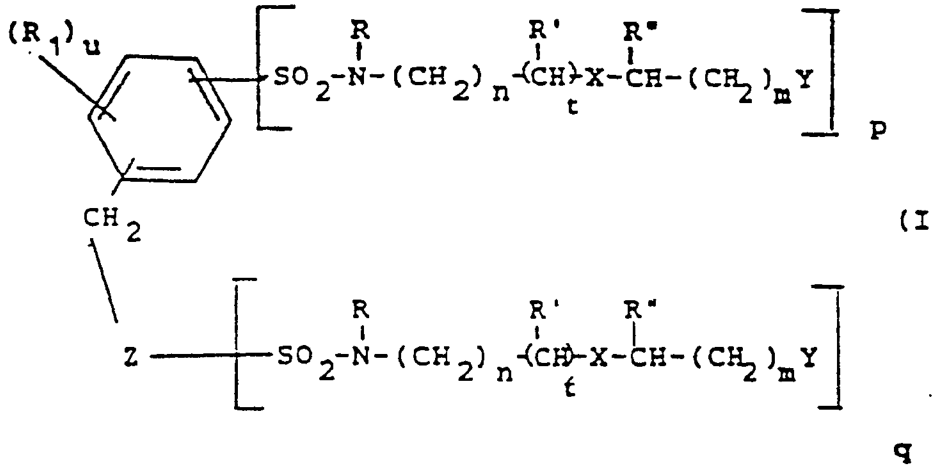

(III) '

4. Verbindungen nach Anspruch 1 der Formel IV,

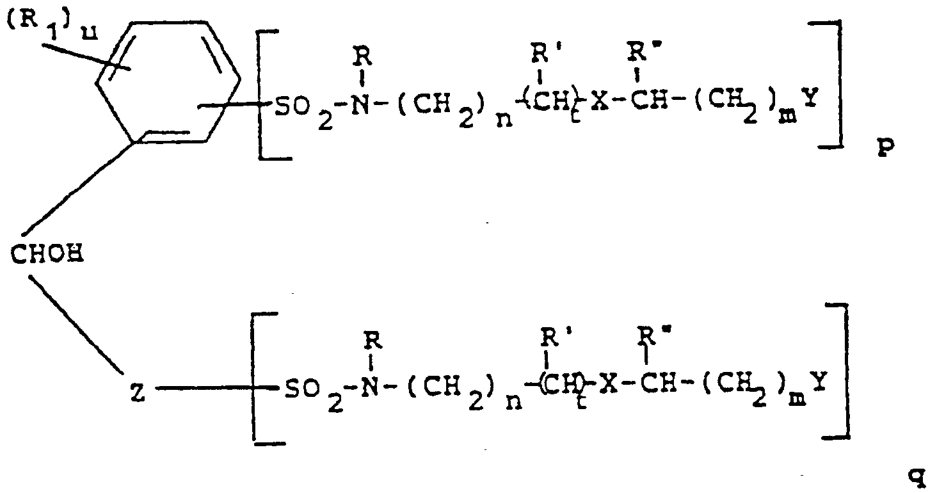

(IV),

in der $R_1$, R, R′, R″, X, Y, Z, m, n, t, p, q und u dasselbe wie in Anspruch 1 bedeuten.
5. Verbindungen nach Anspruch 2 der Formel V,

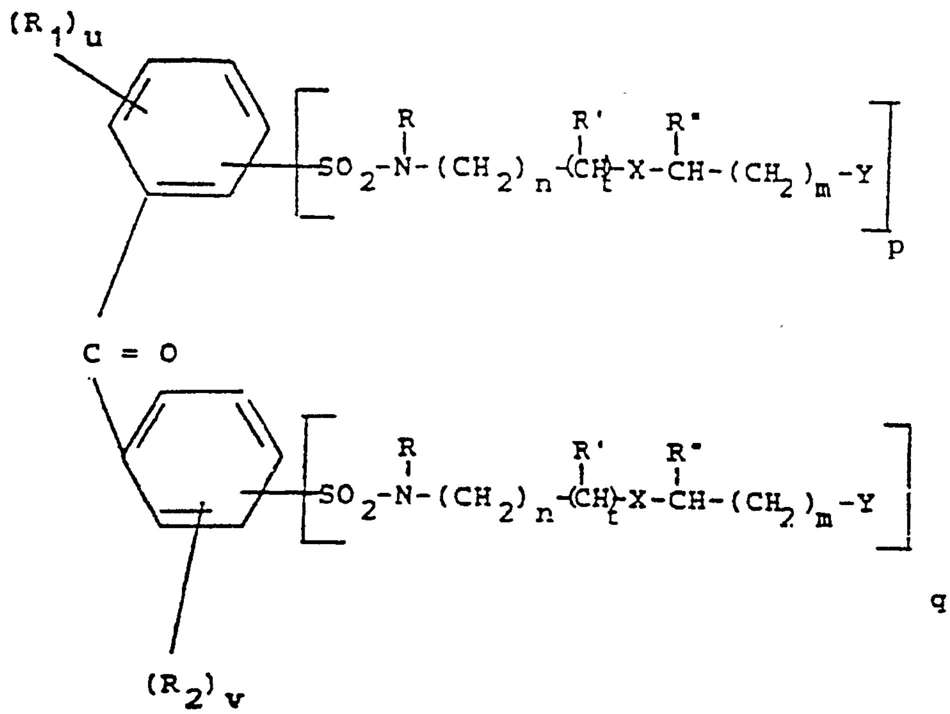

(V),

in der $R_1$, $R_2$, R, R′, R″, X, Y, Z, m, n, p, q, t, u und v dasselbe wie in Anspruch 1 bedeuten.

6. Verbindungen nach Anspruch 5 der Formel V, in der Y $COOR_3$ bedeutet, wobei $R_3$ H oder $C_{1-4}$-Alkyl darstellt.

7. Verbindungen nach Anspruch 5 der Formel V, in der Y $NH_2$, $NHR_6$ oder $NR_6R_7$ bedeutet, wobei $R_6$ und $R_7$ dasselbe wie in Anspruch 1 bedeuten.

8. Verbindungen nach Anspruch 5 der Formel V, in der p oder q gleich O sind.

9. Verbindungen nach Anspruch 8, dadurch gekennzeichnet, daß

– $u + v \geqq 1$ ist.

und

– die Gesamtzahl der Kohlenstoffatome der Kette

$$(CH_2)_n - (\dot{C}H)_t - X - \dot{C}H - (CH_2)_m - Y \geq 5 \text{ ist.}$$

10. Verbindungen nach Anspruch 5 der Formel V mit folgenden Definitionen :

– P = 1,

– q = 1,

– $u + v \geqq 1$

und

– die Gesamtzahl der Kohlenstoffatome der Kette

$$(CH_2)_n - (\dot{C}H_t) - X - \dot{C}H - (CH_2)_m - Y \text{ ist } \geq 5.$$

11. Verbindungen nach Anspruch 1 der Formel VI,

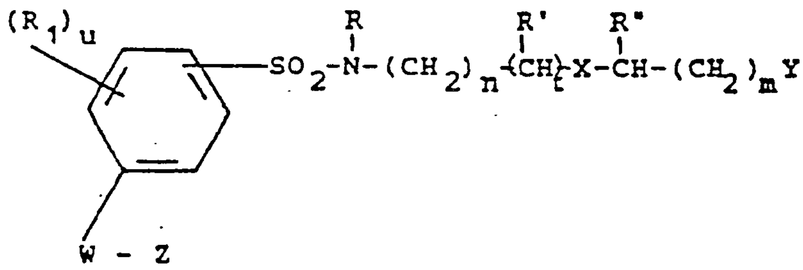

(VI),

in der $R_1$, R, R', R", X, Y, Z, W, m, n, u und t dasselbe wie in Anspruch 1 bedeuten.

12. Verbindungen nach Anspruch 11 der Formel VII,

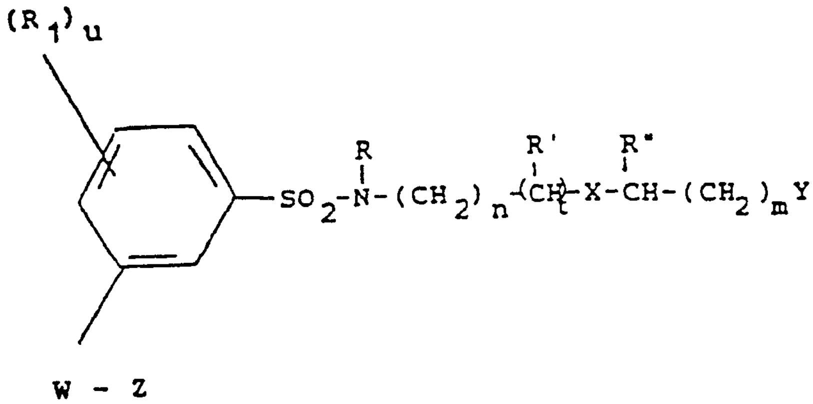

(VII),

in der $R_1$, R, R', R", X, Y, Z, W, m, n, u und t dasselbe wie in Anspruch 1 bedeuten.

13. Verbindungen nach Anspruch 12 der Formel VII, in der Y $COOR_3$ bedeutet, wobei $R_3$ H oder $C_{1-4}$-Alkyl darstellt.

14. Verbindungen nach Anspruch 13 der Formel VIII,

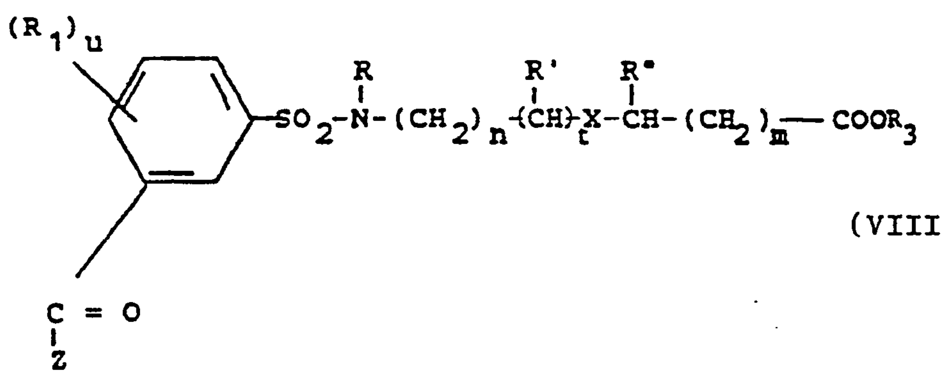

(VIII),

in der $R_1$, $R_3$, R, R', R", X, Z, m, n, u und t dasselbe wie in Anspruch 1 bedeuten.

15. Verbindungen nach Anspruch 13 der Formel IX,

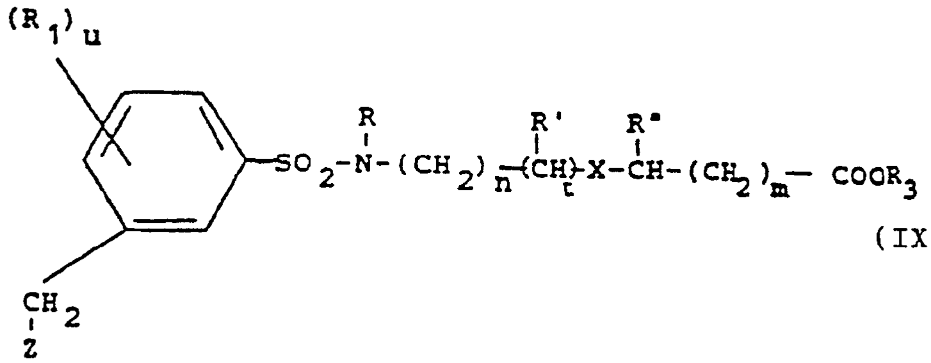

(IX),

in der $R_1$, $R_3$, R, R', R", X, Z, m, n, u und t dasselbe wie in Anspruch 1 bedeuten.

16. Verbindungen nach Anspruch 13 der Formel X,

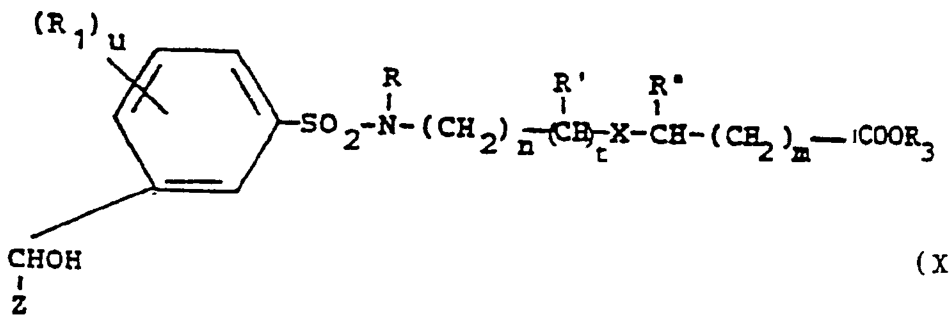

(X),

in der $R_1$, $R_3$, R, R', R", X, Z, m, n, u und t dasselbe wie in Anspruch 1 bedeuten.

17. Verbindungen nach Anspruch 14 der Formel VIII, in der die Gesamtzahl der Kohlenstoffatome der Kette

$$(CH_2)_n - (CH)_t - X - CH - (CH_2)_m - Y \geq 5 \text{ ist.}$$

18. Verbindungen nach Anspruch 15 der Formel IX, in der Gesamtzahl der Kohlenstoffatome der Kette

$$(CH_2)_n -(CH)_t\ X - CH - (CH_2)_m - Y \geq 5 \text{ ist.}$$

19. Verbindungen nach Anspruch 16 der Formel X, in der die Gesamtzahl der kohlenstoffatome der Kette

$$(CH_2)_n -(CH)_t- X - CH - (CH_2)_m - Y \geq 5 \text{ ist.}$$

20. Verbindungen nach Anspruch 13 der Formel XI,

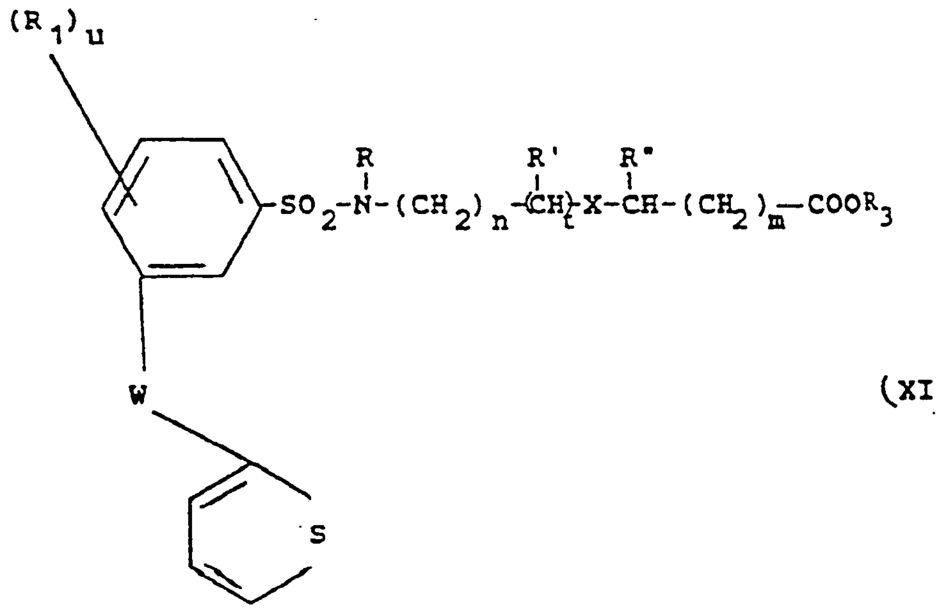

(XI),

in der
die Gesamtzahl der Kohlenstoffatome der Kette

$$(CH_2)_n - (CH)_t\ X - CH - (CH_2)_m - Y \geq 5 \text{ ist}$$

und
$R_1$, $R_3$, R, R′, R″, X, W, m, n, t und u dasselbe wie in Anspruch 1 bedeuten.

21. Verbindungen nach Anspruch 11 der Formel VIbis,

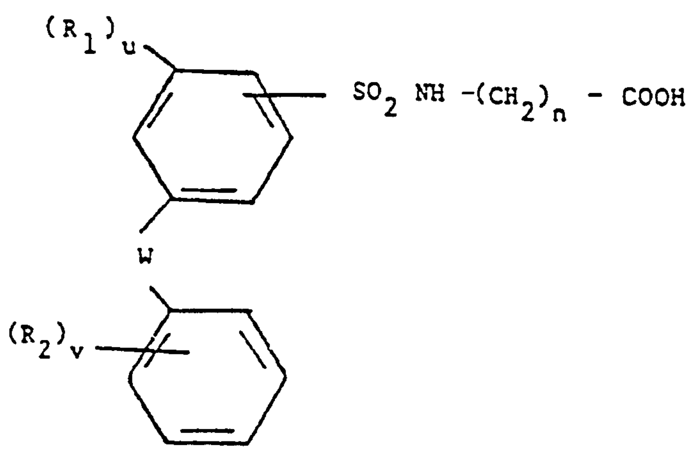

,

in der bedeuten :
- $R_1$ und $R_2$ ein Halogen,
- u O bis 2,
- v O bis 2,
- W C=O, CHOH oder $CH_2$

und
- n 3 bis 11.

22. Verbindungen nach Anspruch 11 der Formel VIter,

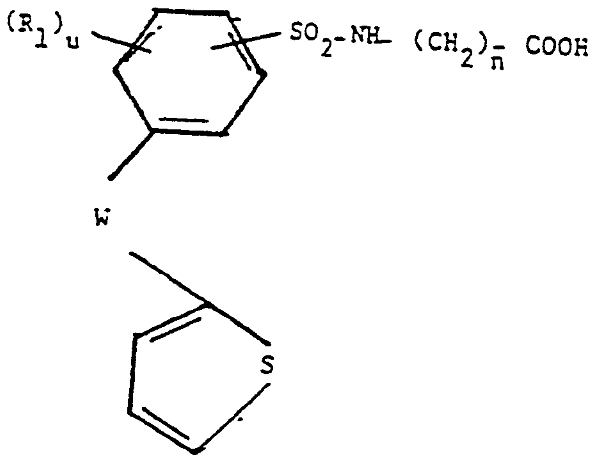

,

in der bedeuten :

- $R_1$ ein Halogen,
- u O bis 2,
- W C=O, CHOH oder $CH_2$

und

- n 3 bis 11.

23. Verbindungen nach Anspruch 1 der folgenden Formeln :

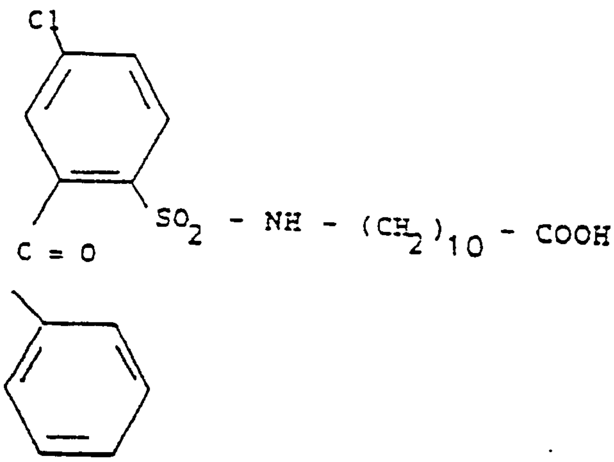

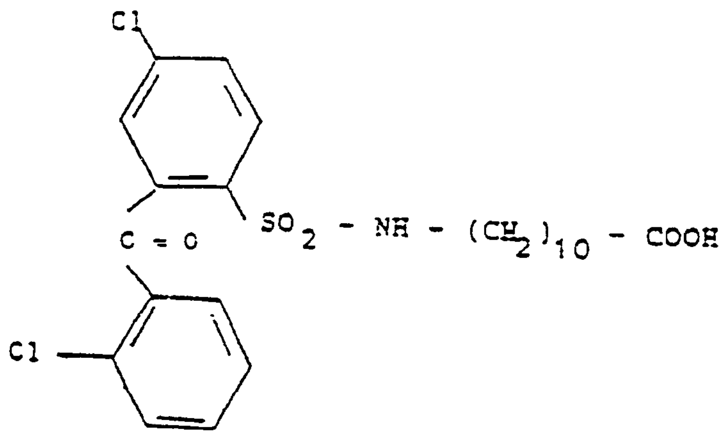

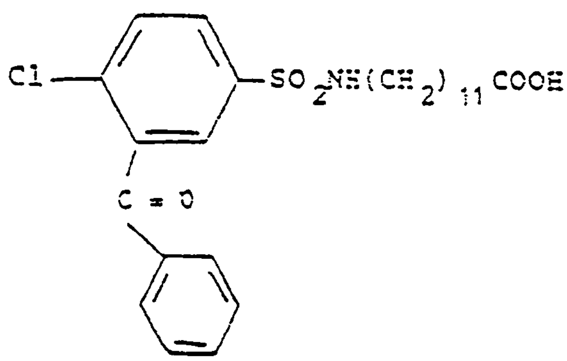

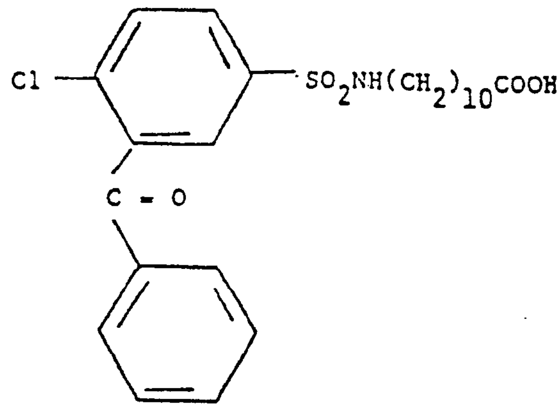

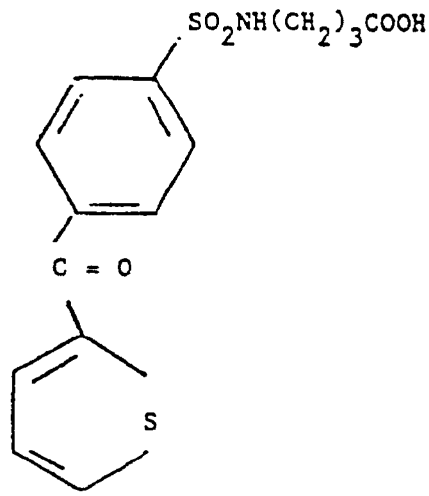

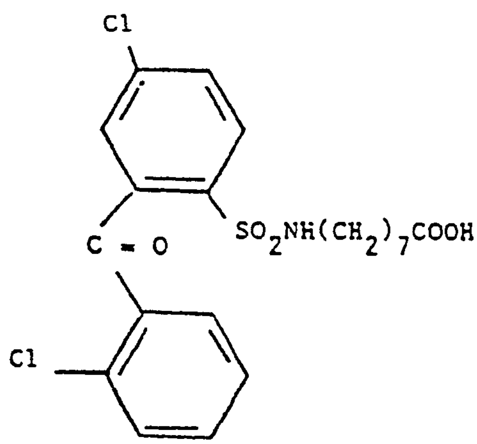

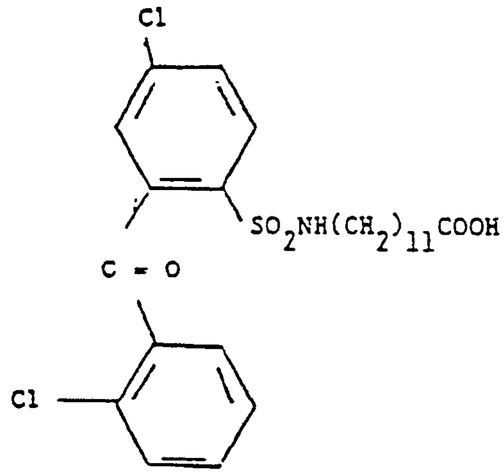

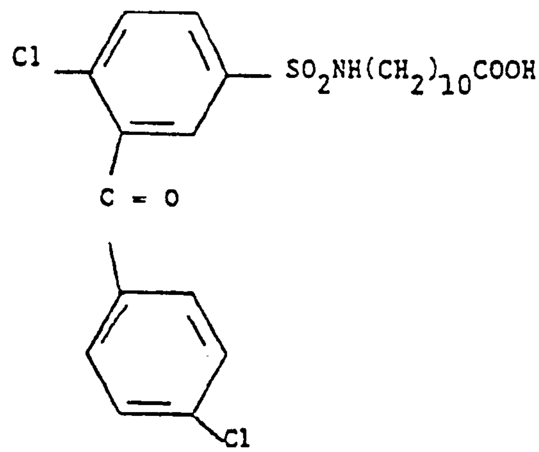

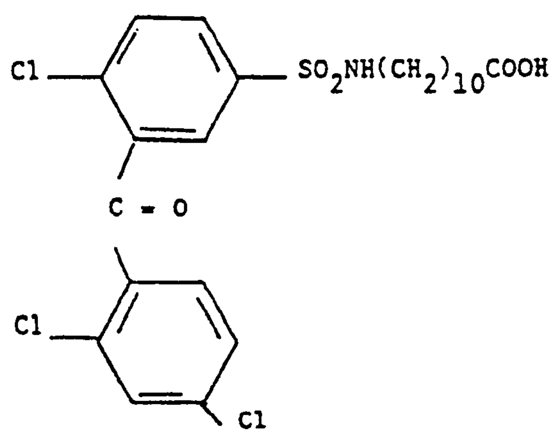

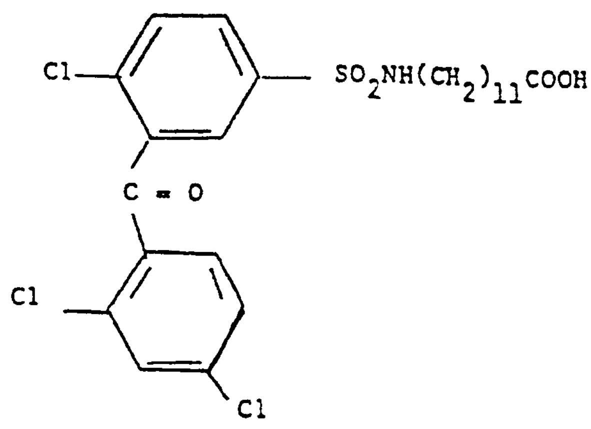

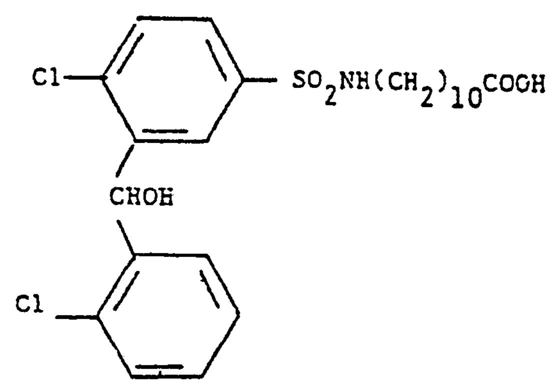

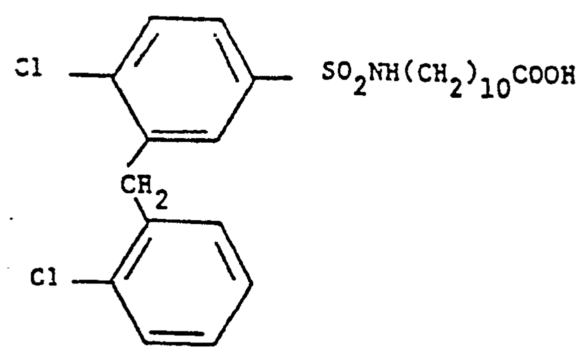

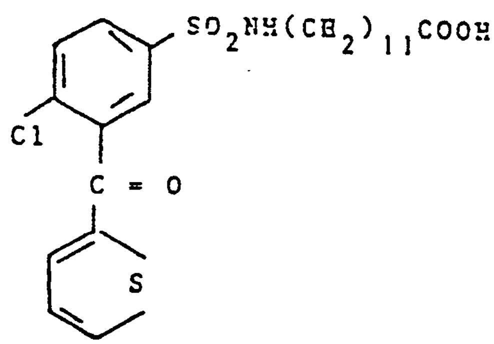

24. Verfahren zur Herstellung der Verbindungen der Formel I nach Anspruch 1, gekennzeichnet durch

. gegebenenfalls Reduktion einer Verbindung der Formel

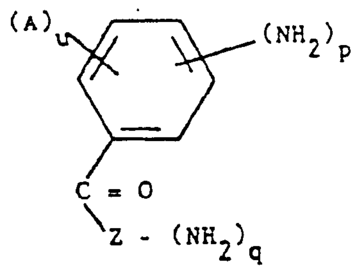

,

worin bedeuten :

– A dasselbe wie $R_1$ mit Ausnahme von $NH_2$, d.h. Cl, F, Br, $NO_2$, $CF_3$, $C_{1-4}$-Alkyl, $C_{1-4}$-Alkoxy, Acetamido oder Benzamido,

– Z

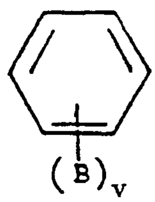

oder

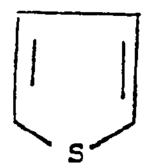

,

– B dasselbe wie $R_2$ mit Ausnahme von $NH_2$, d.h. Cl, $NO_2$, $CF_3$, $C_{1-4}$-Alkyl, $C_{1-4}$-Alkoxy, Acetamido oder Benzamido,

– u eine ganze Zahl von O bis 2,

– v eine ganze Zahl von O bis 2,

– p O oder 1

und

– q O oder 1, wobei p + q ≧ 1 ist,

insbesondere mit Aluminiumchlorid und Lithiumalanat, zur Reduktion von C=O zu $CH_2$ unter Erhalt der Verbindung der Formel

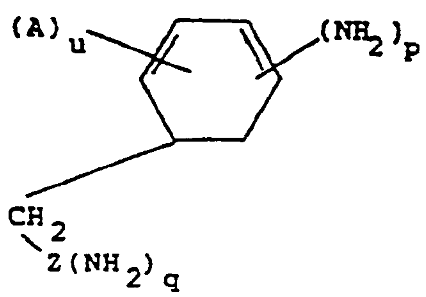

,

in der A, Z, u, p und q die obige Bedeutung besitzen,

. Sandmeyer-Reaktion mit einer der Aminverbindungen der oben angegebenen Formel unter Erhalt eines Sulfohalogenids der Formel

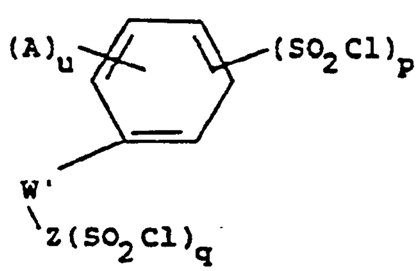

,

in der A, Z, u, p und q die obige Bedeutung besitzen und W′ C=O oder $CH_2$ darstellt, und

. Umsetzung des Sulfohalogenids, insbesondere des Sulfochlorids, der obigen Formel mit einer oder zwei Verbindungen der Formel

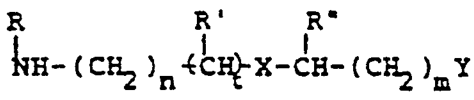,

in der bedeuten :

- R H, $C_{1-6}$-Alkyl oder Benzyl,
- R′ und R″ H oder $C_{1-4}$-Alkyl,
- X $(CH_2)r$, wobei r O oder 1 bedeutet, -CH=CH,

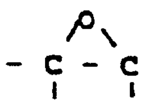 oder 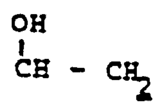,

- Y $COOR_3$, wobei $R_3$ H oder $C_{1-4}$-Alkyl darstellt,

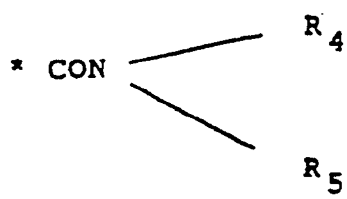,

worin $R_4$ und $R_5$ H oder $C_{1-4}$-Alkyl darstellen,

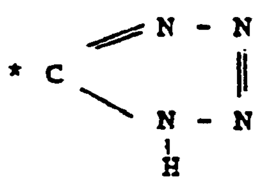,

* -C ≡ N oder

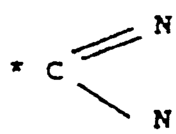,

wobei

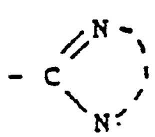

einen nichtaromatischen 5- oder 6-gliedrigen Ring darstellt, in dem die zwei oder drei Atome, mit denen der Ring vervollständigt wird, Kohlenstoffatome und/oder Sauerstoffatome und/oder Stickstoffatome sind, oder

* $NH_2$, $NHR_6$ oder $NR_6R_7$,
  wobei $R_6$ und $R_7$ $C_{1-4}$-Alkyl darstellen oder mit dem Stickstoffatom ein nichtaromatisches cyclisches Amin bilden können,
– mit der Maßgabe, daß X nicht

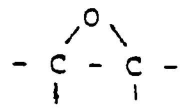

bedeutet, wenn Y $NH_2$ darstellt,
– n eine ganze Zahl von O bis 1O,
– m eine ganze Zahl von O bis 1O
und
– t O oder 1,
– wobei die Gesamtzahl der Atome der Kette

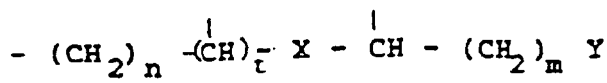

2 bis 2O beträgt,
zur Verbindung der Formel

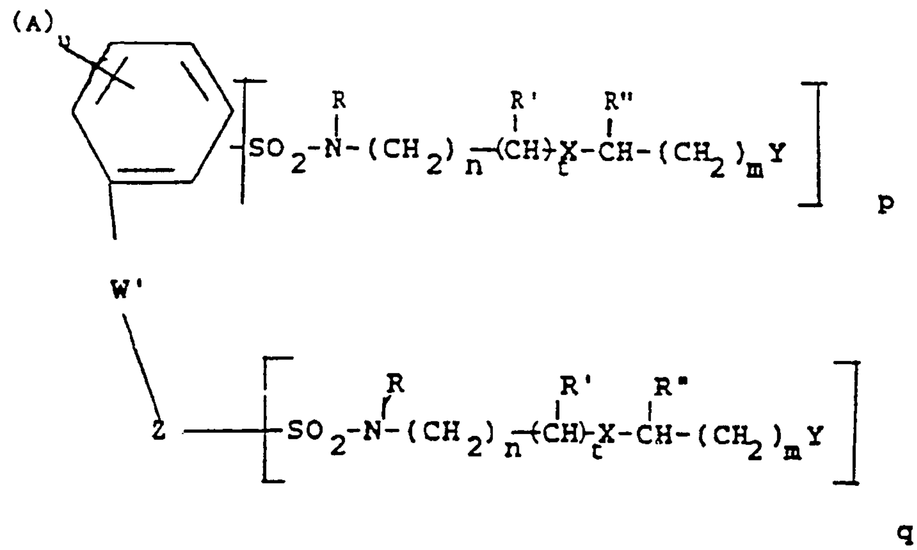

,

in der A, Z, W', R, R', R", X, Y, m, n, t, q und u die obige Bedeutung besitzen,
. und gegebenenfalls, wenn W' C=O darstellt, Reduktion der obigen Verbindung, insbesondere mit einem Alkaliborhydrid, wie Natriumborhydrid, zur Reduktion von C=O zu CHOH
. sowie gegebenenfalls anschließende Reduktion, wenn A und/oder B $NO_2$ darstellen.
25. Verfahren nach Anspruch 24 zur Herstellung der Verbindungen der Formel II,

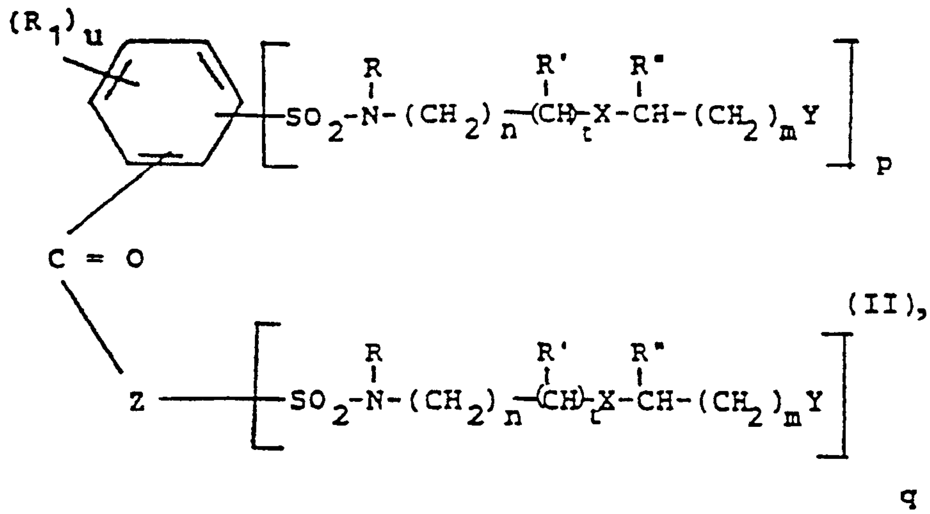

(II),

in der $R_1$, R, R′, R″, X, Y, Z, m, n, p, q, u und t dasselbe wie in Anspruch 2 bedeuten, gekennzeichnet durch Umsetzung eines Sulfohalogenids, insbesondere eines Sulfochlorids, der Formel

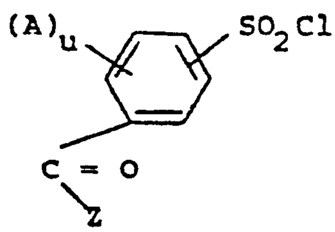

,

in der A, Z und u dasselbe wie in Anspruch 24 bedeuten, oder eines Disulfohalogenids, insbesondere eines Disulfochlorids, der Formel

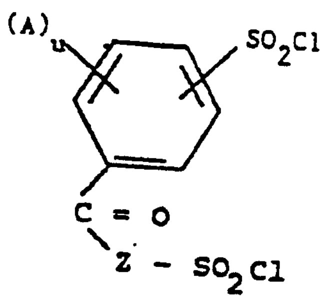

,

in der A, u und Z die obige Bedeutung besitzen, mit einer oder zwei Verbindungen der Formel

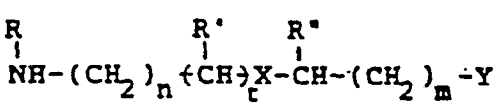

,

in der R, R′, R″, n, m, t, X und Y die Bedeutung von Anspruch 24 besitzen, sowie gegebenenfalls anschließende Reduktion, wenn A und/oder B $NO_2$ bedeuten.

26. Verfahren nach Anspruch 25, gekennzeichnet durch Umsetzung eines Sulfochlorids der Formel

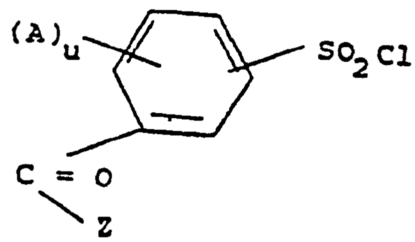

,

in der A, u und Z dasselbe wie in Anspruch 25 bedeuten,
oder eines Disulfochlorids der Formel

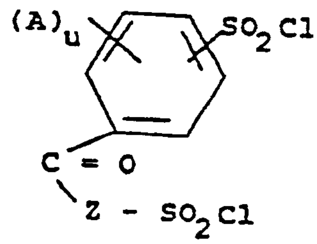

,

in der A, u und Z dasselbe wie Anspruch 25 bedeuten,
mit einer oder zwei Aminosäuren der Formel

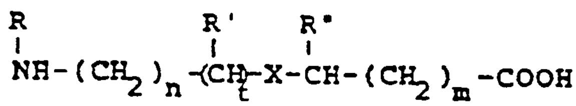

,

in der R, R′, R″, n, m, t und X dasselbe wie in Anspruch 25 bedeuten.

27. Verfahren nach Anspruch 25,
gekennzeichnet durch
– Veresterung einer Verbindung wie gemäß Anspruch 26 erhalten, in deren Formel Y COOH bedeutet, zu einer Verbindung der Formel II, in der Y $COOR_3$ darstellt, wobei $R_3$ $C_{1-4}$-Alkyl bedeutet,
oder
– Umwandlung einer Verbindung wie gemäß Anspruch 26 erhalten in eine Verbindung der Formel II,
in der
Y

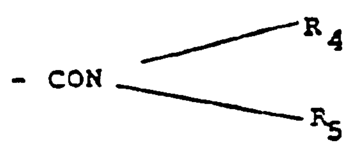

,

worin $R_4$, $R_5$ H oder $C_{1-4}$-Alkyl darstellen,

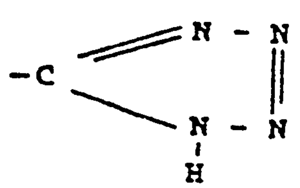 , $-C\equiv N$ oder 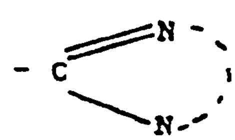

bedeutet.

28. Verfahren nach Anspruch 25,
gekennzeichnet durch Umsetzung eines Sulfochlorids der Formel

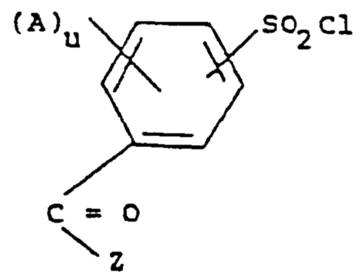,

wobei bedeuten :

– A dasselbe wie für $R_1$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido,

– Z

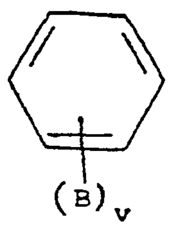 oder 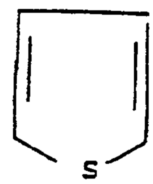,

– B dasselbe wie für $R_2$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido,

– u eine ganze Zahl von O bis 2

und

– v eine ganze Zahl von O bis 2,

oder eines Disulfohalogenids, insbesondere eines Disulfochlorids der Formel

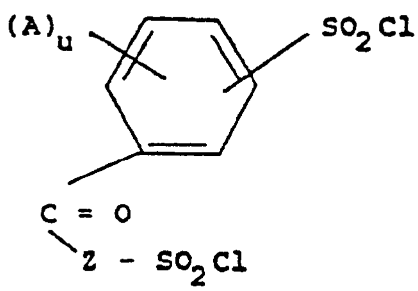,

in der A, u und Z die obige Bedeutung besitzen, mit einem Aminoalkylamin der Formel

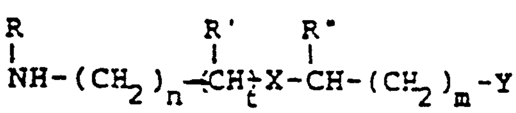,

in der bedeuten :

– R H, $C_{1-6}$-Alkyl oder Benzyl,

– R′ und R″ H oder $C_{1-4}$-Alkyl,

– n eine ganze Zahl von O bis 1O,

– m eine ganze Zahl von O bis 1O,

– t O oder 1,

– Y $NH_2$, $NHR_6$ oder $NR_6R_7$, wobei $R_6$ und $R_7$ $C_{1-4}$-Alkyl darstellen oder mit dem Stickstoff ein nichtaromatisches cyclisches Amin bilden,

und

– X dasselbe wie in Formel I.

29. Verfahren nach Anspruch 25, dadurch gekennzeichnet, daß die eingesetzte Aminosäure oder das eingesetzte Dialkylaminoalkylamin unter

$$- NH_2 - (CH_2)_n - COOH,$$

wobei n 1 bis 11 bedeutet, beziehungsweise

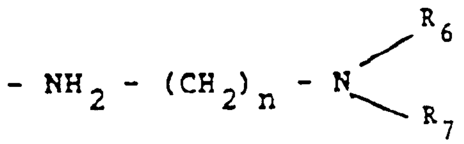

,

worin n 2 oder 3 bedeutet und $R_6$ und $R_7$ $C_{1-4}$-Alkyl oder zusammen mit dem Stickstoffatom einen Morpholinring darstellen, ausgewählt wird und das eingesetzte Sulfochlorid ausgewählt wird unter

- 2-Benzoyl-4-nitrobenzolsulfonylchlorid,
- 2-Benzoyl-4-chlorbenzolsulfonylchlorid,
- 4-Chlor-2-(2-chlorbenzoyl)-benzolsulfonylchlorid,
- 2-Benzoylbenzolsulfonylchlorid,
- 2-(4-Chlorbenzoyl)-benzolsulfonylchlorid,
- 4-Benzoylbenzolsulfonylchlorid,
- 3-Benzoylbenzolsulfonylchlorid,
- 3-(2,4-Dichlorbenzoyl)-4-chlorbenzolsulfonylchlorid
- 3-Benzoyl-4-chlorbenzolsulfonylchlorid,
- 3-(4-Chlorbenzoyl)-4-chlorbenzolsulfonylchlorid,
- 4-Benzoyl-3-chlorbenzolsulfonylchlorid und
- 4-Chlor-3-(2-thenoyl)-benzolsulfonylchlorid.

3O. Verfahren nach Anspruch 24 zur Herstellung der Verbindungen der Formel III,

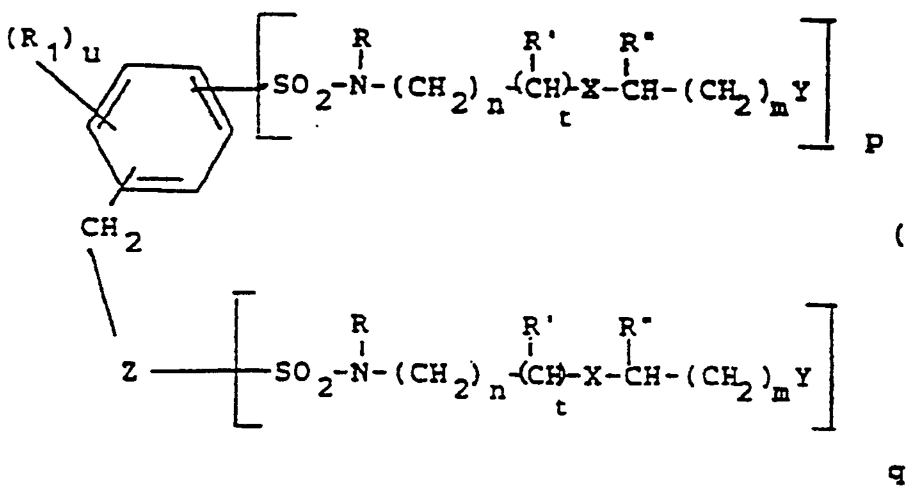

(III),

in der $R_1$, R, R′, R″, X, Y, Z, m, n, t, p, q und u die Bedeutung von Anspruch 3 besitzen, gekennzeichnet durch Reduktion einer Verbindung der Formel

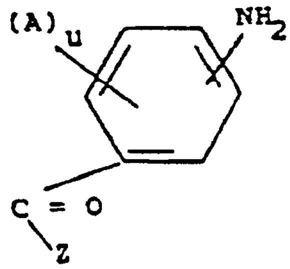

oder der Formel

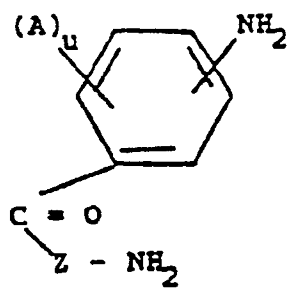

,

worin A dasselbe wie in Anspruch 24 bedeutet,
– zur Reduktion von C=O zur $CH_2$ unter Erhalt der Verbindung der Formel

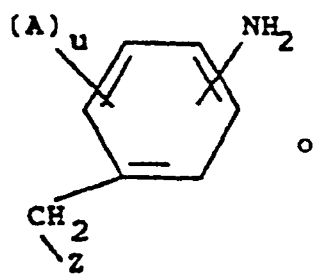

oder

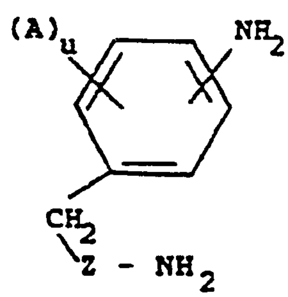

)

– Sandmeyer-Reaktion mit einer dieser Verbindungen unter Erhalt der Verbindungen der Formel

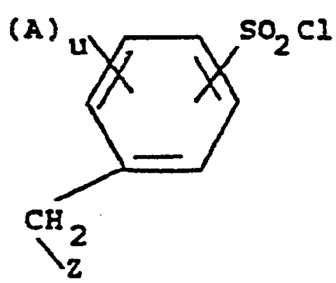

oder

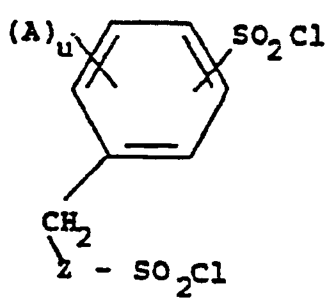

und
– kondensation des Sulfohalogenids oder Disulfohalogenids der obigen Formel mit einer Verbindung der Formel

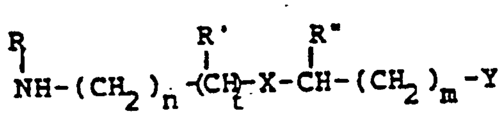

,

in der R, R', R", X, Y, n, m und t die Bedeutung von Anspruch 24 besitzen, sowie gegebenenfalls

– anschließende Reduktion, wenn A und/oder B $NO_2$ bedeuten.

31. Verfahren nach Anspruch 3O, dadurch gekennzeichnet, daß die eingesetzte Aminosäure oder das eingesetzte Dialkylaminoalkylamin unter

$$- NH_2 -(CH_2)_n - COOH ,$$

wobei n 1 bis 11 bedeutet, beziehungsweise

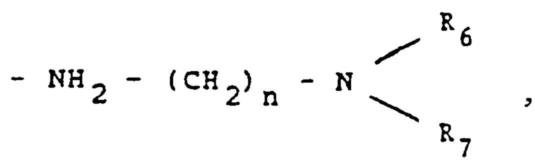

wobei n 2 oder 3 bedeutet und $R_6$ und $R_7$ $C_{1-4}$-Alkyl oder zusammen mit dem Stickstoffatom einen Morpholinring darstellen,

ausgewählt werden

und die Verbindung der Formel

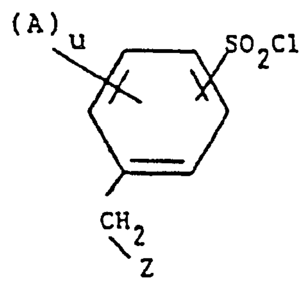

ausgewählt wird unter

– 3-(2,4-Dichlorbenzyl)-4-chlorbenzolsulfonylchlorid,
– 3-(4-Chlorbenzyl)-4-chlorbenzolsulfonylchlorid,
– 2-Benzyl-4-chlorbenzolsulfonylchlorid,
– 2-(2,4-Dichlorbenzyl)-4-chlorbenzolsulfonylchlorid,
– 2-(4-Chlorbenzyl)-4-chlorbenzolsulfonylchlorid,
– 4-Benzyl-3-chlorbenzolsulfonylchlorid,
– 4-(2,4-Dichlorbenzyl)-3-chlorbenzolsulfonylchlorid,
– 4-(4-Chlorbenzyl)-3-chlorbenzolsulfonylchlorid und
– 3-Benzyl-4-chlorbenzolsulfonylchlorid.

32. Verfahren nach Anspruch 24 zur Herstellung der Verbindungen der Formel IV,

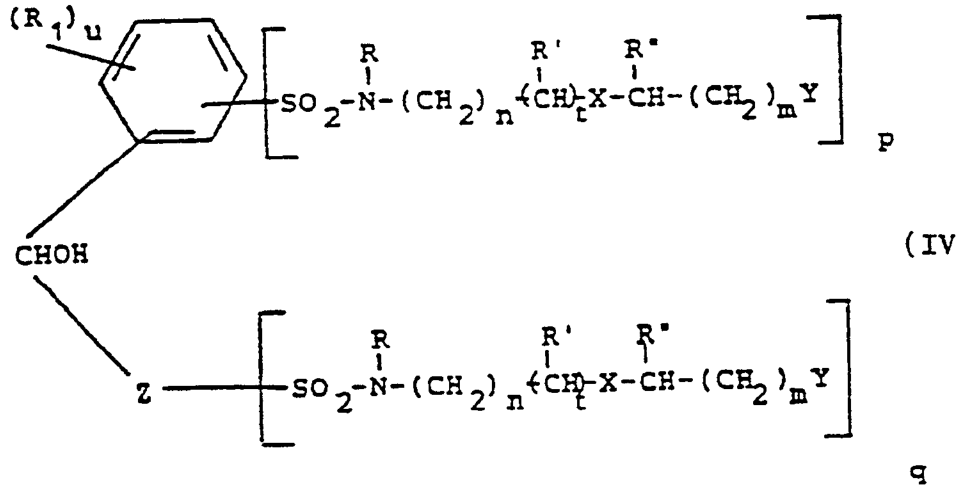

(IV),

in der $R_1$, R, R', R", X, Y, m, n, t, p, q und u die Bedeutung von Anspruch 4 besitzen,
gekennzeichnet durch
Reduktion einer Verbindung der Formel

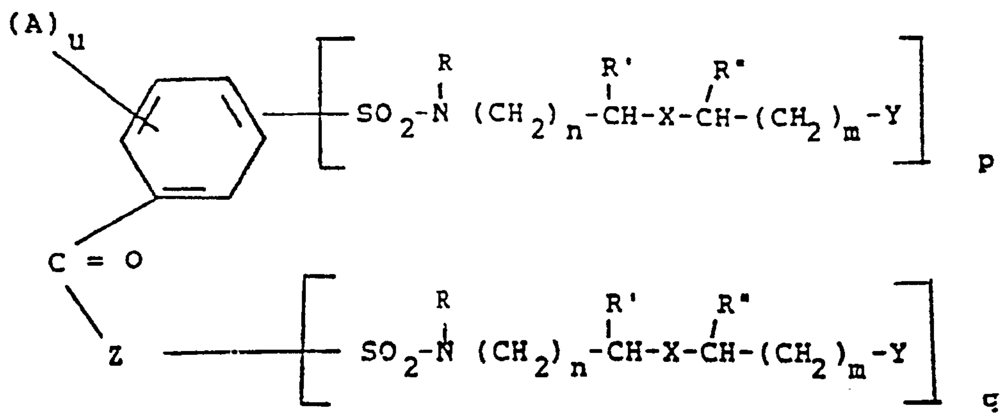

,

in der A, X, Y, Z, R, R', R", m, n, p, q, t und u dasselbe wie in Anspruch 24 bedeuten,
und wie gemäß Anspruch 25 erhalten, vor einer gegebenenfalls vorgenommenen Reduktion von A und/oder B, wenn diese $NO_2$ darstellen,
zur Umwandlung von C=O in CHOH mit einem Alkaliborhydrid.

33. Verfahren nach Anspruch 32,
dadurch gekennzeichnet, daß
die eingesetzte Aminosäure oder das eingesetzte Dialkylaminoalkylamin unter

$$- NH_2 -(CH_2)_n- COOH ,$$

worin n 1 bis 11 bedeutet,
beziehungsweise

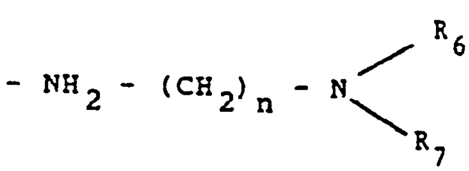

,

worin n 2 oder 3 bedeutet und $R_6$ und $R_7$ $C_{1-4}$-Alkyl oder zusammen mit dem Stickstoffatom einen Morpholinring darstellen, ausgewählt werden
und das eingesetzte Sulfochlorid ausgewählt wird unter

- 2-Benzoyl-4-nitrobenzolsulfonylchlorid,
- 2-Benzoyl-4-chlorbenzolsulfonylchlorid,
- 4-Chlor-2-(2-chlorbenzoyl)-benzolsulfonylchlorid,
- 2-Benzoylbenzolsulfonylchlorid,
- 2-(4-Chlorbenzoyl)-benzolsulfonylchlorid,
- 4-Benzoylbenzolsulfonylchlorid,
- 3-Benzoylbenzolsulfonylchlorid,
- 3-(2,4-Dichlorbenzoyl)-4-chlorbenzolsulfornylchlorid,
- 3-Benzoyl-4-chlorbenzolsulfonylchlorid,
- 3-(4-Chlorbenzoyl)-4-chlorbenzolsulfonylchlorid,
- 4-Benzoyl-3-chlorbenzolsulfonylchlorid und
- 4-Chlor-3-(2-thenoyl)-benzolsulfonylchlorid.

34. Verbindungen nach einem der Ansprüche 1 bis 23 als therapeutische Wirkstoffe.

35. Pharmazeutische Zusammensetzungen,
dadurch gekennzeichnet, daß sie als Wirkstoff eine der Verbindungen nach einem der Ansprüche 1 bis 23 zusammen mit einem pharmazeutisch akzeptablen Träger enthalten.

36. Pharmazeutische Zusammensetzung zur Behandlung entzündlicher, allergischer oder asthmatischer Zustände, dadurch gekennzeichnet, daß sie als Wirkst off eine der Verbindungen nach einem der Ansprüche 1 bis 23 enthält.

37. Pharmazeutische Zusammensetzung zur Behandlung von Niereninsuffizienz und cardiovasculären Erkrankungen, dadurch gekennzeichnet, daß sie als Wirkstoff eine der Verbindungen nach einem der Ansprüche 1 bis 23 enthält.

38. Pharmazeutische Zusammensetzungen, die als Wirkstoff mindestens eine der Verbindungen nach einem der Ansprüche 1 bis 23 zusammen mit einem pharmazeutisch akzeptablen Träger enthalten, in Form von injizierbaren Präparationen, die 10 bis 100 mg Wirkstoff pro Dosiereinheit enthalten.

39. Pharmazeutische Zusammensetzungen, die als Wirkstoff mindestens eine der Verbindungen nach einem der Ansprüche 1 bis 23 zusammen mit einem pharmazeutisch akzeptablen Träger enthalten, in Form von zur oralen Verabreichung vorgesehenen Darreichungsformen, die 10 bis 500 mg Wirkstoff pro Dosiereinheit enthalten.

**Patentanspruche für folgenden Vertragsstaat : GR**

1. Verbindungen der Formel I,

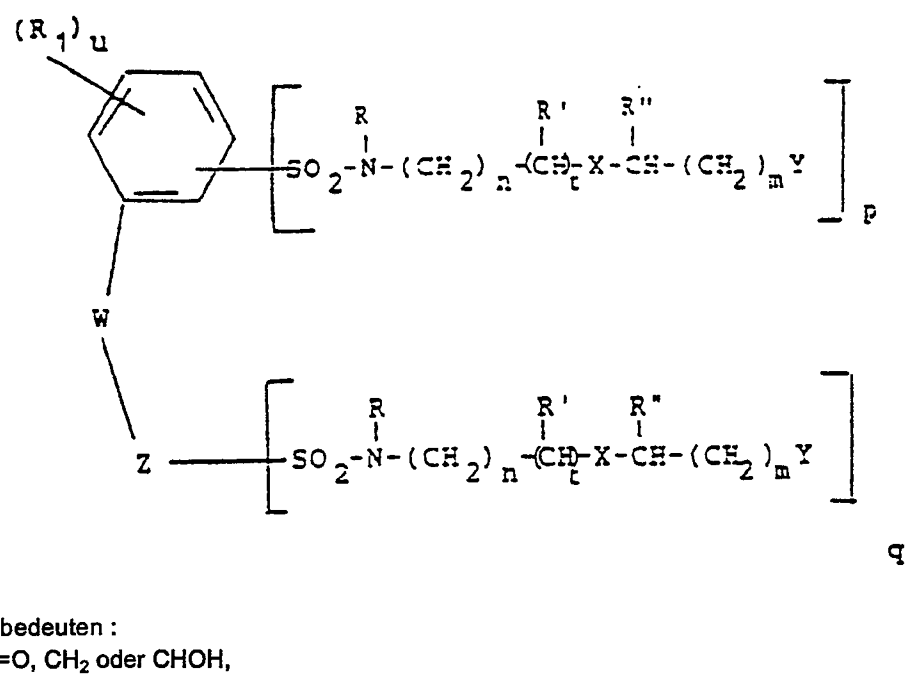

(I),

wobei bedeuten :
- W C=O, $CH_2$ oder CHOH,
- Z

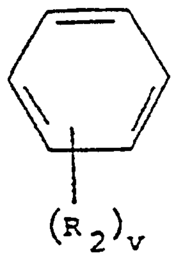

oder

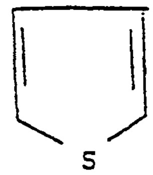

,

– $R_1$ Cl, F, Br, $NO_2$, $NH_2$, $CF_3$, $C_{1-4}$-Alkyl, insbesondere Methyl oder Ethyl, $C_{1-4}$-Alkoxy, Acetamido oder Benzamido,

– $R_2$ Cl, F, Br, $NO_2$, $NH_2$, $CF_3$, $C_{1-4}$-Alkyl, $C_{1-4}$-Alkoxy, Acetamido oder Benzamido,

– R H, $C_{1-6}$-Alkyl oder Benzyl,

– R' und R" H oder $C_{1-4}$-Alkyl,

– X $(CH_2)_r$, wobei r O oder 1 bedeutet, -CH=CH,

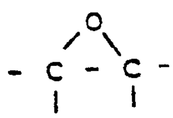

oder

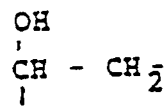

,

– Y $COOR_3$, wobei $R_3$ H oder $C_{1-4}$-Alkyl darstellt,

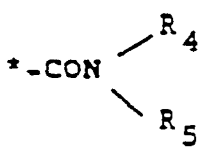

,

worin $R_4$ und $R_5$ H oder $C_{1-4}$-Alkyl darstellen,

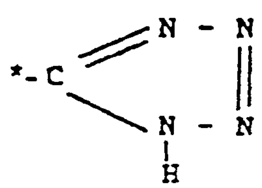

,

* - C ≡ N oder

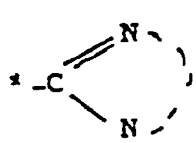

,

wobei

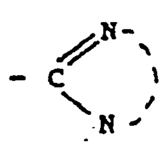

einen nichtaromatischen 5- oder 6-gliedrigen Ring darstellt, in dem die zwei oder drei Atome, mit denen

der Ring vervollständigt wird, Kohlenstoffatome und/oder Sauerstoffatome und/oder Stickstoffatome sind, oder

* $NH_2$, $NHR_6$ oder $NR_6R_7$, wobei $R_6$ und $R_7$ $C_{1-4}$-Alkyl darstellen oder mit dem Stickstoffatom ein nichtaromatisches cyclisches Amin bilden können,

– mit der Maßgabe, daß X nicht

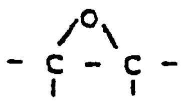

bedeutet, wenn Y $NH_2$ darstellt,

– u eine ganze Zahl von O bis 2,
– v eine ganze Zahl von O bis 2,
– p O oder 1,
– q O oder 1, wobei p + q $\geqq$ 1 ist,
– n eine ganze Zahl von O bis 1O,
– m eine ganze Zahl von O bis 1O

und

– t O oder 1,
– wobei die Gesamtzahl der Kohlenstoffatome der Kette

$$-(CH_2)_n-(CH)_t-X-CH-(CH_2)_m\,Y$$

2 bis 20 beträgt,

sowie ihre physiologisch akzeptablen Salze.

2. Verbindungen nach Anspruch 1 der Formel II,

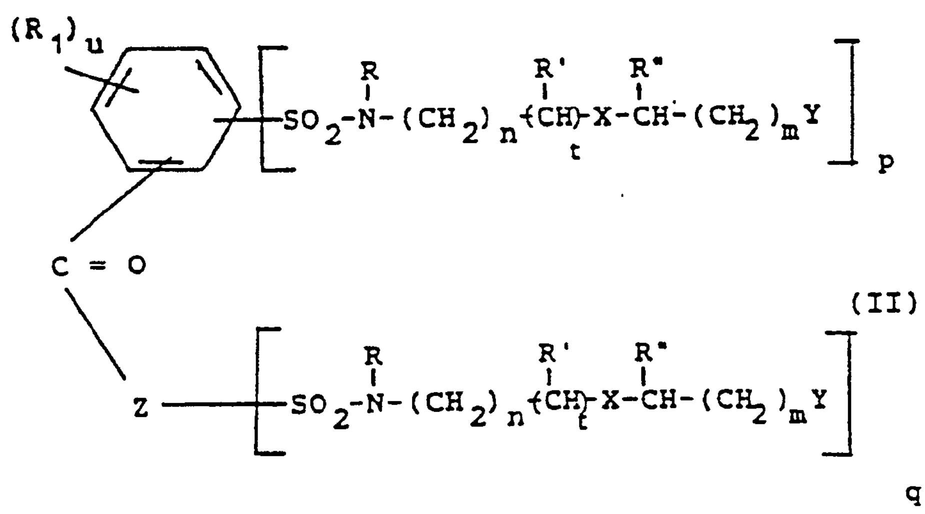

(II) ,

in der $R_1$, R, R', R", X, Y, Z, m, n, t, p, q und u dasselbe wie in Anspruch 1 bedeuten.

3. Verbindungen nach Anspruch 1 der Formel III,

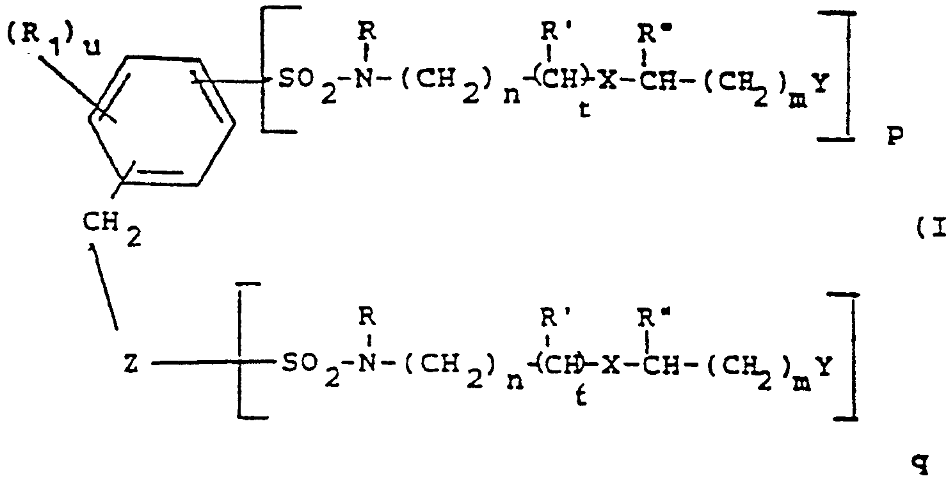

(III) ,

4. Verbindungen nach Anspruch 1 der Formel IV,

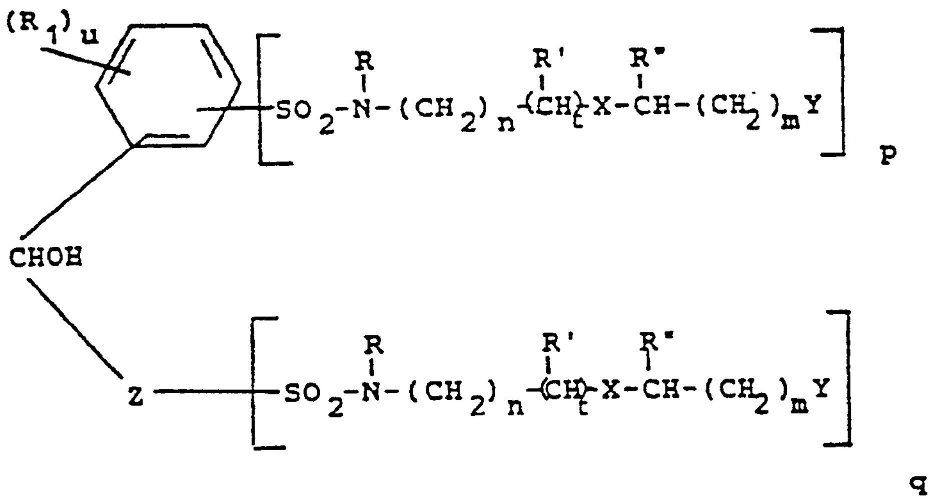

(IV),

in der $R_1$, R, R', R", X, Y, Z, m, n, t, p, q und u dasselbe wie in Anspruch 1 bedeuten.

5. Verbindungen nach Anspruch 2 der Formel V,

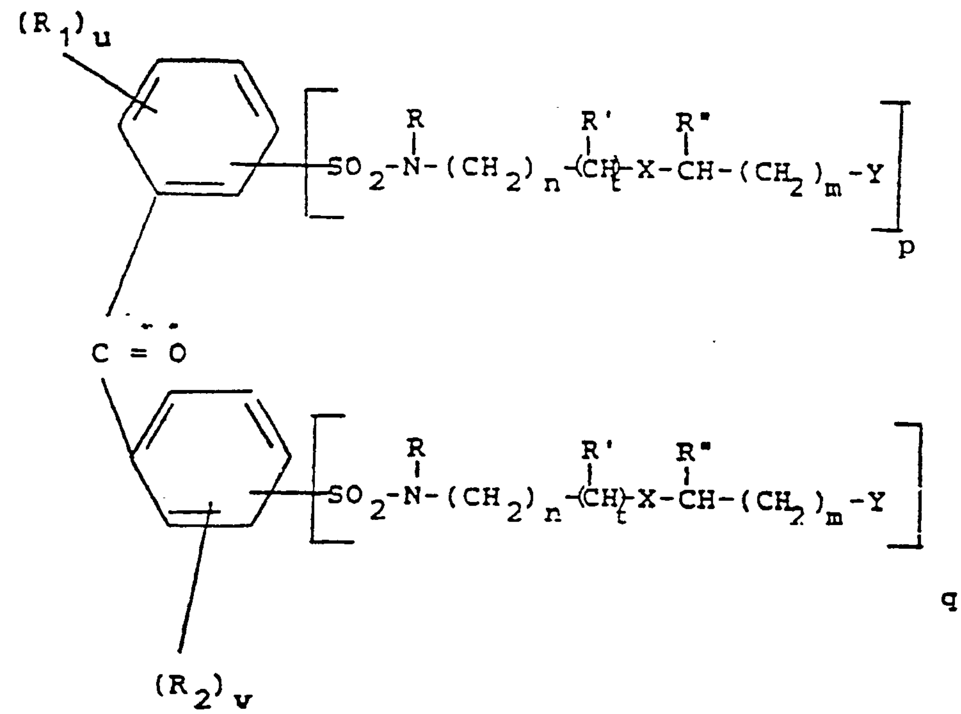

(V),

in der $R_1$, $R_2$, R, R', R'', X, Y, Z, m, n, p, q, t, u und v dasselbe wie in Anspruch 1 bedeuten.

6. Verbindungen nach Anspruch 5 der Formel V, in der Y $COOR_3$ bedeutet, wobei $R_3$ H oder $C_{1-4}$-Alkyl darstellt.

7. Verbindungen nach Anspruch 5 der Formel V, in der Y $NH_2$, $NHR_6$ oder $NR_6R_7$ bedeutet, wobei $R_6$ und $R_7$ dasselbe wie in Anspruch 1 bedeuten.

8. Verbindungen nach Anspruch 5 der Formel V, in der p oder q gleich O sind.

9. Verbindungen nach Anspruch 8, dadurch gekennzeichnet, daß

- u + v ≧ 1 ist

und

- die Gesamtzahl der Kohlenstoffatome der Kette

$$(CH_2)_n - (CH)_t - X - CH - (CH_2)_m - Y \geq 5 \text{ ist.}$$

10. Verbindungen nach Anspruch 5 der Formel V mit folgenden Definitionen :

- P = 1,
- q = 1,
- u + v ≧ 1

und

- die Gesamtzahl der Kohlenstoffatome der Kette

$$(CH_2)_n - (CH_t) - X - CH - (CH_2)_m - Y \text{ ist } \geq 5.$$

11. Verbindungen nach Anspruch 1 der Formel VI,

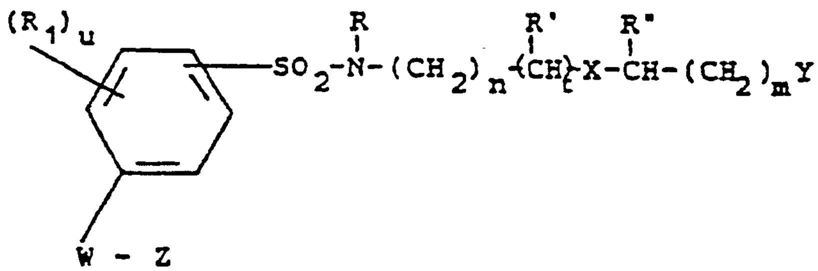

(VI),

in der $R_1$, R, R′, R″, X, Y, Z, W, m, n, u und t dasselbe wie in Anspruch 1 bedeuten.

12. Verbindungen nach Anspruch 11 der Formel VII,

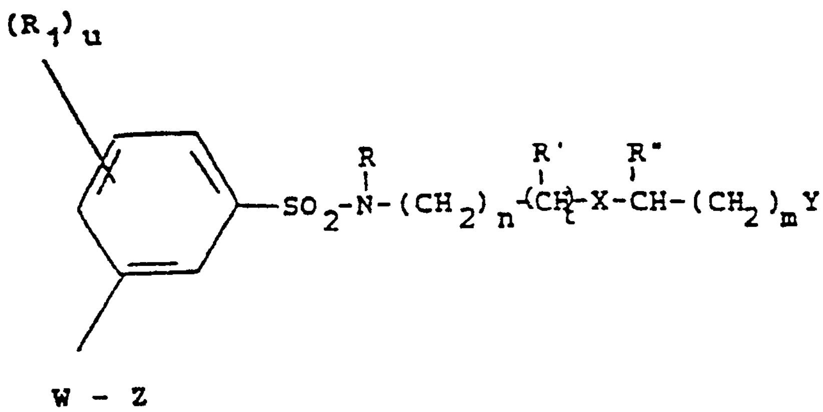

(VII),

in der $R_1$, R, R′, R″, X, Y, Z, W, m, n, u und t dasselbe wie in Anspruch 1 bedeuten.

13. Verbindungen nach Anspruch 12 der Formel VII, in der Y $COOR_3$ bedeutet, wobei $R_3$ H oder $C_{1-4}$-Alkyl darstellt.

14. Verbindungen nach Anspruch 13 der Formel VIII,

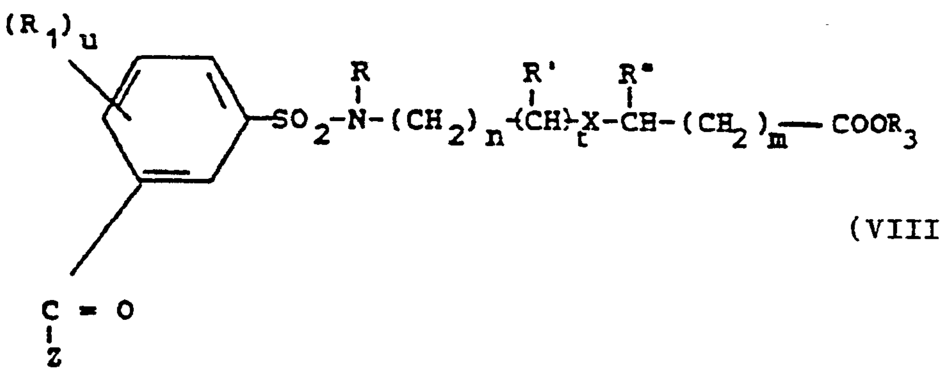

(VIII),

in der $R_1$, $R_3$, R, R′, R″, X, Z, m, n, u und t dasselbe wie in Anspruch 1 bedeuten.

15. Verbindungen nach Anspruch 13 der Formel IX,

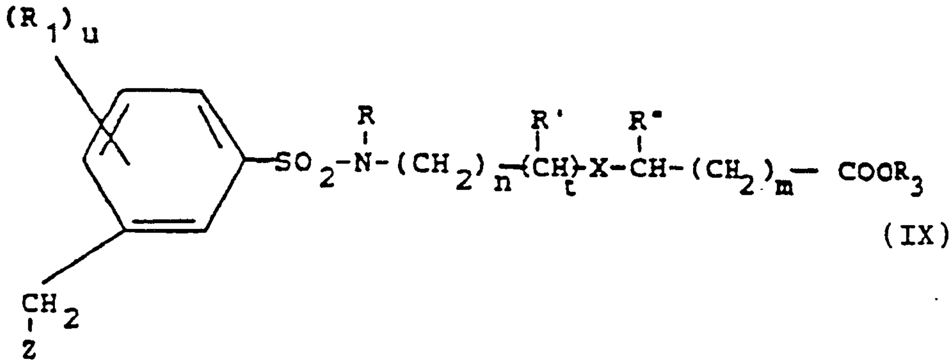

(IX),

in der $R_1$, $R_3$, R, R', R", X, Z, m, n, u und t dasselbe wie in Anspruch 1 bedeuten.

16. Verbindungen nach Anspruch 13 der Formel X,

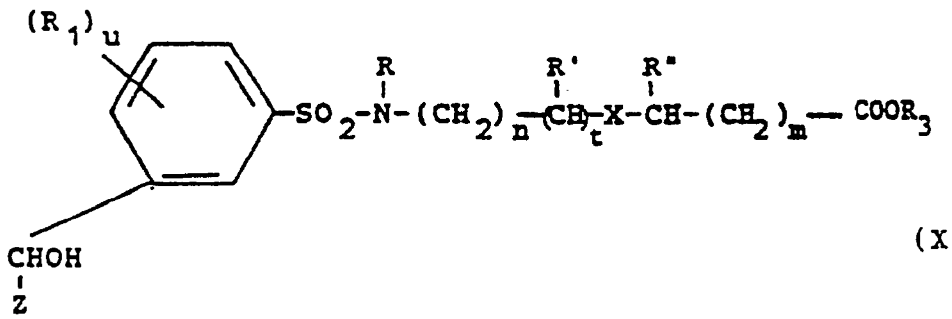

(X),

in der $R_1$, $R_3$, R, R', R", X, Z, m, n, u und t dasselbe wie in Anspruch 1 bedeuten.

17. Verbindungen nach Anspruch 14 der Formel VIII, in der die Gesamtzahl der kohlenstoffatome der Kette

$$(CH_2)_n - (\overset{|}{C}H)_t - X - \overset{|}{C}H - (CH_2)_m - Y \geq 5 \text{ ist.}$$

18. Verbindungen nach Anspruch 15 der Formel IX, in der Gesamtzahl der kohlenstoffatome der kette

$$(CH_2)_n - (\overset{|}{C}H)_t\ X - \overset{|}{C}H - (CH_2)_m - Y \geq 5 \text{ ist.}$$

19. Verbindungen nach Anspruch 16 der Formel X, in der die Gesamtzahl der kohlenstoffatome der Kette

$$(CH_2)_n - (\overset{|}{C}H)_t - X - \overset{|}{C}H - (CH_2)_m - Y \geq 5 \text{ ist.}$$

20. Verbindungen nach Anspruch 13 der Formel XI,

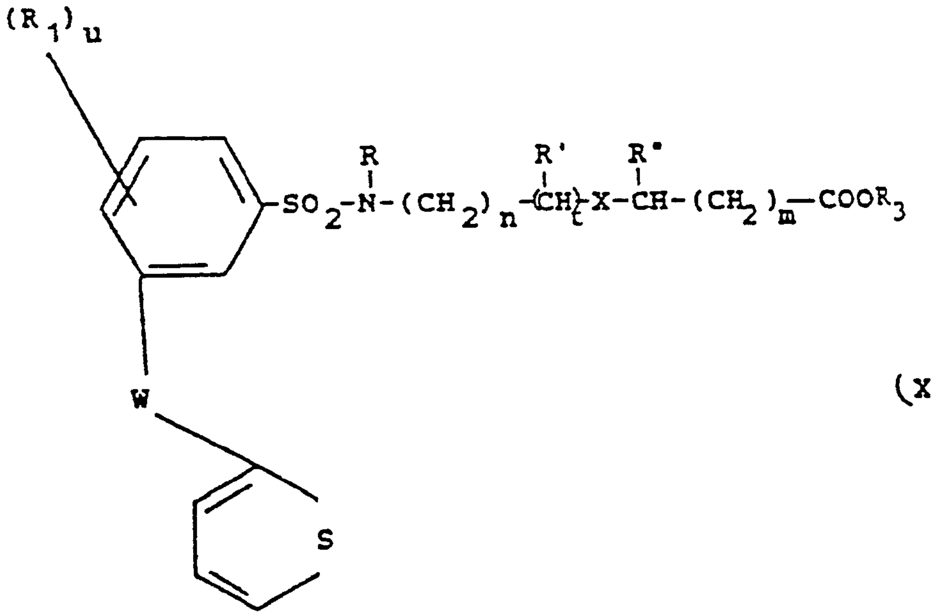

(XI),

in der die Gesamtzahl der Kohlenstoffatome der Kette

$$(CH_2)_n - (CH)_t - X - CH - (CH_2)_m - Y \geq 5 \text{ ist}$$

und $R_1$, $R_3$, R, R', R", X, W, m, n, t und u dasselbe wie in Anspruch 1 bedeuten.

21. Verbindungen nach Anspruch 11 der Formel VIbis,

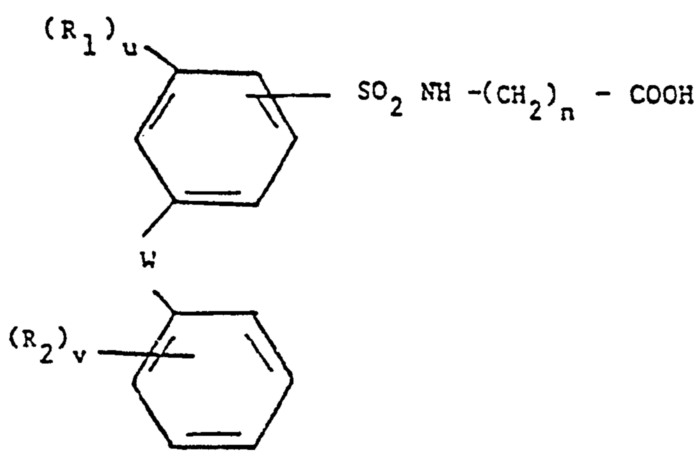

,

in der bedeuten :

- $R_1$ und $R_2$ ein Halogen,
- u O bis 2,
- v O bis 2,
- W C=O, CHOH oder $CH_2$

und

- n 3 bis 11.

22. Verbindungen nach Anspruch 11 der Formel VIter,

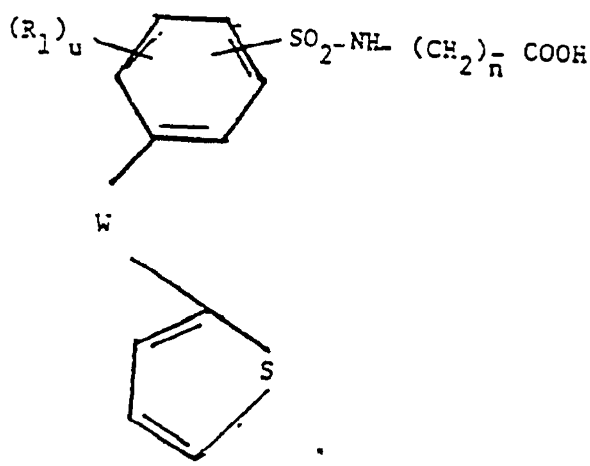

,

in der bedeuten :

– $R_1$ ein Halogen,

– u O bis 2,

– W C=O, CHOH oder $CH_2$

und

– n 3 bis 11.

23. Verbindungen nach Anspruch 1 der folgenden Formeln :

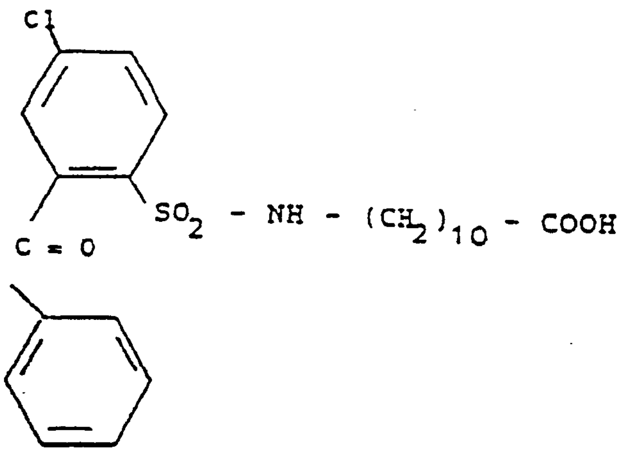

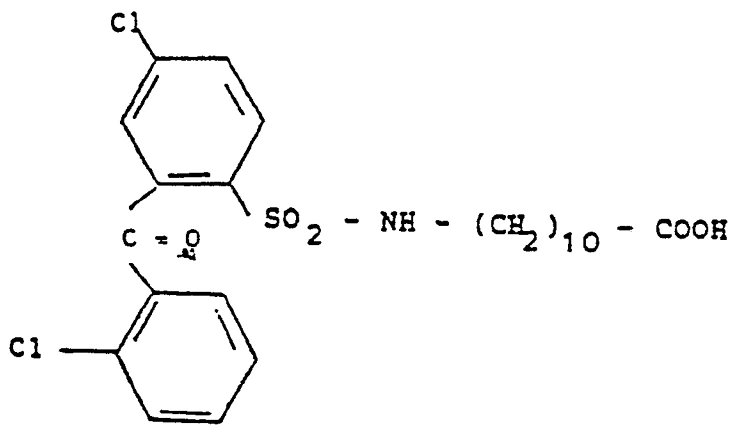

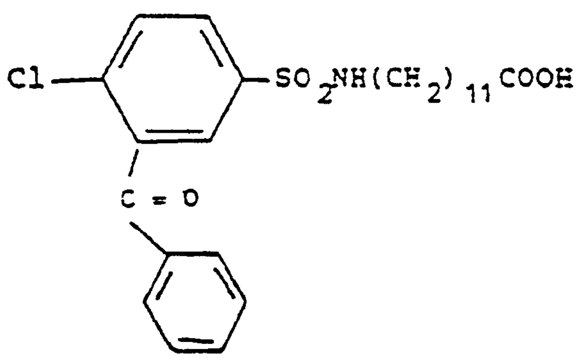

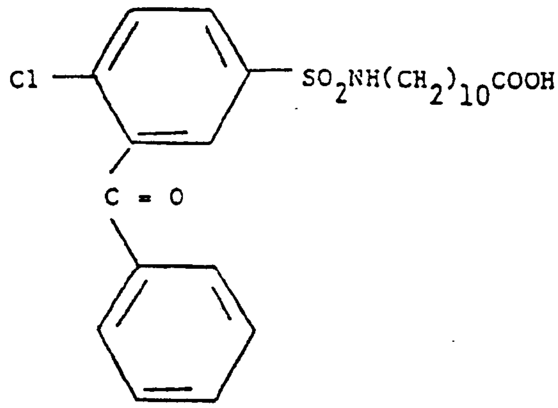

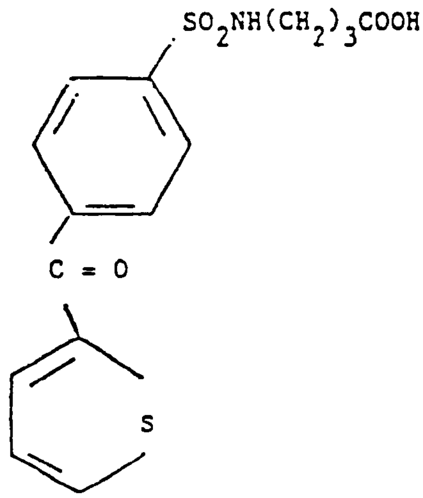

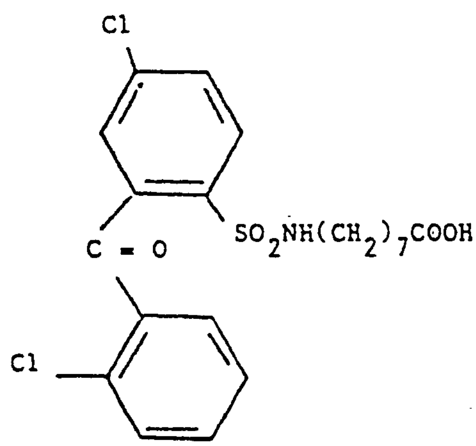

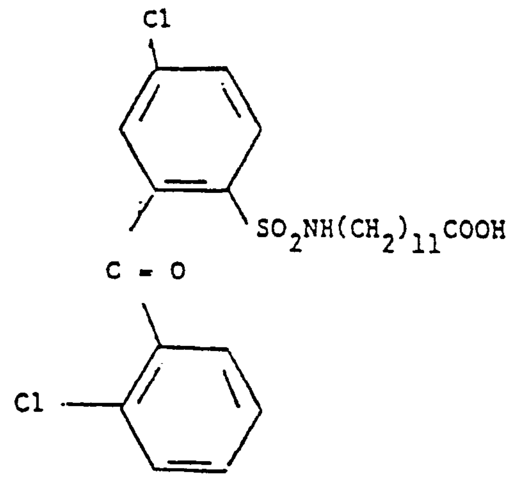

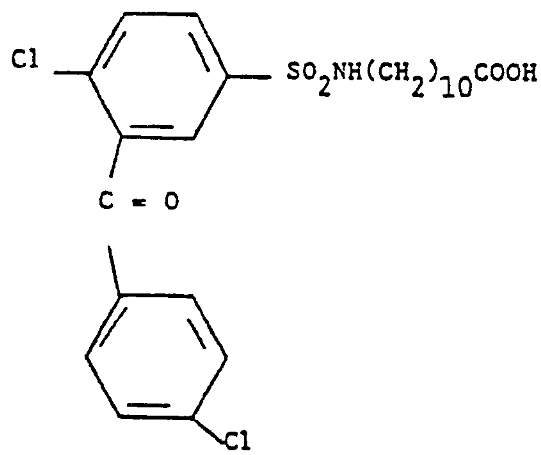

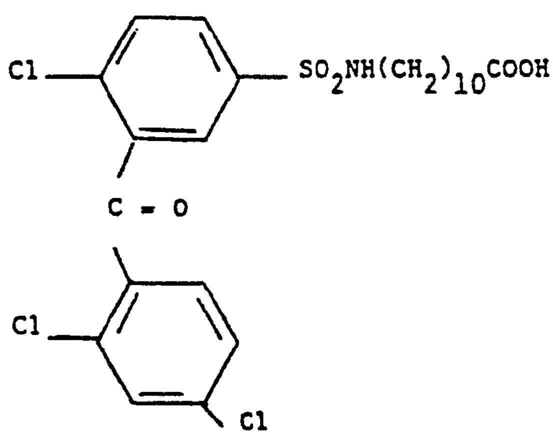

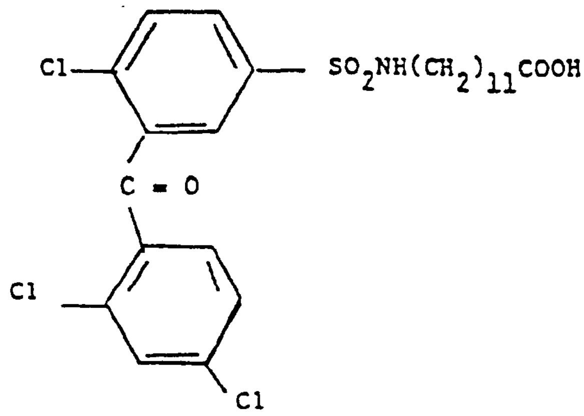

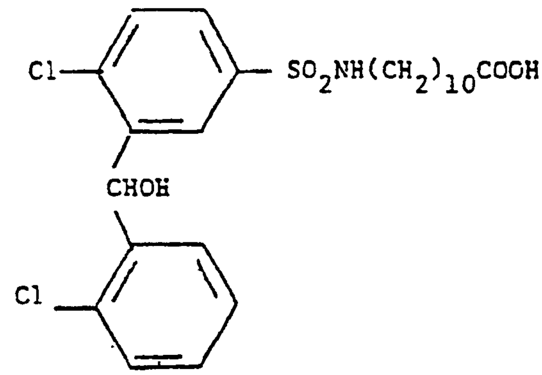

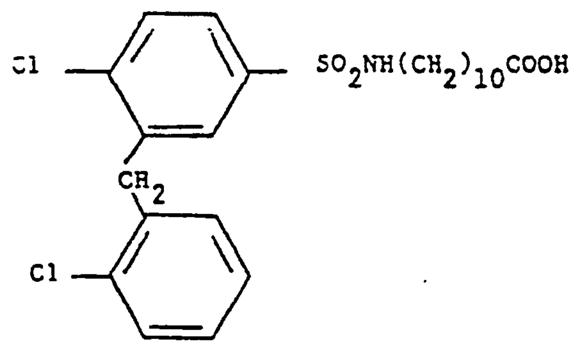

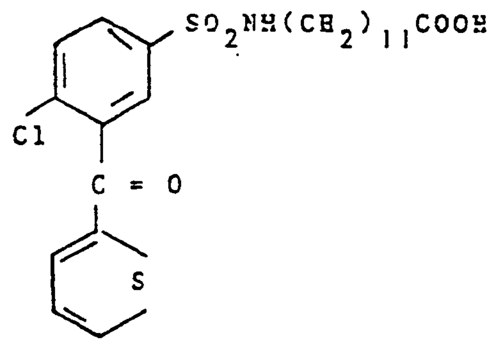

24. Verfahren zur Herstellung der Verbindungen der Formel I nach Anspruch 1, gekennzeichnet durch
. gegebenenfalls Reduktion einer Verbindung der Formel

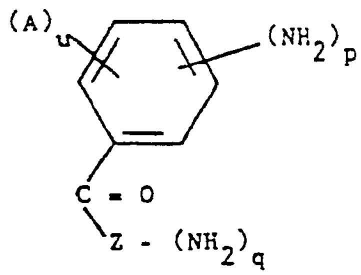

,

worin bedeuten :

– A dasselbe wie $R_1$ mit Ausnahme von $NH_2$, d.h. Cl, F, Br, $NO_2$, $CF_3$, $C_{1-4}$-Alkyl, $C_{1-4}$-Alkoxy, Acetamido oder Benzamido,

– Z

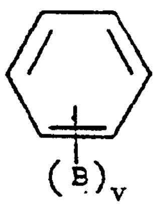

oder

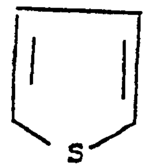

,

– B dasselbe wie $R_2$ mit Ausnahme von $NH_2$, d.h. Cl, $NO_2$, $CF_3$, $C_{1-4}$-Alkyl, $C_{1-4}$-Alkoxy, Acetamido oder Benzamido,

– u eine ganze Zahl von O bis 2,

– v eine ganze Zahl von O bis 2,

– p O oder 1

und

– q O oder 1, wobei p + q $\geqq$ 1 ist,

insbesondere mit Aluminiumchlorid und Lithiumalanat, zur Reduktion von C=O zu $CH_2$ unter Erhalt der Verbindung der Formel

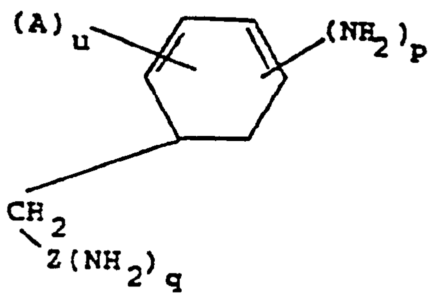

,

in der A, Z, u, p und q die obige Bedeutung besitzen,

. Sandmeyer-Reaktion mit einer der Aminverbindungen der oben angegebenen Formel unter Erhalt eines Sulfohalogenids der Formel

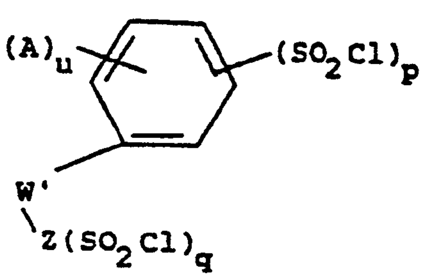

,

in der A, Z, u, p und q die obige Bedeutung besitzen und W′ C=O oder $CH_2$ darstellt, und

. Umsetzung des Sulfohalogenids, insbesondere des Sulfochlorids, der obigen Formel mit einer oder zwei Verbindungen der Formel

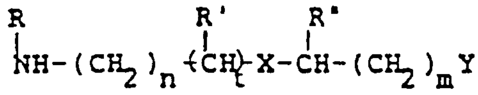

,

in der bedeuten :

– R H, $C_{1-6}$-Alkyl oder Benzyl,

– R′ und R″ H oder $C_{1-4}$-Alkyl,

– X $(CH_2)r$, wobei r O oder 1 bedeutet, -CH=CH,

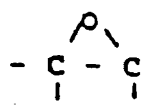

oder

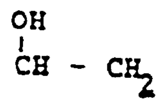

,

– Y $COOR_3$, wobei $R_3$ H oder $C_{1-4}$-Alkyl darstellt,

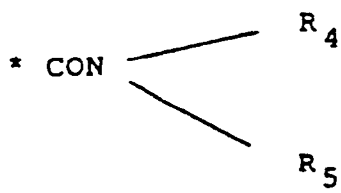

,

worin $R_4$ und $R_5$ H oder $C_{1-4}$-Alkyl darstellen,

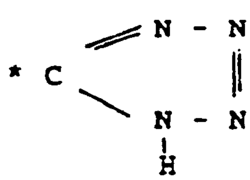

,

* -C ≡ N oder

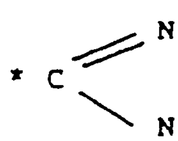

,

wobei

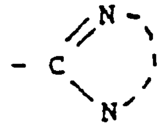

einen nichtaromatischen 5- oder 6-gliedrigen Ring darstellt, in dem die zwei oder drei Atome, mit denen der Ring vervollständigt wird, Kohlenstoffatome und/oder Sauerstoffatome und/oder Stickstoffatome sind, oder

* $NH_2$, $NHR_6$ oder $NR_6R_7$,
  wobei $R_6$ und $R_7$ $C_{1-4}$-Alkyl darstellen oder mit dem Stickstoffatom ein nichtaromatisches cyclisches Amin bilden können,
– mit der Maßgabe, daß X nicht

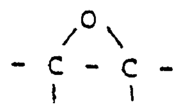

bedeutet, wenn Y $NH_2$ darstellt,
– n eine ganze Zahl von O bis 1O,
– m eine ganze Zahl von O bis 1O
und
– t O oder 1,
– wobei die Gesamtezahl der Atome der Kette

$$-(CH_2)_n-(CH)_t-X-CH-(CH_2)_m\ Y$$

2 bis 2O beträgt,
zur Verbindung der Formel

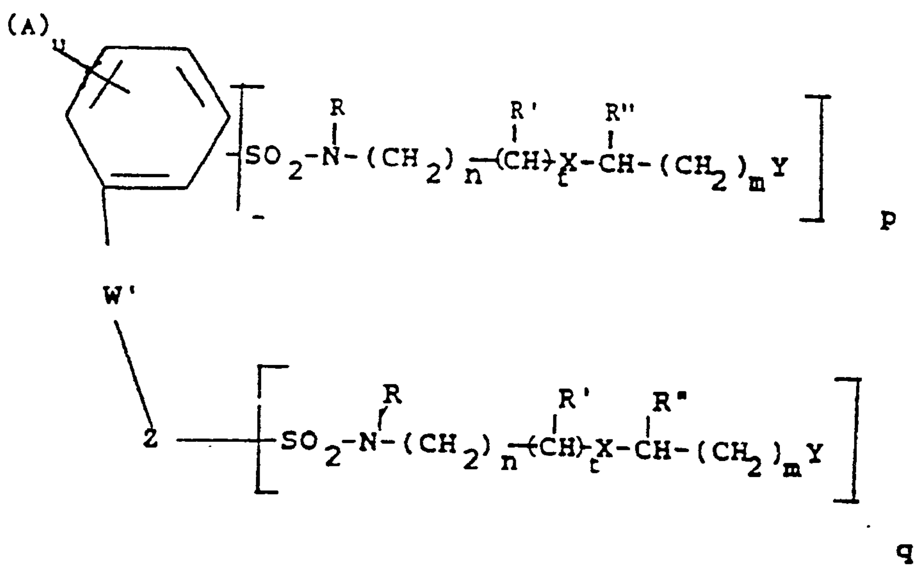

,

in der A, Z, W′, R, R′, R″, X, Y, m, n, t, q und u die obige Bedeutung besitzen,
. und gegebenenfalls, wenn W′ C=O darstellt, Reduktion der obigen Verbindung, insbesondere mit einem Alkaliborhydrid, wie Natriumborhydrid, zur Reduktion von C=O zu CHOH
. sowie gegebenenfalls anschließende Reduktion, wenn A und/oder B $NO_2$ darstellen.
25. Verfahren nach Anspruch 24 zur Herstellung der Verbindungen der Formel II,

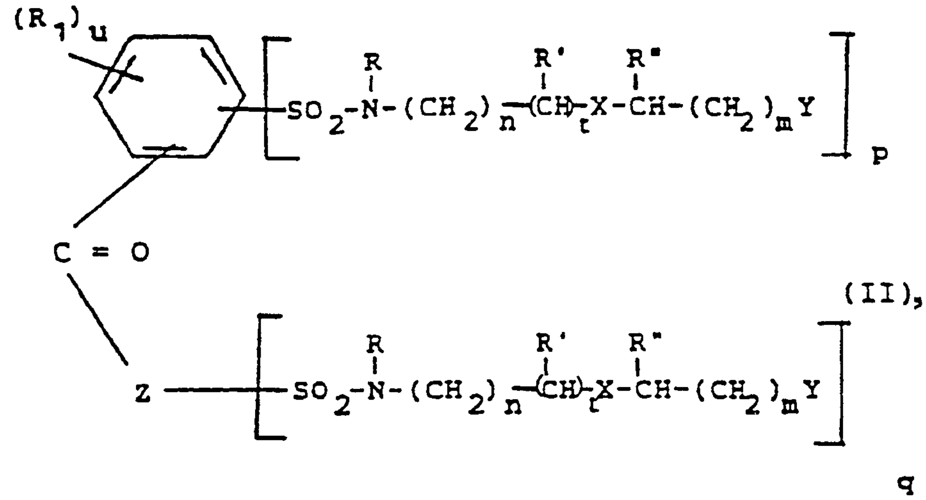

(II),

in der $R_1$, R, R', R'', X, Y, Z, m, n, p, q, u und t dasselbe wie in Anspruch 2 bedeuten, gekennzeichnet durch Umsetzung eines Sulfohalogenids, insbesondere eines Sulfochlorids, der Formel

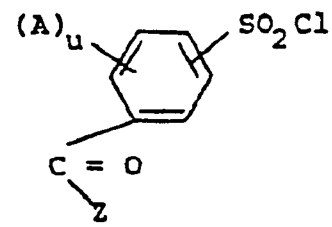

,

in der A, Z und u dasselbe wie in Anspruch 24 bedeuten, oder eines Disulfohalogenids, insbesondere eines Disulfochlorids, der Formel

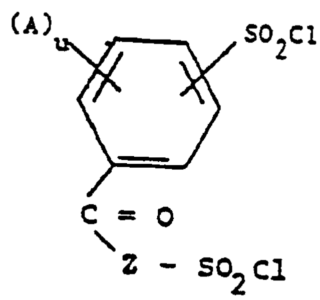

,

in der A, u und Z die obige Bedeutung besitzen, mit einer oder zwei Verbindungen der formel

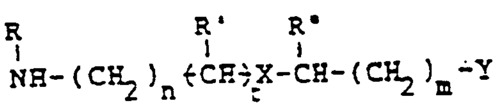

,

in der R, R', R'', n, m, t, X und Y die Bedeutung von Anspruch 24 besitzen, sowie gegebenenfalls anschließende Reduktion, wenn A und/oder B $NO_2$ bedeuten.

26. Verfahren nach Anspruch 25, gekennzeichnet durch Umsetzung eines Sulfochlorids der Formel

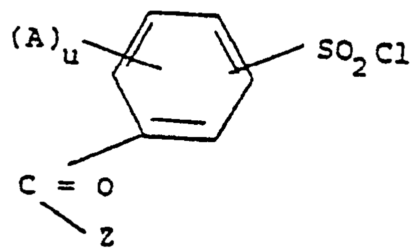

,

in der A, u und Z dasselbe wie in Anspruch 25 bedeuten, oder eines Disulfochlorids der Formel

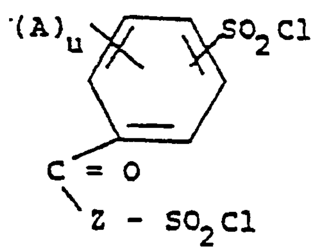

,

in der A, u und Z dasselbe wie Anspruch 25 bedeuten, mit einer oder zwei Aminosäuren der Formel

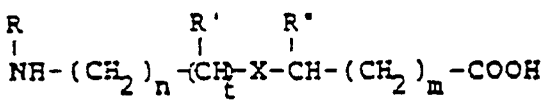

,

in der R, R′, R″, n, m, t und X dasselbe wie in Anspruch 25 bedeuten.

27. Verfahren nach Anspruch 25,
gekennzeichnet durch
– Veresterung einer verbindung wie gemäß Anspruch 26 erhalten, in deren Formel Y COOH bedeutet, zu einer Verbindung der Formel II, in der Y $COOR_3$ darstellt, wobei $R_3$ $C_{1-4}$-Alkyl bedeutet,
oder
– Umwandlung einer Verbindung wie gemäß Anspruch 26 erhalten in eine Verbindung der Formel II, in der
Y

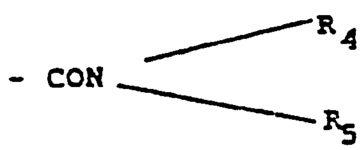

,

worin $R_4$, $R_5$ H oder $C_{1-4}$-Alkyl darstellen,

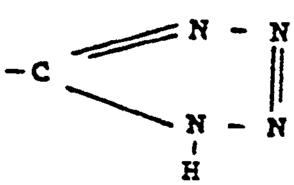

, - C≡N oder

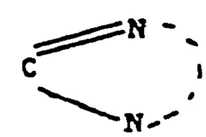

bedeutet.

28. Verfahren nach Anspruch 25,

gekennzeichnet durch Umsetzung eines Sulfochlorids der Formel

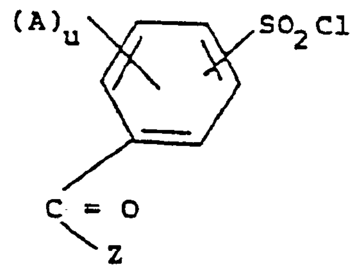

,

wobei bedeuten :

- A dasselbe wie für $R_1$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido,
- Z

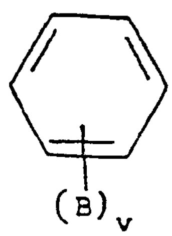

oder

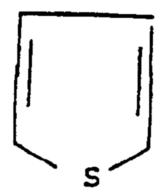

,

- B dasselbe wie für $R_2$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido,
- u eine ganze Zahl von O bis 2

und

- v eine ganze Zahl von O bis 2,

oder eines Disulfohalogenids, insbesondere eines Disulfochlorids der Formel

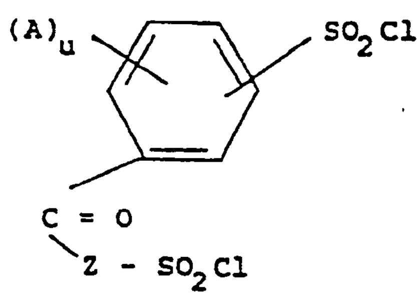

,

in der A, u und Z die obige Bedeutung besitzen, mit einem Aminoalkylamin der Formel

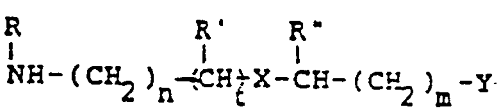

,

in der bedeuten :

- R H, $C_{1-6}$-Alkyl oder Benzyl,
- R' und R" H oder $C_{1-4}$-Alkyl,
- n eine ganze Zahl von O bis 1O,
- m eine ganze Zahl von O bis 1O,
- t O oder 1,
- Y $NH_2$, $NHR_6$ oder $NR_6R_7$, wobei $R_6$ und $R_7$ $C_{1-4}$-Alkyl darstellen oder mit dem Stickstoff ein nicht-aromatisches cyclisches Amin bilden,
und
– X dasselbe wie in Formel I.

29. Verfahren nach Anspruch 25,
dadurch gekennzeichnet, daß
die eingesetzte Aminosäure oder das eingesetzte Dialkylaminoalkylamin unter

$$- NH_2 - (CH_2)_n - COOH,$$

wobein n 1 bis 11 bedeutet,
beziehungsweise

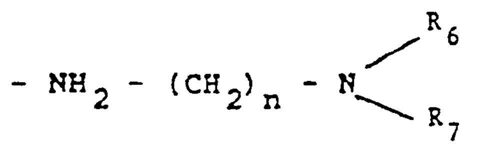

,

worin n 2 oder 3 bedeutet und $R_6$ und $R_7$ $C_{1-4}$-Alkyl oder zusammen mit dem Stickstoffatom einen Morpholinring darstellen,
ausgewählt wird
und
das eingesetzte Sulfochlorid ausgewählt wird unter
– 2-Benzoyl-4-nitrobenzolsulfonylchlorid,
– 2-Benzoyl-4-chlorbenzolsulfonylchlorid,
– 4-Chlor-2-(2-chlorbenzoyl)-benzolsulfonylchlorid,
– 2-Benzoylbenzolsulfonylchlorid,
– 2-(4-Chlorbenzoyl)-benzolsulfonylchlorid,
– 4-Benzoylbenzolsulfonylchlorid,
– 3-Benzoylbenzolsulfonylchlorid,
– 3-(2,4-Dichlorbenzoyl)-4-chlorbenzolsulfonylchlorid
– 3-Benzoyl-4-chlorbenzolsulfonylchlorid,
– 3-(4-Chlorbenzoyl)-4-chlorbenzolsulfonylchlorid,
– 4-Benzoyl-3-chlorbenzolsulfonylchlorid und
– 4-Chlor-3-(2-thenoyl)-benzolsulfonylchlorid.

30. Verfahren nach Anspruch 24 zur Herstellung der Verbindungen der Formel III,

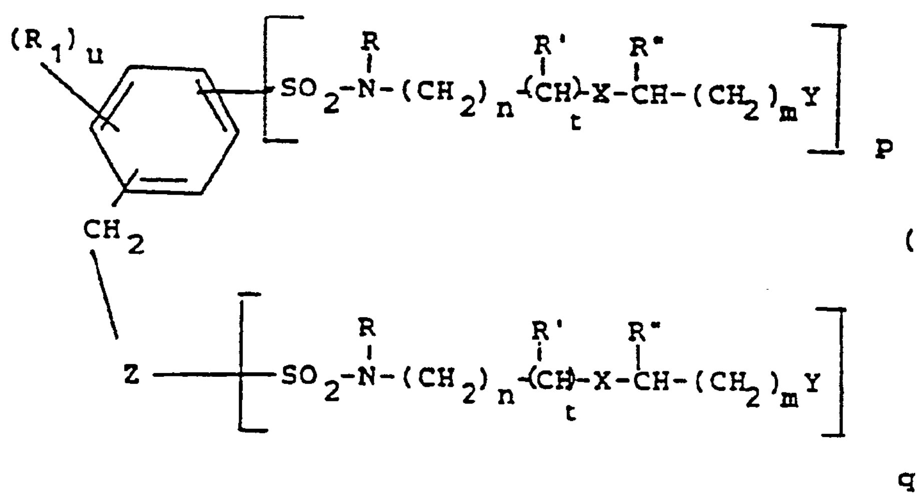

(III),

in der $R_1$, R, R′, R″, X, Y, Z, m, n, t, p, q und u die Bedeutung von Anspruch 3 besitzen,
gekennzeichnet durch

Reduktion einer Verbindung der Formel

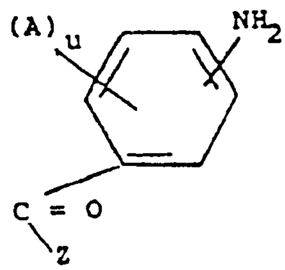

oder der Formel

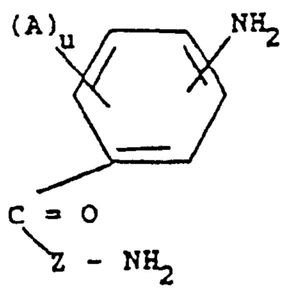

,

worin A dasselbe wie in Anspruch 24 bedeutet,

– zur Reduktion von C=O zur $CH_2$ unter Erhalt der Verbindung der Formel

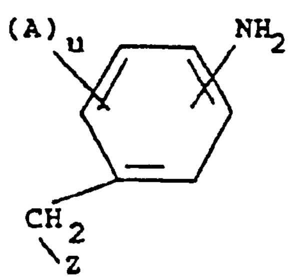

oder

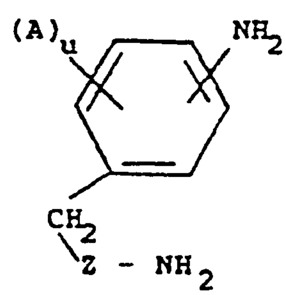

,

– Sandmeyer-Reaktion mit einer dieser Verbindungen unter Erhalt der Verbindungen der Formel

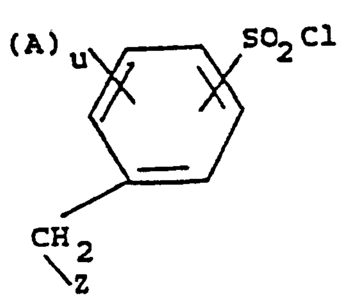

oder

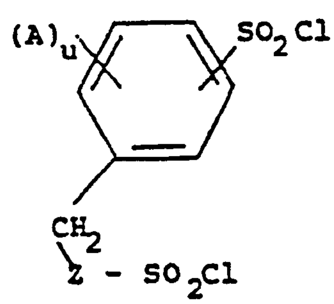

und

– kondensation des Sulfohalogenids oder Disulfohalogenids der obigen Formel mit einer Verbindung der Formel

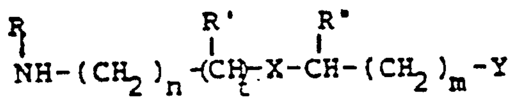
,

in der R, R', R'', X, Y, n, m und t die Bedeutung von Anspruch 24 besitzen, sowie gegebenenfalls

– anschließende Reduktion, wenn A und/oder B $NO_2$ bedeuten.

31. Verfahren nach Anspruch 30, dadurch gekennzeichnet, daß die eingesetzte Aminosäure oder das eingesetzte Dialkylaminoalkylamin unter

$$-NH_2-(CH_2)_n-COOH,$$

wobein n 1 bis 11 bedeutet, beziehungsweise

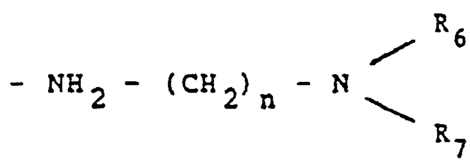
,

wobein 2 oder 3 bedeutet und $R_6$ und $R_7$ $C_{1-4}$-Alkyl oder zusammen mit dem Stickstoffatom einen Morpholinring darstellen, ausgewählt werden und die Verbindung der Formel

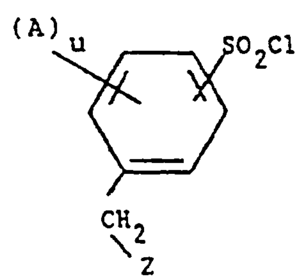

ausgewählt wird unter

– 3-(2,4-Dichlorbenzyl)-4-chlorbenzolsulfonylchlorid,
– 3-(4-Chlorbenzyl)-4-chlorbenzolsulfonylchlorid,
– 2-Benzyl-4-chlorbenzolsulfonylchlorid,
– 2-(2,4-Dichlorbenzyl)-4-chlorbenzolsulfonylchlorid,
– 2-(4-Chlorbenzyl)-4-chlorbenzolsulfonylchlorid,
– 4-Benzyl-3-chlorbenzolsulfonylchlorid,
– 4-(2,4-Dichlorbenzyl)-3-chlorbenzolsulfonylchlorid,
– 4-(4-Chlorbenzyl)-3-chlorbenzolsulfonylchlorid und
– 3-Benzyl-4-chlorbenzolsulfonylchlorid.

32. Verfahren nach Anspruch 24 zur Herstellung der Verbindungen der Formel IV,

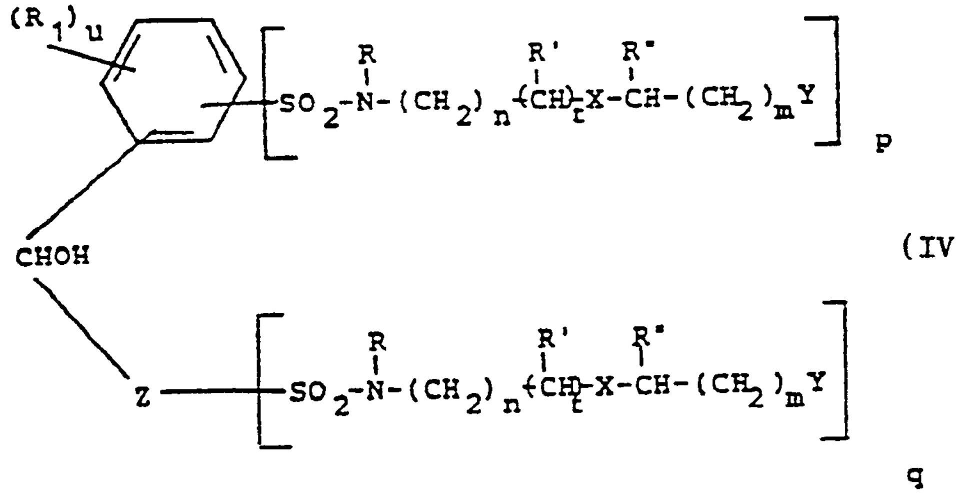

(IV),

in der $R_1$, R, R', R", X, Y, m, n, t, p, q und u die Bedeutung von Anspruch 4 besitzen, gekennzeichnet durch Reduktion einer Verbinduing der Formel

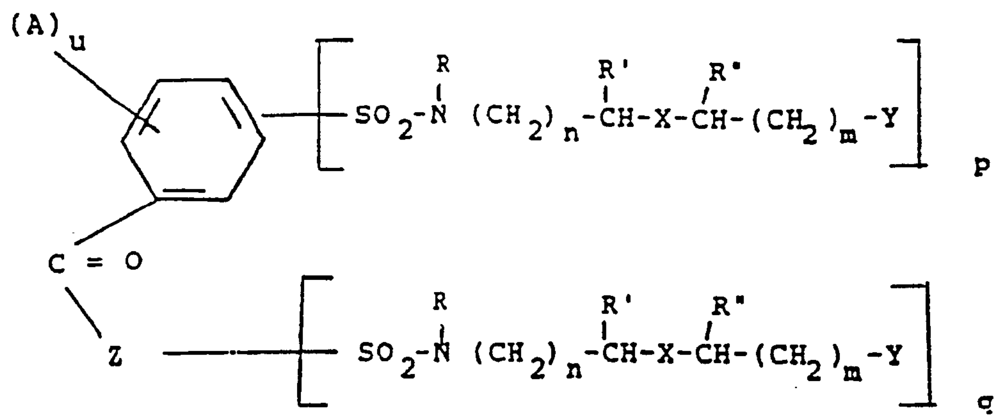

,

in der A, X, Y, Z, R, R', R", m, n, p, q, t und u dasselbe wie in Anspruch 24 bedeuten, und wie gemäß Anspruch 25 erhalten, vor einer gegebenenfalls vorgenommenen Reduktion von A und/oder B, wenn diese $NO_2$ darstellen, zur Umwandlung von C=O in CHOH mit einem Alkaliborhydrid.

33. Verfahren nach Anspruch 32, dadurch gekennzeichnet, daß die eingesetzte Aminosäure oder das eingesetzte Dialkylaminoalkylamin unter

$$- NH_2 -(CH_2)_n- COOH ,$$

worin n 1 bis 11 bedeutet, beziehungsweise

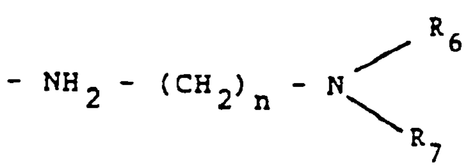

,

worin n 2 oder 3 bedeutet und $R_6$ und $R_7$ $C_{1-4}$-Alkyl oder zusammen mit dem Stickstoffatom einen Morpholinring darstellen, ausgewählt werden
und das eingesetzte Sulfochlorid ausgewählt wird unter
- 2-Benzoyl-4-nitrobenzolsulfonylchlorid,
- 2-Benzoyl-4-chlorbenzolsulfonylchlorid,
- 4-Chlor-2-(2-chlorbenzoyl)-benzolsulfonylchlorid,
- 2-Benzoylbenzolsulfonylchlorid,
- 2-(4-Chlorbenzoyl)-benzolsulfonylchlorid,
- 4-Benzoylbenzolsulfonylchlorid,
- 3-Benzoylbenzolsulfonylchlorid,
- 3-(2,4-Dichlorbenzoyl)-4-chlorbenzolsulfonylchlorid,
- 3-Benzoyl-4-chlorbenzolsulfonylchlorid,
- 3-(4-Chlorbenzoyl)-4-chlorbenzolsulfonylchlorid,
- 4-Benzoyl-3-chlorbenzolsulfonylchlorid und
- 4-Chlor-3-(2-thenoyl)-benzolsulfonylchlorid.

**Patentanspruche für folgenden Vertragsstaat : ES**

1. Verfahren zur Herstellung von Verbindungen der Formel I,

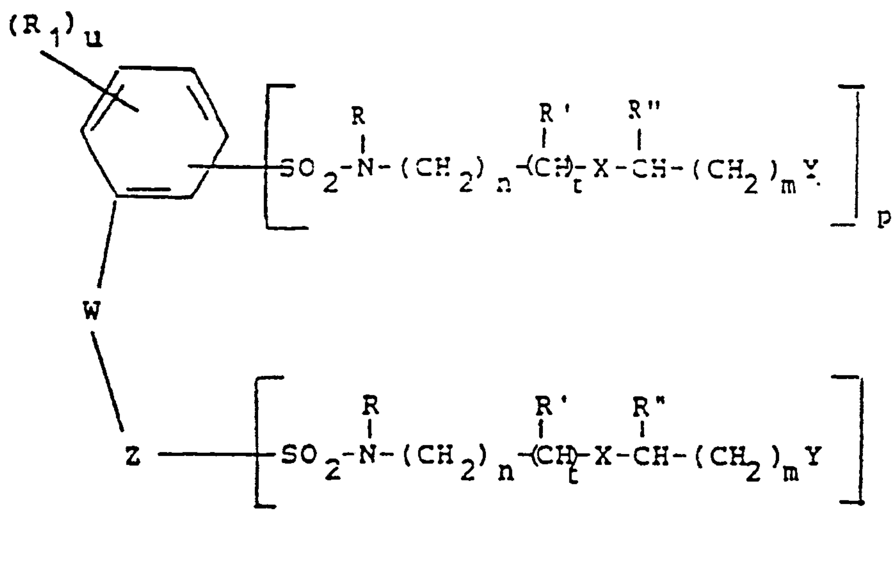

(I),

wobei bedeuten :
- W C=O, $CH_2$ oder CHOH,
- Z

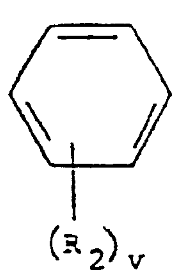

oder

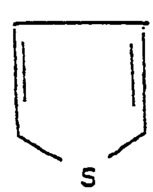

,

- $R_1$ Cl, F, Br, $NO_2$, $NH_2$, $CF_3$, $C_{1-4}$-Alkyl, insbesondere Methyl oder Ethyl, $C_{1-4}$-Alkoxy, Acetamido oder Benzamido,
- $R_2$ Cl, F, Br, $NO_2$, $NH_2$, $CF_3$, $C_{1-4}$-Alkyl, $C_{1-4}$-Alkoxy, Acetamido oder Benzamido,

– R H, $C_{1-6}$-Alkyl oder Benzyl,
– R' und R'' H oder $C_{1-4}$-Alkyl,
– X $(CH_2)_r$, wobei r O oder 1 bedeutet, -CH=CH,

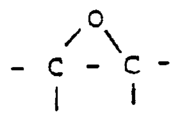 oder 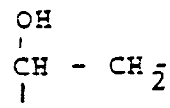 ,

– Y $COOR_3$, wobei $R_3$ H oder $C_{1-4}$-Alkyl darstellt,

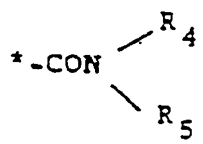 ,

worin $R_4$ und $R_5$, H oder $C_{1-4}$-Alkyl darstellen,

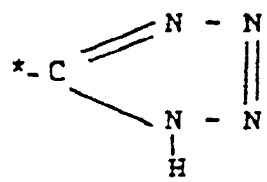 ,

* -C≡N oder

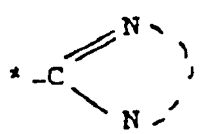 ,

wobei

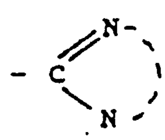

einen nichtaromatischen 5- oder 6-gliedrigen Ring darstellt, in dem die zwei oder drei Atome, mit denen der Ring vervollständigt wird, Kohlenstoffatome und/oder Sauerstoffatome und/oder Stickstoffatome sind, oder

* $NH_2$, $NHR_6$ oder $NR_6R_7$,
wobei $R_6$ und $R_7$ $C_{1-4}$-Alkyl darstellen oder mit dem Stickstoffatom ein nichtaromatisches cyclisches Amin bilden können,

– mit der Maßgabe, daß X nicht

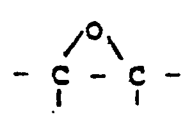

bedeutet, wenn Y $NH_2$ darstellt,

- u eine ganze Zahl von O bis 2,
- v eine ganze Zahl von O bis 2,
- p O oder 1,
- q O oder 1, wobei p + q ≧ 1 ist,
- n eine ganze Zahl von O bis 1O,
- m eine ganze Zahl von O bis 1O

und

- t O oder 1,
- wobei die Gesamtzahl der Kohlenstoffatome der Kette

$$-(CH_2)_n-(\overset{|}{C}H)_t-X-\overset{|}{C}H-(CH_2)_m-Y$$

2 bis 20 beträgt,
gekennzeichnet durch

. gegebenenfalls Reduktion einer Verbindung der Formel

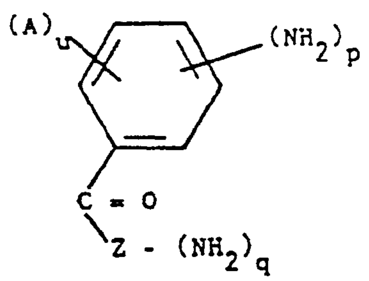

,

wobei bedeuten :

- A dasselbe wie $R_1$ mit Ausnahme von $NH_2$, d.h. Cl, F, Br, $NO_2$, $CF_3$, $C_{1-4}$-Alkyl, $C_{1-4}$-Alkoxy, Acetamido oder Benzamido und vorzugsweise Cl oder $NO_2$, Z

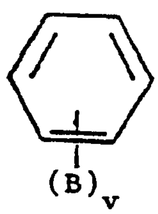

oder

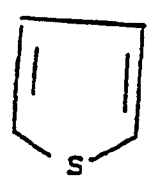

,

- B dasselbe wie $R_2$ mit Ausnahme von $NH_2$, d.h. Cl, $NO_2$, $CF_3$, $C_{1-4}$-Alkyl, $C_{1-4}$-Alkoxy, Acetamido oder Benzamido und vorzugsweise Cl oder $NO_2$

und

- u, v, p und q dasselbe wie oben, insbesondere mit Aluminiumchlorid und Lithiumalanat, zur Reduzierung von C=O zu $CH_2$

unter Erhalt einer Verbindung der formel

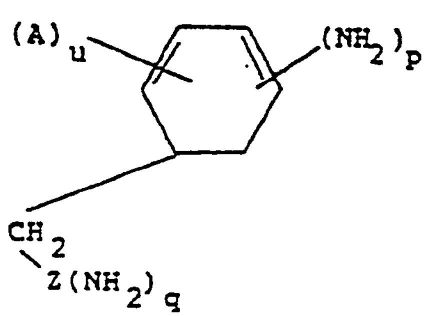

,

in der A, Z, u, p und q die obige Bedeutung besitzen,

. Sandmeyer-Reaktion mit einer der Aminverbindungen der oben angegebenen Formel unter Erhalt eines Sulfohalogenids der Formel

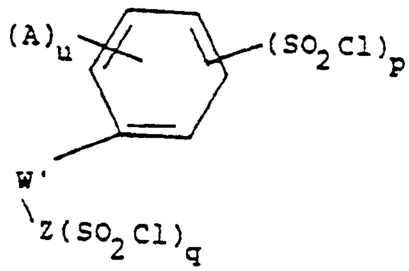

,

in der A, Z, u, p und q die obige Bedeutung besitzen und W' C=O oder $CH_2$ darstellt, und

. Umsetzung des Sulfohalogenids der obigen Formel mit einer Verbindung der Formel

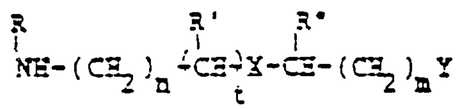

in der R, R', R", m, n, t, X und Y die obige Bedeutung besitzen, zur Verbindung der Formel

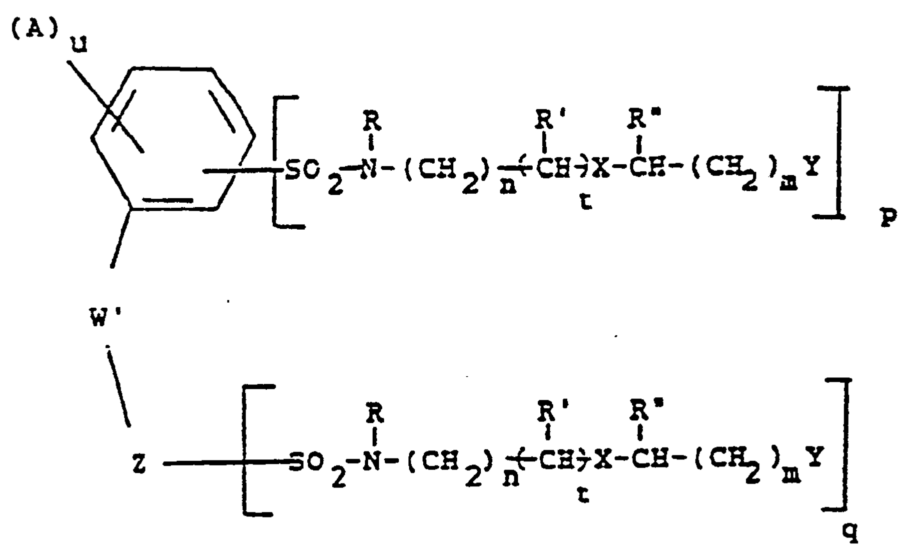

,

in der $R_1$, R', R", X, Y, A, Z, W', m, n, t, p und q die obige Bedeutung besitzen,

. und gegebenenfalls, wenn W' C=O darstellt, Reduktion der obigen Verbindung, insbesondere mit einem Alkaliborhydrid, zur Reduktion von C=O zu CHOH,

. sowie gegebenenfalls anschließende Reduktion, wenn A und/oder B $NO_2$ bedeuten.

2. Verfahren nach Anspruch 1 zur Herstellung der Verbindungen der Formel II

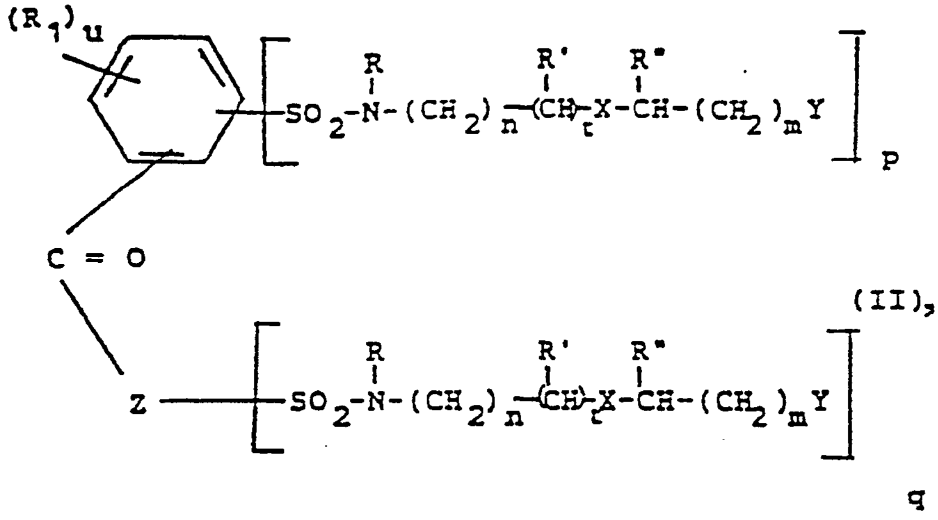

(II),

in der $R_1$, R, R', R", X, Y, Z, m, n, p, q, u und t dasselbe wie in Anspruch 1 bedeuten, gekennzeichnet durch
Umsetzung eines Sulfohalogenids, insbesondere eines Sulfochlorids, der Formel

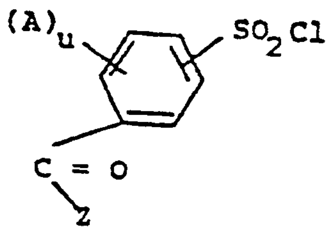

,

in der A, Z und u dasselbe wie in Anspruch 1 bedeuten,
oder eines Disulfohalogenids der Formel

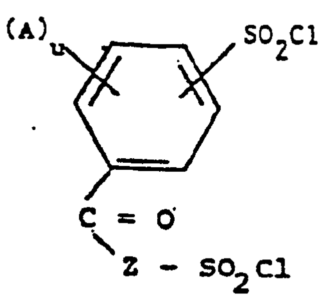

,

in der A, u und Z die obige Bedeutung besitzen, mit einer Verbindung der Formel

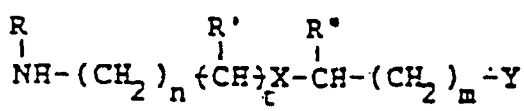

,

in der R, R', R", n, m, t, X und Y die Bedeutung von Anspruch 1 besitzen,
sowie gegebenenfalls anschließende Reduktion, wenn A Lind/oder B $NO_2$ bedeuten.
3. Verfahren nach Anspruch 2 zur Herstellung der Verbindungen der Formel V,

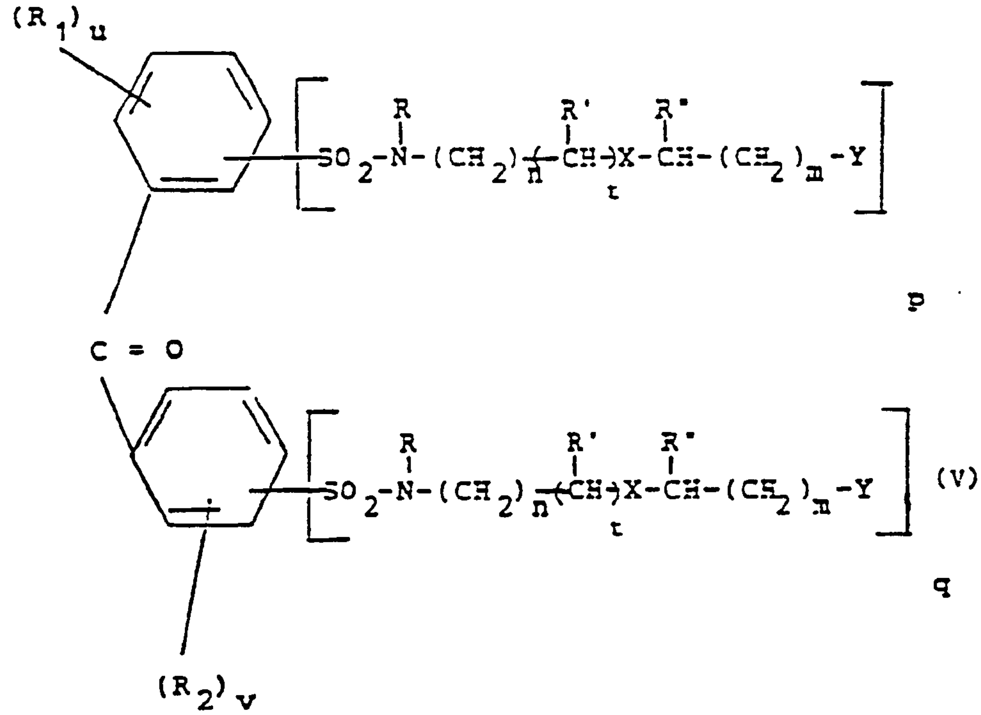

(V),

in der $R_1$, R, R', R", X, Y, Z, m, n, t, p, q und u dasselbe wie in Anspruch 1 bedeuten, gekennzeichnet durch

Umsetzung eines Sulfohalogenids der Formel

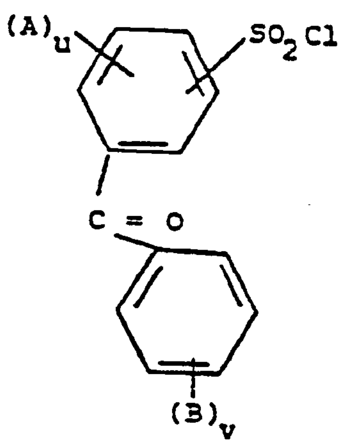

,

in der A, B, u und v die Bedeutung von Anspruch 2 besitzen, oder eines Disulfohalogenids der Formel

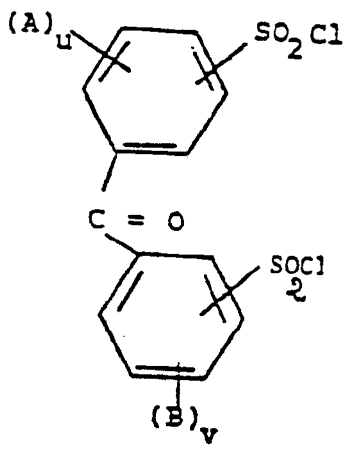

,

in der A, B, u und v die obige Bedeutung besitzen, mit einer oder zwei Verbindungen der Formel

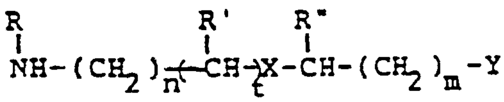

,

in der R, R', R", m, n, t, X und Y die Bedeutung von Anspruch 2 besitzen, sowie gegebenenfalls anschließende Reduktion, wenn A und/oder B $NO_2$ bedeuten.

4. Verfahren nach Anspruch 3 zur Herstellung der Verbindungen der Formel V, in der Y $COOR_3$ bedeutet, worin $R_3$ Wasserstoff oder $C_{1-4}$-Alkyl darstellt.

5. Verfahren nach Anspruch 3 zur Herstellung der Verbindungen der Formel V, in der Y $NH_2$, $NHR_6$ oder $NR_6R_7$ bedeutet, wobei $R_6$ und $R_7$ die Bedeutung von Anspruch 1 besitzen.

6. Verfahren nach Anspruch 3 zur Herstellung der Verbindungen der Formel V, in der p oder q O bedeuten.

7. Verfahren nach Anspruch 3 zur Herstellung der Verbindungen der Formel V mit folgenden Definitionen :

- p oder q = 1,
- u + v $\geqq$ 1,
- die Gesamtzahl der kohlenstoffatome der kette

$$(CH_2)_n-(CH)_t-X-CH-(CH_2)_m-Y \text{ ist } \geq 5.$$

8. Verfahren nach Anspruch 3 zur Herstellung der Verbindungen der Formel V mit folgenden Definitionen :

- P = 1,
- q = 1,
- u + v $\geqq$ 1,
- die Gesamtzahl der kohlenstoffatome der kette

$$(CH_2)_n-(CH)_t-X-CH-(CH_2)_m-Y \text{ ist } \geq 5.$$

9. Verfahren nach Anspruch 2 zur Herstellung der Verbindungen der Formel II, gekennzeichnet durch Umsetzung eines Sulfochlorids der Formel

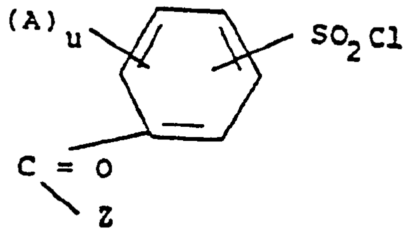

,

in der A, u und Z die Bedeutung von Anspruch 2 besitzen,
oder eines Disulfochlorids der Formel

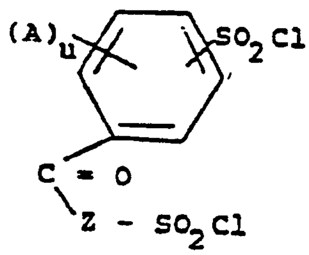

,

in der A, u und Z die Bedeutung von Anspruch 2 besitzen,
mit einer Aminosäure der Formel

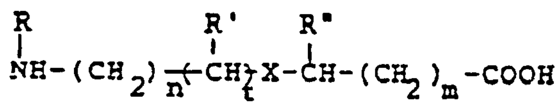

,

in der R, R′, R″, n, m, t und X die Bedeutung von Anspruch 2 besitzen.

10. Verfahren nach Anspruch 2 zur Herstellung der Verbindungen der Formel II,
gekennzeichnet durch

– Veresterung einer Verbindung wie gemäß Anspruch 9 erhalten, in deren Formel Y COOH bedeutet, zu einer Verbindung der Formel II, in der Y $COOR_3$ darstellt, wobei $R_3$ $C_{1-4}$-Alkyl bedeutet,
oder

– Umwandlung einer Verbindung wie gemäß Anspruch 9 erhalten in eine Verbindung der Formel II, in der Y bedeutet :

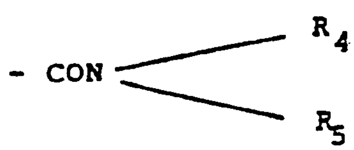

,

worin $R_4$, $R_5$ H oder $C_{1-4}$-Alkyl darstellen,

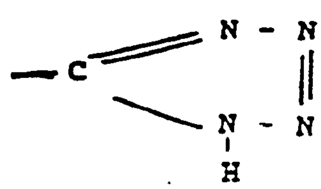

,

- C≡N oder

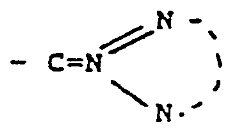

.

11. Verfahren nach Anspruch 2 zur Herstellung der Verbindungen der Formel II, gekennzeichnet durch Umsetzung eines Sulfochlorids der Formel

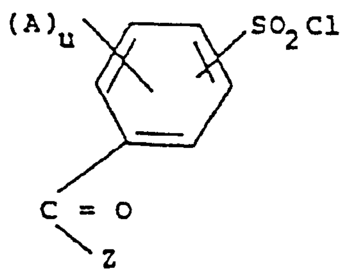

,

wobei bedeuten :

– A dasselbe wie für $R_1$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido,

– Z

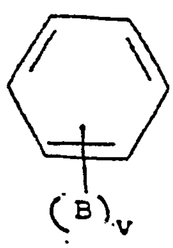

oder

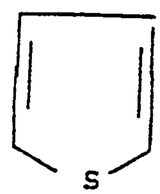

,

– B dasselbe wie für $R_2$ angegeben mit Ausnahme von $NH_2$, d.h. Cl, F, Br, $NO_2$, $CF_3$, $C_{1-4}$-Alkyl, $C_{1-4}$-Alkoxy, Acetamido oder Benzamido und vorzugsweise Cl oder $NO_2$,

– u eine ganze Zahl von O bis 2

und

– v eine ganze Zahl von O bis 2,

oder eines Disulfohalogenids der Formel

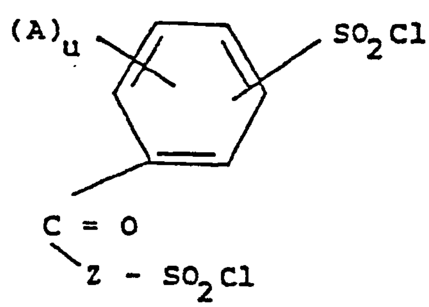

,

in der A, u und Z die obige Bedeutung besitzen, mit einem Aminoalkylamin der Formel

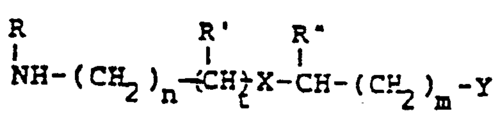

,

in der bedeuten :

- R H, $C_{1-6}$-Alkyl oder Benzyl,
- R' und R" H oder $C_{1-4}$-Alkyl,
- n eine ganze Zahl von O bis 1O,
- m eine ganze Zahl von O bis 1O,
- t O oder 1

und

- Y $NH_2$, $NHR_6$ oder $NR_6R_7$, wobei $R_6$ und $R_7$ $C_{1-4}$-Alkyl darstellen oder mit dem Stickstoff ein nichtaromatisches cyclisches Amin bilden.

12. Verfahren nach Anspruch 2 zur Herstellung der Verbindungen der Formel II, dadurch gekennzeichnet, daß die eingesetzte Aminosäure oder das eingesetzte Dialkylaminoalkylamin unter

$$- NH_2 - (CH_2)_n - COOH,$$

wobein 1 bis 11 bedeutet,

beziehungsweise

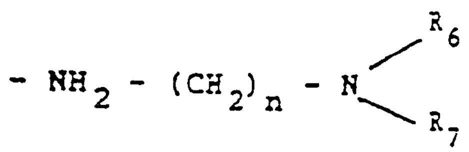,

worin n 2 oder 3 bedeutet und $R_6$ und $R_7$ $C_{1-4}$-Alkyl oder zusammen mit dem Stickstoffatom einen Morpholinring darstellen,

ausgewählt wird

und

das eingesetzte Sulfochlorid ausgewählt wird unter

- 2-Benzoyl-4-nitrobenzolsulfonylchlorid,
- 2-Benzoyl-4-chlorbenzolsulfonylchlorid,
- 4-Chlor-2-(2-chlorbenzoyl)-benzolsulfonylchlorid,
- 2-Benzoylbenzolsulfonylchlorid,
- 2-(4-Chlorbenzoyl)-benzolsulfonylchlorid,
- 4-Benzoylbenzolsulfonylchlorid,
- 3-Benzoylbenzolsulfonylchlorid,
- 3-(2,4-Dichlorbenzoyl)-4-chlorbenzolsulfonylchlorid
- 3-Benzoyl-4-chlorbenzolsulfonylchlorid,
- 3-(4-Chlorbenzoyl)-4-chlorbenzolsulfonylchlorid,
- 4-Benzoyl-3-chlorbenzolsulfonylchlorid und
- 4-Chlor-3-(2-thenoyl)-benzolsulfonylchlorid.

13. Verfahren nach Anspruch 1 zur Herstellung der Verbindungen der Formel III,

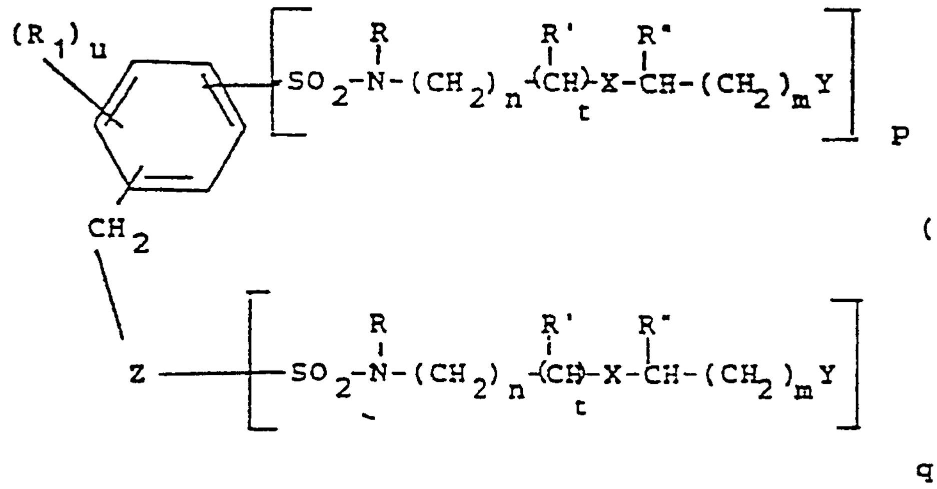

(III),

in der $R_1$, R, R', R'', X, Y, Z, m, n, t, p, q und u die Bedeutung von Anspruch 3 besitzen, gekennzeichnet durch
Reduktion einer Verbindung der Formel

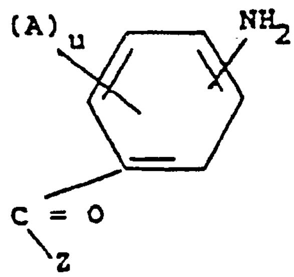

oder der Formel

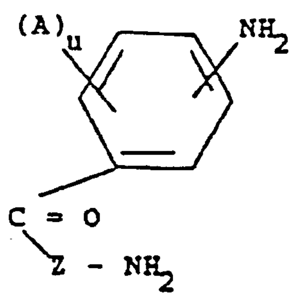

,

insbesondere mit Aluminiumchlorid oder Lithiumalanat, zur Reduktion von C=O zu $CH_2$ unter Erhalt der Verbindung der Formel

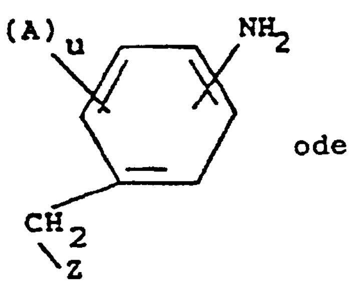

oder

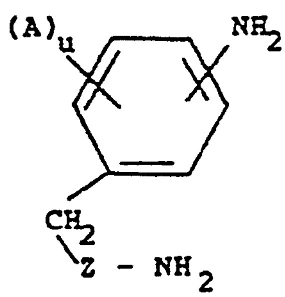

,

– Sandmeyer-Reaktion mit einer dieser Verbindungen unter Erhalt der Verbindungen der Formel

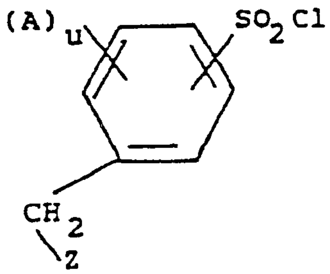

oder

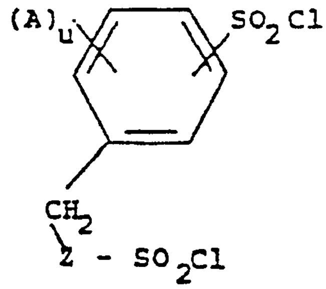

und

– kondensation des Sulfohalogenids oder Disulfohalogenids der obigen Formel mit einer Verbindung der Formel

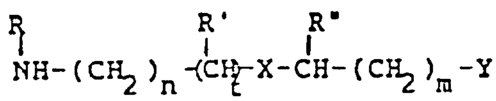

,

in der R, R′, R″, X, Y, n, m und t die Bedeutung von Anspruch 1 besitzen,
sowie gegebenenfalls
anschließende Reduktion, wenn A und/oder B $NO_2$ bedeuten.

14. Verfahren nach Anspruch 13 zur Herstellung der Verbindungen der Formel III, dadurch gekennzeichnet, daß die eingesetzte Aminosäure oder das eingesetzte Dialkylaminoalkylamin unter

$$- NH_2 -(CH_2)_n - COOH ,$$

wobein 1 bis 11 bedeutet,
beziehungsweise

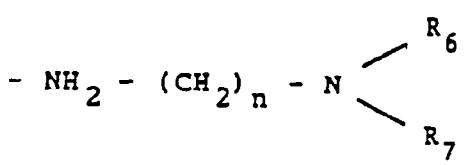

,

wobein 2 oder 3 bedeutet und $R_6$ und $R_7$ $C_{1-4}$-Alkyl oder zusammen mit dem Stickstoffatom einen Morpholinring darstellen,
ausgewählt werden
und die Verbindung der Formel

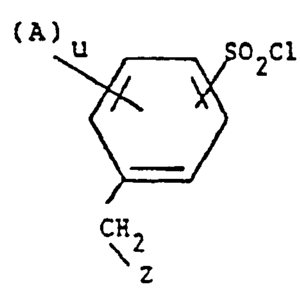

ausgewählt wird unter

– 3-(2,4-Dichlorbenzyl)-4-chlorbenzolsulfonylchlorid,
– 3-(4-Chlorbenzyl)-4-chlorbenzolsulfonylchlorid,
– 2-Benzyl-4-chlorbenzolsulfonylchlorid,
– 2-(2,4-Dichlorbenzyl)-4-chlorbenzolsulfonylchlorid,
– 2-(4-Chlorbenzyl)-4-chlorbenzolsulfonylchlorid,
– 4-Benzyl-3-chlorbenzolsulfonylchlorid,
– 4-(2,4-Dichlorbenzyl)-3-chlorbenzolsulfonylchlorid,
– 4-(4-Chlorbenzyl)-3-chlorbenzolsulfonylchlorid und
– 3-Benzyl-4-chlorbenzolsulfonylchlorid.

15. Verfahren nach Anspruch 1 zur Herstellung der Verbindungen der Formel IV,

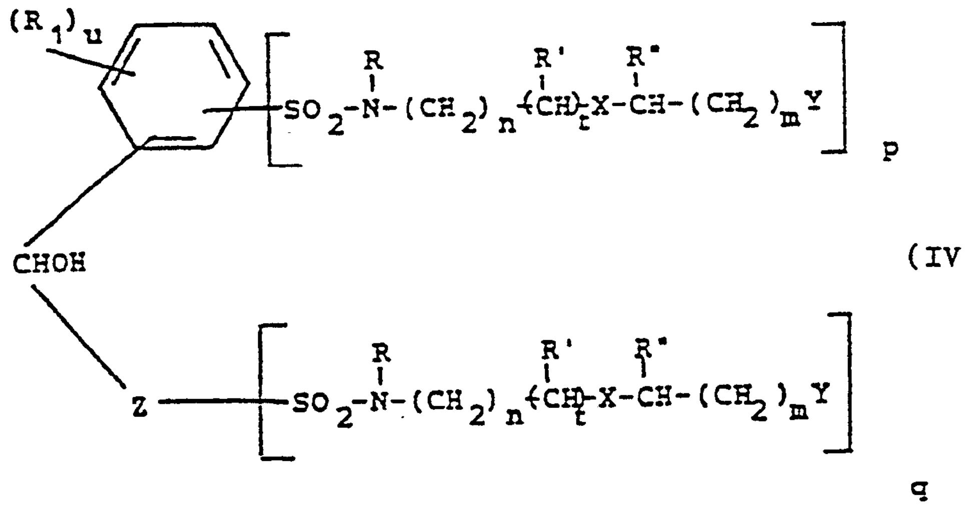

(IV),

in der $R_1$, R, R', R", X, Y, m, n, t, p, q und u die Bedeutung von Anspruch 1 besitzen, gekennzeichnet durch Reduktion einer Verbindung der Formel

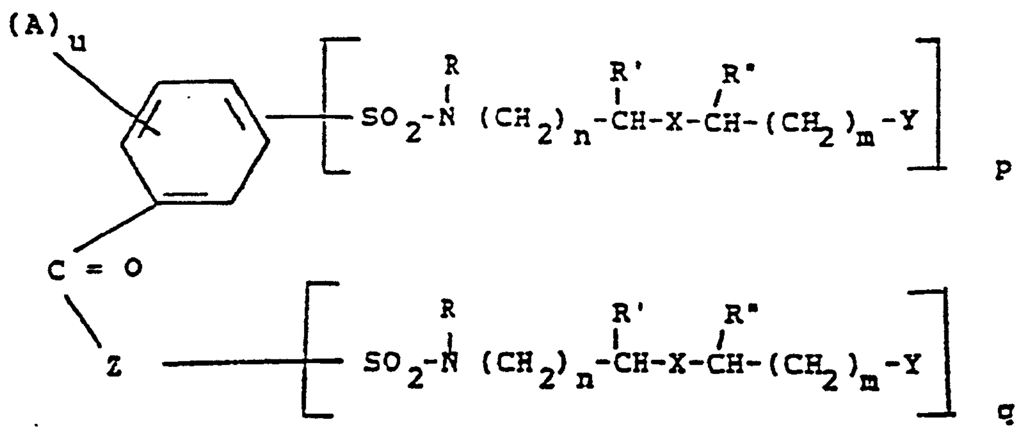

,

in der A, X, Y, Z, R, R', R", m, n, p, q, t und u dasselbe wie in Anspruch 1 bedeuten, und wie gemäß Anspruch 2 erhalten, vor einer gegebenenfalls vorgenommenen Reduktion von A und/oder B, wenn diese $NO_2$ darstellen, zur Umwandlung von C=O in CHOH mit einem Alkaliborhydrid.

16. Verfahren nach Anspruch 15 zur Herstellung von Verbindungen der Formel II, dadurch gekennzeichnet, daß die eingesetzte Aminosäure oder das eingesetzte Dialkylaminoalkylamin unter

$$-NH_2-(CH_2)_n-COOH ,$$

worin n 1 his 11 bedeutet, beziehungsweise

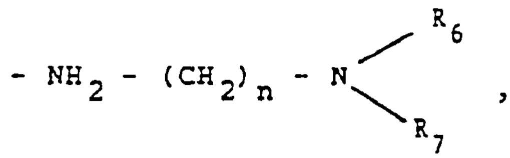

worin n 2 oder 3 bedeutet und $R_6$ und $R_7$ $C_{1-4}$-Alkyl oder zusammen mit dem Stickstoffatom einen Morpholinring darstellen, ausgewählt werden und das eingesetzte Sulfochlorid ausgewählt wird unter

- 2-Benzoyl-4-nitrobenzolsulfonylchlorid,
- 2-Benzoyl-4-chlorbenzolsulfonylchlorid,
- 4-Chlor-2-(2-chlorbenzoyl)-benzolsulfonylchlorid,
- 2-Benzoylbenzolsulfonylchlorid,
- 2-(4-Chlorbenzoyl)-benzolsulfonylchlorid,
- 4-Benzoylbenzolsulfonylchlorid,
- 3-Benzoylbenzolsulfonylchlorid,
- 3-(2,4-Dichlorbenzoyl)-4-chlorbenzolsulfonylchlorid,
- 3-Benzoyl-4-chlorbenzolsulfonylchlorid,
- 3-(4-Chlorbenzoyl)-4-chlorbenzolsulfonylchlorid,
- 4-Benzoyl-3-chlorbenzolsulfonylchlorid und
- 4-Chlor-3-(2-thenoyl)-benzolsulfonylchlorid.

17. Verfahren nach Anspruch 1 zur Herstellung von Verbindungen der Formel VI,

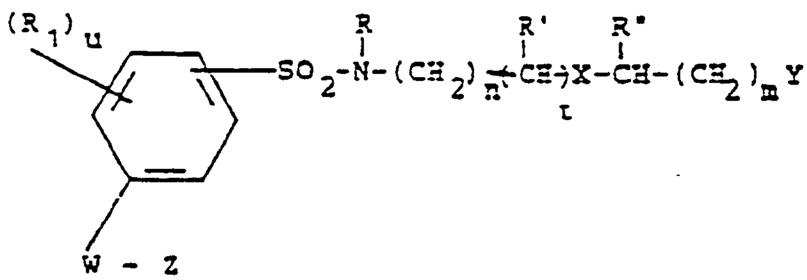

in der $R_1$, R, R', R'', X, Y, Z, W, m, n, t und u die Bedeutung von Anspruch 1 besitzen,
. gegebenenfalls Reduktion einer Verbindung der formel

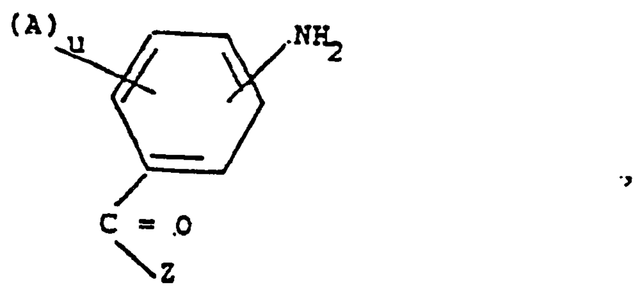

in der A, Z und u die Bedeutung von Anspruch 1 besitzen,
insbesondere mit Aluminiumchlorid und Lithiumalanat, zur Reduzierung von C=O zu $CH_2$ unter Erhalt der Verbindung der Formel

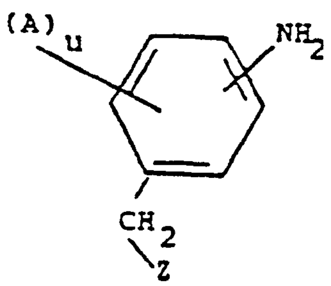

,

in der A, Z und u die obige Bedeutung besitzen,

. Sandmeyer-Reaktion mit einer der Aminverbindungen der oben angegebenen Formel unter Erhalt eines Sulfohalogenids der Formel

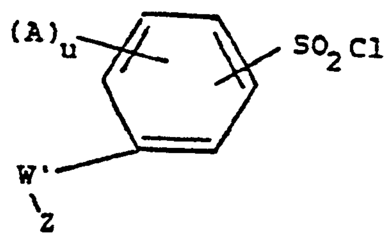

,

in der A, Z und u die obige Bedeutung besitzen und W′ C=O oder $CH_2$ darstellt,
und

. Umsetzung des Sulfohalogenids der obigen Formel mit einer Verbindung der Formel

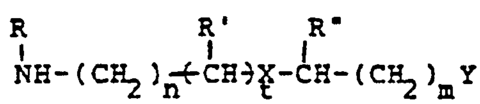

,

in der R, R′, R″, m, n, t, X und Y dasselbe wie in Anspruch 1 bedeuten,
zu einer Verbindung der Formel

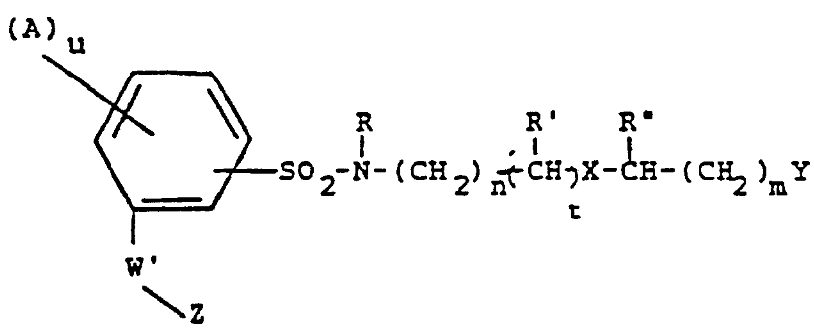

und

. gegebenenfalls, wenn W′ C=O darstellt, Reduktion der obigen Verbindung, insbesondere mit einem Alkaliborhydrid, zur Reduktion von C=O zu CHOH

. sowie gegebenenfalls anschließende Reduktion, wenn A und/oder B $NO_2$ darstellen.

18. Verfahren nach Anspruch 17 zur Herstellung der Verbindungen der Formel VII,

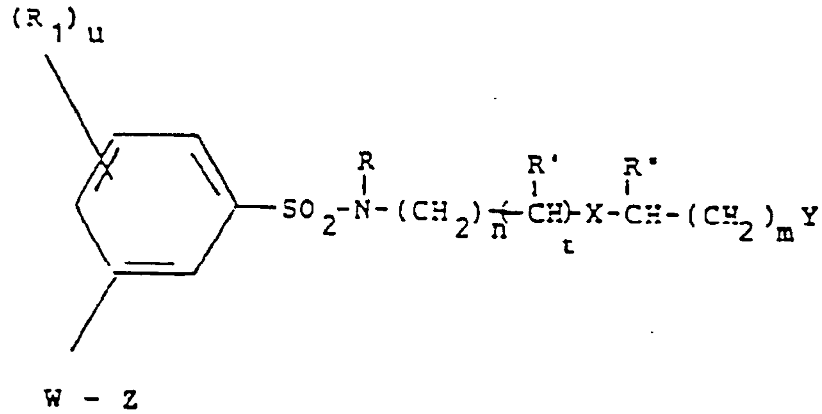

(VII),

in der $R_1$, R, R′, R″, X, Y, Z, W, m, n, t und u die Bedeutung von Anspruch 1 besitzen,

. gegebenenfalls Reduktion einer Verbindung der Formel

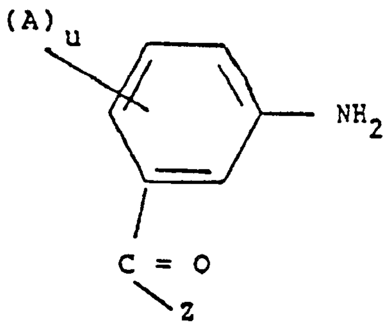

,

in der A, Z und u die Bedeutung von Anspruch 1 besitzen,
insbesondere mit Aluminiumchlorid und Lithiumalanat, zur Reduktion von C=O zu $CH_2$ unter Erhalt einer Verbindung der Formel

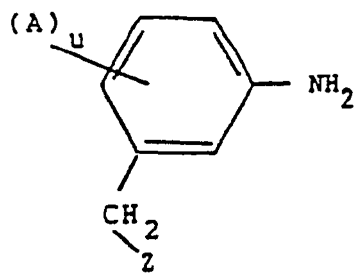

,

in der A, Z und u die obige Bedeutung besitzen,

. Sandmeyer-Reaktion mit einer der oben angegebenen Aminverbindungen unter Erhalt eines Sulfohalogenids der Formel

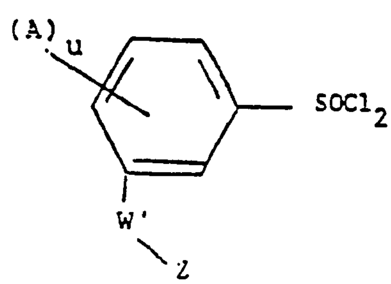

,

in der A, Z und u die obige Bedeutung besitzen und W′ C=O oder $CH_2$ darstellt, und

. Umsetzung des Sulfohalogenids der obigen Formel mit einer Verbindung der Formel

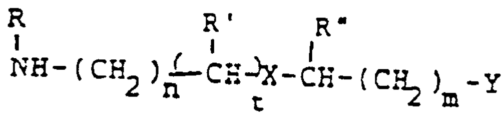

,

in der R, R′, R″, X, Y, m, n und t die oben angegebene Bedeutung besitzen, zur Verbindung der Formel

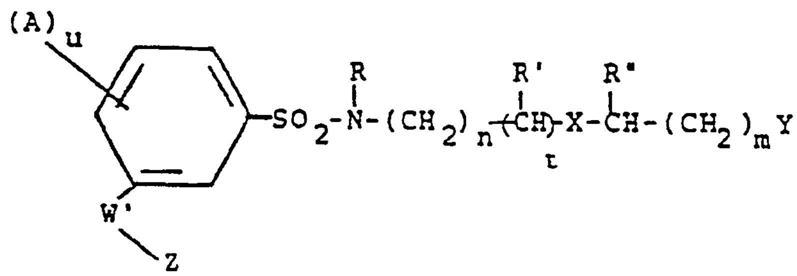

. und gegebenenfalls, wenn W′ C=O darstellt, Reduktion der obigen Verbindung, insbesondere mit einem Alkaliborhydrid, zur Reduktion von C=O zu CHOH

. sowie gegebenenfalls anschließende Reduktion, wenn A und/oder B $NO_2$ darstellen.

19. verfahren nach Anspruch 18 zur Herstellung der verbindungen der Formel VII, in der Y $COOR_3$ darstellt, wobei $R_3$ H oder $C_{1-4}$-Alkyl bedeutet,

. gegebenenfalls Reduktion einer Verbindung der Formel

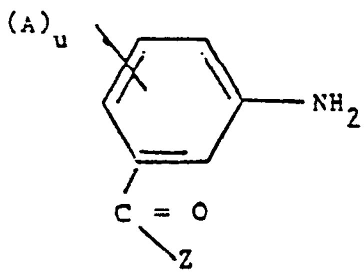

,

in der A, Z und u dasselbe wie in Anspruch 1 bedeuten insbesondere mit Aluminiumchlorid und Lithiumalanat, zur Reduktion von C=O zur $CH_2$ unter Erhalt der Verbindung der Formel

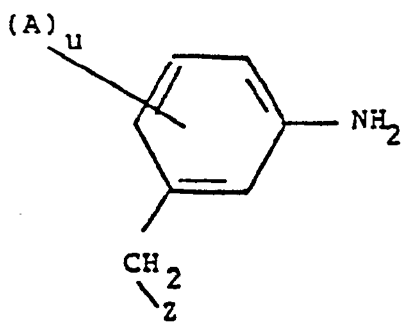

,

in der A, Z und u die obige Bedeutung besitzen,

. Sandmeyer-Reaktion mit einer der Aminverbindungen der oben angegebenen Formel unter Erhalt eines Sulfohalogenids der Formel

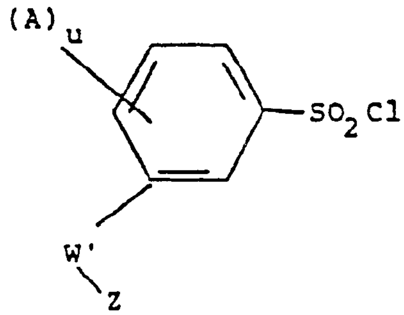

,

in der A, Z und u die obige Bedeutung besitzen und W' C=O oder $CH_2$ darstellt
und

. Umsetzung des Sulfohalogenids, insbesondere des Sulfochlorids, der obigen Formel mit einer oder zwei Verbindungen der Formel

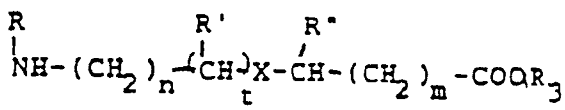

,

in der R, R', R", X, $R_3$, m, n und t dasselbe wie in Anspruch 1 bedeuten, zur Verbindung der Formel

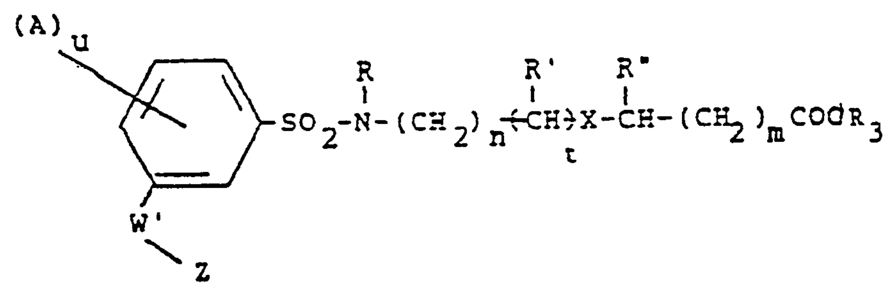

. und gegebenenfalls, wenn W' C=O darstellt, Reduktion der obigen Verbindung, insbesondere mit einem Alkaliborhydrid, wie Natriumborhydrid, zur Reduktion von C=O zu CHOH

. sowie gegebenenfalls anschließende Reduktion, wenn A und/oder B $NO_2$ darstellen.

20. Verfahren nach Anspruch 19 zur Herstellung von Verbindungen der formel VIII,

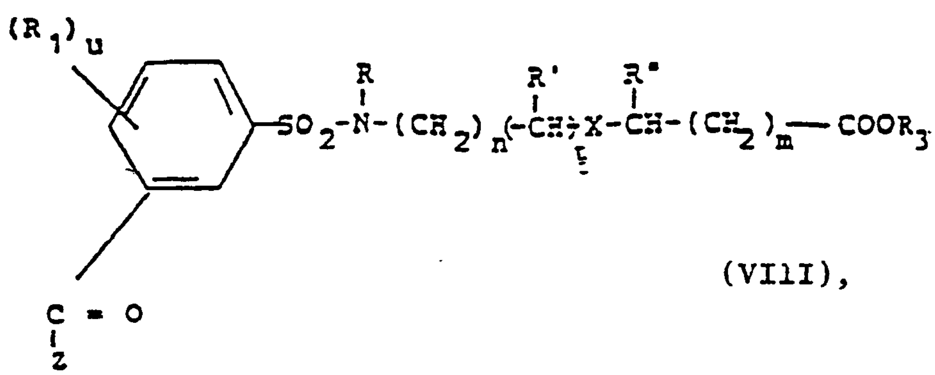

(VIII),

in der $R_1$, $R_3$, R, R', R", X, Z, m, n, t und u dasselbe wie in Anspruch 1 bedeuten.

21. Verfahren nach Anspruch 19 zur Herstellung von Verbindungen der Formel IX,

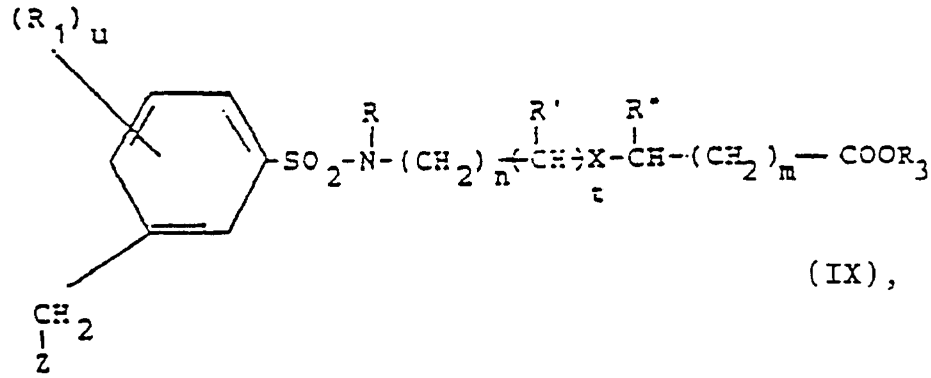

(IX),

in der $R_1$, $R_3$, R, R', R", X, Z, W, m, n, t und und u dasselbe wie in Anspruch 1 bedeuten.

22. Verfahren nach Anspruch 19 zur Herstellung von Verbindungen der Formel X,

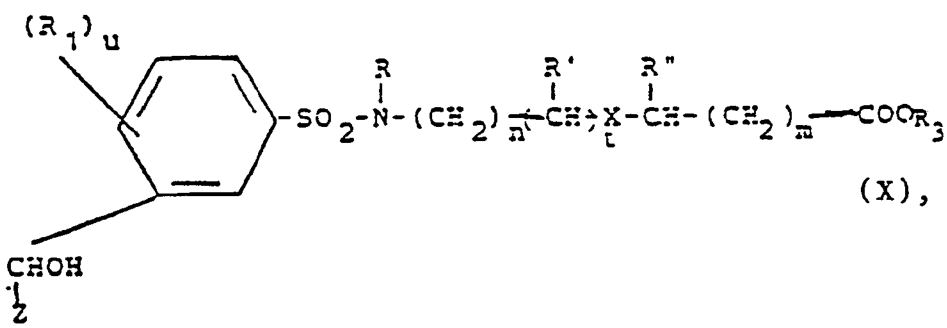

(X),

in der $R_1$, $R_3$, R, R', R", X, Z, W, m, n, t und u dasselbe wie in Anspruch 1 bedeuten.

23. Verfahren nach Anspruch 20 zur Herstellung von Verbindungen der Formel VIII, in der die Gesamtzahl der kohlenstoffatome der kette

$$(CH_2)_n-(\overset{|}{CH})_t-X-\overset{|}{CH}-(CH_2)_m-Y \geq 5 \text{ ist.}$$

24. Verfahren nach Anspruch 21 zur Herstellung von Verbindungen der Formel IX, in der die Gesamtzahl der kohlenstoffatome der Kette

$$(CH_2)_n-(\overset{|}{CH})_t-X-\overset{|}{CH}-(CH_2)_m-Y \geq 5 \text{ ist.}$$

25. Verfahren nach Anspruch 22 zur Herstellung von Verbindungen der Formel X, in der die Gesamtzahl der kohlenstoffatome der kette

$$(CH_2)_n-(\overset{|}{CH})_t-X-\overset{|}{CH}-(CH_2)_m-Y \geq 5 \text{ ist.}$$

26. Verfahren nach Anspruch 1 zur Herstellung von Verbindungen der Formel XI,

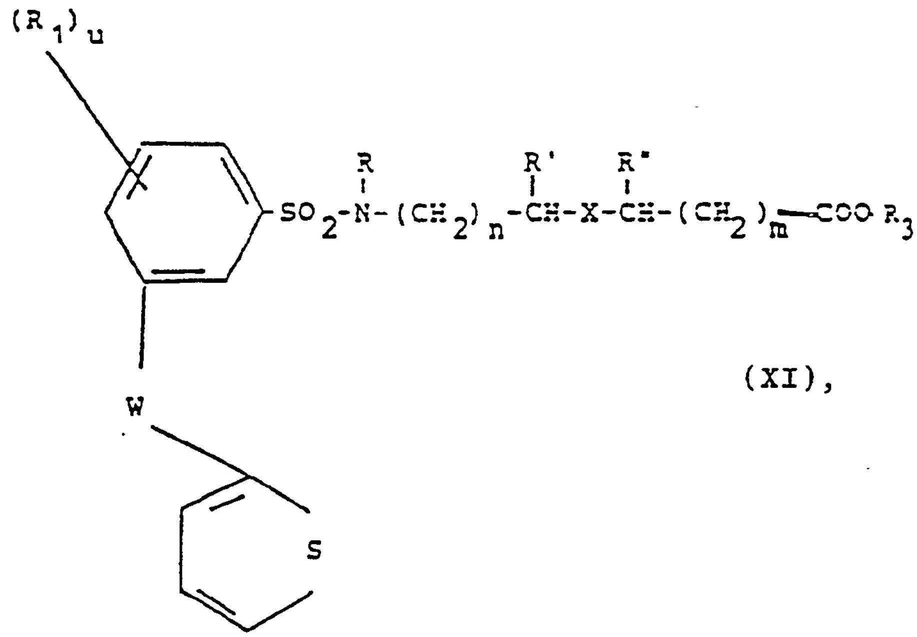

(XI),

wobei
- die Gesamtzahl der kohlenstoffatome der kette

$$(CH_2)_n - (CH)_t - X - CH - (CH_2)_m - Y \geq 5 \text{ ist,}$$

und $R_1$, $R_3$, R, R′, R″, X, W, m, n, t und u dasselbe wie in Anspruch 1 bedeuten, gekennzeichnet durch
. gegebenenfalls Reduktion einer Verbindung der Formel

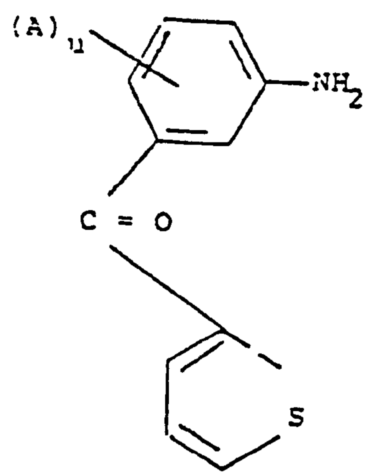

,

in der A und u dasselbe wie in Anspruch 1 bedeuten, insbesondere mit Aluminiumchlorid und Lithiumalanat, zur Reduktion von C=O zu $CH_2$ unter Erhalt der Verbindung der Formel

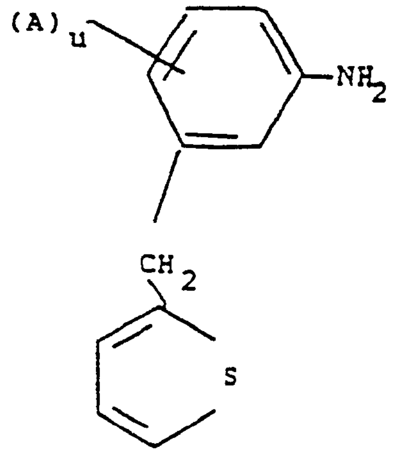

,

in der A und u die obige Bedeutung besitzen,

. Sandmeyer-Reaktion mit einer der Aminverbindungen der oben angegebenen Formel unter Erhalt eines Sulfohalogenids der Formel

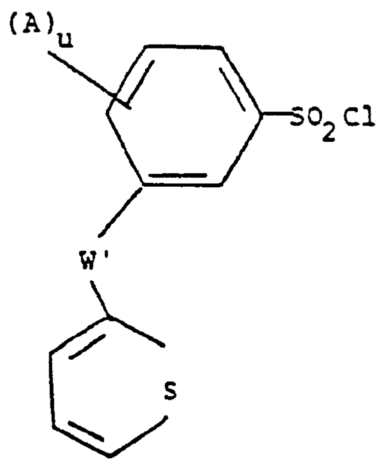

,

in der A und u die obige Bedeutung besitzen und W′ C=O oder $CH_2$ darstellt,

und

. Umsetzung des Sulfohalogenids der obigen Formel mit einer oder zwei Verbindungen der Formel

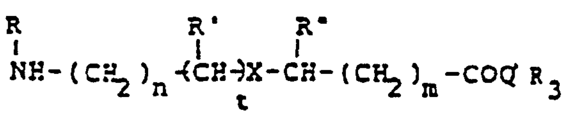

,

in der R, R′, R″, X, m, n, t und $R_1$ dasselbe wie in Anspruch 1 bedeuten,

zur Verbindung der Formel

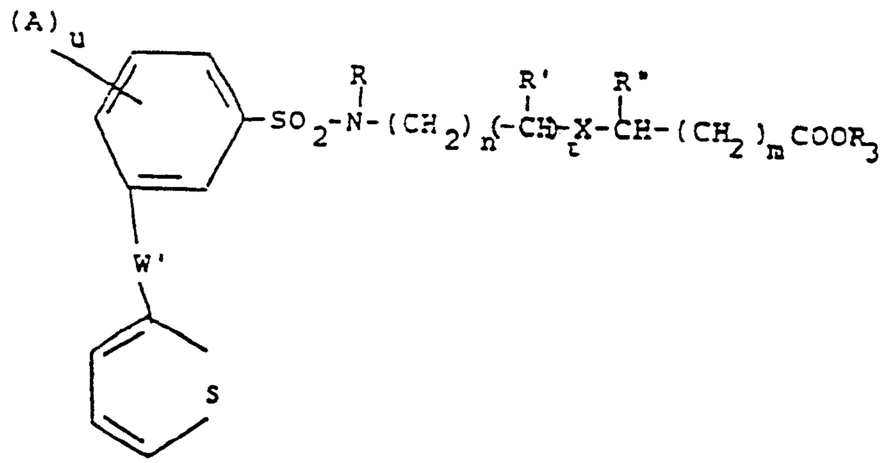

. und gegebenenfalls, wenn W' C=O darstellt, Reduktion der obigen Verbindung, insbesondere mit einem Alkaliborhydrid, zur Reduktion von C=O zu CHOH

. sowie gegebenenfalls anschließende Reduktion, wenn A und/oder B $NO_2$ darstellen.

27. Verfahren zur Herstellunb einer pharmazeutischen Zusammensetzung, gekennzeichnet durch Formulierung einer der aus den Verfahren nach einem der Ansprüche 1 bis 26 resultierenden Verbindungen mit einem pharmazeutisch akzeptablen Träger unter Bedingungen, welche die Herstellung der pharmazeutischen Zusammensetzung erlauben.